# EUROPEAN PATENT SPECIFICATION

(11) **EP 1 778 637 B1**
(45) Date of publication and mention of the grant of the patent: **22.02.2012**
(21) Application number: 05763770.4
(22) Date of filing: 24.06.2005
(51) Int. Cl.: C07D 211/96, C07D 401/12, C07D 405/12, C07D 409/14, A61K 31/445, A61K 31/453, A61K 31/454, A61K 31/4545, A61P 25/00, A61P 25/28

(54) **FKBP-binding composition and pharmaceutical use thereof**
FKBP bindende Zusammensetzung und deren pharmazeutische Verwendung
Composition de liaison aux protéines FKBP et utilisation pharmaceutique associée

(30) Priority: 29.06.2004 US 583740 P; 04.11.2004 US 624946 P
(43) Date of publication of application: 02.05.2007
(73) Proprietor: Aventis Pharmaceuticals Inc., Bridgewater, New Jersey 08807 (US)
(72) Inventor: KOSLEY, JR., Raymond, W., Bridgewater, NJ 08807 (US); BARON, Bruce, Westfield, NJ 07090 (US); JIMONET, Patrick, F-78450 Villepreux (FR); JURCAK, John, G., Bethlehem, Pennsylvania 18020 (US); SHIMSHOCK, Stephen, J., Hillsborough, New Jersey 08844 (US); ZHAO, Xu-Yang, Bridgewater, New Jersey 08807 (US); SHER, Rosy, Bridgewater, New Jersey 08807 (US); MUELLER, Paul, J., Hoboken, New Jersey 07030 (US); BEALL, Jennifer, Geneva, Illinois 60134 (US); BARRAGUE, Matthieu, Union City, New Jersey 07030 (US); GUILES, Joseph, W., Lafayette, CO 80026 (US)
(74) Representative: Le Pennec, Magali
(86) International application number: PCT/US2005/022360
(87) International publication number: WO 2006/012256

(56) References cited:
- WO-A-98/53814
- US-A1- 2002 052 372
- US-A1- 2002 052 410
- US-B1- 6 268 384
- I. O. DONKOR ET AL: "Peptidyl Aldehyde Inhibitors of Calpain Incorporating P2-Proline Mimetics" BIOORGANIC & MEDICINAL CHEMISTRY LETTERS, vol. 13, no. 5, 2003, pages 783-784, XP002362047

## Description

### BACKGROUND OF THE INVENTION

### 1. Field of the Invention

This invention relates to compositions that bind to FKBP proteins and to their use for treating neurological diseases. The invention also relates to methods of treating neurological diseases with these compositions.

### 2. Description of the Related Art

The diseases treated by compounds or compositions of the invention are major health problems and they are poorly addressed by current therapies. Patients seek medical attention after the disease or traumatic injury that has resulted in damage to the brain or peripheral nervous system. Drug treatments are available which offer symptomatic treatment for Parkinson's or Alzheimer's disease (e.g. dopamine or acetylcholine-replacement therapies, respectively).

Neurological diseases are associated with the death of or injury to neuronal cells. For example, the loss of dopaminergic neurons in the substantia nigra is believed to be the basis for Parkinson's disease. Although the molecular mechanism of neurodegeneration in Alzheimer's disease is yet to be established, inflammation and deposition of beta-amyloid protein and other such agents may compromise neuronal function or survival. In patients suffering from brain ischemia or spinal cord injuries, extensive neuronal cell death is observed. Currently, there are no satisfactory treatments for these diseases.

One approach to treating neurological diseases involves the use of drugs capable of inhibiting neuronal cell death. A more recent approach involves the promotion of nerve regeneration by drugs which stimulate neurite outgrowth.

Recently, small molecules have been shown to stimulate axonal outgrowth in vivo. In individuals suffering from a neurological disease, this stimulation of neurite outgrowth may protect neurons from further degeneration, and accelerate the regeneration of nerve cells. For example, estrogen has been shown to promote the growth of axons and dendrites, which are neurites sent out by nerve cells to communicate with each other in a developing or injured adult brain [C. Dominique Toran-Allerand et al., J. Steroid Biochem. Mol. Biol., 56, pp. 169-78 (1996); and B. S. McEwen et al., Brain Res. Dev. Brain. Res., 87, pp. 91-95 (1995)]. The progress of Alzheimer's disease may be slowed in women who take estrogen. Estrogen is hypothesized to complement NGF (nerve growth factor) and other neurotrophins and thereby help neurons differentiate and survive.

It has been reported that compounds with an affinity for the FK506 binding protein (FKBP) also possess nerve growth stimulatory activity [Lyons et al., PNAS, 91, pp. 3191-3195 (1994)]. Many of these compounds also have immunosuppressive activity, which could be a deleterious side effect in treating a human subject having a neurological disease.

FK506 (Tacrolimus), an immunosuppressive drug which binds to FKBP12 and other FKBP proteins, has been demonstrated to act synergistically with NGF in stimulating neurite outgrowth in PC12 cells as well as sensory ganglia [Lyons et al., Proc Nat. Acad. Sci. (1994)]. This compound has also been shown to be neuroprotective in focal cerebral ischemia-[J. Sharkey and S. P. Butcher, Nature, 371, pp. 336-339 (1994)] and to increase the rate of axonal regeneration in injured sciatic nerve [B. Gold et al., J. Neurosci., 15, pp. 7509-16 (1995)].

More recently, sub-classes of FKBP binding compounds which lack immunosuppressive activity have been disclosed for use in stimulating nerve growth [see for example U.S. Pat. Nos. 5,614,547; 5,696,135; WO 96/40633; WO 96/40140; WO 97/1,6190; J. P. Steiner et al., Proc. Natl. Acad. Sci. USA, 94, pp. 2019-23 (1997); and G.S. Hamilton et al., Bioorg. Med. Chem. Lett., 7, pp. 1785-90 (1997)]. These compounds supposedly avoid certain unwanted side effects of immunosuppressive FKBP binding compounds but still bind to FKBP and alter its functioning.

Some compounds bearing a similarity to those of the present invention are disclosed in a published patent application, US 2002/0052410 A1 (published May 2, 2002) and in US6,268,384 patent. However, these documents do not disclose or suggest the compounds of the present invention Furthermore, other compounds bearing a similarity to those of the present invention have been disclosed in another published patent application, US 2002/0052372 A1 (published May 2, 2002). However that application also fails to disclose or suggest the compounds of the present invention.

### SUMMARY OF THE INVENTION

We have now discovered a class of compounds that are effective for treating nerve damage.

The present invention relates to neurotrophic, low molecular weight, small molecule compounds having an affinity for FKBP-type immunophilins. Once bound to these proteins, the compounds are potential potent modulators of the function of immunophilin proteins.

Specifically, the present invention relates to compounds including enantiomers, stereoisomers, rotamers and tautomers of said compounds and pharmaceutically acceptable salts, solvates or derivatives thereof, said compounds having the general structure shown in formula I: 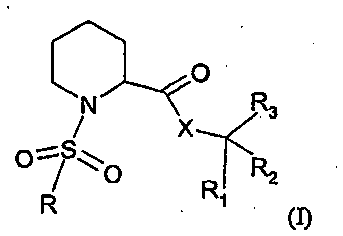
- wherein:: R is fluoroalkyl of the formula CₙHₓF_{y} wherein n is an integer from 1 to 6, x is an integer from 0 to 12, y is an integer from I to 13 and the sum of x and y is 2n+1, aryl, aryl C₁₋₆ alkyl, aryl C₂₋₄ alkenyl, wherein aryl is selected from phenyl, biphenyl and naphthyl, heteroaryl or heteroaryl C₁₋₄ alkyl, wherein heteroaryl is selected from pyridyl, thiophenyl and benzothiophenyl, and heterocyclyl selected from piperidinyl and piperazinyl,
- R₁ is: hydrogen or C₁₋₆ alkyl;
- R₂ is: CONHR₄, benzimidazol-2-yl, CR₅R₅OH, or CR₇R₈NHR₉;
- R₃ is: aryl, aryl C₁₋₄ alkyl or aryl C₂₋₄ alkenyl, wherein aryl is selected from phenyl, biphenyl, naphthyl, bisarylmethyl, heteroaryl and heteroaryl C₁₋₄ alkyl, wherein heteroaryl is selected from indolyl, thiophenyl, benzothiophenyl, furanyl, benzofuranyl, 1,3-benzodioxolyl, 1, 4-benzodioxanyl and C₅₋₇ cycloalkyl C₁ alkyl;
- X is: NR₁₀ or CH₂;
wherein:
- R₄ is: selected from the group consisting of hydrogen, C₁₋₁₀ alkyl, C₂₋₁₀ alkenyl, C₃₋₈ cycloalkyl, C₈₋₁₂ bicycloalkyl, C₁₀₋₁₄ tricycloalkyl, C₃₋₈ cycloalkyl C₁₋₆ alkyl, C₈₋₁₂ bicycloalkyl C₁₋₆ alkyl, C₈₋₁₂ bicycloalkylamino- C₁₋₆ alkyl, C₈₋₁₂ tricycloalkylamino-C₁₋₆ alkyl, C₃₋₈ cycloalkenyl, C₃₋₈ cycloalkenyl C₁₋₆ alkyl, fluoroalkyl of the formula CₙHₓF_{y} wherein n is an integer from 1 to 20, x is an integer from 0 to 40, y is an integer from 1 to 41 and the sum of x and y is 2n+1, amino C₂₋₂₀ alkyl, mono or dialkylamino C₂₋₂₀ alkyl, mono-, di, or trihydroxy C₂₋₂₀ alkyl, C₁₋₆ alkoxy- C₂₋₂₀ alkyl, C₁₋₂₀ thioalkyl, C₁₋₆ alkyl-thio-C₂₋₂₀ alkyl, hydroxy-C₂₋₆ -alkoxy-C₂₋₆ alkyl, C₁₋₆ alkyl-bis-(hydroxy-C₁₋₆ alkyl), aryl, aryl C₁₋₁₀ alkyl, aryl C₃₋₄ alkenyl, wherein aryl is selected from phenyl, biphenyl, indanyl and naphthyl, heteroaryl or heteroaryl C₁₋₁₀ alkyl, wherein heteroaryl is selected from pyridyl, thiophenyl, furanyl, benzofuranyl, benzothiophenyl, pyrrolidinyl, and indolyl; heterocycloalkyl or heterocycloalkyl C₁₋₁₀ alkyl wherein heterocycloalkyl is selected from tetrahydrofuranyl, tetrahydropyranyl, morpholinyl, piperidinyl, pyrrolidinyl, tetrahydrothiophenyl, piperazinyl, dioxolanyl, sulfolanyl and 1,1-dioxotetrahydrothiopyranyl; heterobicyclyl of the formula: 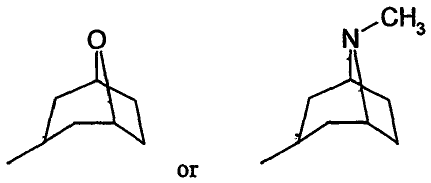 comprising C₅₋₈ cycloalkyl, C₈₋₁₂bicycloallcyl, and/or heterocyclyl, optionally substituted with one or more substituents selected from the group consisting of C₁₋₁₀ alkyl, C₁₋₁₀ alkoxy, hydroxy, hydroxy C₁₋₁₀ alkoxy, hydroxy C₁₋₁₀ alkyl, C₁₋₁₀ acyloxy, amino, C₁₋₁₀ alkylcarbonylamino, hydroxy C₁₋₂₀ alkylcarbonylamino, amino C₂₋₁₀ alkoxy, mono-C₁₋₆ -alkylamino-C₂₋₁₀-alkoxy, di-C₁₋₆-alkylamino-C₂₋₁₀-alkoxy, mono(C₁₋₁₀ alkyl)amino, di-(C₁₋₁₀ alkyl)amino, amino C₁₋₁₀ alkylcarbonylamino, C₁₋₁₀ alkylaminocarbonyl, hydroxy C₁₋₁₀ alkylaminocarbonyl, amino C₂₋₁₀ alkylaminocarbonyl, mono or dialkylamino C₁₋₁₀ alkylaminocarbonyl, oxo, optionally substituted aryl and optionally substituted heteroaryl, wherein aryl is selected from phenyl, naphthyl and biphenyl, and heteroaryl is selected from pyridyl and thiophenyl; and
- wherein:: the aryl or heteroaryl groups of R are optionally substituted with one or more substituents selected from the group consisting of fluorine, chlorine, bromine, C₁₋₂₀ alkyl, fluoroalkyl or fluoroalkoxy of the formula CₙHₓF_{y} or -OCₙHₓF_{y} wherein n is an integer from 1 to 4, x is an integer from 0 to 8, y is an integer from 1 to 9 and the sum of x and y is 2n+1, C₁₋₂₀ alkoxy, C₁₋₂₀ alkenyloxy, C₁₋₁₀-alkoxy-C₁₋₁₀-alkoxy, hydroxy, hydroxy C₁₋₂₀ alkoxy, hydroxy C₁₋₂₀ alkyl, C₁₋₂₀ acyloxy, hydroxy C₁₋₂₀ acyl, nitro, amino, aminosulfonyl, C₁₋₂₀ alkylcarbonylamino, hydroxy C₁₋₂₀ alkylcarbonylamino, amino C₂₋₂₀ alkoxy, amino C₁₋₂₀ alkylcarbonylamino, C₁₋₂₀ alkylaminocarbonyl, hydroxy C₁₋₂₀ alkylaminocarbonyl, amino C₁₋₂₀ alkylaminocarbonyl, mono or dialkylamino C₁₋₂₀ alkylaminocarbonyl, optionally substituted aryl or heteroarylsulfonyl, aminosulfonyl, mono- or di(C₁₋₆)alkylaminosulfonyl, optionally substituted phenyl or thiophenyl, optionally substituted phenyl or thiophenyl C₁₋₁₀ alkyloxy, and cyano;
- wherein:: the phenyl or thiophenyl substituent on the aryl or heteroaryl group of R is optionally substituted with one or more substituents selected from the group consisting of fluorine, chlorine, bromine, C₁₋₅ alkyl, fluoroalkyl or fluoroalkoxy of the formula CₙHₓF_{y} or OCₙHₓF_{y} wherein n is an integer from 1 to 4, x is an integer from 0 to 8, y is an integer from 1 to 9 and the sum of x and y is 2n+1, C₁₋₁₀ alkoxy, C₁₋₁₀ alkenyloxy, C₁₋₅ alkoxy-C₁₋₅ alkoxy, hydroxy, hydroxy C₁₋₁₀ alkoxy, hydroxy C₁₋₁₀ alkyl, hydroxy C₁₋₂₀-alkyloxo, nitro, amino, aminosulfonyl, C₁₋₁₀ alkylcarbonylamino, hydroxy C₁₋₁₀ alkylcarbonylamino, amino C₂₋₁₀ alkoxy, amino C₁₋₁₀ alkylcarbonylamino, C₁₋₁₀ alkylaminocarbonyl, hydroxy C₁₋₁₀ alkylaminocarbonyl, amino C₁₋₁₀ alkylaminocarbonyl, mono or dialkylamino C₁₋₁₀ alkylaminocarbonyl, optionally substituted aryl C₁₋₁₀ alkyloxy, and cyano;
- wherein:: the aryl or heteroaryl of R₃ is optionally substituted with one or more substituents selected from the following: C₃₋₈ cycloalkyloxy, C₁₋₁₀ alkoxy, C₁₋₁₀ alkyl, C₂₋₁₀ alkenyl, C₃₋₁₀ alkenyloxy, mono- (C₁₋₆-alkyl)amino-C₂₋₆alkyloxy, di-(C₁₋₆ alkyl)amino-C₂₋₆-alkyloxy, amino-C₂₋₆alkyloxy, heterocyclyl-C₂₋₆alkyloxy, aryl-C₂₋₆-alkyloxy, heteroaryl-C₂₋₆-alkyloxy, fluoroalkyl or fluoroalkoxy of the formula CₙHₓF_{y} or OCₙHₓF_{y} wherein n is an integer from 1 to 4, x is an integer from 0 to 8, y is an integer from 1 to 9 and the sum of x and y is 2n+1, C₁₋₆-alkoxy-C₂₋₆-alkoxy, aryl-C₁₋₆-alkyloxy, hydroxy, chloro, fluoro, bromo, hydroxy-C₁₋₆-alkoxy, hydroxy-C₁₋₁₀ alkyl, C₁₋₆ alkyloxo, cyano, amino, 4-aryloxo, di-C₁₋₅ alkylphosphityl-C₁₋₅ alkyl, C₁₋₆ alkyloxoamino, C₁₋₆ alkyloxo(C₁₋₆ alkyl)amino, C₁₋₁₀alkylsulfonyl, arylsulfonyl, carboxy, C₁₋₂₀ carboalkoxy, dialkylamino-C₁₋₁₀ alkyl, monoalkylamino-C₁₋₁₀ alkyl, and amino-C₁₋₁₀ alkyl;
- wherein:: the heterocycloalkyl substituent group attached to the alkyloxy group of R₃ is selected from the following: pyrrolidinyl, morpholinyl, piperidinyl, and piperazinyl;
- wherein:: the heteroaryl substituent group attached to the alkyloxy group of R₃ is selected from pyridinyl, and pyrrolidinyl;
- wherein:: the aryl of the arylsulfonyl substituent on the aryl of R₃ is selected from phenyl, indolyl, thiophenyl, and furanyl;
- wherein:: the aryl or heteroaryl groups of R₄ are optionally substituted with one or more substituents selected from the following: mono- or di-(C₁₋₆ alkyl)amino, mono- or di (C₁₋₆)alkylamino-C₂₋₆ alkyl, amino-C₁₋₆ alkyl, heterocycloalkyl, amino, C₁₋₂₀ alkoxy, chloro, fluoro, bromo, C₁₋₁₀ alkyl, aryl and heteroaryl;
- wherein:: the heterocycloalkyl group attached to the aryl of R₄ is selected from the following: morpholinyl, pyrrolidinyl, piperazinyl, and piperidinyl;wherein: the heteroaryl group attached to the aryl of R₄ is selected from pyridyl,pyrrolyl, and furanyl;
R₅, R₆ = independently H, or C₁₋₆ alkyl;
R₇, R₈ = independently H, or C₁₋₆ alkyl;
R₉ = di-(C₁₋₅ alkyl)amino-C₂₋₄ alkyl; mono(C₁₋₅ alkyl)amino-C₂₋₄ alkyl; amino-C₂₋₄ alkyl, or heterocycloalkyl methyl in which heterocycloalkyl is selected from the group consisting of thiophenyl, furanyl, pyrrolyl, and pyridinyl; and
R₁₀ = H, or C₁₋₆ alkyl.

The compounds of the invention have been evaluated for binding affinity at human FK506 binding protein FKBP12. Compounds have been identified with inhibition constants (Ki) better than 1 micromolar, many with Ki better than 100 nM. Compounds of this structural type penetrate cells and bind to FK506 binding proteins, whereby they influence intracellular signaling pathways. Activity is typically evident at concentrations of 1 µM (micromolar) or less.

The new compounds disclosed herein should be useful for treating disorders involving neurological damage, including, but not limited to, disorders such as Parkinson's disease, multiple sclerosis, Alzheimer's disease, stroke, and spinal cord injury.

### DETAILED DESCRIPTION OF THE INVENTION

The compounds disclosed herein may be effectively used in treating a disease state that involves neurodegeneration in its etiology and progression. Such disease states include Parkinson's disease, multiple sclerosis, Alzheimer's disease, organic brain disorder, memory dysfunction, neuropathies, peripheral neuropathies, trigeminal neuralgia, glossopharyngeal neuralgia, Bell's palsy, myasthenia gravis, muscular dystrophy, progressive muscular atrophy, and traumatic injury to spinal, peripheral, or central nervous system tissue.

Peripheral nerve trauma may be used as a test of potential efficacy for such disorders as those listed above. Such a test may be performed as follows: Sciatic or facial nerve is mechanically crushed, and animals (rats or mice) are monitored for behavioral and neurological deficits (e.g. abnormal gait or inability to move the whiskers). Compounds or vehicle are administered at least once daily beginning at the time of injury and continuing throughout the observation period. After 3 weeks, the animals are euthanized, and nerve integrity is visualized histologically. The number, caliber, and myelination state of axons in the damaged nerve are quantitated. Compound efficacy is reflected as a reduction of neurological deficits and an increase in the total number of axons, and especially the fraction of large caliber myelinated fibers.

Clinically, this finding would be exploited by giving a suitable dose of the compound by mouth or by injection to a patient suffering from a traumatic injury, such as a crush or a laceration of a peripheral nerve.

Unless defined otherwise herein, all technical and scientific terms used herein have the same meaning as is commonly understood by one of skill in the art to which this invention belongs.

The compounds of formula I as well as the intermediates and starting materials used in their preparation are named in accordance with IUPAC rules of nomenclature in which characteristic groups have decreasing priority for citation as the principle group as follows: acids, esters, amides, etc. Alternatively, the compounds are namedby AutoNom 4.0 (Beilstein Information Systems, Inc.).

As used in the present application, the following definitions apply:
An "alkyl group" is intended to mean a straight or branched chain monovalent radical of saturated and/or unsaturated carbon atoms and hydrogen atoms, such as methyl, ethyl, propyl, isopropyl, butyl, isobutyl, t-butyl, ethenyl, pentenyl, butenyl, propenyl, ethynyl, butynyl, propynyl, pentynyl, hexynyl, and the like, which may be unsubstituted (i.e., containing only carbon and hydrogen) or substituted by one or more suitable substituents as defined below. Unless otherwise specified herein, "alkyl" indicates C₁-C₆.
A "cycloalkyl group" is intended to mean a non- aromatic, monovalent monocyclic, bicyclic, or tricyclic radical containing 3, 4, 5, 6, 7, 8, 9, 10, 11, 12, 13, or 14 carbon ring atoms, each of which may be saturated or unsaturated, and which may be unsubstituted or substituted by one or more suitable substituents as defined below, and to which may be fused one or more heterocycloalkyl groups, aryl groups, or heteroaryl groups, which themselves may be unsubstituted or substituted by one or more suitable substituents.

Illustrative examples of cycloalkyl groups include the following moieties:

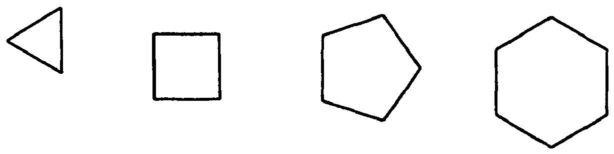

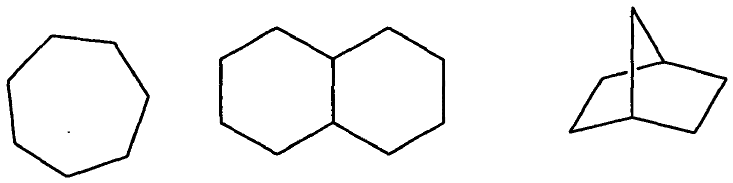

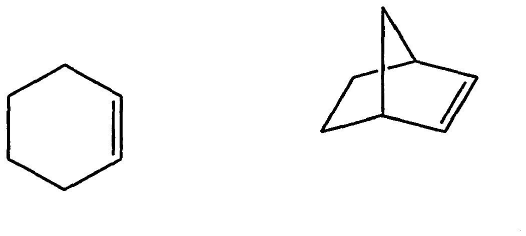

A "heterocycloalkyl group" is intended to mean a non-aromatic, monovalent monocyclic, bicyclic, or tricyclic radical, which is saturated or unsaturated, containing 3, 4, 5, 6, 7, 8, 9, 10, 11, 12, 13, 14, 15, 16, 17, or 18 ring atoms, and which includes 1, 2, 3, 4, or 5 heteroatoms selected from nitrogen, oxygen, and sulfur, wherein the radical is unsubstituted or substituted by one or more suitable substituents as defined below, and to which may be fused one or more cycloalkyl groups, aryl groups, or heteroaryl groups, which themselves may be unsubstituted or substituted by one or more suitable substituents. Illustrative examples of heterocycloalkyl groups include the following moieties:

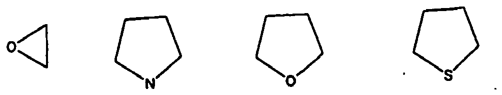

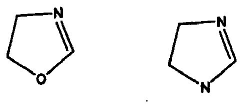

An "aryl group" is intended to mean an aromatic, monovalent monocyclic, bicyclic, or tricyclic radical containing 6, 10, 14, 18 carbon ring atoms, which may be unsubstituted or substituted by one or more suitable substituents as defined below, and to which may be fused one or more cycloalkyl groups, heterocycloalkyl groups, or heteroaryl groups, which themselves may be unsubstituted or substituted by one or more suitable substituents. Illustrative examples of aryl groups include the following moieties:

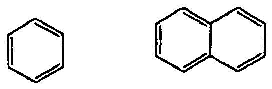

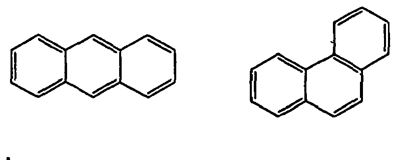

A "heteroaryl group" is intended to mean an aromatic monovalent monocyclic, bicyclic, or tricyclic radical containing 4, 5, 6, 7, 8, 9,10,11,12,13,14, 15, 16, 17, or 18 ring atoms, including 1, 2, 3, 4, or 5 heteroatoms selected from nitrogen, oxygen, and sulfur, which may be unsubstituted or substituted by one or more suitable substituents as defined below, and to which may be fused one or more cycloalkyl groups, heterocycloalkyl groups, or aryl groups, which themselves may be unsubstituted or substituted by one or more suitable substituents. Illustrative examples of heteroaryl groups include the following moieties: benzothiophenyl, thiophenyl and furanyl.

An "acyl group" is intended to mean a -C(O) -R radical, wherein R is any suitable substituent as defined below.

An "aminosulfonyl group" is intended to mean a -SO₂NH₂ radical.

The term "suitable substituent" is intended to mean any of the substituents recognizable, such as by routine testing, to those skilled in the art as not adversely affecting the binding activity of the inventive compounds. Illustrative examples of suitable substituents include hydroxy groups, oxo groups, alkyl groups, acyl groups, sulfonyl groups, mercapto groups, alkylthio groups, alkoxy groups, cycloalkyl groups, heterocycloalkyl groups, aryl groups, heteroaryl groups, carboxy groups, amino groups, alkylamino groups, dialkylamino groups, carbamoyl groups, aryloxy groups, heteroaryloxy groups, arylthio groups, heteroarylthio groups.

The term "suitable organic moiety" is intended to mean any organic moiety recognizable, such as by routine testing, to those skilled in the art as not adversely affecting the binding activity of the inventive compounds. Illustrative examples of suitable organic moieties include, but are not limited to, hydroxy groups, alkyl groups, oxo groups, cycloalkyl groups, heterocycloalkyl groups, aryl groups, heteroaryl groups, acyl groups, sulfonyl groups, mercapto groups, alkylthio groups, alkoxy groups, carboxy groups, amino groups, alkylamino groups, dialkylamino groups, carbamoyl groups, arylthio groups, heteroarylthio groups.

A "hydroxy group" is intended to mean the radical -OH.

An "amino group" is intended to mean the radical NH₂.

An "alkylamino group" is intended to mean the radical -NHR where R is an alkyl group as defined above.

A "dialkylamino group" is intended to mean the radical -NRₐ R_{b} where Rₐ and R_{b} are each independently an alkyl group as defined above.

An "alkoxy group" is intended to mean the radical -OR where R is an alkyl group as defined above, for example, methoxy, ethoxy, propoxy.

A "dialkylaminocarbonyl group" is intended to mean the radical -C(O)NRₐ R_{b} where Rₐ and R_{b} are each independently an alkyl group as defined above.

A "carboxy group" is intended to mean the radical -C(O)OH.

"Optional" or "optionally" means that the subsequently described event or circumstance may or may not occur, and that the description includes instances where said event or circumstance occurs and instances in which it does not. For example, "optionally substituted aryl" means that the aryl radical may or may not be substituted and that the description includes both substituted aryl radicals and aryl radicals having no substitution. Unless otherwise specified herein, "optionally substituted" refers to either mono- or disubstitution.

A "pharmaceutically acceptable solvate" is intended to mean a solvate that retains the biological effectiveness and properties of the biologically active components of compounds of formula I. Examples of pharmaceutically acceptable solvates include compounds of formula I in combination with water, isopropanol, ethanol, methanol, DMSO, ethyl acetate, acetic acid, or ethanolamine.

A "pharmaceutically acceptable salt" is intended to mean a salt that retains the biological effectiveness and properties of the free acids and bases of compounds of formula I and that is not biologically or otherwise undesirable. Examples of pharmaceutically acceptable salts include sulfates, pyrosulfates, bisulfates, sulfites, bisulfites, phosphates, monohydrogenphosphates, dihydrogenphosphates, metaphosphates, pyrophosphates, chlorides, bromides, iodides, acetates, propionates, decanoates, caprylates, acrylates, formates, isobutyrates, caproates, heptanoates, propiolates, oxalates, malonates, succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxybenzoates, phthalates, sulfonates, xylenesulfonates, phenylacetates, phenylpropionates, phenylbutyrates, citrates, lactates, gamma-hydroxybutyrates, glycollates, tartrates, methane-sulfonates, propanesulfonates, naphthalene-1-sulfonates, naphthalene-2-sulfonates, and mandelates.

If the inventive compound is a base, the desired salt may be prepared by any suitable method known to the art, including treatment of the free base with an inorganic acid, such as hydrochloric acid; hydrobromic acid; sulfuric acid; nitric acid; phosphoric acid; or with an organic acid, such as acetic acid; maleic acid; succinic acid; mandelic acid; fumaric acid; malonic acid; pyruvic acid; oxalic acid; glycolic acid; salicylic acid; pyranosidyl acids such as glucouronic acid and galactouronic acid; alpha-hydroxy acids such as citric acid and tartaric acid; amino acids such as aspartic acid and glutamic acid; aromatic acids such as benzoic acid and cinnamic acid; sulfonic acids such as p-toluenesulfonic acid or ethanesulfonic acid.

If the inventive compound is an acid, the desired salt may be prepared by any suitable method known to the art, including treatment of the free acid with an inorganic or organic base, such as an amine (primary, secondary or tertiary); an alkali metal or alkaline earth metal hydroxide. Illustrative examples of suitable salts include organic salts derived from amino acids such as glycine and arginine; ammonia; primary, secondary and tertiary amines; and cyclic amines such as piperidine, morpholine, and piperazine, and inorganic salts derived from sodium, calcium, potassium, magnesium, manganese, iron, copper, zinc, aluminum, and lithium.

In the case of compounds, salts, or solvates that are solids, it is understood by those skilled in the art that the inventive compounds, salts, and solvates may exist in different crystal forms, all of which are intended to be within the scope of the present invention.

The inventive compounds may exist as single stereoisomers, racemates, and/or mixtures of enantiomers and/or diastereomers. All such single stereoisomers, racemates, and mixtures thereof are intended to be within the scope of the present invention. Preferably, the inventive compounds are used in optically pure form.

As used herein, "biological activity" refers to the in vivo activities of a compound or physiological responses that result upon in vivo administration of a compound, composition or other mixture. Biological activity, thus, encompasses therapeutic effects and pharmaceutical activity of such compounds, compositions and mixtures.

As used herein, an "effective amount" of a compound for treating a particular disease is an amount that is sufficient to ameliorate, or in some manner reduce the symptoms associated with the disease. Such amount may be administered as a single dosage or may be administered according to a regimen, whereby it is effective. The amount may cure the disease but, typically, is administered in order to ameliorate the symptoms of the disease. Typically, repeated administration is required to achieve the desired amelioration of symptoms. The amount of a compound of formula (I) which constitutes a "therapeutically effective amount" will vary depending on the compound, the disease-state and its severity, and the age of the human to be treated, but can be determined routinely by one of ordinary skill in the art having regard to his own knowledge and to this disclosure.

"Treating" or "treatment" as used herein cover administration to a subject by any manner in which the symptoms of a condition, disorder or disease are ameliorated or otherwise beneficially altered; where the condition, disorder or disease is characterized by neurodegenerative activity; and include:
(i) preventing the disease-state from occurring in a human, in particular, when such human is predisposed to the disease-state but has not yet been diagnosed as having it;
(ii) inhibiting the disease-state, i.e., slowing or arresting its development; or
(iii) relieving the disease-state, i.e., causing regression of the disease-state.

As used herein, "amelioration" of the symptoms of a particular disorder by administration of a particular pharmaceutical composition refers to any lessening, whether permanent or temporary, lasting or transient that can be attributed to or associated with administration of the composition.

As used herein, the IC₅₀ refers to an amount, concentration or dosage of a particular test compound that achieves a 50% inhibition of a maximal response, such as binding of FK506 to an FKBP, in an assay that measures such response.

As used herein, EC₅₀ refers to a dosage, concentration or amount of a particular test compound that elicits a dose-dependent response at 50% of maximal expression of a particular response that is induced, provoked or potentiated by the particular test compound.

Specifically, the present invention relates to compounds, including enantiomers, stereoisomers, rotamers and tautomers of said compounds and pharmaceutically acceptable salts, solvates or derivatives thereof, said compounds having the general structure shown in formula I: 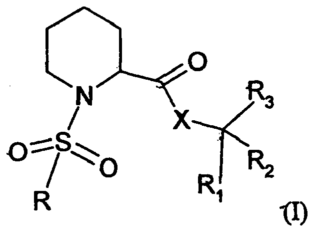 wherein:
- R is: fluoroalkyl of the formula CₙHₓF_{y} wherein n is an integer from 1 to 6, x is an integer from 0 to 12, y is an integer from 1 to 13 and the sum of x and y is 2n+1, aryl, aryl C₁₋₆ alkyl, aryl C₂₋₄ alkenyl, wherein aryl is selected from phenyl, biphenyl and naphthyl, heteroaryl or heteroaryl C₁₋₄ alkyl, wherein heteroaryl is selected from pyridyl, thiophenyl and benzothiophenyl, and heterocyclyl selected from piperidinyl and Piperazinyl,
- R₁ is: hydrogen or C₁₋₆ alkyl;
- R₂ is: CONHR₄, benzimidazol-2-yl, CR₅R₆OH, or CR₇R₈NHR₉;
- R₃ is: aryl, aryl C₁₋₄ alkyl or aryl C₂₋₄ alkenyl, wherein aryl is selected from phenyl, biphenyl, naphthyl, bisarylmethyl, heteroaryl and heteroaryl C₁₋₄ alkyl, wherein heteroaryl is selected from indolyl, thiophenyl, benzothiophenyl, furanyl, benzofuranyl, 1,3-benzodioxolyl, 1, 4-benzodioxanyl and C₅₋₇ cycloalkyl C₁ alkyl;
- X is: NR₁₀ or CH₂;
wherein:
- R₄ is: selected from the group consisting of hydrogen, C₁₋₁₀ alkyl, C₂₋₁₀ alkenyl, C₃₋₈ cycloalkyl, C₈₋₁₂ bicycloalkyl, C₁₀₋₁₄ tricycloalkyl, C₃₋₈ cycloalkyl C₁₋₆ alkyl, C₈₋₁₂ bicycloalkyl C₁₋₆ alkyl, C₈₋₁₂ bicycloalkylamino- C₁₋₆ alkyl, C₈₋₁₂ tricycloalkylamino-C₁₋₆ alkyl, C₃₋₈ cycloalkenyl, C₃₋₈ cycloalkenyl C₁₋₆ alkyl, fluoroalkyl of the formula CₙHₓF_{y} wherein n is an integer from 1 to 20, x is an integer from 0 to 40, y is an integer from 1 to 41 and the sum ofx and y is 2n+1, amino C₂₋₂₀ alkyl, mono or dialkylamino C₂₋₂₀ alkyl, mono-, di, or trihydroxy C₂₋₂₀ alkyl, C₁₋₆ alkoxy- C₂-₂₀ alkyl, C₁₋₂₀ thioalkyl, C₁₋₆ alkyl-thio-C₂₋₂₀ alkyl, hydroxy-C₂₋₆ -alkoxy-C₂₋₆ alkyl, C₁₋₆ alkyl-bis-(hydroxy-C₁₋₆ alkyl), aryl, aryl C₁₋₁₀ alkyl, aryl C₃₋₄ alkenyl, wherein aryl is selected from phenyl, biphenyl, indanyl and naphthyl, heteroaryl or heteroaryl C₁-₁₀ alkyl, wherein heteroaryl is selected from pyridyl, thiophenyl, furanyl, benzofuranyl, benzothiophenyl, pyrrolidinyl, and indolyl; heterocycloalkyl or heterocycloalkyl C₁₋₁₀ alkyl wherein heterocycloalkyl is selected from tetrahydrofuranyl, tetrahydropyranyl, morpholinyl, piperidinyl, pyrrolidinyl, tetrahydrothiophenyl, piperazinyl, dioxolanyl, sulfolanyl and 1,1-dioxotetrahydrothiopyranyl; heterobicyclyl of the formula: 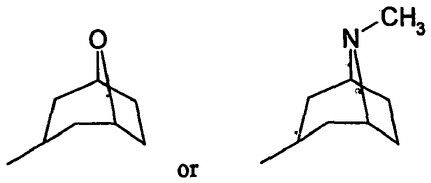 comprising C₅₋₈ cycloalkyl, C₈₋₁₂ bicycloalkyl, and/or heterocyclyl, optionally substituted with one or more substituents selected from the group consisting of C₁₋₁₀ alkyl, C₁₋₁₀ alkoxy, hydroxy, hydroxy C₁₋₁₀ alkoxy, hydroxy C₁₋₁₀ alkyl, C₁₋₁₀ acyloxy, amino, C₁₋₁₀ alkylcarbonylamino, hydroxy C₁₋₂₀ alkylcarbonylamino, amino C₂₋₁₀ alkoxy, mono-C₁₋₆ -alkylamino-C₂-₁₀-alkoxy, di-C₁₋₆-alkylamino-C₂-_{1O}-alkoxy, mono-(C₁₋₁₀ alkyl)amino, di-(C₁₋₁₀ alkyl)amino, amino C₁₋₁₀ alkylcarbonylamino, C₁₋₁₀ alkylaminocarbonyl, hydroxy C₁₋₁₀ alkylaminocarbonyl, amino C₂₋₁₀ alkylaminocarbonyl, mono or dialkylamino C₁₋₁₀ alkylaminocarbonyl, oxo, optionally substituted aryl and optionally substituted heteroaryl, wherein aryl is selected from phenyl, naphthyl and biphenyl, and heteroaryl is selected from pyridyl and thiophenyl; and
- wherein:: the aryl or heteroaryl groups of R are optionally substituted with one or more substituents selected from the group consisting of fluorine, chlorine, bromine, C₁₋₂₀ alkyl, fluoroalkyl or fluoroalkoxy of the formula CₙHₓF_{y} or OCₙHₓFy wherein n is an integer from 1 to 4, x is an integer from 0 to 8, y is an integer from 1 to 9 and the sum ofx and y is 2n+1, C₁₋₂₀ alkoxy, C₁₋₂₀ alkenyloxy, C₁₋₁₀-alkoxy-C₁₋₁₀-alkoxy, hydroxy, hydroxy C₁₋₂₀ alkoxy, hydroxy C₁₋₂₀ alkyl, C₁₋₂₀ acyloxy, hydroxy C₁₋₂ acyl, nitro, amino, aminosulfonyl, C₁₋₂₀ alkylcarbonylamino, hydroxy C₁-₂₀ alkylcarbonylamino, amino C₂₋₂₀ alkoxy, amino C₁₋₂₀ alkylcarbonylamino, C₁₋₂₀ alkylaminocarbonyl, hydroxy C₁₋₂₀ alkylaminocarbonyl, amino C₁₋₂₀ alkylaminocarbonyl, mono or dialkylamino C₁₋₂₀ alkylaminocarbonyl, optionally substituted aryl or heteroarylsulfonyl, aminosulfonyl, mono- or di(C₁₋₆)alkylaminosulfonyl, optionally substituted phenyl or thiophenyl, optionally substituted phenyl or thiophenyl C₁₋₁₀ alkyloxy, and cyano;
- wherein:: the phenyl or thiophenyl substituent on the aryl or heteroaryl group ofR is optionally substituted with one or more substituents selected from the group consisting of fluorine, chlorine, bromine, C₁₋₅ alkyl, fluoroalkyl or fluoroalkoxy of the formula CₙHₓF_{y} or OCₙHₓF_{y} wherein n is an integer from 1 to 4, x is an integer from 0 to 8, y is an integer from 1 to 9 and the sum of x and y is 2n+1, C₁₋₁₀ alkoxy, C₁₋₁₀ alkenyloxy, C₁₋₅ alkoxy-C₁₋₅ alkoxy, hydroxy, hydroxy C₁₋₁₀ alkoxy, hydroxy C₁₋₁₀ alkyl, hydroxy C₁₋₂₀-alkyloxo, nitro, amino, aminosulfonyl, C₁₋₁₀ alkylcarbonylamino, hydroxy C₁₋₁₀ alkylcarbonylamino, amino C₂₋₁₀ alkoxy, amino C₁₋₁₀ alkylcarbonylamino, C₁₋₁₀ alkylaminocarbonyl, hydroxy C₁₋₁₀ alkylaminocarbonyl, amino C₁₋₁₀ alkylaminocarbonyl, mono or dialkylamino C₁₋₁₀ alkylaminocarbonyl, optionally substituted aryl C₁₋₁₀ alkyloxy, and cyano;
- wherein:: the aryl or heteroaryl of R₃ is optionally substituted with one or more substituents selected from the following: C₃₋₈ cycloalkyloxy, C₁₋₁₀ alkoxy, C₁₋₁₀ alkyl, C₂₋₁₀ alkenyl, C₃₋₁₀ alkenyloxy, mono- (C₁₋₆-alkyl)amino-C₂₋₆alkyloxy, di-(C₁₋₆ alkyl)amino-C₂₋₆alkyloxy, amino-C₂₋₆alkyloxy, heterocyclyl-C₂₋₆alkyloxy, aryl-C₂₋₆-alkyloxy, heteroaryl-C₂₋₆-alkyloxy, fluoroalkyl or fluoroalkoxy of the formula CₙHₓF_{y} or OCₙHₓF_{y} wherein n is an integer from 1 to 4, x is an integer from 0 to 8, y is an integer from 1 to 9 and the sum of x and y is 2n+1, C₁₋₆-alkoxy-C₂₋₆-alkoxy, aryl-C₁₋₆-alkyloxy, hydroxy, chloro, fluoro, bromo, hydroxy-C₁₋₆-alkoxy, hydroxy-C₁₋₁₀ alkyl, C₁₋₆ alkyloxo, cyano, amino, 4-aryloxo, di-C₁₋₅ alkylphosphityl-C₁₋₅ alkyl, C₁₋₆ alkyloxoamino, C₁₋₆ alkyloxo(C₁₋₆ alkyl)amino, C₁₋₁₀alkylsulfonyl, arylsulfonyl, carboxy, C₁₋₂₀ carboalkoxy, dialkylamino-C₁₋₁₀ alkyl, monoalkylamino-C₁₋₁₀ alkyl, and amino-C₁₋₁₀ alkyl;
- wherein:: the heterocycloalkyl substituent group attached to the alkyloxy group of R₃ is selected from the following: pyrrolidinyl, morpholinyl, piperidinyl, and piperazinyl;
- wherein:: the heteroaryl substituent group attached to the alkyloxy group of R₃ is selected from pyridinyl, and pyrrolidinyl;
- wherein:: the aryl of the arylsulfonyl substituent on the aryl of R₃ is selected from phenyl, indolyl, thiophenyl, and furanyl;
- wherein:: the aryl or heteroaryl groups of R₄ are optionally substituted with one or more substituents selected from the following: mono- or di-(C₁₋₆ alkyl)amino, mono- or di (C₁₋₆)alkylamino-C₂₋₆ alkyl, amino-C₁₋₆ alkyl, heterocycloalkyl, amino, C₁₋₂₀ alkoxy, chloro, fluoro, bromo, C₁₋₁₀ alkyl, aryl and heteroaryl;
- wherein:: the heterocycloalkyl group attached to the aryl of R₄ is selected from the following: morpholinyl, pyrrolidinyl, piperazinyl, and piperidinyl;
wherein: the heteroaryl group attached to the aryl of R₄ is selected from pyridyl, pyrrolyl, and furanyl;
R₅, R₆= independently H, or C₁₋₆ alkyl;
R₇, R₈ = independently H, or C₁₋₆ alkyl;
R₉ =di-(C₁₋₅ alkyl)amino-C₂₋₄ alkyl; mono(C₁₋₅ alkyl)amino-C₂₋₄ alkyl; amino-C₂₋₄ alkyl, or heterocycloalkyl methyl in which heterocycloalkyl is selected from the group consisting of thiophenyl, furanyl, pyrrolyl, and pyridinyl; and R₁₀ = H, or C₁₋₆ alkyl, except the compound wherein R is a phenyl para-substituted by a methyl group, R₁ is hydrogen, R₂ is CR5R6OH, R₃ is phenylmethyl, X is NR₁₀, R₅, R₆ and R₁₀ are hydrogen.
The compound wherein R is a phenyl substituted by a methyl group, R₁ is hydrogen, R₂ is CRSR6OH, R₃ is phenylmethyl, X is NR₁₀, R₅, R₆ and R₁₀ are hydrogen is described in Donkor IA et al. (2003) Bioorganic & Medicinal Chemistry Letters 13(5):783-784.

Preferred compounds of the present invention include enantiomers, stereoisomers, rotamers and tautomers of said compounds and pharmaceutically acceptable salts, solvates or derivatives thereof, said compounds having the general structure shown in formula I, wherein: R is selected from the group consisting of aryl, aryl C₁₋₆ alkyl, aryl C₂₋₄ alkenyl, wherein aryl is selected from phenyl, biphenyl or naphthyl; heteroaryl or heteroaryl C₁₋₄alkyl, wherein heteroaryl is selected from pyridyl, thiophenyl or benzothiophenyl; and heterocyclyl selected from piperidinyl or piperazinyl;
R₁ is hydrogen or methyl;
R₂ is CONHR₄, benzimidazol-2-yl, CR₅R₆OH, or CR₇R₈NHR₉;
R₃ is selected from the group consisting of phenyl and aryl C₁₋₄ alkyl or aryl C₂₋₄ alkenyl, wherein aryl is selected from phenyl, biphenyl, naphthyl or bisarylmethyl, and heteroaryl or heteroaryl C₁₋₄ alkyl, wherein heteroaryl is selected from indolyl and thiophenyl.
X is NR₁₀ or CH₂;
wherein R₄ is selected from the group consisting of hydrogen, C₁₋₁₀ alkyl, C₂₋₁₀ alkenyl, C₃₋₈ cycloalkyl, C₁₋₁₂ bicycloalkyl, C₁₀₋₁₄ tricycloalkyl , C₃₋₈ cycloalkyl- C₁₋₆ alkyl, C₈₋₁₂ bicycloalkylC₁₋₆ alkyl, C₈₋₁₂ bicycloalkylamino- C₁₋₆ alkyl, C₈₋₁₂ tricycloalkylamino-C₁₋₆ alkyl, C₃₋₈ cycloalkenyl, C₃₋₈ cycloalkenyl C₁₋₆ alkyl, fluoroalkyl of the formula CₙHₓF_{y} wherein n is an integer from 1 to 10, x is an integer from 0 to 20, y is an integer from 1 to 21 and the sum of x and y is 2n+1, amino C₂₋₁₀ alkyl, mono or dialkylamino C₂₋₁₀ alkyl, mono-, di, or trihydroxy C₂₋₁₀ alkyl, C₁₋₆alkoxy-C₂₋₁₀ alkyl, C₁₋₁₀ thioalkyl, C₁₋₆alkyl-thio-C₂₋₁₀alkyl, hydroxy-C₂₋₆-alkoxy-C₂₋₆alkyl, C₁₋₆alkyl-bis-(hydroxy-C₁₋₆alkyl), aryl, aryl C₁₋₆ alkyl, and aryl C₃₋₄ alkenyl, wherein aryl is selected from phenyl, biphenyl, indanyl and naphthyl, heteroaryl or heteroaryl C₁₋₁₀ alkyl, wherein heteroaryl is selected from pyridyl, thiophenyl, furanyl, benzofuranyl, benzothiophenyl, pyrrolidinyland indolyl; heterocycloalkyl or heterocycloalkyl C₁₋₁₀ alkyl wherein heterocycloalkyl is selected from tetrahydrofuranyl, tetrahydropyranyl, morpholinyl, piperidinyl, pyrrolidinyl, piperazinyl, dioxolanyl, sulfolanyl and 1,1-dioxotetrahydrothiopyranyl;
wherein C₅₋₈ cycloalkyl, C₈₋₁₂ bicycloalkyl, or heterocycloalkyl is optionally substituted with one or more substituents selected from the group consisting of C₁₋₆ alkyl, C₁₋₆ alkoxy, hydroxy, hydroxy C₁₋₆ alkoxy, hydroxy C₁₋₆ alkyl, amino, C₁₋₆ alkylcarbonylamino, hydroxy C₁₋₆ alkylcarbonylamino, amino C₂₋₆ alkoxy, mono-C₁₋₆-alkylamino-C₂₋₆-alkoxy, di-C₁₋₆-alkylamino-C₂₋₆-alkoxy, mono-(C₁₋₆ alkyl)amino, di-(C₁₋₆ alkyl)amino, amino C₁₋₆ alkylcarbonylamino, C₁₋₆ alkylaminocarbonyl, hydroxy C₁₋₆ alkylaminocarbonyl, amino C₂₋₆ alkylaminocarbonyl, mono or dialkylamino C₁₋₆ alkylaminocarbonyl, oxo, optionally substituted aryl and optionally substituted heteroaryl; wherein aryl is selected from phenyl, naphthyl and biphenyl and heteroaryl is selected from pyridyl and thiophenyl;
wherein: the aryl or heteroaryl groups of R are optionally substituted with one or more substituents selected from the group consisting of fluorine, chlorine, bromine, C₁₋₁₀ alkyl, fluoroalkyl or fluoroalkoxy of the formula CₙHₓF_{y} or OCₙHₓF_{y} wherein n is an integer from 1 to 4, x is an integer from 0 to 8, y is an integer from 1 to 9 and the sum of x and y is 2n+1, C₁₋₁₀ alkoxy, C₁₋₁₀ alkenyloxy, C₁₋₁₀ -alkoxy- C₁₋₁₀ -alkoxy, hydroxy, hydroxy C₁₋₁₀ alkoxy, hydroxy C₁₋₁₀ alkyl, hydroxy C₁₋₁₀ acyl, nitro, amino, aminosulfonyl, C₁₋₁₀ alkylcarbonylamino, hydroxy C₁₋₁₀ alkylcarbonylamino, amino C₂₋₁₀ alkoxy, amino C₁₋₁₀ alkylcarbonylamino, C₁₋₁₀ alkylaminocarbonyl, hydroxy C₁₋₁₀ alkylaminocarbonyl, amino C₁₋₁₀ alkylaminocarbonyl, mono or dialkylamino C₁₋₁₀ alkylaminocarbonyl, optionally substituted aryl or heteroarylsulfonyl, aminosulfonyl, mono- or di(C₁₋₆)alkylaminosulfonyl, optionally substituted phenyl or thiophenyl, optionally substituted phenyl or thiophenyl C₁₋₁₀ alkyloxy, and cyano;
wherein the phenyl or thiophenyl substituent on the aryl or heteroaryl group of R is optionally substituted with one or more substituents selected from the group consisting of fluorine, chlorine, bromine, C₁₋₅ alkyl, CF₃, OCF₃, C₁₋₆ alkoxy, C₁₋₆ alkenyloxy, C₁₋₅ alkoxy-C₁₋₅ alkoxy, hydroxy, hydroxy C₁₋₁₀ alkoxy, hydroxy C₁₋₁₀ alkyl, nitro, amino, aminosulfonyl, C₁₋₁₀ alkylcarbonylamino, hydroxy C₁₋₆ alkylcarbonylamino, amino C₂₋₆ alkoxy, amino C₁₋₆ alkylcarbonylamino, C₁₋₆ alkylaminocarbonyl, hydroxy C₁₋₆ alkylaminocarbonyl, amino C₁₋₆ alkylaminocarbonyl, mono or dialkylamino C₁₋₆ alkylaminocarbonyl, optionally substituted aryl C₁₋₆ alkyloxy, and cyano; wherein:
the aryl or heteroaryl of R₃ is optionally substituted with one or more substituents selected from the following: C₃₋₆ cycloalkyloxy, C₁₋₆ alkoxy, C₁₋₆ alkyl, C₂₋₆ alkenyl, C₃₋₆ alkenyloxy, mono- (C₁₋₆ alkyl)amino-C₂₋₆ alkyloxy, di-(C₁₋₆ alkyl)amino-C₂₋₆ alkyloxy, amino-C₂₋₆ alkyloxy, heterocyclyl- C₂₋₆ alkyloxy, aryl- C₂₋₆ alkyloxy, heteroaryl- C₂₋₆ alkyloxy, fluoroalkyl or fluoroalkoxy of the formula CₙHₓF_{y} or OCₙHₓF_{y} wherein n is an integer from 1 to 4, x is an integer from 0 to 8, y is an integer from 1 to 9 and the sum of x and y is 2n+1, C₁₋₆ alkoxy - C₂₋₆ alkyloxy, aryl- C₁₋₆ alkoxy, hydroxy, chloro, fluoro, bromo, hydroxy- C₁₋₆ alkoxy, hydroxy-C₁₋₁₀ alkyl, C₁₋₆alkyloxo, cyano, amino, 4-aryloxo, C₁₋₆ alkyloxoamino, C₁₋₆ alkyloxo(C₁₋₆ alkyl)amino, carboxy, dialkylamino- C₁₋₆ alkyl, monoalkylamino- C₁₋₆ alkyl, and amino-C₁₋₆ alkyl;
   wherein: the heterocyclyl substituent group attached to the alkyloxy group of R₃ is selected from the following: pyrrolidinyl, morpholinyl, piperidinyl, and piperazinyl;
   wherein: the heteroaryl substituent group attached to the alkyloxy group of R₃ is selected from pyridinyl, and pyrrolidinyl;
   wherein:
   the aryl or heteroaryl groups of R₄ are optionally substituted with one or more substituents selected from the following: mono- or di-( C₁₋₆ alkyl)amino, mono- or di-(C₁₋₆)alkylamino-C₂₋₆-alkyl, amino- C₁₋₆ alkyl, heterocyclyl, amino, C₁₋₁₀ alkoxy, chloro, fluoro, bromo, C₁₋₁₀ alkyl, aryl and heteroaryl;
   wherein the heterocyclyl group attached to the aryl of R₄ is selected from the following: morpholinyl, pyrrolidinyl, piperazinyl, piperidinyl; wherein the heteroaryl group attached to the aryl of R₄ is selected from pyridyl, pyrrolyl, and furanyl;
   R₅, R₆ = H;
   R_{7,} R₈ = H;
   R₉ = di-(C₁₋₅ alkyl)amino-C₂₋₄ alkyl; mono(C₁₋₅ alkyl)amino-C₂₋₄ alkyl; amino-C₂₋₄ alkyl, heterocycloalkyl methyl in which heterocycloalkyl is selected from group consisting of thiophenyl, furanyl pyrrolyl, pyridinyl; and R₁₀ = H, CH₃ , except the compound wherein R is a phenyl para-substituted by a methyl group, R₁ is hydrogen, R₂ is CR5R6OH, R₃ is phenylmethyl, X is NR₁₀, R₅, R₆ and R₁₀ are hydrogen.
   The compound wherein R is a phenyl substituted by a methyl group, R₁ is hydrogen, R₂ is CR5R60H, R₃ is phenylmethyl, X is NR₁₀, R₅, R₆ and R₁₀ are hydrogen is described in Donkor IA et al. (2003) Bioorganic & Medicinal Chemistry Letters 13(5):783-784.

More preferred compounds according to the present invention include enantiomers, stereoisomers, rotamers and tautomers of said compound and pharmaceutically acceptable salts, solvates or derivatives thereof, said compound having the general structure shown in formula I, wherein:
R is aryl selected from phenyl, biphenyl and naphthyl, or heteroaryl selected from pyridyl and thiophenyl,
R₁ is hydrogen or methyl,
R₂ is CONHR₄,
R₃ is aryl-C₁, alkyl wherein aryl is selected from phenyl, biphenyl and naphthyl, or heteroaryl-C₁ alkyl, wherein heteroaryl is selected from indolyl and thiophenyl,
X is NR₁₀,
wherein R₄ is selected from the group consisting of hydrogen, C₁₋₁₀ alkyl, C₂₋₁₀ alkenyl, C₃-₈ cycloalkyl, C₈-₁₂ bicycloalkyl, C₁₀₋₁₄ tricycloalkyl , C₃₋₈ cycloalkyl- C₁₋₆ alkyl, C₈₋₁₂ bicycloalkylC₁₋₆ alkyl, C₈₋₁₂ bicyoloamino-C₁₋₆ alkyl, C₈₋₁₂ tricycloamino-C₁₋₆ alkyl, C₃-₈ cycloalkenyl, C₃₋₈ cycloalkenyl C₁₋₆ alkyl, fluoroalkyl of the formula CₙHₓF_{y} wherein n is an integer from 1 to 6, x is an integer from 0 to 12, y is an integer from 1 to 13 and the sum of x and y is 2n+1, amino C₂₋₈ alkyl, mono or dialkylamino C₂₋₈ alkyl, mono-, di-, or trihydroxy C₂₋₁₀ alkyl, C₁₋₆-alkoxy-C₂₋₁₀ alkyl, C₁₋₆ thioalkyl, C₁₋₆ alkyl-thio- C₂₋₆ alkyl, hydroxy- C₂₋₆-alkoxy C₂₋₆ alkyl, C₁₋₆ alkyl-bis-(hydroxy-C₁₋₆ alkyl), aryl, aryl C₁₋₆ alkyl, wherein aryl is selected from phenyl, biphenyl, naphthyl, heteroaryl or heteroaryl C₁₋₆ -alkyl, wherein heteroaryl is selected from pyridyl, thiophenyl, heterocycloalkyl or heterocycloalkyl C₁₋₆ alkyl wherein heterocycloalkyl is selected from tetrahydrofuranyl, tetrahydropyranyl, morpholinyl, piperidinyl, pyrrolidinyl, and piperazinyl;
wherein: the aryl or heteroaryl groups of R are optionally substituted with one or more substituents selected from the group consisting of fluorine, chlorine, bromine, C₁₋₁₀ alkyl, CF₃, OCF₃, C₁₋₁₀ alkoxy, C₁₋₁₀ alkenyloxy, C₁₋₅ alkoxy-C₁-₁₀ -alkoxy, hydroxy, hydroxy C₁₋₁₀ alkoxy, hydroxy C₁₋₁₀ alkyl, nitro, amino, C₁₋₁₀ alkylcarbonylamino, hydroxy C₁₋₁₀ alkylcarbonylamino, amino C₂₋₁₀ alkoxy, amino C₁₋₁₀ alkylcarbonylamino, optionally substituted phenyl or thiophenyl, optionally substituted phenyl or thiophenyl C₁₋₁₀ alkyloxy, and cyano;
wherein the phenyl or thiophenyl substituent on the aryl or heteroaryl group of R is optionally substituted with one or more substituents selected from the group consisting of fluorine, chlorine, bromine, C₁₋₅ alkyl, CF₃, OCF₃, C₁-₆alkoxy, C₁₋₆ alkenyloxy, C₁₋₅ alkoxy- C₁₋₅ alkoxy, hydroxy, hydroxy C₁₋₁₀ alkoxy, hydroxy C₁₋₁₀ alkyl, nitro, optionally substituted aryl C₁₋₆ alkyloxy, and cyano;
wherein: the aryl or heteroaryl of R₃ is optionally substituted with one or more substituents selected from: C₃₋₆ cycloalkyloxy, C₁₋₆ alkoxy, C₁₋₆ alkyl, C₂₋₆ alkenyl, C₃₋₆ alkenyloxy, mono- (C₁₋₆ alkyl)amino- C₂₋₆ alkyloxy, di-( C₁₋₆ alkyl)amino- C₂₋₆ -alkyloxy, amino- C₂₋₆ alkyloxy, heterocyclyl- C₂₋₆ alkyloxy, aryl- C₂₋₆ -alkyloxy, heteroaryl- C₂₋₆ -alkyloxy, fluoroalkyl or fluoroalkoxy of the formula CₙHₓF_{y} or OCₙHₓF_{y} wherein n is an integer from 1 to 4, x is an integer from 0 to 8, y is an integer from 1 to 9 and the sum of x and y is 2n+1, C₁₋₆ -alkoxy- C₂₋₆-alkoxy, aryl- C₁₋₆ -alkyloxy, hydroxy, chloro, fluoro, bromo, nitro, hydroxy- C₁₋₆ -alkoxy, hydroxy-C₁₋₆, C₁₋₆ alkyloxo, cyano, amino, 4-aryloxo, C₁₋₆ alkyloxoamino, carboxy, dialkylamino- C₁₋₆ alkyl, monoalkylamino- C₁₋₆ -alkyl, and amino- C₁₋₆ alkyl;
wherein: the heterocycloalkyl substituent group attached to the alkyloxy group of R₃ is selected from: pyrrolidinyl, morpholinyl, and piperidinyl;
wherein: the heteroaryl substituent group attached to the alkyloxy group of R₃ is selected from pyridinyl, and pyrrolidinyl;
wherein: the aryl or heteroaryl groups of R₄ are optionally substituted with one or more substituents selected from the following: mono- or di-( C₁₋₆ - . alkyl)amino, mono- or di (C₁₋₆)alkylamino-C₂₋₆-alkyl, amino- C₁₋₆ -alkyl, heterocycloalkyl, amino, C₁₋₆ alkoxy, chloro, fluoro, bromo, C₁₋₆ -alkyl, aryl and heteroaryl;
wherein the heterocycloalkyl group attached to the aryl of R₄ is selected from the following: morpholinyl, pyrrolidinyl, piperazinyl, and piperidinyl; and wherein the heteroaryl group attached to the aryl of R₄ is selected from pyridyl and pyrrolyl.

Still more preferred compounds of the invention include compounds of Formula I, wherein:
- R is: aryl, wherein aryl is selected from phenyl and naphthyl,
- R₁ is: hydrogen or methyl ,
- R₂ is: CONHR₄,
- R₃ is: aryl-C₁-alkyl, wherein aryl is selected from phenyl, indolyl, and thiophenyl;
- X is: NH wherein R₄, is selected from the group consisting of hydrogen, C₁₋₁₀ alkyl, C₃₋₆ cycloalkyl, adamantyl, adamantyl-C₁-alkyl, 2-adamantylaminoethyl, C₃₋₆ -cycloalkyl-C₁ alkyl, 3-propenyl, trifluoromethyl-C₁-alkyl, 4-amino-n-butyl, 4-(methylamino)-n-butyl, 4-(dimethylamino)-n-butyl; 4-diethylaminobutyl, 2-hydroxyethyl, 2-(2-hydroxyethoxy)ethyl, 3-hydroxypropyl, 2-methoxyethylaryl, wherein aryl is selected from phenyl and naphthyl; aryl C₁-alkyl, wherein aryl is selected from phenyl, and biphenyl, heteroaryl C₁-alkyl, wherein heteroaryl is selected from 2-thiophenyl and 2-pyridinyl, heterocycloalkyl, wherein heterocycloalkyl is selected from 4-tetrahydropyranyl, 4-piperidinyl, 2,2,6,6-tetramethylpiperidinyl and 1-ethyl-4-piperidinyl, heterocycloalkyl-C₁-alkyl, wherein heterocycloalkyl is selected from 4-tetrahydropyranyl and 2-tetrahydrofuranyl, 2- heterocyclodkyl-C₂-alkyl, wherein heterocycloalkyl is selected from 4-tetrahydropyranyl and 1-piperazinyl;
- wherein:: the phenyl of R is optionally substituted with one or two substituents in the 3-, 4-, or 5-positions selected from the group consisting of fluorine, chlorine, bromine, trifluoromethyl, C₁₋₇ alkyloxy, C₁₋₇ alkenyloxy, Clog alkyl, and C₂₋₃ hydroxypropyl; the naphthyl of R is optionally substituted with one of the substituents selected from chlorine, fluorine, and dimethylamino; the phenyl of R₃ is optionally substituted with one or two substituents selected from the group consisting of C₁₋₅ alkyl, C₁₋₅ alkyloxy, 2-dimethylaminoethoxy, 2-pyrrolidino-ethoxy, 3-fluoropropyloxy, 3-methoxypropyloxy, cyclopentoxy, benzyloxy, hydroxy, chloro, fluoro, bromo, trifluoromethyl, 1-hydroxyethyl, acetyl, allyloxy, cyano, amino, 4-benzoyl, and acetoxy; the phenyl of R₄ is optionally substituted with one or two substituents selected from the group consisting of 4-dimethylamino, 4-methylamino, 4-amino, methoxy, ethoxy, chloro, 4-morpholino, fluoro, and methyl.

A compound of the present invention, according to Formula I, may also be selected from:
(S)-1-(3-Trifluoromethyl-benzenesulfonyl)-piperidine-2-carboxylic acid [(S)-1-(4-dimethylamino-phenylcarbamoyl)-2-(4-methoxy-phenyl)-ethyl]-amide;
(S)-1-(3-Trifluoromethyl-benzenesulfonyl)-piperidine-2-carboxylic acid [S)-2-(4-aoetyl-phenyl)-1-(4-ethoxy-phenylcarbamoyl)-ethyl]-amide;
(S)-1-(3-Trifluoromethyl-benzenesulfonyl)-piperidine-2-carboxylic acid [(S)-1-(4-morpholin-4-yl-phenylcarbamoyl)-2-(4-trifluoromethyl-phenyl)-ethyl]-amide;
(S)-1-(3-Trifluoromethyl-benzenesulfonyl)-piperidine-2-carboxylic acid [(S)-2-(4-methoxyphenyl)-1-(tetrahydro-pyran-4-ylcarbamoyl)-ethyl]-amide;
(S)-1-(3-Trifluoromethyl-benzenesulfonyl)-piperidine-2-carboxylic acid [(S)-2-(2-methyl-4-methoxy-phenyl)-1-(tetrahydro-pyran-4-ylcarbamoyl)-ethyl]-amide;
(S)-1-(3-Trifluoromethyl-benzenesulfonyl)-piperidine-2-carboxylic acid [(S)-2-(3,4-dimethoxy-phenyl)-1-(tetrahydro-pyran-4-yl-carbamoyl)-ethyl]-amide;
(S)-1-(3-Trifluoromethyl-benzenesulfonyl)-piperidine-2-carboxylic acid [(S)-2-(4-methoxyphenyl)-1-(4-morpholin-4-yl-phenylcarbamoyl)-ethyl]-amide;
(S)-1-(3-Methoxy-benzenesulfonyl)-piperidine-2-carboxylic acid ((S)1-carbamoyl-2-phenylethyl)-amide;
(S)-1-(3-Hydroxy-benzenesulfonyl)-piperidine-2-carboxylic acid ((S)-1-carbamoyl-2-phenylethyl)-amide;
(S)-1-(3-Propoxy-benzenesulfonyl)-piperidine-2-carboxylic acid ((S)-1-carbamoyl-2-phenylethyl)-amide;
(S)-1-(3-Trifluoromethyl-benzenesulfonyl)-piperidine-2-carboxylic acid [(S)-2-(4-(1-hydroxyethyl)-phenyl]-1-(4-morpholin-4-yl-phenylcarbamoyl)-ethyl]-amide;
(S)-1-(3-Trifluoromethyl-benzenesulfonyl)-piperidine-2-carboxylic acid {(S)-(4-ethoxy-phenylcarbamoyl)-2-[4-(1-hydroxy-ethyl)-phenyl]-ethyl}-amide;
(S)-1-(3-Methoxy-benzenesulfonyl)-piperidine-2-carboxylic acid [(S)-2-(indol-3-yl)-1-(naphthalen-2-ylcarbamoyl)-ethyl]-amide;
(S)-1-(3-Trifluoromethyl-benzenesulfonyl)-piperidine-2-carboxylic acid {(S)-1-(4-dimethylamino-phenylcarbamoyl)-2-[4-(1-hydroxy-ethyl)-phenyl]-ethyl}-amide;
(S)-1-(3,5-Dichloro-benzenesulfonyl)-piperidine-2-carboxylic acid [(S)-2-[1H-indol-3-yl)-1-methylcarbamoyl-ethyl]-amide;
(S)-1-(3-Trifluoromethyl-benzenesulfonyl)-piperidine-2-carboxylic acid [(S)-2-(4-acetylphenyl)-1-(4-morpholin-4-yl-phenylcarbamoyl)-ethyl]-amide;
(S)-1-(3-Fluoro-benzenesulfonyl)-piperidine-2-carboxylic acid [(S)-2-(4-cyclopentyloxy-phenyl)-1-methyl-1-(tetrahydro-pyran-4-ylcarbamoyl)-ethyl]-amide;
(S)-1-(3-Fluoro-benzenesulfonyl)-piperidine-2-carboxylic acid [(S)-2-(4-isopropoxy-phenyl)-1-(2,2,6,6-tetramethyl-piperidin-4-ylcarbamoyl)-ethyl]-amide;
(S)-1-(3-Fluoro-benzenesulfonyl)-(2S)-piperidine-2-carboxylic acid [(S)-2-[4-(3-fluoro-propoxy)-phenyl]-1-(2,2,6,6-tetramethyl-piperidin-4-ylcarbamoyl)-ethyl]-amide;
(S)-1-(3-Fluoro-benzenesulfonyl)-piperidine-2-carboxylic acid [(S)-2[4-(3-fluoro-propoxy)-phenyl]-1-((S)-2-methoxy-1-methyl-ethylcarbamoyl)-ethyl]-amide;
(S)-1-(3-Trifluoromethyl-benzenesulfonyl)-piperidine-2-carboxylic acid [(S)-2-(4-isopropoxyphenyl)-1-methylcarbamoyl-ethyl]-amide;
(S)-1-(3-Trifluoromethyl-benzenesulfonyl)-piperidine-2-carboxylic acid [(S)-2-(4-isopropoxyphenyl)-1-(tetrahydro-pyran-4-ylcarbamoyl)-ethyl]-amide;
(S)-1-(3-Trifluoromethyl-benzenesulfonyl)-piperidine-2-carboxylic acid [(S)-2-(phenyl)-1-(benzimidazol-2-yl)-ethyl]-amide;
(S)-1-(3-Trifluoromethyl-benzenesulfonyl)-piperidine-2-carboxylic acid [(S)-2-(4-(benzyloxy)phenyl)-1-(benzimidazol-2-yl)-ethyl]-amide;
(S)-1-(3-Fluoro-benzenesulfonyl)-piperidine-2-carboxylic acid [(S)-2-(4-methoxy-phenyl)-1-(tetrahydropyran-4-yl-carbamoyl)-ethyl]-amide;
(S)-1-Benzenesulfonyl-piperidine-2-carboxylic acid ((S)-1-amido-2-(2-methylphenyl)-ethyl)-amide;
(S)-1-Benzenesulfonyl-piperidine-2-carboxylic acid ((S))-1-cyclohexylatnido-2-(4-methylphenyl)-ethyl)-amide;
(S)-1-Benzenesulfonyl-piperidine-2-carboxylic acid [(S)-2-(4-allyloxy-phenyl)-1-cyclohexylamido-ethyl]-amide;
(S)-1-(3-Trifluoromethyl-benzenesulfonyl)-piperidine-2-carboxylic acid [(S)-2-(4-allyloxy-phenyl)-1-cyclohexylcarbamoyl-ethyl]-amide;
(S)-1-(3-Trifluoromethyl-benzenesulfonyl)-piperidine-2-carboxylic acid ((S)-1-cyclohexylamido-2-(4-methylphenyl)-ethyl)-amide;
(S)-1-((E)-2-Phenyl-ethenesulfonyl)-piperidine-2-carboxylic acid [(S)-1-amido-2-(1H-indol-3-yl)-ethyl]-amide;
(S)-1-(4,5-Dibromo-thiophene-2-sulfonyl)-piperidine-2-carboxylic acid [(S)-1-amido-2-(1H-indol-3-yl)-ethyl]-amide;
(S)-1-Benzenesulfonyl-piperidine-2-carboxylic acid [(S)-1-tert-butylamido-2-(1H-indol-3-yl)-ethyl]-amide;
(S)-1-Benzenesulfonyl-piperidine-2-carboxylic acid [(S)-1-(3-hydroxy-2,2-dimethyl-propylamido)-2-(4-isopropyl-phenyl)-ethyl]-amide;
(S)-1-Benzenesulfonyl-piperidine-2-carboxylic acid methyl-((S)-1-methylamido-2-(phenyl)-ethyl)-amide;
(S)-1-Benzenesulfonyl-piperidine-2-carboxylic acid [(S)-1-(3,3-dimethyl-butylamido)-2-(4-methoxyphenyl)-ethyl]-amide;
(S)-1 -Benzenesulfonyl-piperidine-2-carboxylic acid [(S)-1-(2,2-dimethyl-propylamido)-2-(phenyl)-ethyl]-amide;
(4- {(S)-2-[((S)-1-Benzenesulfonyl-piperidine-2-carbonyl)-arnino]-2-amido-ethyl}-benzyl)-phosphonic acid diethyl ester;
(S)-1-Benzenesulfonyl-piperidine-2-carboxylic acid [(S)-2-(4-(acetylamino)-phenyl)-1-amido-ethyl]-amide;
(S)-1-Benzenesulfonyl-piperidine-2-carboxylic acid [(S)-1-amido-2-(4-(hydroxymethyl)-phenyl)-ethyl]-amide;
(S)-1-(Naphthalene-2-sulfonyl)-piperidine-2-carboxylic acid [(S)-1-amido-2-(1H-indol-3-yl)-ethyl]-amide;
(S)-1-(3-Trifluoromethyl-benzenesulfonyl)-piperidine-2-carboxylic acid [(S)-1-(cyclohexylmethyl-amido)-2-(4-methylphenyl)ethyl]-amide;
(S)-1-(3-Trifluoromethyl-benzenesulfonyl)-piperidine-2-carboxylic acid [(S)-1-(4-amino-butylamido)-2-(4-methylphenyl)-ethyl]-amide;
(S)-1-Benzenesulfonyl-piperidine-2-carboxylic acid [(S)-1-amido-2-(1H-indol-3-yl)-ethyl]-amide;
(S)-1-Benzenesulfonyl-piperidine-2-carboxylic acid ((S)-1-amido-2-(pyridin-4-yl)-ethyl)-amide;
(S)-1-Benzenesulfonyl-piperidine-2-carboxylic acid ((S)-1-amido-2-cyclohexyl-ethyl)-amide;
(S)-1-Benzenesulfonyl-piperidine-2-carboxylic acid {(S)-2-phenyl-1-(thiophen-2-ylmethyl)-amido]-ethyl}-amide;
(S)-1-(3-Fluoro-benzenesulfonyl)-piperidine-2-carboxylic acid [(S)-1-(4-dimethylalnino-butylcarbamoyl)-2-(4-fluoro-phenyl)-ethyl]-amide;
(S)-1-(3-Fluoro-benzenesulfonyl)-piperidine-2-carboxylic acid [(S)-2[4-(3-methoxy-propoxy)-phenyl]-1-(tetrahydro-pyran-4-ylcarbamoyl)-ethyl]-amide;
(S)-1-(3-Trifluoromethyl-benzenesulfonyl)-piperidine-2-carboxylic acid {(S)-1-cyclohexylcarbamoyl-2-[4-(2-dimethylamino-ethoxy)-phenyl]-ethyl}-amide;
(S)-1-(3-Trifluoromethyl-benzenesulfonyl)-piperidine-2-carboxylic acid [(S)-2-(4-methoxyphenyl)-1-(2-piperazin-1-yl-ethylcarbamoyl)-ethyl]-amide;
(S)-1-(3-Trifluoromethyl-benzenesulfonyl)-piperidine-2-carboxylic acid [{S)-1-(2-hydroxy-1,1-dimethyl-ethylcarbamoyl)-2-(4-methoxy-phenyl)-ethyl]-amide;
(S)-1-Benzenesulfonyl-piperidine-2-carboxylic acid ((S)-1-carbamoyl-2-phenyl-ethyl)-amide;
(S)-1-(3-Fluoro-benzenesulfonyl)-piperidine-2-carboxylic acid {(S)-2-[4-(3-methoxy-propoxy)-phenyl]-1-methylcarbamoyl-ethyl}-amide;
(S)-1-(3-Fluoro-benzenesulfonyl)-piperidine-2-carboxylic acid {(S)-1-((S)-2-methoxy-1-methyl-ethylcarbamoyl)-2-[4-(3-methoxy-propoxy)-phenyl]-ethyl}-amide; and
(S)-1-(3-Hydroxy-benzenesulfonyl)-piperidine-2-carboxylic acid [(S)-2-(1H-indol-3-yl)-1-(naphthalen,-2-ylcarbamoyl)-ethyl]-amide.

Included in the present invention as an additional aspect of the invention is a pharmaceutical composition, comprising a compound, including enantiomers, stereoisomers, rotamers and tautomers of said compound and pharmaceutically acceptable salts, solvates or derivatives thereof, having the general structure shown in formula I; wherein:
- R is: fluoroalkyl of the formula CₙHₓF_{y} wherein n is an integer from 1 to 6, x is an integer from 0 to 12, y is an integer from 1 to 13 and the sum of x and y is 2n+1, aryl, aryl C₁₋₆ alkyl, aryl C₂₋₄ alkenyl, wherein aryl is selected from phenyl, biphenyl or naphthyl, heteroaryl or heteroaryl C₁₋₄ alkyl, wherein heteroaryl is selected from pyridyl, thiophenyl or benzothiophenyl, and heterocyclyl selected from piperidinyl or piperazinyl,
- R₁ is: hydrogen or C₁₋₆ alkyl;
- R₂ is: CONHR₄, benzimidazol-2-yl, CR₅R₆OH, or CR₇R₈NHR₉;
- R₃ is: aryl and aryl C₁₋₄ alkyl or aryl C₂₋₄ alkenyl, wherein aryl is selected from phenyl, biphenyl, naphthyl or bisarylmethyl, heteroaryl or heteroaryl C₁₋₄ alkyl, wherein heteroaryl is selected from indolyl or thiophenyl, benzothiophenyl, furanyl, benzofuranyl, 1,3-benzodioxolyl, 1, 4-benzodioxanyl or C₅₋₇ cycloalkyl C₁ alkyl,;
- X is: NR₁₀ or CH₂; wherein R₄ is selected from the group consisting of hydrogen, C₁₋₁₀ alkyl, C₂₋₁₀ alkenyl, C₃₋₈ cycloalkyl, C₈₋₁₂ bicycloalkyl, C₁₀₋₁₄ tricycloalkyl , C₃₋₈ cycloalkyl C₁₋₆ alkyl, C₈₋₁₂ bicycloalkylC₁₋₆ alkyl, C₈₋₁₂ bicycloalkylamino- C₁₋₆ alkyl, C₈₋₁₂ tricycloalkylamino-C₁₋₆ alkyl, C₃₋₈ cycloalkenyl, C₃₋₈ cycloalkenyl C₁₋₆ alkyl, fluoroalkyl of the formula CₙHₓF_{y} wherein n is an integer from 1 to 20, x is an integer from 0 to 40, y is an integer from 1 to 41 and the sum of x and y is 2n+1, amino C₂₋₂₀ alkyl, mono or dialkylamino C₂₋₂₀ alkyl, mono-, di, or trihydroxy C₂₋₂₀ alkyl, C₁₋₆ alkoxy- C₂₋₂₀ alkyl, C₁₋₂₀ thioalkyl, C₁₋₆ alkyl-thio-C₂₋₂₀ alkyl, hydroxy-C₂₋₆ -alkoxy-C₂₋₆ alkyl, C₁₋₆ alkyl-bis(hydroxy-C₁₋₆ alkyl), aryl, aryl C₁₋₁₀ alkyl, aryl C₃₋₄ alkenyl, wherein aryl is selected from phenyl, biphenyl, indanyl or naphthyl, heteroaryl or heteroaryl C₁₋₁₀ alkyl, wherein heteroaryl is selected from pyridyl, thiophenyl, furanyl, benzofuranyl, benzothiophenyl, pyrrolidinyl, indolyl, heterocycloalkyl or heterocycloalkyl C₁₋₁₀ alkyl wherein heterocycloalkyl is selected from tetrahydrofuranyl, tetrahydropyranyl, morpholinyl, piperidinyl, pyrrolidinyl, tetrahydrothiophenyl, piperazinyl, dioxolanyl, sulfolanyl or 1,1-dioxotetrahydrothiopyranyl; heterobicyclyl of the formula: 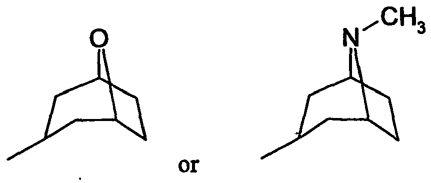 wherein C₅₋₈ cycloallcyl, C₈₋₁₂ bicycloalkyl, or heterocyclyl is optionally substituted with one or more substituents selected from the group consisting of C₁₋₁₀ alkyl, C₁₋₁₀ alkoxy, hydroxy, hydroxy C₁₋₁₀ alkoxy, hydroxy C₁₋₁₀ alkyl, C₁₋₁₀ acyloxy, amino, C₁₋₁₀ alkylcarbonylamino, hydroxy C₁₋₂₀ alkylcarbonylamino, amino C₂₋₁₀ alkoxy, mono-C₁₋₆ -alkylamino-C₂₋₁₀-alkoxy, di-C₁₋₆-alkylamino-C₂₋₁₀-alkoxy, mono-(C₁₋₁₀ alkyl)amino, di-(C₁₋₁₀ alkyl)amino, amino C₁₋₁₀ alkylcarbonylamino, C₁₋₁₀ alkylaminocarbonyl, hydroxy C₁₋₁₀ alkylaminocarbonyl, amino C₂₋₁₀ alkylaminocarbonyl, mono or dialkylamino C₁₋₁₀ alkylaminocarbonyl, oxo, optionally substituted aryl or optionally substituted heteroaryl, wherein aryl is selected from phenyl, naphthyl or biphenyl and heteroaryl is selected from pyridyl or thiophenyl.
- wherein:: the aryl or heteroaryl groups of R are optionally substituted with one or more substituents selected from the group consisting of fluorine, chlorine, bromine, C₁₋₂₀ alkyl, fluoroalkyl or fluoroalkoxy of the formula CₙHₓF_{y} or OCₙHₓF_{y} wherein n is an integer from 1 to 4, x is an integer from 0 to 8, y is an integer from 1 to 9 and the sum of x and y is 2n+1, C₁₋₂₀ alkoxy, C₁₋₂₀ alkenyloxy, C₁₋₁₀-alkoxy-C₁₋₁₀-alkoxy, hydroxy, hydroxy C₁₋₂₀ alkoxy, hydroxy C₁₋₂₀ alkyl, C₁₋₂₀ acyloxy, hydroxy C₁₋₂₀ acyl, nitro, amino, aminosulfonyl, C₁₋₂₀ alkylcarbonylamino, hydroxy C₁₋₂₀ alkylcarbonylamino, amino C₂₋₂₀ alkoxy, amino C₁₋₂₀ alkylcarbonylamino, C₁₋₂₀ alkylaminocarbonyl, hydroxy C₁₋₂₀ alkylaminocarbonyl, amino C₁₋₂₀ alkylaminocarbonyl, mono or dialkylamino C₁₋₂₀ alkylaminocarbonyl, optionally substituted aryl or heteroarylsulfonyl, aminosulfonyl, mono- or di(C₁₋₆)alkylaminosulfonyl, optionally substituted phenyl or thiophenyl, optionally substituted phenyl or thiophenyl C₁₋₁₀ alkyloxy, and cyano; wherein the phenyl or thiophenyl substituent on the aryl or heteroaryl group of R is optionally substituted with one or more substituents selected from the group consisting of fluorine, chlorine, bromine, C₁₋₅ alkyl, fluoroalkyl or fluoroalkoxy of the formula CₙHₓF_{y} or OCₙHₓF_{y} wherein n is an integer from 1 to 4, x is an integer from 0 to 8, y is an integer from 1 to 9 and the sum of x and y is 2n+1, C₁₋₁₀alkoxy, C₁₋₁₀ alkenyloxy, C₁₋₅ alkoxy-C₁₋₅ alkoxy, hydroxy, hydroxy C₁₋₁₀ alkoxy, hydroxy C₁₋₁₀ alkyl, hydroxy C₁₋₂₀-alkyloxo, nitro, amino, aminosulfonyl, C₁₋₁₀ alkylcarbonylamino, hydroxy C₁₋₁₀ alkylcarbonylamino, amino C₂₋₁₀ alkoxy, amino C₁₋₁₀ alkylcarbonylamino, C₁₋₁₀ alkylaminocarbonyl, hydroxy C₁₋₁₀ alkylaminocarbonyl, amino C₁₋₁₀ alkylaminocarbonyl, mono or dialkylamino C₁₋₁₀ alkylaminocarbonyl, optionally substituted aryl C₁₋₁₀ alkyloxy, and cyano;
- wherein:: the aryl or heteroaryl of R₃ is optionally substituted with one or more substituents selected from the following: C₃₋₈ cycloalkyloxy, C₁₋₁₀ alkoxy, C₁₋₁₀ alkyl, C₂₋₁₀ alkenyl, C₃₋₁₀ alkenyloxy mono- (C₁₋₆-alkyl)amino-C₂₋₆alkyloxy, di-(C₁₋₆ alkyl)amino-C₂₋₆-alkyloxy, amino-C₂₋₆alkyloxy, heterocyclyl-C₂₋₆alkyloxy, aryl-C₂₋₆-alkyloxy, heteroaryl-C₂₋₆-alkyloxy, fluoroalkyl or fluoroalkoxy of the formula CₙHₓF_{y} or OCₙHₓF_{y} wherein n is an integer from 1 to 4, x is an integer from 0 to 8, y is an integer from 1 to 9 and the sum of x and y is 2n+1, C₁₋₆-alkoxy-C₂₋₆-alkoxy, aryl-C₁₋₆-alkyloxy, hydroxy, chloro, fluoro, bromo, hydroxy-C₁₋₆-alkoxy, hydroxy-C₁₋₁₀ alkyl, C₁₋₆ alkyloxo, cyano, amino, 4-aryloxo, di-C₁₋₅ alkylphosphityl-C₁₋₅ alkyl, C₁₋₆ alkyloxoamino, C₁₋₆ alkyloxo(C₁₋₆ alkyl)amino, C₁₋₁₀ alkylsulfonyl, arylsulfonyl, carboxy, C₁₋₂₀ carboalkoxy, dialkylamino-C₁₋₁₀ alkyl, monoa1kylamino-C₁₋₁₀ alkyl, amino-C₁₋₁₀ alkyl;
- wherein:: the heterocycloalkyl substituent group attached to the alkyloxy group of R₃ is selected from the following: pyrrolidinyl, morpholinyl, piperidinyl, and piperazinyl;
- wherein:: the heteroaryl substituent group attached to the alkyloxy group of R₃ is selected from pyridinyl, pyrrolidinyl, wherein: the aryl of the arylsulfonyl substituent on the aryl of R₃ is selected from phenyl, indolyl, thiophenyl, furanyl; wherein: the aryl or heteroaryl groups of R₄ are optionally substituted with one or more substituents selected from the following: mono- or di-(C₁₋₆ alkyl)amino, mono- or di (C₁₋₆)alkylamino-C₂₋₆ alkyl, amino-C₁₋₆ alkyl, heterocycloalkyl, amino, C₁₋₂₀ alkoxy, chloro, fluoro, bromo, C₁₋₁₀ alkyl, aryl or heteroaryl; wherein the heterocycloalkyl group attached to the aryl of R₄ is selected from the following: morpholinyl, pyrrolidinyl, piperazinyl, piperidinyl; wherein the heteroaryl group attached to the aryl of R₄ is selected from pyridyl, pyrrolyl, furanyl; R₅, R₆ = independently H, C₁₋₆ alkyl; R₇, R₈ = independently H, C₁₋₆ alkyl; R₉ = di-(C₁₋₅ alkyl)amino-C₂₋₄ alkyl; mono(C₁₋₅ alkyl)amino-C₂₋₄ alkyl; amino-C₂₋₄ alkyl, heterocycloalkyl methyl in which heterocycloalkyl is selected from the group consisting of thiophenyl, furanyl, pyrrolyl, pyridinyl; and R₁₀ = H, C₁₋₆ alkyl.

It is to be understood that the pharmaceutical composition may comprise a compound wherein the compound is selected from:
(S)-1-(3-Trifluoromethyl-benzenesulfonyl)-piperidine-2-carboxylic acid [(S)-1-(4-dimethylamino-phenylcarbamoyl)-2-(4-methoxy-phenyl)-ethyl]-amide;
(S)-1-(3-Trifluoromethyl-benzenesulfonyl)-piperidine-2-carboxylic acid [(S)-2-(4-acetylphenyl)-1-(4-ethoxy-phenylcarbamoyl)-ethyl]-amide;
(S)-1-(3-Trifluoromethyl-benzenesulfonyl)-piperidine-2-carboxylic acid [(S)-1-(4-morpholin-4-yl-phenylcarbamoyl)-2-(4-trifluoromethyl-phenyl)-ethyl]-amide;
(S)-1-(3-Trifluoromethyl-benzenesulfonyl)-piperidine-2-carboxylic acid [(S)-2-(4-methoxyphenyl)-1-(tetrahydro-pyran-4-ylcarbamoyl)-ethyl)-amide;
(S)-1-(3-Trifluoromethyl-benzenesulfonyl)-piperidine-2-carboxylic acid [(S)-2-(2-methyl-4-methoxy-phenyl)-1-(tetrahydro-pyran-4-ylcarbamoyl)-ethyl]-amide;
(S)-1-(3-Trifluoromethyl-benzenesulfonyl)-piperidine-2-carboxylic acid [(S)-2-(3,4-dimethoxy-phenyl)-1-(tetrahydro-pyran-4-yl-carbamoyl)-ethyl]-amide;
(S)-1-(3-Trifluoromethyl-benzenesulfonyl)-piperidine-2-carboxylic acid [(S)-2-(4-methoxyphenyl)-1-(4-morpholin-4-yl-phenylcarbamoyl)-ethyl]-amide;
(S)-1-(3-Methoxy-benzenesulfonyl)-piperidine-2-carboxylic acid ((S)1-carbamoyl-2-phenylethyl)-amide;
(S)-1-(3-Hydroxy-benzenesulfonyl)-piperidine-2-carboxylic acid ((S)-1-carbamoyl-2-phenylethyl)-amide;
(S)-1-(3 -Propoxy-benzenesulfonyl)-piperidine-2-carboxylic acid ((S)-1-carbamoyl-2-phenylethyl)-amide;
(S)-1-(3-Trifluoromethyl-benzenesulfonyl)-piperidine-2-carboxylic acid [(S)-2-[4-(1-hydroxyethyl)-phenyl]-1-(4-morpholin-4-yl-phenylcarbamoyl)-ethyl]-amide;
(S)-1-(3-Trifluoromethyl-benzenesulfonyl)-piperidine-2-carboxylic acid {(S)-1-(4-ethoxy-phenylcarbamoyl)-2-[4-(1-hydroxy-ethyl)-phenyl]-ethyl} -amide;
(S)-1-(3-Methoxy-benzenesulfonyl)-piperidine-2-carboxylic acid [(S)-2-(indol-3-yl)-1-(naphthalen-2-ylcarbamoyl)-ethyl]-amide;
(S)-1-(3-Trifluoromethyl-benzenesulfonyl)-piperidine-2-carboxylic acid {(S)-1-(4-dimethylamino-phenylcarbamoyl)-2-[4-(1-hydroxy-ethyl)-phenyl]-ethyl}-amide;
(S)-1-(3,5-Dichloro-benzenesulfonyl)-piperidine-2-carboxylic acid [(S)-2-(1H-indol-3-yl)-1-methylcarbamoyl-ethyl]-amide;
(S)-1-(3-Trifluoromethyl-benzenesulfonyl)-piperidine-2-carboxylic acid [(S)-2-(4-acetyl-phenyl)-1-(4-morpholin-4-yl-phenylcarbamoyl)-ethyl]-amide;
(S)-1-(3-Fluoro-benzenesulfonyl)-piperidine-2-carboxylic acid [(S)-2-(4-cyclopentyloxy-phenyl)-1-methyl-1-(tetrahydro-pyran-4-ylcarbamoyl)-ethyl]-amide;
(S)-1-(3-Fluoro-benzenesulfonyl)-piperidine-2-carboxylic acid [(S)-2-(4-isopropoxy-phenyl)-1-(2,2,6,6-tetramethyl-piperidin-4-ylcarbamoyl)-ethyl]-amide;
(S)-1-(3-Fluoro-benzenesulfonyl)-(2S)-piperidine-2-carboxylic acid [(S)-2-[4-(3-fluoro-propoxy)-phenyl]-1-(2,2,6,6-tetramethyl-piperidin-4-ylcarbamoyl)-ethyl]-amide;
(S)-1-(3-Fluoro-benzenesulfonyl)-piperidine-2-carboxylic acid [(S)-2-(4-(3-fluoro-propoxy)-phenyl]-1-((S)-2-methoxy-1-methyl-ethylcarbamoyl)-ethyl]-amide;
(S)-1-(3-Trifluoromethyl-benzenesulfonyl)-piperidine-2-carboxylic acid [(S)-2-(4-isopropoxyphenyl)-1-methylcarbamoyl-ethyl]-amide;
(S)-1-(3-Trifluoromethyl-benzenesulfonyl)-piperidine-2-carboxylic acid [(S)-2-(4-isopropoxyphenyl)-1-(tetrahydro-pyran-4-ylcarbamoyl)-ethyl]-amide;
(S)-1-(3-Trifluoromethyl-benzenesulfonyl)-piperidine-2-carboxylic acid [(S)-2-phenyl)-1-(benzimidazol-2-yl)-ethyl]-amide;
(S)-1-(3-Trifluoromethyl-benzenesulfonyl)-piperidine-2-carboxylic acid [(S)-2-(4-(benzyloxy)phenyl)-1-(benzimidazol-2-yl)-ethyl]-amide;
(S)-1-(3-Fluoro-benzenesulfonyl)-piperidine-2-carboxylic acid [(S)-2-4-methoxy-phenyl)-1-(tetrahydropyran-4-yl-carbamoyl)-ethyl]-amide;
(S)-1-Benzenesulfonyl-piperidine-2-carboxylic acid ((S)-1-amido-2-{2-methylphenyl)-ethyl)-amide;
(S)-1-Benzenesulfonyl-piperidine-2-carboxylic acid ((S)-1-cyclohexylamido-2-(4-methylphenyl)-ethyl)-amide;
(S)-1-Benzenesulfonyl-piperidine-2-carboxylic acid [(S)-2-(4-allyloxy-phenyl)-1-cyclohexylamido-ethyl]-amide;
(S)-1-(3-Trifluoromethyl-benzenesulfonyl)-piperidine-2-carboxylic acid [(S)-2-(4-allyloxy-phenyl)-1-cyclohexylcarbamoyl-ethyl]-amide;
(S)-1-(3-Trifluoromethyl-benzenesulfonyl)-piperidine-2-carboxylic acid ((S)-1-cyclohexylamido-2-(4-methylphenyl)-ethyl)-amide;
(S)-1-((E)-2-Phenyl-ethenesulfonyl)-piperidine-2-carboxylic acid [(S)-1-amido 2-(1H-indol-3-yl)-ethyl]-amide;
(S)-1-(4,5-Dibromo-thiophene-2-sulfonyl)-piperidine-2-carboxylic acid [(S)-1-amido-2-(1H-indol-3-yl)-ethyl]-amide;
(S)-1-Benzenesulfonyl-piperidine-2-carboxylic acid [(S)-1-tert-butylamido-2-(1H-indol-3-yl)-ethyl]-amide;
(S)-1-Benzenesulfonyl-piperidine-2-carboxylic acid [(S)-1-(3-hydroxy-2,2-dimethyl-propylamido)-2-(4-isopropyl-phenyl)-ethyl]-amide;
(S)-1-Benzenesulfonyl-piperidine-2-carboxylic acid methyl-((S)-1-methylamido-2-(phenyl)-ethyl)-amide;
(S)-1-Benzenesulfonyl-piperidine-2-carboxylic acid [(S)-1-(3,3-dimethyl-butylamido)-2-(4-methoxyphenyl)-ethyl]-amide;
(S)-1-Benzenesulfonyl-piperidine-2-carboxylic acid [(S)-1-(2,2-dimethyl-propylamido)-2-(phenyl)-ethyl]-amide;
(4-{(S)-2-[((S)-1-Benzenesulfonyl-piperidine-2-carbonyl)-amino]-2-amido-ethyl}-benzyl)-phosphonic acid diethyl ester;
(S)-1-Benzenesulfonyl-piperidine-2-carboxylic acid [(S)-2-(4-(acetylamino)-phenyl)-1-amido-ethyl]-amide;
(S)-1-Benzenesulfonyl-piperidine-2-carboxylic acid [(S)-1-amido-2-(4-(hydroxymethyl)-phenyl)-ethyl]-amide;
(S)-1-(Naphthalene-2-sulfonyl)-piperidine-2-carboxylic acid [(S)-1-amido-2-(1H-indol-3-yl)-ethyl]-amide;
(S)-1-(3-Trifluoromethyl-benzenesulfonyl)-piperidine-2-carboxylic acid [(S)-1-(cyclohexylmethyl-amido)-2-(4-methylphenyl)ethyl]-amide;
(S)-1-(3-Trifluoromethyl-benzenesulfonyl)-piperidine-2-carboxylic acid [(S)-1-(4-amino-butylamido)-2-(4-methylphenyl)-ethyl]-amide;
(S)-1-Benzenesulfonyl-piperidine-2-carboxylic acid [(S)-1-amido-2-(1H-indol-3-yl)-ethyl]-amide;
(S)-1-Benzenesulfonyl-piperidine-2-carboxylic acid ((S)-1-amido-2-(pyridin-4-yl)-ethyl)-amide;
(S)-1-Benzenesulfonyl-piperidine-2-carboxylic acid ((S)-1-amido-2cyclohexyl-ethyl)-amide;
(S)-1-Benzenesulfonyl-piperidine-2-carboxylic acid {(8)-2-phenyl-1-[(thiophen-2-yimethyl)-amido]-ethyl}-amide;
(S)-1-(3-Fluoro-benzenesulfonyl)-piperidine-2-carboxylic acid [(S)-1-(4-dimethylamino-butylcarbamoyl)-2-(4-fluoro-phenyl)-ethyl]-amide;
(S)-1-(3-Fluoro-benzenesulfonyl)-piperidine-2-carboxylic acid [(S)-2-[4-(3-methoxy-pmpoxy)phenyl]-1-(tetrahydro-pyran-4-ylcarbamoyl)-ethyl]-amide;
(S)-1-(3-Trifluoromethyl-benzenesulfonyl)-piperidine-2-carboxylic acid {(S)-1-cyclohexylcarbamoyl-2-[4-(2-dimethylamino-ethoxy)-phenyl]-ethyl}-amide;
(S)-1-(3-Trifluoromethyl-benzenesulfonyl)-piperidine-2-carboxylic acid [(S)-2-(4-methoxyphenyl)-1-(2-piperazin-1-yl-ethylcarbamoyl)-ethyl]-amide;
(S)-1-(3-Trifluoromethyl-benzenesulfonyl)-piperidine-2-carboxylic acid [(S)-1-(2-hydroxy-1,1-dimethyl-ethylcarbamoyl)-2-(4-methoxy-phenyl)-ethyl]-amide;
(S)-1-Benzenesulfonyl-piperidine-2-carboxylic acid ((S)-1-carbamoyl-2-phenyl-ethiyl)-amide;
(S)-1-(3-Fluoro-benzenesulfonyl)-piperidine-2-carboxylic acid {(S)-2-[4-(3-methoxy-propoxy)-phenyl]-1-methylcarbamoyl-ethyl}-amide;
(S)-1-(3-Fluoro-benzenesulfonyl)-piperidine-2-carboxylic acid {(S)-1-((S)-2-methoxy-1-methyl-ethylcarbamoyl)-2-[4-(3-methoxy-propoxy)-phenyl]-ethyl}-amide; and
(S)-1-(3-Hydroxy-benzenesulfonyl)-piperidine-2-carboxylic acid [(S)-2-(1H-indol-3-yl)-1-(naphthalen-2-ylcarbamoyl)-ethyl]-amide.

The present disclosure also includes as an additional aspect a method of treating a disorder involving neurological damage, comprising: administering to a subject in need thereof, an effective amount of a compound, including enantiomers, stereoisomers, rotamers and tautomers of said compound and pharmaceutically acceptable salts, solvates or derivatives thereof, with said compound having the general structure shown in formula I, wherein:
- R is: fluoroalkyl of the formula CₙHₓF_{y} wherein n is an integer from 1 to 6, x is an integer from 0 to 12, y is an integer from 1 to 13 and the sum of x and y is 2n+1, aryl, aryl C₁₋₆ alkyl, aryl C₂₋₄ alkenyl, wherein aryl is selected from phenyl, biphenyl or naphthyl, heteroaryl or heteroaryl C₁₋₄ alkyl, wherein heteroaryl is selected from pyridyl, thiophenyl or benzothiophenyl, and heterocyclyl selected from piperidinyl or piperazinyl,
- R₁ is: hydrogen or C₁₋₆ alkyl;
- R₂ is: CONER₄, benzimidazol-2-yl, CR₅R₆OH, or CR₇R₈NHR₉;
- R₃ is: aryl and aryl C₁₋₄ alkyl or aryl C₂₋₄ alkenyl, wherein aryl is selected from phenyl, biphenyl, naphthyl or bisarylmethyl, heteroaryl or heteroaryl C₁₋₄ alkyl, wherein heteroaryl is selected from indolyl or thiophenyl, benzothiophenyl, furanyl, benzofuranyl, 1,3-benzodioxolyl, 1, 4-benzodioxanyl or C₅₋₇ cycloalkyl C₁ alkyl;
- X is: NR₁₀ or CH₂; wherein R₄ is selected from the group consisting of hydrogen, C₁₋₁₀ alkyl, C₂₋₁₀ alkenyl, C₃₋₈ cycloalkyl, C₈₋₁₂ bicycloalkyl, C₁₀₋₁₄ tricycloalkyl , C₃₋₈ cycloalkyl C₁₋₆ alkyl, C₈₋₁₂ bicycloalkylC₁₋₆ alkyl, C₈₋₁₂ bicycloalkylamino- C₁₋₆ alkyl, C₈₋₁₂ tricycloalkylamino-C₁₋₆ alkyl, C₃₋₈ cycloalkenyl, C₃₋₈ cycloalkenyl C₁₋₆ alkyl, fluoroalkyl of the formula CₙHₓF_{y} wherein n is an integer from 1 to 20, x is an integer from 0 to 40, y is an integer from 1 to 41 and the sum of x and y is 2n+1, amino C₂₋₂₀ alkyl, mono or dialkylamino C₂₋₂₀ alkyl, mono-, di, or trihydroxy C₂₋₂₀ alkyl, C₁₋₆ alkoxy- C₂₋₂₀ alkyl, C₁₋₂₀ thioalkyl, C₁₋₆ alkyl-thio-C₂₋₂₀ alkyl, hydroxy-C₂₋₆ -alkoxy-C₂₋₆ alkyl, C₁₋₆ alkyl-bis-(hydroxy-C₁₋₆ alkyl), aryl, aryl C₁₋₁₀ alkyl, aryl C₃₋₄ alkenyl, wherein aryl is selected from phenyl, biphenyl, indanyl or naphthyl, heteroaryl or heteroaryl C₁₋₁₀ alkyl, wherein heteroaryl is selected from pyridyl, thiophenyl, furanyl, benzofuranyl, benzothiophenyl, pyrrolidinyl, indolyl, heterocycloalkyl or heterocycloalkyl C₁₋₁₀ alkyl wherein heterocycloalkyl is selected from tetrahydrofuranyl, tetrahydropyranyl, morpholinyl, piperidinyl, pyrrolidinyl, tetrahydrothiophenyl, piperazinyl, dioxolanyl, sulfolanyl or 1,1-dioxotetrahydrothiopyranyl; heterobicyclyl of the formula: 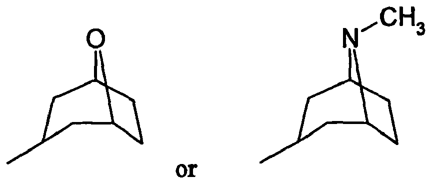 wherein C₅₋₈ cycloalkyl, C₈₋₁₂ bicycloalkyl, or heterocyclyl is optionally substituted with one or more substituents selected from the group consisting of C₁₋₁₀ alkyl, C₁₋₁₀ alkoxy, hydroxy, hydroxy C₁₋₁₀ alkoxy, hydroxy C₁₋₁₀ alkyl, C₁₋₁₀ acyloxy, amino, C₁₋₁₀ alkylcarbonylamino, hydroxy C₁₋₂₀ alkylcarbonylamino, amino C₂₋₁₀ alkoxy, mono-C₁₋₆ -alkylamino-C₂₋₁₀-alkoxy, di-C₁₋₆-alkylamino-C₂₋₁₀-allcoxy, mono-(C₁₋₁₀ alkyl)amino, di-(C₁₋₁₀ alkyl)amino, amino C₁₋₁₀ alkylcarbonylamino, C₁₋₁₀ alkylaminocarbonyl, hydroxy C₁₋₁₀ alkylaminocarbonyl, amino C₂₋₁₀ alkylaminocarbonyl, mono or dialkylamino C₁₋₁₀ alkylaminocarbonyl, oxo, optionally substituted aryl or optionally substituted heteroaryl, wherein aryl is selected from phenyl, naphthyl or biphenyl and heteroaryl is selected from pyridyl or thiophenyl.
- wherein:: the aryl or heteroaryl groups of R are optionally substituted with one or more substituents selected from the group consisting of fluorine, chlorine, bromine, C₁₋₂₀ alkyl, fluoroalkyl or fluoroalkoxy of the formula CₙHₓF_{y} or OCₙHₓF_{y} wherein n is an integer from 1 to 4, x is an integer from 0 to 8, y is an integer from 1 to 9 and the sum of x and y is 2n+1, C₁₋₂₀ alkoxy, C₁₋₂₀ alkenyloxy, C₁₋₁₀-alkoxy-C₁₋₁₀-alkoxy, hydroxy, hydroxy C₁₋₂₀ alkoxy, hydroxy C₁₋₂₀ alkyl, C₁₋₂₀ acyloxy, hydroxy C₁₋₂₀ acyl, nitro, amino, aminosulfonyl, C₁₋₂₀ alkylcarbonylamino, hydroxy C₁₋₂₀ alkylcarbonylamino, amino C₂₋₂₀ alkoxy, amino C₁₋₂₀ alkylcarbonylamino, C₁₋₂₀ alkylaminocarbonyl, hydroxy, ciao alkylaminocarbonyl, amino C₁₋₂₀ all,ylaminocarbonyl, mono or dialkylamino C₁₋₂₀ alkylaminocarbonyl, optionally substituted aryl or heteroarylsulfonyl, aminosulfonyl, mono- or di(C₁₋₆)alkylaminosulfonyl, optionally substituted phenyl or thiophenyl, optionally substituted phenyl or thiophenyl C₁₋₁₀ alkyloxy, and cyano; wherein the phenyl or thiophenyl substituent on the aryl or heteroaryl group of R is optionally substituted with one or more substituents selected from the group consisting of fluorine, chlorine, bromine, C₁₋₅alkyl, fluoroalkyl or fluoroalkoxy of the formula CₙHₓF_{y} or OCₙHₓF_{y} wherein n is an integer from 1 to 4, x is an integer from 0 to 8, y is an integer from 1 to 9 and the sum of x and y is 2n+1, C₁₋₁₀alkoxy, C₁₋₁₀ alkenyloxy, C₁₋₅ alkoxy-C₁₋₅ alkoxy, hydroxy, hydroxy C₁₋₁₀alkoxy, hydroxy C₁₋₁₀alkyl, hydroxy C₁₋₂₀-alkyloxo, nitro, amino, aminosulfonyl, C₁₋₁₀alkylcarbonylamino, hydroxy C₁₋₁₀ alkylcarbonylamino, amino C₂₋₁₀alkoxy, amino C₁₋₁₀alkylcarbonylamino, C₁₋₁₀ alkylaminocarbonyl, hydroxy C₁₋₁₀alkylaminocarbonyl, amino C₁₋₁₀ alkylaminocarbonyl, mono or dialkylamino C₁₋₁₀alkylaminocarbonyl, optionally substituted aryl C₁₋₁₀alkyloxy, and cyano;
- wherein:: the aryl or heteroaryl of R₃ is optionally substituted with one or more substituents selected from the following: C₃₋₈cycloalkyloxy, C₁₋₁₀alkoxy, C₁₋₁₀ alkyl, C₂₋₁₀alkenyl, C₃₋₁₀ alkenyloxy mono- (C₁₋₆-alkyl)amino-C₂₋₆alkyloxy, di-(C₁₋₆alkyl)amino-C₂₋₆-alkyloxy, amino-C₂₋₆alkyloxy, heterocyclyl-C₂₋₆alkyloxy, aryl-C₂₋₆-alkyloxy, heteroaryl-C₂₋₆-alkyloxy, fluoroalkyl or fluoroalkoxy of the formula CₙHₓF_{y} or OCₙHₓF_{y} wherein n is an integer from 1 to 4, x is an integer from 0 to 8, y is an integer from 1 to 9 and the sum of x and y is 2n+1, C₁₋₆-alkoxy-C₂₋₆-alkoxy, aryl-C₁₋₆-alkyloxy, hydroxy, chloro, fluoro, bromo, hydroxy-C₁₋₆-alkoxy, hydroxy-C₁₋₁₀ alkyl, C₁₋₆alkyloxo, cyano, amino, 4-aryloxo, di-C₁₋₅alkylphosphityl-C₁₋₅ alkyl, C₁₋₆alkyloxoamino, C₁₋₆alkyloxo(C₁₋₆ alkyl)amino, C₁₋₁₀alkylsulfonyl, arylsulfonyl, carboxy, C₁₋₂₀carboalkoxy, dialkylamino-C₁₋₁₀ alkyl, monoalkylamino-C₁₋₁₀ alkyl, amino-C₁₋₁₀ alkyl;
- wherein:: the heterocycloalkyl substituent group attached to the alkyloxy group of R₃ is selected from the following: pyrrolidinyl, morpholinyl, piperidinyl, and piperazinyl;
- wherein:: the heteroaryl substituent group attached to the alkyloxy group of R₃ is selected from pyridinyl, pyrrolidinyl, wherein: the aryl of the arylsulfonyl substituent on the aryl of R₃ is selected from phenyl, indolyl, thiophenyl, furanyl; wherein: the aryl or heteroaryl groups of R₄ are optionally substituted with one or more substituents selected from the following: mono- or di-(C₁₋₆ alkyl)amino, mono- or di (C₁₋₆)alkylamino-C₂₋₆ alkyl, amino-C₁₋₆ alkyl, heterocycloalkyl, amino, C₁₋₂₀ alkoxy, chloro, fluoro, bromo, C₁₋₁₀ alkyl, aryl or heteroaryl; wherein the heterocycloalkyl group attached to the aryl of R₄ is selected from the following: morpholinyl, pyrrolidinyl, piperazinyl, piperidinyl; wherein the heteroaryl group attached to the aryl of R₄ is selected from pyridyl, pyrrolyl, furanyl; R₅, R₆ = independently H, C₁₋₆ alkyl; R₇, R₈ = independently H, C₁₋₆ alkyl; R₉ = di-(C₁₋₅ alkyl)amino-C₂₋₄ alkyl; mono(C₁₋₅ alkyl)amino-C₂₋₄ alkyl; amino-C₂₋₄ alkyl, heterocycloalkyl methyl in which heterocycloalkyl is selected from the group consisting of thiophenyl, furanyl, pyrrolyl, pyridinyl; and R₁₀ = H, C₁₋₆ alkyl.

And preferred methods of treating a disorder involving neurological damage include the method wherein the disorder is Parkinson's disease, the method wherein the disorder is multiple sclerosis, the method wherein the disorder is Alzheimer's disease, the method wherein the disorder is stroke, and the method wherein the disorder is spinal cord injury.

Furthermore, a more preferred method for treating a neurological disorder, as described hereinabove includes the method wherein:
- R is: aryl, aryl C₁₋₆ alkyl, aryl C₂₋₄ alkenyl, wherein aryl is selected from phenyl, biphenyl or naphthyl, heteroaryl or heteroaryl C₁₋₄ alkyl, wherein heteroaryl is selected from pyridyl, thiophenyl or benzothiophenyl, and heterocyclyl selected from piperidinyl or piperazinyl,
- R₁ is: hydrogen or methyl
- R₂ is: CONHR₄, benzimidazol-2-yl, CR₅R₆OH, or CR₇R₈NHR₉;
- R₃ is: phenyl and aryl C₁₋₄ alkyl or aryl C₂₋₄ alkenyl, wherein aryl is selected from phenyl, biphenyl, naphthyl or bisarylmethyl, heteroaryl or heteroaryl C₁₋₄ alkyl, wherein heteroaryl is selected from indolyl or thiophenyl.
- X is: NR₁₀ or CH₂; wherein R₄ is selected from the group consisting of hydrogen, C₁₋₁₀ alkyl, C₂₋₁₀ alkenyl, C₃₋₈ cycloalkyl, C₈₋₁₂ bicycloalkyl, C₁₀₋₁₄ tricycloalkyl , C₃₋₈ cycloalkyl-C₁₋₆ alkyl, C₈₋₁₂ bicycloalkylC₁₋₆ alkyl, C₈₋₁₂ bicycloalkylamino-C₁₋₆ alkyl, C₈₋₁₂ tricycloalkylamino-C₁₋₆ alkyl, C₃₋₈ cycloalkenyl, C₃₋₈ cycloalkenyl C₁₋₆ alkyl, fluoroalkyl of the formula CₙHₓF_{y} wherein n is an integer from 1 to 10, x is an integer from 0 to 20, y is an integer from 1 to 21 and the sum ofx and y is 2n+1, amino C₂₋₁₀ alkyl, mono or dialkylamino C₂₋₁₀ alkyl, mono-, di, or trihydroxy C₂₋₁₀ alkyl, C₁₋₆-alkoxy-C₂₋₁₀ alkyl, C₁₋₁₀ thioalkyl, C₁₋₆alkyl-thio-C₂₋₁₀alkyl, hydroxy-C₂-₆-alkoxy-C₂₋₆alkyl, C₁₋₆alkyl-bis(hydroxy-C₁₋₆alkyl), aryl, aryl C₁₋₆ alkyl, aryl C₃₋₄ alkenyl, wherein aryl is selected from phenyl, biphenyl, indanyl or naphthyl, heteroaryl or heteroaryl C₁₋₁₀ alkyl, wherein heteroaryl is selected from pyridyl, thiophenyl, furanyl, benzofuranyl, benzothiophenyl, pyrrolidinyl, indolyl, heterocycloalkyl or heterocycloalkyl C₁₋₁₀ alkyl wherein heterocycloalkyl is selected from tetrahydrofuranyl, tetrahydropyranyl, morpholinyl, piperidinyl, pyrrolidinyl, piperazinyl, dioxolanyl, sulfolanyl or 1,1-dioxotetrahydrothiopyranyl; wherein C₅₋₈ cycloalkyl, C₈₋₁₂ bicycloalkyl, or heterocycloalkyl is optionally substituted with one or more substituents selected from the group consisting of C₁₋₆ alkyl, C₁₋₆ alkoxy, hydroxy, hydroxy C₁₋₆ alkoxy, hydroxy C₁₋₆ alkyl, amino, C₁₋₆ alkylcarbonylamino, hydroxy C₁₋₆ alkylcarbonylamino, amino C₂₋₆ alkoxy, mono-C₁₋₆-alkylamino-C₂₋₆-alkoxy, di-C ₁₋₆-alkylamino-C₂₋₆-alkoxy, mono-(C₁₋₆ alkyl)amino, di-(C₁₋₆ alkyl)amino, amino C₁₋₆ alkylcarbonylamino, C₁₋₆ alkylaminocarbonyl, hydroxy C₁₋₆ alkylaminocarbonyl, amino C₂₋₆ alkylaminocarbonyl, mono or dialkylamino C₁₋₆ alkylaminocarbonyl, oxo, optionally substituted aryl or optionally substituted heteroaryl, wherein aryl is selected from phenyl, naphthyl or biphenyl and heteroaryl is selected from pyridyl or thiophenyl;
wherein:
the aryl or heteroaryl groups of R are optionally substituted with one or more substituents selected from the group consisting of fluorine, chlorine, bromine, C₁₋₁₀ alkyl, fluoroalkyl or fluoroalkoxy of the formula CₙHₓF_{y} or OCₙHₓF_{y} wherein n is an integer from 1 to 4, x is an integer from 0 to 8, y is an integer from 1 to 9 and the sum of x and y is 2n+1, C₁₋₁₀ alkoxy, C₁₋₁₀ alkenyloxy, C₁₋₁₀ -alkoxy- C₁₋₁₀ -alkoxy, hydroxy, hydroxy C₁₋₁₀ alkoxy, hydroxy C₁₋₁₀ alkyl, hydroxy C₁₋₁₀ acyl, nitro, amino, aminosulfonyl, C₁₋₁₀ alkylcarbonylamino, hydroxy C₁₋₁₀ alkylcarbonylamino, amino C₂₋₁₀ alkoxy, amino C₁₋₁₀ alkylcarbonylamino, C₁₋₁₀ alkylaminocarbonyl, hydroxy C₁₋₁₀ alkylaminocarbonyl, amino C₁₋₁₀ alkylaminocarbonyl, mono or dialkylamino C₁₋₁₀ alkylaminocarbonyl, optionally substituted aryl or heteroarylsulfonyl, aminosulfonyl, mono- or di(C₁₋₆)alkylaminosulfonyl, optionally substituted phenyl or thiophenyl, optionally substituted phenyl or thiophenyl-C₁₋₁₀ alkyloxy, and cyano;
wherein the phenyl or thiophenyl substituent on the aryl or heteroaryl group of R is optionally substituted with one or more substituents selected from the group-consisting of fluorine, chlorine, bromine, C₁₋₅ alkyl, CF₃, OCF₃, C₁₋₆ alkoxy, C₁₋₆ alkenyloxy, C₁₋₅ alkoxy-C₁₋₅ alkoxy, hydroxy, hydroxy C₁₋₁₀ alkoxy, hydroxy C₁₋₁₀ alkyl, nitro, amino, aminosulfonyl, C₁₋₁₀ alkylcarbonylamino, hydroxy C₁₋₆ alkylcarbonylamino, amino C₂₋₆ alkoxy, amino C₁₋₆ alkylcarbonylamino, C₁₋₆ alkylaminocarbonyl, hydroxy C₁₋₆ alkylaminocarbonyl, amino C₁₋₆ alkylaminocarbonyl, mono or dialkylamino C₁₋₆ alkylaminocarbonyl, optionally substituted aryl C₁₋₆ alkyloxy, and cyano;
wherein:
the aryl or heteroaryl of R₃ is optionally substituted with one or more substituents selected from the following: C₃₋₆ cycloalkyloxy, C₁₋₆ alkoxy, C₁₋₆ alkyl, C₂₋₆ alkenyl, C₃₋₆ alkenyloxy,
mono- (C₁₋₆ alkyl)amino-C₂₋₆ alkyloxy, di-(C₁₋₆ alkyl)amino-C₂₋₆ alkyloxy, amino-C₂₋₆ alkyloxy, heterocyclyl- C₂₋₆ alkyloxy, aryl- C₂₋₆ alkyloxy, heteroaryl- C₂₋₆ alkyloxy, fluoroalkyl or fluoroalkoxy of the formula CₙHₓF_{y} or OCₙHₓF_{y} wherein n is an integer from 1 to 4, x is an integer from 0 to 8, y is an integer from 1 to 9 and the sum of x and y is 2n+1, C₁₋₆ alkoxy - C₂₋₆ alkyloxy, aryl- C₁₋₆ alkoxy, hydroxy, chloro, fluoro, bromo, hydroxy- C₁₋₆ alkoxy, hydroxy-C₁₋₁₀ alkyl, C₁₋₆ alkyloxo, cyano, amino, 4-aryloxo, C₁₋₆ alkyloxoamino, C₁₋₆ alkyloxo(C₁₋₆ alkyl)amino, carboxy, dialkylamino- C₁₋₆ alkyl, monoalkylamino- C₁₋₆ alkyl, amino- C₁₋₆ alkyl;
wherein: the heterocyclyl substituent group attached to the alkyloxy group of R₃ is selected from the following: pyrrolidinyl, morpholinyl, piperidinyl, and piperazinyl;
wherein: the heteroaryl substituent group attached to the alkyloxy group of R₃ is selected from pyridinyl, pyrrolidinyl;
wherein:
the aryl or heteroaryl groups of R₄ are optionally substituted with one or more substituents selected from the following: mono- or di-(C₁₋₆ alkyl)amino,
mono- or di (C₁₋₆)alkylamino-C₂₋₆-alkyl, amino- C₁₋₆ alkyl,
heterocyclyl, amino, C₁₋₁₀ alkoxy, chloro, fluoro, bromo, C₁₋₁₀ alkyl, aryl or heteroaryl;
wherein the heterocyclyl group attached to the aryl of R₄ is selected from the following: morpholinyl, pyrrolidinyl, piperazinyl, piperidinyl;
wherein the heteroaryl group attached to the aryl of R₄ is selected from, pyridyl, pyrrolyl, furanyl;
R₅, R₆ = H;
R₇, R₈ = H;
R₉ = di-(C₁₋₅ alkyl)amino-C₂₋₄ alkyl; mono(C₁₋₅ alkyl)amino-C₂₋₄ alkyl; amino-C₂₋₄ alkyl, heterocycloalkyl methyl in which heterocycloalkyl is selected from group consisting of thiophenyl, furanyl, pyrrolyl, pyridinyl; and
R₁₀ = H, CH₃.

And still more preferred methods of treating a disorder involving neurological damage include the method wherein the disorder is Parkinson's disease, the method wherein the disorder is multiple sclerosis, the method wherein the disorder is Alzheimer's disease, the method wherein the disorder is stroke, and the method wherein the disorder is spinal cord injury.

The synthesis of the compounds of the present invention will be understood by a person having skill in the art of chemical synthesis, by reference to the following synthetic schemes.

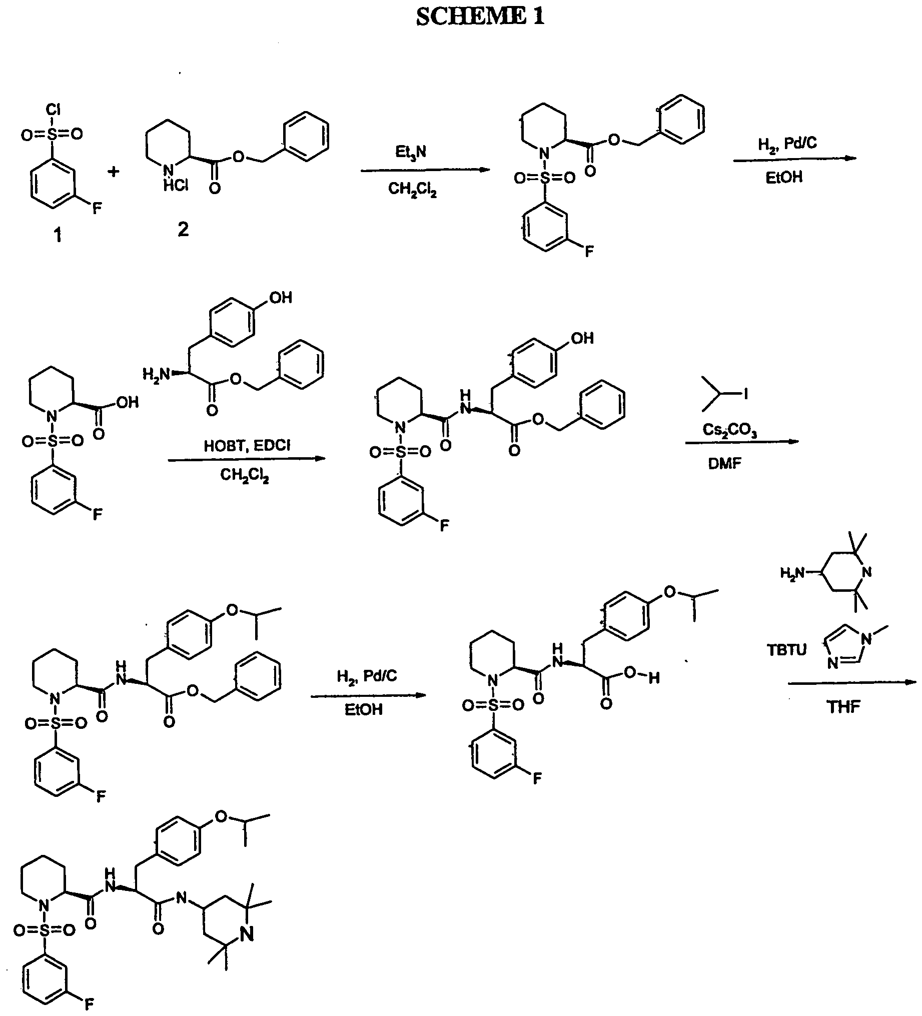

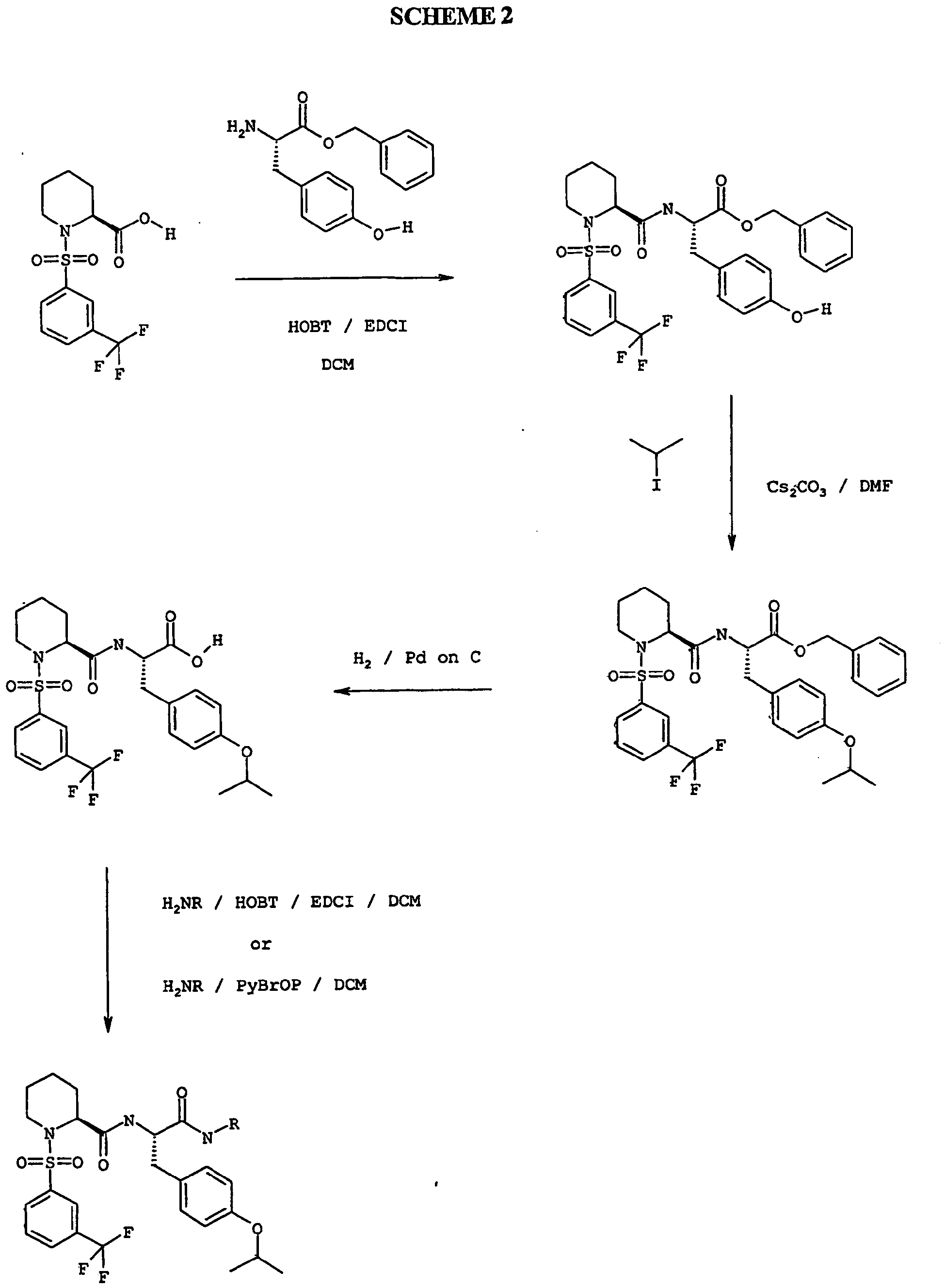

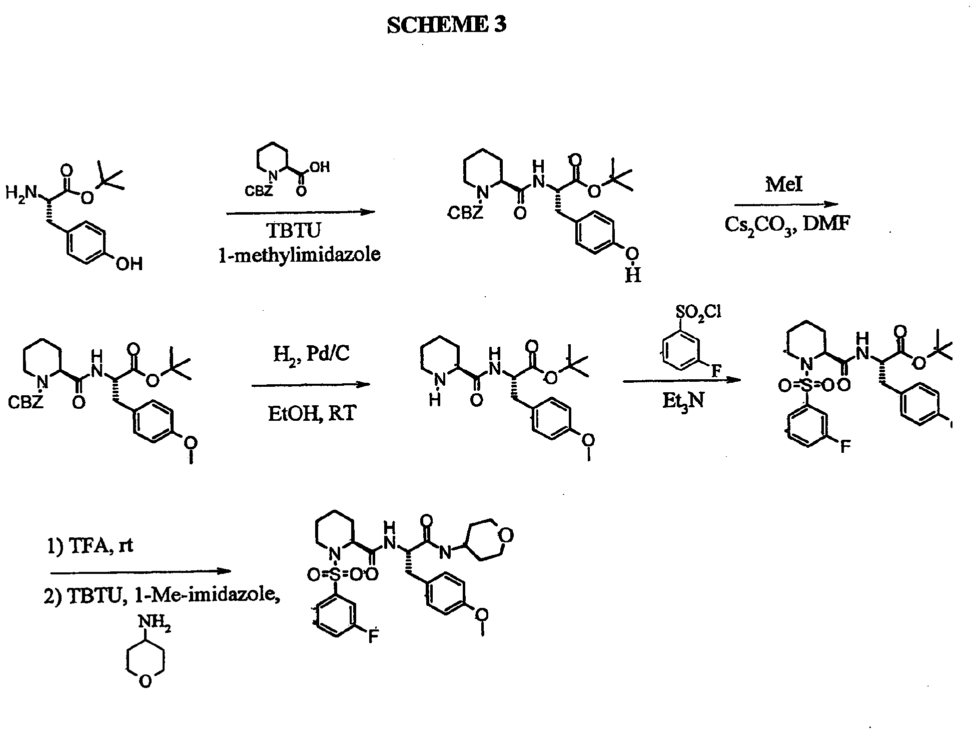

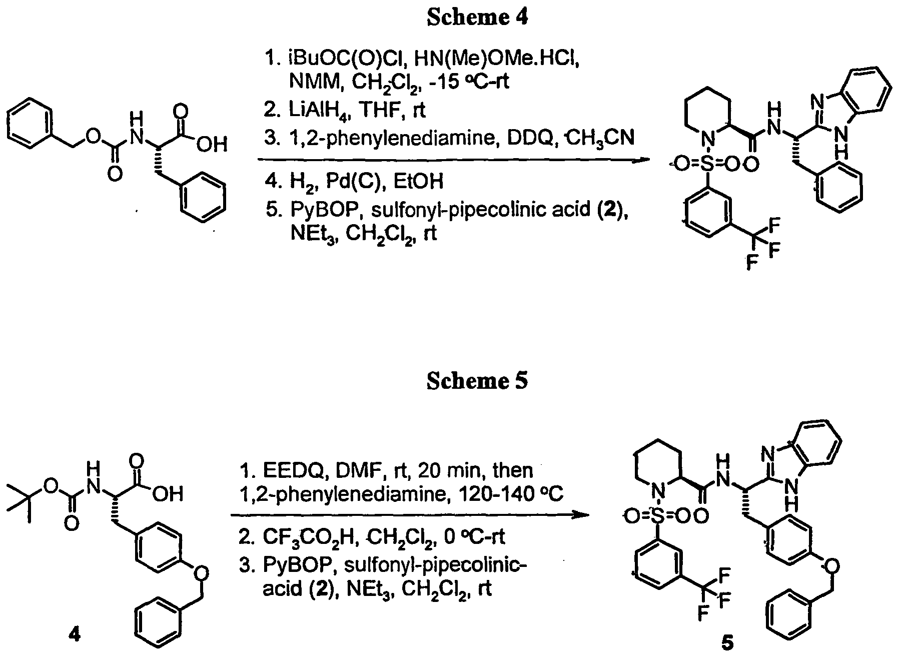

For examples 28 through 38, the following conditions were used for characterizing the reaction products. High Pressure Liquid Chromatography/ Mass Spectrometry (LC-MS) conditions for determination of retention times (RT) were as follows: 3 micron Luna^{®} C18 (2) HPLC column (30mm x 4.6mm) eluting with (i) mixture of 0.05% trifluoroacetic acid in acetonitrile and 0.05% trifluoroacetic acid in water (1:19, v/v) for 2 minutes, (ii) mixture of 0.05% trifluoroacetic acid in acetonitrile and 0.05% trifluoroacetic acid in water (1:19 to 19:1, v/v) gradient elution over 10 minutes, (iii) mixture of 0.05% trifluoroacetic acid in acetonitrile and 0.05% trifluoroacetic acid in water (19:1, v/v) for 2 minutes, (iv) mixture of 0.05% trifluoroacetic acid in acetonitrile and 0.05% trifluoroacetic acid in water (1:19 to 1:19, v/v) gradient elution over 2 minutes; flow rate 2 ml/minute with approximately 200 ml/minute split to the Mass Spectrometer; injection volume 10-40 ml; in line Diode Array (220-450 nm), in line Evaporative Light Scattering (ELS) detection ELS - temperature 50°C, Gain 8 - 1.8 ml/minute; Source temperature 150 °C. HPLC purifications were performed on a 10 micron C18 reverse phase column (4.6mm x 10cm) eluting with 10-100% acetonitrile and water containing 0.1% trifluoroacetic acid. All NMR spectra were measured in d6-DMSO at 300 MHz unless otherwise indicated.

### Compounds Prepared on a Solid Phase

Reaction mixtures in Methods A, B, and C were agitated on a Thermolyne^{®} RotoMix^{®} Type 50800 orbital platform shaker.

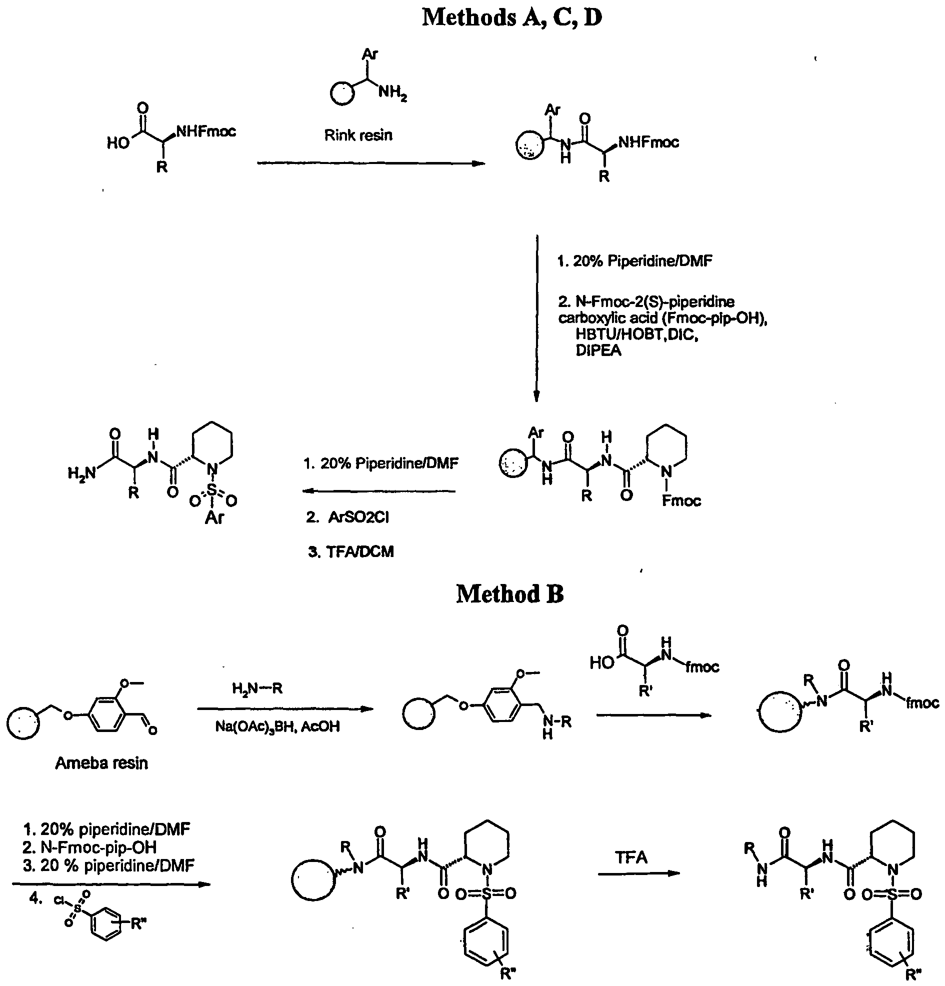

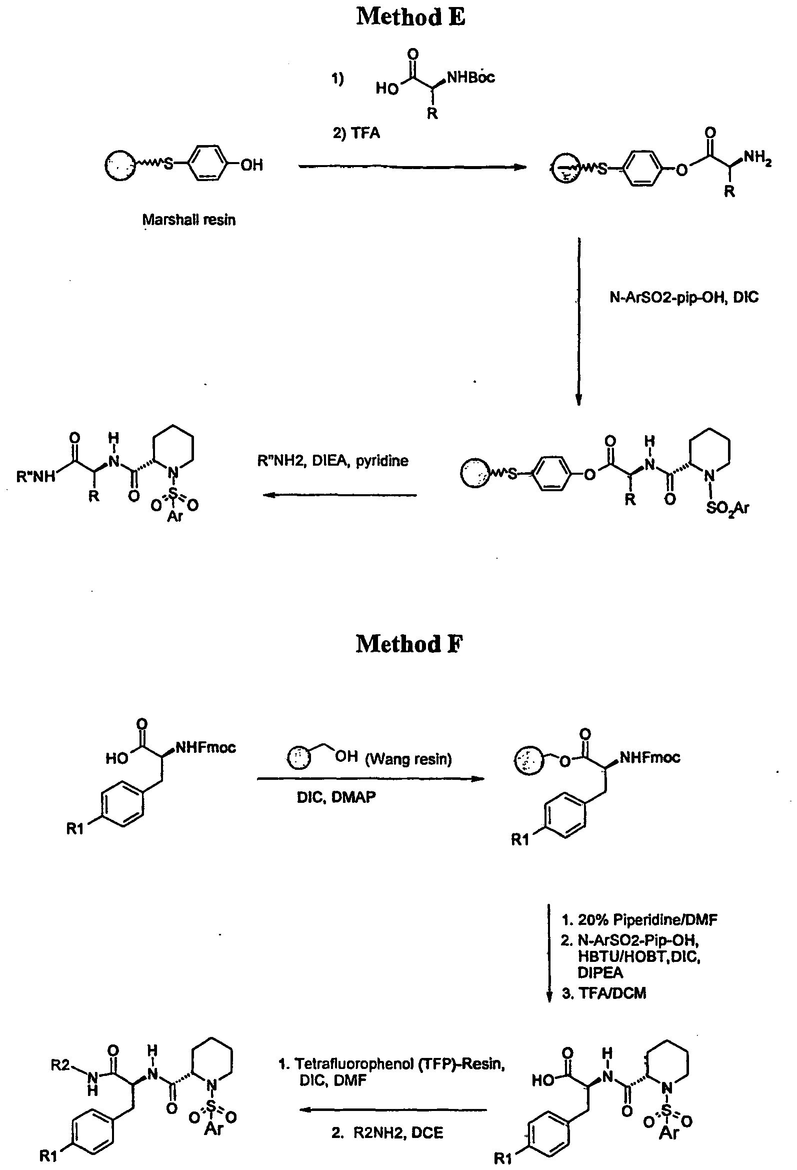

The active compounds of the present invention may be administered orally as pharmaceutical composition, for example, with an inert diluent or with an edible carrier. They may be enclosed in gelatin capsules or compressed into tablet. For the purpose of oral therapeutic administration, the aforesaid compounds may be incorporated with excipients and used in the form of tablets, troches, capsules, elixirs, suspensions, syrups, wafers, chewing gums and the like. These preparations should contain at least 0.5% of active compounds, but may be varied depending upon the particular form and may conveniently be between 4% to about 75% of the weight of the unit. The amount of present compound in such composition is such that a suitable dosage will be obtained. Preferred compositions and preparations according to the present invention are prepared so that an oral dosage unit form contains between 1.0-300 mgs of active compound.

The tablets, pills, capsules, troches and the like may also contain the following as pharmaceutically acceptable excipients or ingredients: a binder such as microcrystalline cellulose, gum tragacanth or gelatin; an excipient such as starch or lactose, a disintegrating agent such as alginic acid, Primogel, corn starch and the like; a lubricant such as magnesium stearate or Sterotes; a glidant such as colloidal silicon dioxide; and a sweetening agent such as sucrose or saccharin or a flavoring agent such as peppermint, methyl, salicylate, or orange flavoring may be added. When the dosage unit is a capsule it may contain, in addition to materials of the above type, a liquid carrier such as fatty oil. Other dosage unit forms may contain other various materials which modify the physical form of the dosage unit, for example, as coatings. Thus tablets or pills may be coated with sugar, shellac, or other enteric coating agents. A syrup may contain, in addition to the active compounds, sucrose as a sweetening agent and certain preservatives, dyes and colorings and flavors. Materials used in preparing these various compositions should be pharmaceutically pure and non-toxic in the amounts used.

For the purposes of parenteral therapeutic administration, the active compounds of the invention may be incorporated into a solution or suspension. These preparations should contain at least 0.1 % of the aforesaid compound, but may be varied between 0.5 and about 50% of the weight thereof. The amount of active compound in such-compositions is such that a suitable dosage will be obtained. Preferred compositions and preparations according to the present invention are prepared so that a parenteral dosage unit contains between 0.5 to 100 mgs of the active compound.

The solutions or suspensions may also include the following components: a sterile diluent such as water for injection, saline solution, fixed oils, polyethylene-glycols, glycerin, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparations can be enclosed in ampules, disposable syringes or multiple vials made of glass or plastic.

The following reagents may be used in the synthetic schemes. DMAP, EDCI, HOAT, HOBT, HOAT, DMSO, PyBOP, HATU, HBTU, DCM, CBZ, PyBOP, and TBTU. DMAP-dimethylaminopropylamine or 4-dimethylaminopyridine. EDCI-1-ethyl-3-[3-(dimethylamino)-propyl]carbodiimide hydrochloride. HOBT-1-hydroxybenzotriazole. DCM- dichloromethane. CBZ- carbobenzyloxy or N-benzyloxycarbonyloxy-. HOAT-1-hydroxy-7-azabenzotriazole. DMSO- dimethylsulfoxide. PyBOP- Benzotriazole-1-yl-oxy-tris-pyrrolidino-phosphonium hexafluorophosphate. TBTU- 2-(1H-Benzotriazole-1-yl)-1,1,3,3-tetramethyluronium tetrafluoroborate. HATU- O-(7-azabenzotriazol-1-yl)-1,1,3,3-tetramethyluronium hexafluorophosphate. HBTU- O-(benzotriazol-1-yl)-1,1,3,3-tetramethyluronium hexafluorophosphate. NMR refers to nuclear magnetic resonance. "psi" refers to pounds per square inch.

### Measurement of binding affinity to FK506 binding proteins:

A construct containing the FKBP coding sequence (NCBI Locus BC005147, as shown in Figure 2) is expressed in frame with an eight amino acid tag ((histidine)6 -aspartate-tyrosine) on the N-terminus to yield His6-FKBP12. Purified His6-FKBP12 protein is used for measurement of binding affinity in 96 well microtiter format. All wells contain 3.5 nM [³H]FK506, 50 mM Hepes-NaOH (pH 7.7), 0.05% bovine serum albumin, 90 µg yttrium silicate copper chelate scintillation proximity beads (Amersham^{®}), and 12 ng His6-FKBP12 in 200 µl final volume. Total and non-specific binding is defined as that observed in the absence and presence of 1 µM unlabeled FK506. Compounds of the present invention were evaluated for FKBP target affinity by including various concentrations of the unlabeled test compounds in the incubation mixtures. In cases where solvents such as DMSO were used for solubilizing the drug, a comparable concentration of the solvent was included in all wells. The reaction was initiated by adding the His6-FKBP12, then the plates were sealed and placed on a laboratory shaker for 1 hour at room temperature. Bound radioactivity was determined using a plate-based scintillation counter (TopCount^{®}, Packard^{®}). Inhibition constants (IC₅₀ values) were obtained using non-linear regression analysis and converted to Ki values using the Cheng-Prusoff correction procedure.

The binding of the compounds of the present invention to FKBP12 is exemplified by the Ki values of the following Table 1.

**TABLE 1**

| Compound | FKBP K_{¡} nM | Compound | FKBP Kᵢ nM |
|---|---|---|---|
| 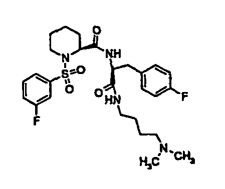 | 19.8 | 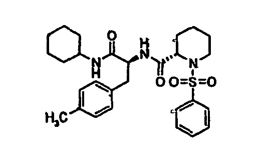 | 42.3 |
| 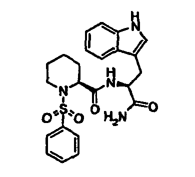 | 191 | 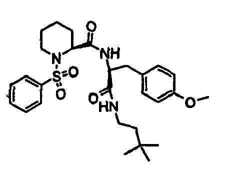 | 2.16 |
| 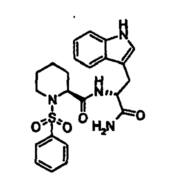 | 26,400 | 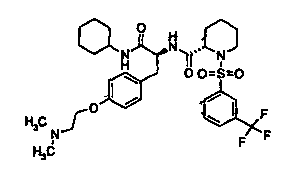 | 4.72 |
| 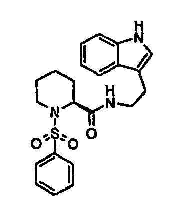 | > 100,000 | 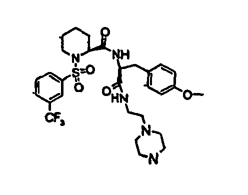 | 3.62 |
| 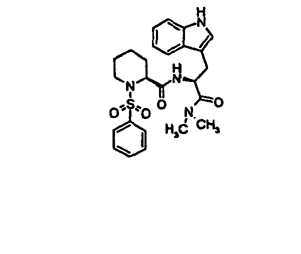 | 3870 | 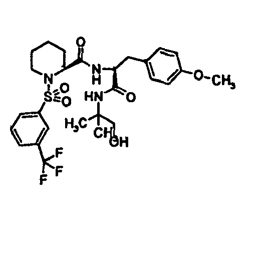 | 9.13 |
| 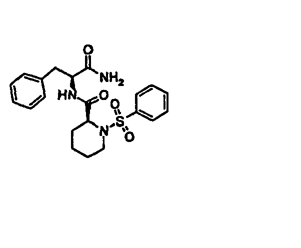 | 517 | 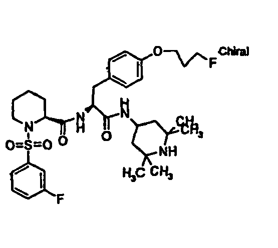 | 4.54 |
| 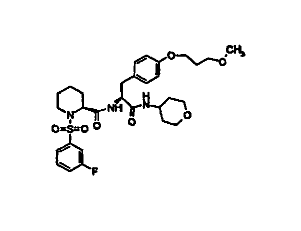 | 3.84 | 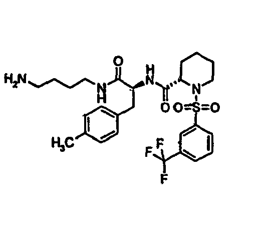 | 1.55 |
| 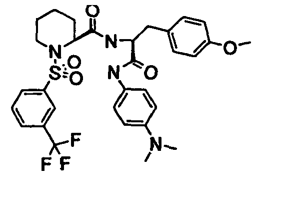 | 7.25 | 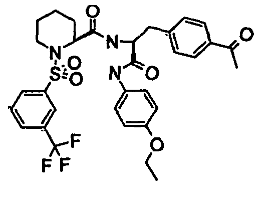 | 2.54 |
| 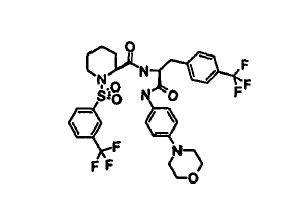 | 2.16 | 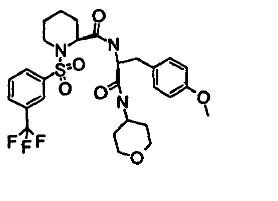 | 11.3 |
| 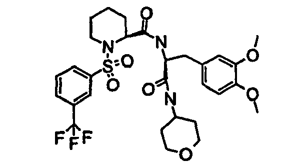 | 2.91 | 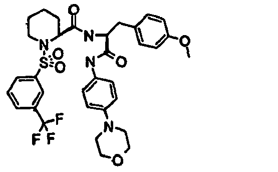 | 0.24 |
| 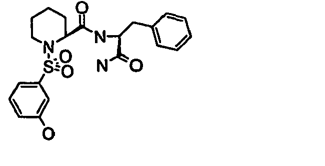 | 556 | 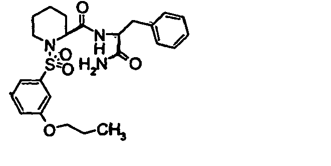 | 331 |
| 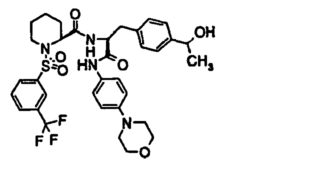 | 2.59 | 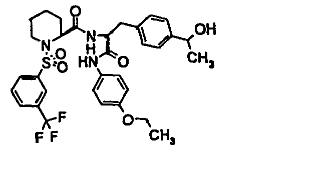 | 5.47 |
| 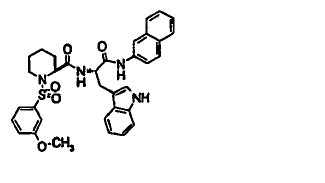 | 27.1 | 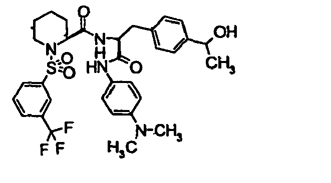 | 18.1 |
| 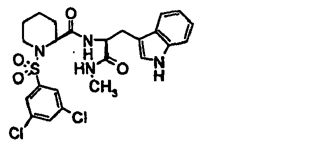 | 25.7 | 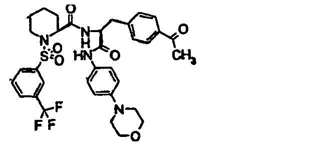 | 8.47 |
| 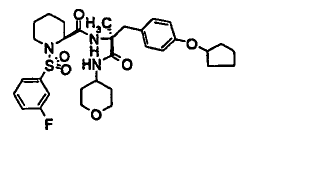 | 22.2 | 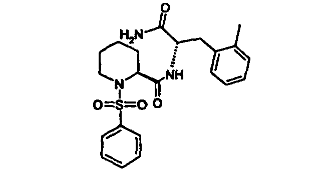 | 456 |
| 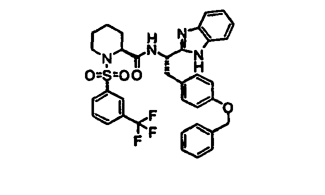 | 88.1 | 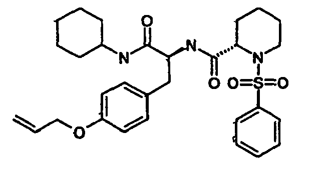 | 12.8 |
| 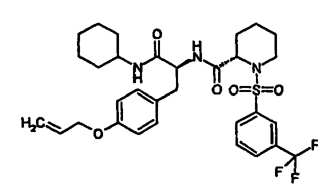 | 5.08 | 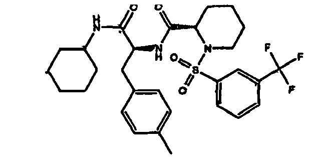 | 30.1 |
| 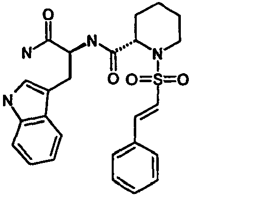 | 842 | 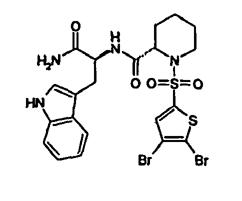 | 79.0 |
| 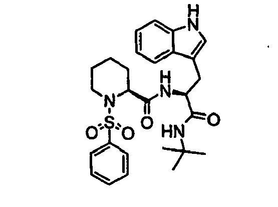 | 151 | 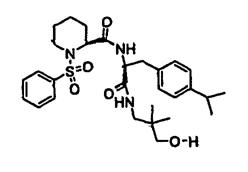 | 1.91 |
| 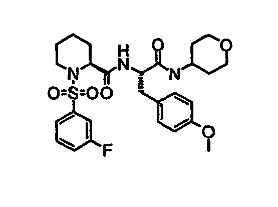 | 281 | 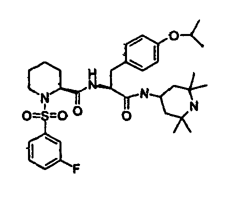 | 4.93 |
| 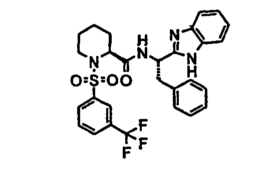 | 97.1 | 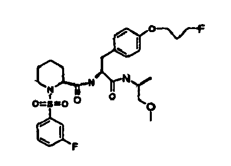 | 40.2 |
| 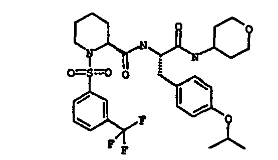 | 2.70 | | |

### Measurement of Activity on FKBP-Mediated Cellular Function:

Background: FK506 and rapamycine are believed to affect cellular function by binding to cytosolic FKBP12. The ligand-bound FKBP12 complexes display specific, high affinity interactions with other protein partners, forming a ternary ligand-protein-protein complex. Although both FK506 and rapamycine each bind to FKBP12, they facilitate distinct protein-protein interactions and modulate different intracellular signal transduction pathways. We have exploited this phenomenon to create an assay for measuring the activity of novel substances that bind to FK506 binding proteins. Compounds are assessed in the presence or absence of FK506 for their effects on reporter gene activation. Such changes are indicative of the compounds entering the cell, interacting with the FKBP12 target, and altering intracellular signaling.

The human IFNγ promoter (see Figure 1) was cloned into the expression vector pGL3NEO (*Sst*I/H*ind* III) to give IFNγ-Luc. Jurkat cells were (grown in RPMI 1640, 10% FBS, 1% antibiotic/antimycotic), transfected with the above DNA [as described by Staynov et al.: Staynov, D.Z., Cousins, D.J., and Lee, T.K. (1995), Proc. Natl. Acad. Sci. USA 92:3606-3610)] and selected against 800 µg/ml of G418 (Geneticin). Monoclonal lines were derived from the stable transfected polyclonal population and used for the assay. The monoclonal cell lines were maintained in culture using the above-described medium in the presence of 400 µg/ml G418.

Jurkat cells containing IFNγ-Luc (monoclonal lines) were resuspended in 1% fetal bovine serum (FBS) containing RPMI Medium 1640 and dispensed into 96-well plates (40,000 cells / well). The plates were then incubated for 3 hours at 37°C in an atmosphere of 5%CO₂. In order to assess intrinsic effects of compounds on FKBP12-mediated signaling, various concentrations of the test compounds (or vehicle) were added to the cells. Then cells were stimulated with 10 ng/ml of phorbol 12-myristate 13-acetate (PMA) and 1.0 µM calcium ionophore (A23187) in a final volume of 100 µl. In other cases, the ability of compounds to inhibit the effects of FK506 was monitored. In this paradigm, fixed concentrations of the test compound (or vehicle) were added to the cell suspension and incubated for 45 minutes at 37°C in the cell culture incubator. The plates were then removed from the incubator and various concentrations of FK506 (typically 10⁻¹² M to 10⁻⁷ M final concentration) were added to the respective wells. Cells were then challenged with PMA/ A23187 as described above and returned to the cell culture incubator.

After an overnight incubation, the plates were removed from the incubator and the response was terminated by addition of 50 µl of lysis buffer (25mM Tris phosphate, pH 7.8, 2mM EDTA, 100ml/L Glycerol, 1% Triton-X100 (v/v)). The cells were incubated 15 minutes at room temperature, and mixed by repeated up and down pipetting to ensure complete lysis. Then 75µl of the cell lysate was transferred to OptiPlate^{™} white bottomed plates and assayed for luciferase activity by adding 75µl of a substrate/cofactor mixture (containing: 0.4 mM Luciferin, 0.5mM ATP, 2.7mM Coenzyme A, 32.5 mM DTT in 2X Assay buffer: 40mM Tricine, 2mM (MgCO₃)₄Mg(OH)₂x5H₂O, 10mM MgSO₄, 0.2mM EDTA). The plates were sealed using an adhesive film and luminescence was quantified in a Packard TopCount-NXT^{™} plate reader.

The intrinsic activity of the test compounds was determined from their ability to inhibit PMA/ A23187- mediated increases in reporter activity. Concentrations producing half-maximal inhibition (EC₅₀) were derived using non-linear regression analysis. For reference, the EC₅₀ of FK506 in this paradigm was 0.7 nM. In other cases, the activity of the test compounds to prevent the action of FK506 was assessed. This was quantitated as the ratio of the EC₅₀ of FK506 observed in the presence of 1 uM test compound divided by the respective EC₅₀ value measured in the absence of the test compound. For reference, rapamycine (1µM) produces a ratio value of 15-30. Compounds interacting with cellular FKBP12 demonstrate either direct inhibition of PMA/ A23187-mediated effects with EC₅₀ values less than 10 µM or when administered at 1 µM produce statistically significant increases in the EC₅₀ of FK506. See: Staynov, D.Z., Cousins, D.J., and Lee, T.K. (1995), Proc. Natl. Acad. Sci. USA 92:3606-3620.

Measurement of Activity on FKBP-mediated cellular function in Jurkat cells of the compounds of the present invention is exemplified by the "Jurkat Shift" values of the following Table 2. The Jurkat Shift values represent the ratio of the EC₅₀ of FK506 observed in the presence of 1 µM test compound divided by the respective EC₅₀ value in the absence of test compound.

**Table 2**

| Compound | Jurkat Shift | Compound | Jurkat Shift |
|---|---|---|---|
| 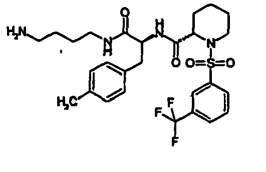 | 4.5 | 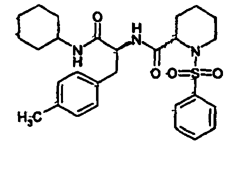 | 10.4 |
| 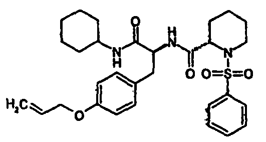 | 13.4 | 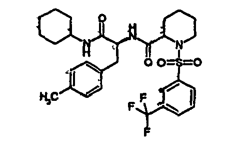 | 69.5 |
| 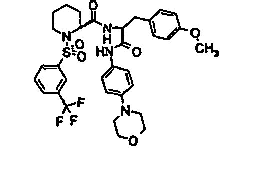 | 83.1 | 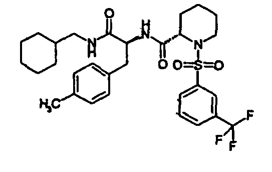 | 2.8 |
| 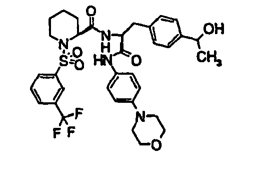 | 45.7 | 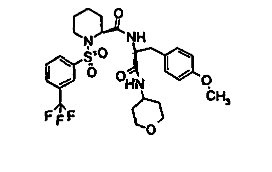 | 14.4 |
| 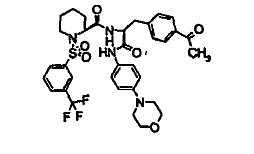 | 12.3 | 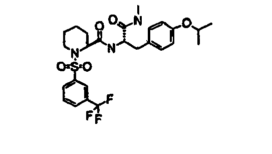 | 3.4 |
| 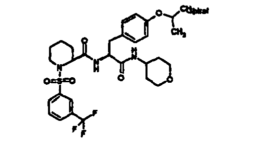 | 7.5 | 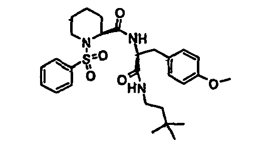 | 6.5 |
| 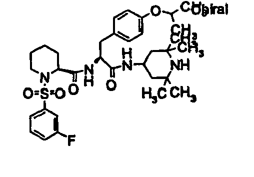 | 69.4 | 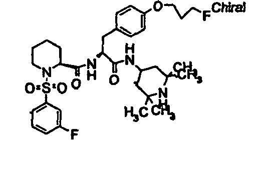 | 34.6 |
| 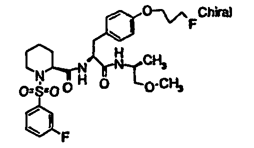 | 7.1 | 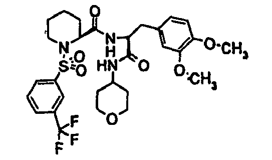 | 31.4 |

### Neurite outgrowth assay:

PC12 cells, obtained from the American Type Culture Collection, were grown in RPMI-1640 medium supplemented with 10% heat-inactivated horse serum (HS) and 5% FBS. Cells were seeded in collagen 4-coated 96 well plates in low serum (0.5% HS, 0.25% FBS) media at a density of 2,000 cells/well. The cells were then primed with 1 µg/ml of NGF for 3 days. On the fourth day, the culture media was replaced with one containing 50 ng/ml of NGF and, where indicated, test compounds. The cells were then incubated for another 4 days following which they were fixed and immunostained with a primary antibody specific for neuronal cells bodies and processes, followed by Alexa Fluor 488-coupled secondary antibody, and the nuclear counterstain Hoechst^{®} Dye (HitKit^{®} Cat#K07-0001-1, Cellomics^{®}). Neurite outgrowth in the stained specimens were quantitated using a computer-controlled, microscope-based imaging system (ArrayScan II^{®}, Cellomics^{®}). Compounds of the present invention were found to significantly increase both the fraction of total cells bearing neurites and the average neurite length.

### Measurement of Immunosuppressant Activity in Human T-cells:

Purified CD4 positive lymphocytes were obtained from human, peripheral blood. Cells were stimulated for 24 hours using anti-CD3 and CD28 monoclonal antibodies crosslinked to protein G. Three days after stimulation, IL-2 production was measured in the culture media supernatant using a commercially available ELISA kit (BD Biosciences). FK506 was employed in all experiments as a positive control. IL-2R expression was measured by flow cytometry using fluorescent-labeled anti-CD25 and anti-CD 122 monoclonal antibodies (binding to the alpha and beta chains of the IL-2R, respectively). Cell proliferation was assessed by tritiated-thymidine incorporation measured at 3 and 6 days post-stimulation. Immunosuppressant activity was defined as activity qualitatively similar to that displayed by FK506.

The present invention is further exemplified by the following examples.

### EXAMPLES

### EXAMPLE 1

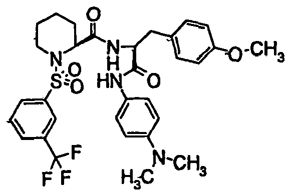

1.
   (S)-1-(3-Trifluoromethyl-benzenesulfonyl)-piperidine-2-carboxylic acid [(S)-1-(4-dimethylamino-phenylcarbamoyl)-2-(4-methoxy-phenyl)-ethyl]-amide
1a)
   (S)-1-(3-Trifluoromethyl-benzenesulfonyl)-piperidine-2-carboxylic acid benzyl ester To 4.0 g (15.64 mmol or millimoles) of (S)-piperidine-2-carboxylic acid benzyl ester hydrochloride and 3.15 g (31.28 mmol) of triethylamine in (200 ml) was added 4.21 g (17.20 mmol) of 3-trifluoromethylbenzenesulfonyl chloride. The mixture was stirred at room temperature for 1 hour. The reaction mixture was then washed with water, brine and dried (sodium sulfate). Chromatography on silica gel, eluting with dichloromethane provided 6.7 g (15.64 mmol) of (S)-1-(3-Trifluoromethyl-benzenesulfonyl)-piperidine-2-carboxylic acid benzyl ester. LCMS (M+H): m/z 428, retention time 3.9 min.
1b)
   (S)-1-(3-Trifluoromethyl-benzenesulfonyl)-piperidine-2-carboxylic acid To 12.20 g (28.85 mmol) ofN-3-trifluoromethyl-(S)-piperidine-2-carboxylic acid benzyl ester in 300 ml of 1/1 methanol/ethyl acetate was added 2.0 g of 10 % palladium on carbon. The resulting suspension was stirred under 1 atmosphere of hydrogen for 2 hours, after which the catalyst was filtered and the filtrate was concentrated to provided 8.0 g (23.73 mmol) of (S)-1-(3-Trifluoromethyl-benzenesulfonyl)-piperidine-2-carboxylic acid, which was used without further purification. The 300 MHz ¹H NMR was consistent with the structure. LCMS (M+H): m/z 338, retention time 2.77 min.
1c)
   (S)-3-(4-Hydroxy-phenyl)-2-{([(S)-1-(3-trifluoromethyl-benzenesulfonyl)-piperidine-2-carbonyl]-amino}-propionic acid methyl ester
   To a solution of 0.96 g (2.85 mmol) of compound 1b and 1.26g (2.85 mmol) of the p-toluenesulfonic acid salt of tyrosine benzyl ester in 20 ml of dichloromethane was added 1.2 g (14.24 mmol) of sodium bicarbonate followed by 0.93 g (6.84 mmol) of HOAT and 1.52 g (8.00 mmol) of EDCI. The mixture was stirred at room temperature for 17 hours after which it was purified by flash chromatography on silica gel eluting with 30-50% ethyl acetate / heptane to provide 1.2 g (20.33 mmol) of (S)-3-(4-hydroxy-phenyl)-2-{[(S)-1-(3-trifluoromethyl-benzenesulfonyl)-piperidine-2-carbonyl]-amino}-propionic acid methylester. LCMS (M+H): m/z 591, retention time 3.59 min.
1d)
   (S)-3-(4-Methoxy-phenyl)-2-{[(S)-1-(3-trifluoromethyl-benzenesulfonyl)-piperidine-2-carbonyl]-amino}-propionic acid benzyl ester
   To a solution of 0.4 g (0.68 mmol) benzyl ester (1c) in 4 ml of DMSO was added 1.10 g (3.39 mmol) of cesium carbonate followed by 0.96 g (6.78 mmol) of iodomethane. This mixture was stirred at room temperature overnight. Reaction mixture was then diluted with 20 ml of ethyl acetate, washed with water three times, brine three times. It was purified by chromatography on silica gel, eluting with 50 % ethyl acetate/ heptane to give 0.41 g (0.68 mmol) of (S)-3-(4-methoxy-phenyl)-2-{[(S)-1-(3-trifluoromethyl-benzenesulfonyl)-piperidine-2-carbonyl]-amino}-propionic acid benzyl ester. LCMS (M+H): m/z 605, retention time 3.79 min.
1e)
   (S)-3-(4-Methoxy-phenyl)-2-{[(S)-1-(3-trifluoromethyl-benzenesulfonyl)-piperidine-2-carbonyl]-amino}-propionic acid
   To 0.41g (0.68 mmol) of benzyl ester (1d) in 6 ml of 1/1 methanol/ethyl acetate under nitrogen was added 0.082 g of 10 % palladium on carbon. The resulting suspension was stirred under 1 atm of hydrogen for 2 hours, after which the suspension was filtered and the filtrate concentrated to provide 0.34 g (0.68 mmol) of (S)-3-(4-methoxy-phenyl)-2-{[(S)-1-(3-trifluoromethyl-benzenesulfonyl)-piperidine-2-carbonyl]-amino}-propionic acid which was used without further purification. The 300 MHz ¹H NMR was consistent with the structure. LCMS (M+H): m/z 515, retention time 3.37 min.
1f)
   (S)-1-(3-Trifluoromethyl-benzenesulfonyl)-piperidine-2-carboxylic acid [(S)-1-(4-dimethylamino-phenylcarbamoyl)-2-(4-methoxy-phenyl)-ethyl]-amide To a solution of 38 mg (0.28 mmol) of 4-dimethylaminoaniline and 72 mg (0.14 mmol) of carboxylic acid (1e) in 3 ml of dichloromethane was added 70 mg (0.82 mmol) of sodium bicarbonate followed by 46 mg (0.34 mmol) of HOAT and 74 mg (0.392 mmol) of EDCI. The mixture was stirred at room temperature for 17 hours after which it was purified by flash chromatography on silica gel, eluting with 30-50% ethyl acetate / heptane. The resulting solid was washed with 30% ethyl acetate / heptane and dried to provide 53 mg (83.8 mmol) of the title compound. ¹H NMR (400 MHz, CDCl₃): 1.13-1.46 (m, 5H); 2.14-2.16 (bd, 1H); 2.60(t, 1H); 2.91(s, 6H); 3.06 (dd, 1H); 3.23 (dd, 1H); 3.65-3.68 (bd, 1H); 3.78(s, 3H); 4.42(b, 1H); 4.71(q, 1H); 6.87(d, 3H); 7.18(d, 2H);7.30(d, 2H); 7.62(s, 1H); 7.69(t, 1H); 7.86(d, 1H); 8.01(d, 1H). Example 1 exhibits FKBP12 binding with a Kᵢ of 6.11E-10.

### EXAMPLE 2

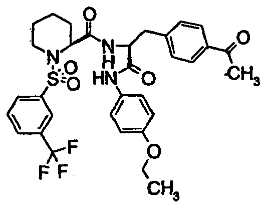

2.
   (S)-1-(3-Trifluoromethyl-benzenesulfonyl)-piperidine-2-carboxylic acid [(S)-2-(4-acetylphenyl)-1-(4-ethoxy-phenylcarbamoyl)-ethyl]-amide
   The title compound was prepared from carboxylic acid compound 15a and 4-ethoxyaniline employing the procedure used to synthesize compound 15. ¹H NMR (300 MHz, CDCl₃): 1.09-1.42(m, 8H); 2.04-2.12(bd, 1H); 2.51-2.54(m, 1H); 2.59(s, 3H); 3.22(dd, 1H); 3.42(dd, 1H); 3.58-3.62(bd, 1H); 4.00(q, 2H); 4.37(b,1H); 4.86(q, 1H); 6.84(d, 1H); 6.90(d, 1H); 7.38(d, 4H); 7.70(t, 1H); 7.86-7.94(m, 4H); 8.00(d, 1H); 8.08(s, 1H).

### EXAMPLE 3

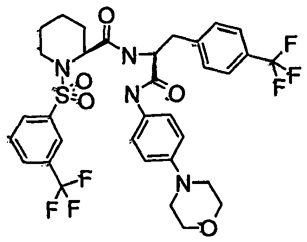

3. (S)-1-(3-Trifluoromethyl-benzenesulfonyl)-piperidine-2-carboxylic acid [(S)-1-(4-morpholin-4-yl-phenylcarbamoyl)-2-(4-trifluoromethyl-phenyl)-ethyl]-amide
   The title compound was prepared from the corresponding 4-(trifluoromethyl) phenylalanine carboxylic acid derivative and 4-morpholinoaniline as described for step (1f). ¹H NMR (400 MHz, CDCl₃): 0.95-1.45(m, 5H); 2.16-2.05(bd, 1H); 2.35(dd,1H); 3.02-3.20(m, 5H); 3.41-3.60(m, 2H); 3.84-3.87(m, 4H); 4.36(b, 1H); 4.80-4.90(m, 1H);6.85-6.88(m, 3H); 7.38-7.43(m, 4H); 7.60(d, 2H); 7.70(t, 1H); 7.88(d, 1H); 7.95(s, 1H); 8.01(d, 1H); 8.08(s, 1H).

### EXAMPLE 4

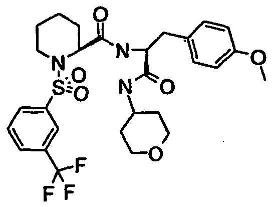

4.
   (S)-1-(3-Trifluoromethyl-benzenesulfonyl)-piperidine-2-carboxylic acid [(S)-2-(4-methoxyphenyl)-1-(tetrahydro-pyran-4-ylcarbamoyl)-ethyl]-amide
   To a solution of 0.094g (0.94 mmol) of 4-aminotetrahydropyran and 0.48g (0.94 mmol) of (S)-3-(4-methoxy-phenyl)-2-{[(S)-1-(3-trifluoromethyl-benzenesulfonyl)-piperidine-2-carbonyl]-amino}-propionic acid in 50 ml of dichloromethane was added 0079g (0.94 mmol) of sodium bicarbonate followed by 0.128g (0.94 mmol) of HOAT and 0.179g (0.94 mmol) of EDCI. The mixture was stirred at room temperature for 17 hours after which it was washed with brine and purified by flash chromatography on silica gel, eluting with 50-100% ethyl acetate / heptane to give 0.50g (0.83 mmol) of the title compound. ¹H NMR (300 MHz, CDCl₃): 1.00-1.21(m, 3H); 1.32-1.43 (m, 4H); 1.80(b, 2H); 2.14(bd, 1H); 2.69(t, 1H); 2.99(dd, 1H); 3.13(dd, 1H); 3.45(s, 2H); 3.65(bd, 1H); 3.78(s, 3H); 3.87-4.00(m, 3H); 4.39(b, 1H); 4.58(q, 1H); 5.88(d, 1H); 6.78(d, 1H); 6.85(d, 2H); 7.13(d, 2H); 7.70(t, 1H); 7.88(d, 1H); 8.00(d, 1H); 8.08(s, 1H).

### EXAMPLE 5

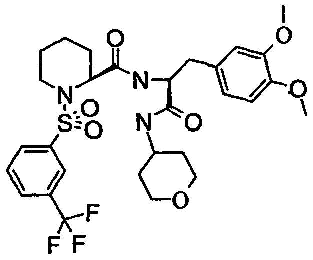

5.
   (S)-1-(3-Trifluoromethyl-benzenesulfonyl)-piperidine-2-carboxylic acid [(S)-2-(3,4-dimethoxy-phenyl)-1-(tetrahydro-pyran-4-ylcarbamoyl)-ethyl]-amide
   To a solution of 90mg (0.29 mmol) of (S)-2-amino-3-(3,4-dimethoxy-phenyl)-(tetrahydropyran-4-yl)-propionamide and 98mg (0.29 mmol) of (S)-1-(3-trifluoromethylbenzenesulfonyl)-piperidine-2-carboxylic acid in 2 ml of dichloromethane was added 25mg (0.29 mmol) of sodium bicarbonate followed by 40mg (0.29 mmol) of HOAT and 56mg (0.29 mmol) of EDCI. The mixture was stirred at room temperature for 17 hours after which it was purified by flash chromatography on silica gel, eluting with 50-100% ethyl acetate / heptane to give 0.162g (0.25 mmol) of the title compound. ¹H NMR (300 MHz, CDCl₃): 1.00-1.40(m, 7H); 1.84(b, 2H); 2.12(bd, 1H); 2.60(t, 1H); 2.99(dd, 1H); 3.18(dd, 1H); 3.45(t, 2H); 3.61(bd, 1H); 3.79-4.00(m, 9H); 4.38(b, 1H); 4.62(q, 1H); 5.96(d, 1H); 6.74-6.83(m, 4H); 7.70(t, 1H); 7.87(d, 1H); 8.00(d, 1H); 8.07(s, 1H).

### EXAMPLE 6

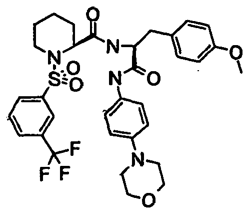

6.
(S)-1-(3-Trifluoromethyl-benzenesulfonyl)-piperidine-2-carboxylic acid [(S)-2-(4-methoxyphenyl)-1-(4-morpholin-4-yl-phenylcarbamoyl)-ethyl]-amide
   The title compound was prepared from carboxylic acid compound 1e and 4-morpholino aniline according to the method described for the preparation of compound 1. ¹H NMR (300 MHz, CDCl₃): 1.00-1.52(m, 5H); 2.12-2.14(bd, 1H); 2.56(dd, 1H); 3.02-3.13(m, 5H); 3.25(dd, 1H); 3.65(bd, 1H); 3.79(s, 3H); 3.83-3.86(m, 4H); 4.40(b, 1H); 4.73(q, 1H); 6.83-6.88(m, 5H); 7.18(d, 2H); 7.38(d, 2H); 7.69(t, 1H); 7.78(s, 1H); 7.87(d, 1H); 8.01(d, 1H); 8.09(s, 1H).

### EXAMPLE 7

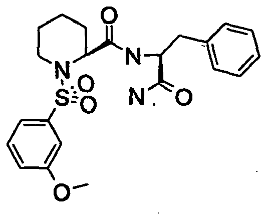

7.
   (S)-1-(3-Methoxy-benzenesulfonyl)-piperidine-2-carboxylic acid [(S)-1-carbamoyl-2-phenylethyl}-amide
7a)
   (S)-1-(3-Methoxy-benzenesulfonyl)-piperidine-2-carboxylic acid
   To a solution of 7.4-g (36.00 mmol) of (S)-piperidine-2-carboxylic acid and sodium carbonate in 50 ml of water was added 3- methoxybenzenesulfonyl chloride. The mixture was stirred at room temperature for 12 hours. After that reaction mixture was washed with ether, acidified to pH 2 with 1N HCl and extracted with ethyl acetate. The organic phase was washed with water, dried (sodium sulfate) and purified by flash chromatography on silica-gel eluting with 50-100% ethyl acetate/ heptane to provide 3.34 g (11.17 mmol) of compound 7a. LCMS (M+H): m/z 300, retention time 1.60 min.
7b)
   (S)-1-(3-Methoxy-benzenesulfonyl)-piperidine-2-carboxylic acid ((S)-1-carbamoyl-2-phenylethyl)-amide
   To a solution of B-2528-158-0 (1.56 g, 5.21 mmol) and (S)-2-amino-3-phenyl-propionamide (1.02 g, 6.25 mmol) in 30 ml of dichloromethane was added 2.74 g (27.09 mmol) of 4-methylmorpholine followed by 1.68g (12.50 mmol) of HOBT, 0.32 g (2.60 mmol) of DMAP and 2.78g (14.58 mmol) of EDCI. The mixture was stirred at room temperature for 17 hours after which it was purified by flash chromatography on silica gel, eluting with 50% ethyl acetate / heptane to provide 1.69 g (3.79 mmol) of the title compound. ¹H NMR (300 MHz, CDCl₃): 0.91-1.38(m, 5H); 2.10(bd, 1H); 2.33(dd, 1H); 2.99(dd, 1H); 3.35(dd, 1H); 3.55(bd, 1H); 3.86(s, 3H); 4.34(b, 1H); 4.75(m, 1H); 5.38(b, 1H); 6.24(b, 1H); 6.82(d, 1H); 7.13(d, 1H); 7.20-7.47(m, 8H).

### EXAMPLE 8

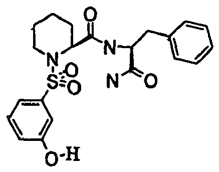

8.
   (S)-1-(3-Hydroxy-benzenesulfonyl)-piperidine-2-carboxylic acid ((S)-1-carbamoyl-2-phenylethyl)-amide
   To a solution of 1.47 g (3.29 mmol) of compound 7 in 200 ml dichloromethane was slowly added 26.39 ml of tribromoborane (26.39 mmol, 1M in dichloromethane). The resulting mixture was stirred at 0 for 3 hours. Methanol (5 ml) was then added. The reaction mixture was neutralized with aqueous sodium carbonate and stirred overnight. The reaction mixture was diluted with DCM, extracted three times with DCM, washed with brine and purified by chromatography on silica gel eluting with 1% 7N NH₃ in MeOH / 50% ethyl acetate/ heptane to give 1.26 g of the title compound as a white solid. LCMS (M+H): m/z 432, retention time 1.55 min.

### EXAMPLE 9

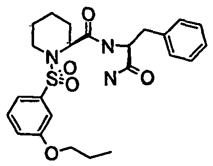

9.
   (S)-1-(3-Propoxy-benzenesulfonyl)-piperidine-2-carboxylic acid ((S)-1-carbamoyl-2-phenylethyl)-amide
   To a solution of 50mg (0.116 mmol) of compound 8 in DMF (0.5 ml) was added n-bromopropane and cesium carbonate. The mixture was stirred at room temperature for 17 hours. It was then purified by flash chromatography on silica gel, eluting with 1% 7N NH₃ in MeOH / ethyl acetate. 4 mg of the title compound was obtained. ¹H NMR (300 MHz, CDCl₃): 0.91-1.38(m, 8H); 1.77-1.89(m, 2H); 2.07(bd, 1H); 2.29(dd, 1H); 2.99(dd, 1H); 3.36(dd, 1H); 3.55(bd, 1H); 3.95(t, 2H); 4.35(b, 1H); 4.71-4.78(m, 1H); 5.33(b, 1H); 6.25(b, 1H); 6.80(d, 1H); 7.12(d, 1H); 7.20-7.45(m, 8H).

### EXAMPLE 10

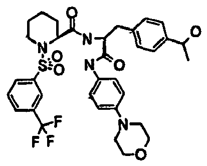

10.
   (S)-1-(3-Trifluoromethyl-benzenesulfonyl)-piperidine-2-carboxylic acid [(S)-2-[4-(1-hydroxyethyl)-phenyl]-1-(4-morpholin-4-yl-phenylcarbamoyl)-ethyl]-amide
10a)
   (S)-3-(4-Acetyl-phenyl)-2-{[(S)-1-(3-trifluoromethyl-benzenesulfonyl)-piperidine-2-carbonyl]-amino}-propionic acid benzyl ester
   To a solution of 0.23 g 0.67 mmol) of compound 1b and 0.2g (0.67 mmol) of 4-acetyl phenylalanine, benzyl ester in 6 ml of dichloromethane was added 0.28 g (3.36 mmol) of sodium bicarbonate followed by 0.22 g (1.61 mmol) of HOAT and 0.36 g (1.88 mmol) of EDCI. The mixture was stirred at room temperature for 17 hours after which it was purified by flash chromatography on silica gel, eluting with 20-50% ethyl acetate / heptane to provide 0.42 g (0.67 mmol) of compound 10a. LCMS (M+H): m/z 617, retention time 3.4 min.
10b)
   (S)-3-[4-(1-Hydroxy-ethyl)-phenyl]-2-{[(S)-1-(3-trifluoromethyl-benzenesulfonyl)-piperidine-2-carbonyl]-amino}-propionic acid
   To 0.15 g (0.24 mmol) of (S)-3-(4-acetyl-phenyl)-2-{[(S)-1-(3-trifluoromethylbenzenesulfonyl)-piperidine-2-carbonyl]-amino}-propionic acid benzyl ester in 30 ml of methanol was added 0.030 g of 10 % palladium on carbon. The resulting suspension was stirred under 50 psi of hydrogen for 2 hours, after which the suspension was filtered and the filtrate was concentrated to provide 0.12 g (0.24 mmol) of the title compound which was used without further purification. The 300 MHz ¹H NMR was consistent with the structure. LCMS (M+Na): m/z 551, retention time 3.05 min.
10c)
   (S)-1-(3-Trifluoromethyl-benzenesulfonyl)-piperidine-2-carboxylic acid [(S)-2-[4-(1-hydroxyethyl)-phenyl]-1-(4-morpholin-4-yl-phenylcarbamoyl)-ethyl]-amide
   To a solution of 50 mg (0.0956 mmol) of compound 10b and 17 mg (0.0956 mmol) of 4-morpholinoaniline in dichloromethane (2 ml) was added 6 mg (0.045 mmol) DMAP followed by addition of sodium bicarbonate 48 mg (0.48 mmol), HOAT 31 mg (0.23 mmol) and EDCI (51 mg, 0.27 mmol). The mixture was stirred at room temperature overnight. The reaction mixture was then loaded on silica gel column, eluted with 50% ethyl acetate / heptane to provide 28 mg (0.041mmol) of compound 10. ¹H NMR (300 MHz, CDCl₃): 1.08-1.82(m, 7H); 1.85(dd, 1H); 2.11-2.16(bd, 1H); 2.55(dd, 1H); 3.10-3.16(m, 5H); 3.30(dt, 1H); 3.56-3.62(bd, 1H); 3.84-3.87(t, 4H); 4.36(b, 1H); 4.81-4.89(m, 2H); 6.80-6.87(m, 3H); 7.19-7.40(m, 6H); 7.69(t, 1H);7.86-7.89(m, 2H); 8.00(d,1H); 8.07(s, 1H).

### EXAMPLE 11

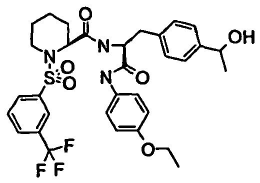

11.
   (S)-1-(3-Trifluoromethyl-benzenesulfonyl)-piperidine-2-carboxylic acid {(S)-1-(4-ethoxy-phenylcarbamoyl)-2-[4-(1-hydroxy-ethyl)-phenyl]-ethyl}-amide
   The title compound was prepared from compound 10b and 4-ethoxyaniline according to the method described for the preparation of compound 10. LCMS (M+H-H₂O): m/z 630, retention time 3.12 min.

### EXAMPLE 12

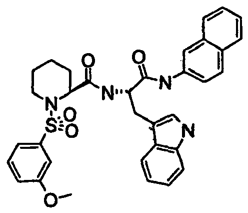

12.
   (S)-1-(3-Methoxy-benzenesulfonyl)-piperidine-2-carboxylic acid [(S)-2-(indol-3-yl)-1-(naphthalen-2-ylcarbamoyl)-ethyl]-amide
   To a solution of 0.78g (2.60 mmol) of (S)-1-(3-methoxy-benzenesulfonyl)-piperidine-2-carboxylic acid and 1.03g (3.12 mmol) of (S)-2-amino-3-(1-indol-3-yl)-naphthalen-2-yl-propionamide in dichloromethane (100 ml) was added 0.16g (1.30 mmol) DMAP followed by addition of 4-methylmorpholine1.37.g (13.54 mmol), HOBT 0.84g (6.25mmol) and EDCI 1.39g (7.29 mmol). The mixture was stirred at room temperature overnight. The reaction mixture was then washed with water, dried (sodium sulfate). Chromatography on silica gel eluting with 50% ethyl acetate / heptane proved 1.2g (1.96 mmol) of the title compound. ¹H NMR (300 MHz, CDCl₃): 1.34-1.01(m, 5H); 2.07(bd, 1H); 2.21(dd, 1H); 3.43-3.48(m, 3H); 3.83(s, 3H); 4.34(b, 1H); 4.98(q, 1H); 6.98(d, 1H); 7.09-7.47(m, 11H); 7.68-7.80(m, 4H); 8.15(s, 1H); 8.21(s, 1H); 8.40(s, 1H).

### EXAMPLE 13

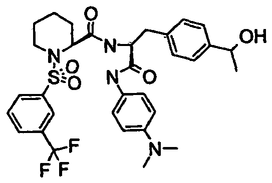

13.
   (S)-1-(3-Trifluoromethyl-benzenesulfonyl)-piperidine-2-carboxylic acid {(S)-1-(4-dimethylamino-phenylcarbamoyl)-2-[4-(1-hydroxy-ethyl)-phenyl]-ethyl}-amide The title compound was prepared from compound 10a and 4-dimethylaminoaniline according to the procedure employed to prepared compound 10. LCMS (M+H-H₂O): m/z 628, retention time 2.59 min.

### EXAMPLE 14

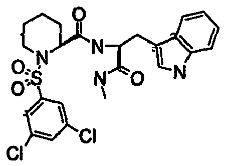

14.
   (S)-1-(3,5-Dichloro-benzenesulfonyl)-piperidine-2-carboxylic acid [(S)-2-(1H-indol-3-yl)-1-methylcarbamoyl-ethyl]-amide
14a)
   [(S)-2-(indol-3-yl)-1-methylcarbamoyl-ethyl]-carbamic acid benzyl ester To a solution of 3.38 g (10.0 mmol) ofN-CBZ-tryptophan in the solution of dichloromethane (100 ml) was added 6 ml of methylamine (12 mmol, 2M in THF) followed by 0.51g (5.20 mmol) of 4-methyl-morpholine, DMAP 0.61g (5.00 mmol), HOBT 3.24g (24.0 mmol) and EDCI 5.30g (28.0 mmol). The mixture was stirred at room temperature overnight, after which it was loaded onto a silica gel column and eluted with ethyl acetate to provide 2.43g (6.92mmol) of compound 14a. LCMS (M+H): m/z 352, retention time 1.62 min.
14b)
   (S)-2-Amino-3-(indol-3-yl)-N-methyl-propionamide
   To 1.89 g (5.37 mmol) of compound 14a in 240 ml of 1/1 methanol/ethyl acetate was added 0.38 g of 10 % palladium on carbon under nitrogen. The resulting suspension was stirred under 1 atm of hydrogen for 1.5 hours, after which the suspension was filtered and the filtrate concentrated to provide 1.16 g (5.37 mmol) of compound 14b which was used without further purification. The 300 MHz NMR was consistent with the structure. LCMS (M+H): m/z 218, retention time 0.93 min.
14c)
   (S)-1-CBZ-Piperidine-2-carboxylic acid [(S)-2-(indol-3-yl)-1-methylcarbamoyl-ethyl]-amide To a solution of 1.77 g (8.15 mmol) of compound 14b and 2.14 g (8.15 mmol) of N-CBZ-(S)-piperidine- 2-carboxylic acid in 50 ml of dichloromethane was added 0.50 g (4.07 mmol) of DMAP followed by 2.65 g (19.63 mmol) of HOBT, 4.28 g (42.38 mmol) of 4-methyl morpholine and 4.37 g (22.90 mmol) of EDCI. The mixture was stirred at room temperature for 17 hours after which it was purified by flash chromatography on silica gel, eluting with 50-100% ethyl acetate / heptane to provide 2.52 g (5.45 mmol) of compound 14c. LCMS (M+H): m/z 463, retention time 1.73 min.
14d)
   (S)-Piperidine-2-carboxylic acid [(S)-2-(indol-3-yl)-1-methylcarbamoyl-ethyl]-amide To 2.46 g (5.32 mmol) of compound 10c in 80 ml of 1/1 methanol/ethyl acetate was added 0.5 g of 10 % palladium on carbon. The resulting suspension was stirred under 1 atm of hydrogen for 2 hours, after which the suspension was filtered and the filtrate concentrated to provide 1.51 g (4.60 mmol) of compound 14d which was used without further purification. The 300 MHz NMR was consistent with the structure. LCMS (M+H): m/z 329, retention time 1.02 min.
14e)
   (S)-1-(3,5-Dichloro-benzenesulfonyl)-piperidine-2-carboxylic acid [(S)-2-indol-3-yl)-1-methylcarbamoyl-ethyl]-amide
   50mg (0.152 mmol) of compound 14d was dissolved in 1 ml of 1/1 dichloromethane / tetrahydrofuran and 0.03 ml (0.30 mmol) triethylamine and 52 mg (0.213 mmol) of 3, 5-dichlorobenzenesulphonyl chloride were added. This mixture was stirred at room temperature for 2 hours. Chromatography on silica gel column provided 81 mg (0.125 mmol) of the title compound. ¹H NMR (300 MHz, CDCl₃): 1.04-1.43(m, 5H); 2.11-2.15(bd, 1H); 2.40(t, 1H); 2.73(d, 3H); 3.29(d, 2H); 3.49-3.59(bd, 1H); 4.38(bd, 1H); 4.69(q, 1H); 5.89(b, 1H); 6.80(d, 1H); 7.12-7.23(m, 4H); 7.36(d, 1H); 7.55(s, 1H); 7.61-7.64(m, 1H); 8.11(s, 1H).

### EXAMPLE 15

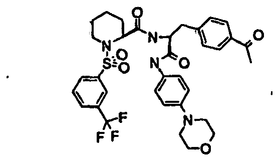

15.
   1-(3-Trifluoromethyl-benzenesulfonyl)-piperidine-2-carboxylic acid [2-(4-acetyl-phenyl)-1-(4-morpholin-4-yl-phenylcarbamoyl)-ethyl]-amide
15a)
   (S)-3-(4-Acetyl-phenyl)-2-{[(S)-1-(3-trifluoromethyl-benzenesulfonyl)-piperidine-2-carbonyl]-amino}-propionic acid
   To 0.15 g (0.24 mmol) of the title compound in 30 ml of ethyl acetate was added 0.030 g of 10 % palladium on carbon under nitrogen. The resulting suspension was stirred under 1 atmosphere of hydrogen for 4 hours, after which the catalyst was filtered and the filtrate was concentrated to provide 0.12 g (0.24 mmol) of the title compound which was used without further purification. The 300 MHz ¹H NMR was consistent with the structure. LCMS (M+H): m/z 527, retention time 3.20 min.
15b)
   (S)-3-(4-Acetyl-phenyl)-2-{[(S)-1-(3-trifluoromethyl-benzenesulfonyl)-piperidine-2-carbonyl]-amino}-propionic acid
   To 84 mg (0.16 mmol) of carboxylic acid compound 15a and 28 mg (0.16 mmol) of 4-morpholinoaniline in dichloromethane (2 ml) was added 13 mg (0.16 mmol) of sodium bicarbonate followed by 22 mg (0.16mmol) of HOAT and 31 mg (0.16 mmol) of EDCI. The mixture was stirred at room temperature for 17 hours after which it was purified by flash chromatography on silica gel eluting with 50% ethyl acetate / heptane, to provide 83 mg (0.12 mmol) of compound 15. ¹H NMR (300 MHz, CDCl₃): 1.08-1.42(m, 5H); 2.01-2.09(m, 1H); 2.54-2.59(m, 4H); 3.10-3.13(m, 4H); 3.18(dd, 1H); 3.44(dd, 1H); 3.60(bd, 1H); 3.84-3.87(m, 4H); 4.37(bs, 1H); 4.86(q, 1H); 6.85-6.90(m, 3H); 7.41(d, 4H); 7.70(t, 1H); 7.86-7.94(m, 4H); 8.01(d, 1H); 8.08(s, 1H).

### EXAMPLE 16

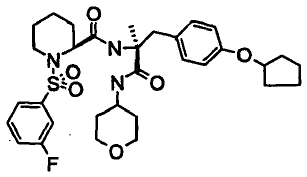

16.
   (S)-1-(3-Fluoro-benzenesulfonyl)-piperidine-2-carboxylic acid [2-(4-cyclopentyloxy-phenyl)-1-methyl-1-(tetrahydro-pyran-4-ylcarbamoyl)-ethyl]-amide
16a)
   (S)-2-(BOC-Amino-3-(4-hydroxy-phenyl)-2-methyl-N-(tetrahydro-pyran-4-yl)-propionamide To 0.50g (1.70 mmol) of a-methyl -N-BOC-tyrosine and 0.17 g (1.71 mmol) of tetrahydropyran-4-yl-amine in 6 ml of dichloromethane was added 0.14g (1.70 mmol) of sodium bicarbonate, 0.23 g (1.70 mmol) of HOAT and 0.32 g (1.70 mmol)of EDCI. This mixture was stirred at room temperature overnight. Reaction mixture was then washed with water and purified by chromatography on silica gel, eluting with 50-100%ethyl acetate/heptane to provide 0.46g (1.22 mmol) of compound 16a. LCMS (M+H): m/z 379, retention time 2.52 min.
16b)
   (S)-2-(BOC-Amino-3-(4-cyclopentyloxy-phenyl)-2-methyl-N-(tetrahydro-pyran-4-yl)-propionamide
   To a solution of 0.13g (0.34 mmol) of compound 16a in 2 ml of DMSO was added 0.33 g (1.00 mmol) of cesium carbonate followed by 0.15 g (0.34 mmol) of bromo-cyclopentane. This mixture was stirred at room temperature overnight. Reaction mixture was then diluted with 12 ml of ethyl acetate, washed twice with water, and with brine. The product was purified by chromatography on silica gel, eluting with 50 % ethyl acetate/ heptane to give 0.091 g (0.20 mmol) of compound 16b. LCMS (M+H): m/z 447, retention time 3.55 min.
16c)
   (S)-2-Amino-3-(4-cyclopentyloxy-phenyl)-2-methyl-N-(tetrahydro-pyran-4-yl)-propionamide To a solution of 0.091g (0.20 mmol) of compound 16b in 6 ml of dichloromethane was added 3 ml oftrifluoroacetic acid. The mixture was stirred at room temperature for 1 hour. The pH was adjusted to 9 with saturated sodium carbonate. The solution was extracted three times with DCM, washed with brine, dried (sodium sulfate) and concentrated to a residue, which was used without further purification. LCMS (M+H): m/z 347.
16d)
   (S)-1-(3-Fluoro-benzenesulfonyl)-piperidine-2-carboxylic acid [(S)-2-(4-cyclopentyloxy-phenyl)-1-methyl-1-(tetrahydro-pyran-4-ylcarbamoyl)-ethyl]-amide
   To a solution of 0.056g (0.19 mmol) of (S)-1-(3-fluorobenzenesulfonyl)-piperidine-2-carboxylic acid (prepared by the method employed for the synthesis of compound 1b) and 0.067 g (0.19 mmol) of compound 16c in 2 ml of dichloromethane was added 0.024g (0.19 mmol) of DMAP, 0.026 g (0.019mmol) of HOAT and 0.037 g (0.19 mmol) of EDCI. The mixture was stirred at room temperature overnight. Reaction mixture was purified by flash chromatograph on silica gel, eluting with 50-100% ethyl acetate/heptane to provide 0.040g (0.065 mmol) of the title compound. ¹H NMR (300 MHz, CDCl₃):1.00-2:00(m, 20H); 2.17(bd, 1H); 2.81(t, 1H); 3.13(q, 2H); 3.46-3.52(m, 2H); 3.69(bd, 1H); 3.85-4.05(m, 3H); 4.29(b, 1H); 4.73(b, 1H); 6.82(d, 1H); 7.05(d, 2H); 6.95-7.06(m, 3H); 7.09-7.34(m, 1H); 7.48-7.62(m, 3H).

MS method: LCMS. Ionization type: ESI. HPLC condition: (A) 100% Water + 0.1% TFA (B) 100% Acetonitrile + 0.1 TFA. Gradient composition: 5% (B) to 100% (B) in 5 min, 1 ml / min. Hypersil C18 column (4.6 x 50 mm).

### EXAMPLE 17

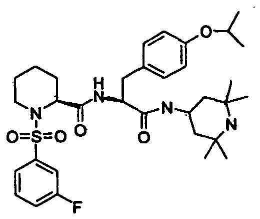

17.
   (S)-1-(3-Fluoro-benzenesulfonyl)-(2S)-piperidine-2-carboxylic acid [(S)-2-(4-isopropoxyphenyl)-1-(2,2,6,6-tetramethyl-piperidin-4-ylcarbamoyl)-ethyl]-amide This may be prepared by the general method of Scheme 1 above.
17a)
   (S)-1-(3-(Fluoro)benzenesulfonyl)-piperidine-2-carboxylic acid benzyl ester
   To a mixture of (S)-piperidine-2-carboxylic acid benzyl ester hydrochloride (5.00 g, 19.5 mmol) and triethylamine (5.50 mL, 39.1 mmol) in dichloromethane (150 mL) was added 3-fluorobenzenesulfonyl chloride (3.80 g, 19.5 mmol). The mixture was stirred at room temperature for 5 h as it turned to a white suspension. The suspension was washed twice with distilled water. The organic layer was then dried over magnesium sulfate and concentrated to afford 9 g of crude oil. Flash column chromatography on silica gel (ethyl acetate/n-heptane) provided 6.51 g (88.3 %) of the title compound as a yellow oil.
17b)
   (S)-(1-(3-(Fluoro)-benzenesulfonyl)-piperidine-2-carboxylic acid
   A mixture of (S)-1-(3-fluoro-benzenesulfonyl)-piperidine-2-carboxylic acid benzylester(6.51 g, 17.3 mmol), ethanol (200 mL) and 10 % Pd on carbon (1.83 g, 1.70 mmol) was hydrogenated at 30 psi for 3 h. The suspension was then filtered through celite and washed with ethyl acetate. The filtrate was concentrated under reduced pressure. The resulting oil was chromatographed on silica gel (dichloromethane/ n-heptane, 9/1) to afford 4.10 g (82.9%) of the title compound.
17c)
   (S)-2-{1-(3-Fluoro-benzenesulfonyl)-piperidine-2-carbonyl]-amino}-3-(4-hydroxy-phenyl)-propionic acid benzyl ester.
   (S)-1-(3)-(Fluoro)-benzenesulfonyl)-piperidine-2-carboxylic acid (500 mg, 1.70 mmol), (S)-2-amino-3-(4-hydroxy-phenyl)-propionic acid benzyl ester (567 mg, 2.10 mmol), 1-hydroxybenzotriazole (330 mg, 2.40 mmol) and 1-[3-(dimethylamino)propyl]-3-ethylcarbodiimide hydrochloride (434 mg, 2.30 mmol) were added to dichloromethane (10 mL). The mixture was stirred overnight and then washed with a saturated aqueous sodium bicarbonate solution. The organic layer was dried over magnesium sulfate and concentrated. The resulting oil was chromatographed on silica gel (methanol/dichloromethane, 5/95) to provide 0.735 mg (78.2 %) of the title compound.
17d)
   (S)-2-{(S)-[1-(3-Fluoro-benzenesulfonyl)-piperidine-2-carbonyl]-amino}-3-(4-isopropoxyphenyl)-propionic acid benzyl ester
   Isopropyl iodide (0.62 mL, 6.2 mmol) was added to a mixture of (S)-2-{[1-(3-(fluoro)-benzenesulfonyl)-piperidine-2-carbonyl]-amino}-3-(4-hydroxy-phenyl)-propionic acid benzyl ester (700 mg, 1.3 mmol) and cesium carbonate (2.4 g, 7.4 mmol) in dry DMF (5 mL). The suspension was stirred at room temperature for 4 h. The reaction mixture was then washed with water, dried over magnesium sulfate and concentrated under reduced pressure. The resulting oil was chromatographed (ethyl acetate/ n-heptane) on silica gel to produce 620 mg (82.1 %) of the title compound.
17e)
   (S)-2-{[1-(3-Fluoro-benzenesulfonyl)-piperidine-2-carbonyl]-amino}-3-(4-isopropoxyphenyl)-propionic acid.
   A mixture of (S)-2-{[(S)1-(3-fluoro-benzenesulfonyl)-piperidine-2-carbonyl]-amino}-3-(4-isopropoxy-phenyl)-propionic acid benzyl ester (0.59 g, 1.00 mmol), ethanol (20 mL) and 10 % Pd on carbon (108mg, 0.1mmol) was hydrogenated at 30 psi for 2 h. The catalyst was filtered through Celite^{®}. The filtrate was concentrated to afford 410 mg (83.0 %) of the title compound.
17f)
   (S)-1-(3-Fluoro-benzenesulfonyl)-piperidine-2-carboxylic acid [(S)-2-(4-isopropoxy-phenyl)-1-(2,2,6,6-tetramethyl-piperidin-4-yl-carbamoyl)-ethyl]-amide
   (S)-2-{[(S)-1-(3-Fluoro-benzenesulfonyl)-piperidine-2-carbonyl]-amino}-3-(4-isopropoxyphenyl)-propionic acid (60 mg, 0.12 mmol), 4-amino-2,2,6,6-tetramethylpiperidine (25 mg, 0.16 mmol), TBTU (51 mg, 1.60 mmol) and N-methyl imidazole (13 mL, 0.16 mmol) were mixed in anhydrous THF (2mL). The mixture was stirred overnight at room temperature. A saturated aqueous sodium bicarbonate solution was added and the mixture was extracted with ethyl acetate. The organic layer was dried over magnesium sulfate and concentrated under reduced pressure. The resulting oil was chromatographed (dichloromethane/ methanol, 95/ 5) on silica gel to afford 48 mg (63 %) of the title compound as a clear glass. MS: m/e 631.3 (M+H). 1H NMR (300 MHz ,d6-DMSO): δ 0.70-1.60 (m, 28 H), 1.90 (d, 1 H, J= 12 Hz), 2.75 (m, 2 H), 3.20 (m, 1 H), 3.60 (d, 1 H, J = 12 Hz), 3.90 (m, 1 H), 4.25 (dd, 1 H, J1 = 9 Hz, J2 = 6 Hz), 4.50 (m, 2 H), 6.80 (d, 2 H, J =12 Hz), 7.10 (d, 2 H, J = 12 Hz), 7.46 (m, 4 H), 7.65 (d, 1 H, J = 12 Hz), 7.90 (d, 1 H, J = 12 Hz).

### EXAMPLE 18

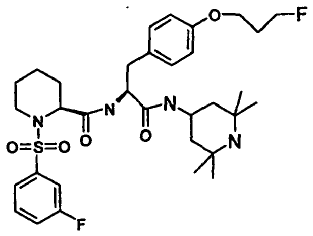

18.
   (S)-1-(3-Fluoro-benzenesulfonyl)-piperidine-2-carboxylic acid [(S)-2-[4-(3-fluoro-propoxy)-phenyl]-1-(2,2,6,6-tetramethyl-piperidin-4-ylcarbamoyl)-ethyl]-amide
18a)
   (S)-2-{[(S)-1-(3-Fluoro-benzenesulfonyl)-piperidine-2-carbonyl]-amino}-3-[4-(3-fluoro-propoxy)-phenyl]-propionic acid benzyl ester.
   3-Bromo-1-fluoro propane (1.6 g, 11 mmol) was added to a mixture of (S)-2-{[(S)-1-(3-fluoro-benzenesulfonyl)-piperidine-2-carbonyl]-amino}-3-(4-hydroxy-phenyl)-propionic acid benzyl ester (compound 17c)(2.0 g, 3.7mmol) and cesium carbonate (3.6 g, 11 mmol) in DMF (60 mL). The suspension was stirred overnight at room temperature. The reaction mixture was diluted in ethyl acetate and washed with water, dried over magnesium sulfate and concentrated. The resulting oil was chromatographed (dichloromethane/ methanol, 9/ 1) on silica gel to produce 1.89 g (85.1%) of the title compound.
18b)
   (S)-2-{[(S)-1-(3-Fluoro-benzenesulfonyl)-piperidine-2-carbonyl]-amino}-3-,[4-(3-fluoro-propoxy)-phenyl]-propionic acid
   To a mixture of (S)-2-{[(S)-1-(3-fluoro-benzenesulfonyl)-piperidine-2-carbonyl]-amino}-3-[4-(3-fluoro-propoxy)-phenyl]-propionic acid benzyl ester (1.9 g, 3.1 mmol)(compound 18a), ethanol (50 mL), and 10 % Pd on carbon was hydrogenated at 30 psi for 3 h. The suspension was filtered through Celite^{®}, washed with dichloromethane and the filtrate concentrated. The resulting oil was chromatographed (dichloromethane/methanol, 9/ 1) on silica gel to provide 1.04 g (64.7 %) of the title compound as a white powder.
18c)
   (S)-(1-(3-Fluoro-benzenesulfonyl)-piperidine-2-carboxylic acid [(S)-2-[4-(3-fluoro-propoxy)-phenyl]-1-(2,2,6,6-tetramethyl-piperidin-4-yl-carbamoyl)-ethyl]-amide
   (S)-2-{[(S)-1-(3-Fluoro-benzenesulfonyl)-piperidine-2-carbonyl]-amino}-3-[4-(3-fluoro-propoxy)-phenyl]-propionic acid (80 mg, 0.16 mmol), 4-amino-2,2,6,6-tetramethylpiperidine (25 mg, 0.16 mmol), 1-hydroxybenzotriazole (30 mg, 0.22 mmol) and 1-[3-(dimethylamino)propyl]-3-ethylcarbodiimide hydrochloride (39 mg, 0.20 mmol) were mixed in dichloromethane (2mL). The mixture was stirred overnight at room temperature. The reaction mixture was diluted with dichloromethane and washed twice with a saturated aqueous sodium bicarbonate solution, and the organic fraction dried over magnesium sulfate. After filtration and subsequent concentration, the resulting oil was chromatographed (dichloromethane/ methanol, 9/1) on silica gel to produce 27 mg (27 %) of the title compound. MS: m/e 649.33 (M+H). ¹H NMR (300 MHz, d6-DMSO): δ 0.70-2.20 (m, 27 H), 2.80 (m, 2 H), 3.20 (m, 1 H), 3.65 (d, 1 H, J = 12 Hz), 4.00 (m, 3 H), 4.30 (q, 1 H, J = 6Hz), 4.50 (m, 2 H), 4.65 (t, 1 H, J = 6 Hz), 6.90 (d, 2 H, J = 9 Hz), 7.15 (d, 2 H, J = 9 Hz), 7.50 (m, 4 H), 8.00 (m, 2 H).

### EXAMPLE 19

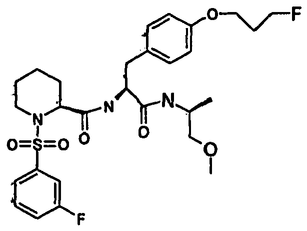

19.
   (S)-1-(3-Fluoro-benzenesulfonyl)-piperidine-2-carboxylic acid [(S)-2-[4-(3-fluoro-propoxy)-phenyl]-1-((S)-2-methoxy-1-methyl-ethylcarbamoyl)-ethyl]-amide (S)-2-{[1-(3-Fluoro-benzenesulfonyl)-piperidine-2-carbonyl]-amino}-3-[4-(3-fluoro-propoxy)-phenyl]-propionic acid (compound 18b) (80 mg, 0.16 mmol), (S)-1-methoxy-2-propylamine (14 mg, 0.16 mmol), 1-hydroxybenzotriazole (30 mg, 0.22 mmol) and 1-[3-(dimethylamino)propyl]-3-ethylcarbodiimide hydrochloride (39 mg, 0.20 mmol) were mixed in dichloromethane (2 mL). The mixture was stirred overnight at room temperature. The reaction mixture was diluted with dichloromethane, washed twice with a saturated aqueous sodium bicarbonate solution, and the organic layer was dried over magnesium sulfate. After manual filtration and subsequent concentration the resulting oil was chromatographed on silica gel (dichloromethane/ methanol, 9/ 1) on silica gel to provide 66 mg (72 %) of the title compound. MS: m/e 582.2 (M+H). ¹H NMR (300 MHz, d6-DMSO): δ 0.75-1.60 (m, 9 H), 1.85-2.15 (m, 3 H), 2.75 (m, 1 H), 2.85 (m, 1 H), 3.10 (m, 1 H), 3.20 (m, 1 H), 3.60 (d, 1 H, J = 12 Hz), 3.85 (m, 1 H), 4.00 (t, 2 H, J = 6 Hz), 4.30 (q, 1 H J = 7.5 Hz), 4.50 (m, 2 H), 4.65 (t, 1 H, J = 6 Hz), 6.85 (d, 2 H, J = 9 Hz), 7.15 (d, 2 H, J = 9 Hz), 7.40 (m, 4 H), 7.75 (d, 1 H, J = 9 Hz), 7.90 (d, 1 H, J = 6 Hz).

### EXAMPLE 20

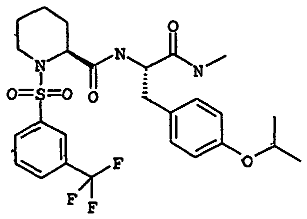

20.
   (S)-1-(3-Trifluoromethyl-benzenesulfonyl)-piperidine-2-carboxylic acid [(S)-2-(4-isopropoxyphenyl)-1-methylcarbamoyl-ethyl]-amide
   To a room temperature solution of (S)-3-(4-isopropoxy-phenyl)-2-{[(S)-1-(3-trifluoromethylbenzenesulfonyl)-piperidine-2-carbonyl]-amino}-propionic acid (319 mg, 0.588 mmol) (prepared according to the method described for the preparation of compound 17e), PyBroP (301 mg, 0.645 mmol), and methylene chloride (8 mL) was added methylamine (3.0 mL of a 2M THF solution) in one portion. The reaction mixture was shaken at room temperature in a sealed vessel for 2.5 h. The reaction mixture was then diluted with methylene chloride, washed with 0.5 N HCl and water successively, dried over MgSO₄, filtered, and evaporated. The residue was chromatographed on silica gel eluting with ethyl acetate. Homogenous fractions with Rf = 0.32 (silica gel, ethyl acetate) material were combined and concentrated to afford 197 mg (60%) of the title compound as a white foam. LC/MS: RT 3.38 min, Area% 100, m/e 556 (M + H). ¹H NMR (400 MHz, d6-DMSO) δ 8.01 d (J=8 Hz; Tyr NH), 7.99 brd (J=7.5 Hz; H-6 of ArSO2), 7.94 brs (H-2 of ArSO2), 7.84 q (J=4.5 Hz; CONHMe), 7.72 brd (J=8 Hz; H-4 of ArSO2), 7.59 dd (J=8, 7.5 Hz; H-5 of ArSO2), 7.11 m (2H, AA'-part of AA'MM'; H-2/H-6 of C6H4), 6.82 m (2H, MM'-part of AA'MM; H-3/H-5 of C6H4), 4.56 brd (J=5 Hz; Pip H-2), 4.53 qq (J=6 Hz, 6; OiPr), 4.21 ddd (J=9, 8, 5.5 Hz; Tyr Ha), 3.65 dm (J=12.5; Pip H-6eq), 3.17 ddd (J=13, 12.5, 3; Pip H-6ax), 2.84 dd (J=13.5, 5.5; Tyr Hb), 2.66 dd (J=13.5, 9; Tyr H'b), 2.53 d (3H, J=4.5; CONHMe), 1.95 brd (J=13.5; Pip H-3eq), 1.49 dm (J=12.5 Hz; Pip H-5eq), 1.40 m (Pip H-4eq), 1.36 m (Pip H-3ax), 1.21 d (3H, J=6 Hz; OiPr), 1.19 d (3H, J=6 Hz; OiPr), 1.16 m (Pip H-5ax), 1.06 m (Pip H-4ax).

### EXAMPLE 21

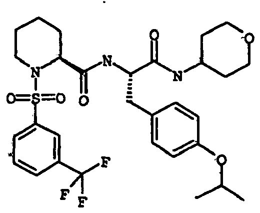

21.
   (S)-1-(3-Trifluoromethyl-benzenesulfonyl)-piperidine-2-carboxylic acid[(S)-2-(4-isopropoxyphenyl)-1-(tetrahydro-pyran-4-ylcarbamoyl)-ethyl]-amide
   To a room temperature mixture of (S)-3-(4-isopropoxy-phenyl)-2-{[(S)-1-(3-trifluoromethylbenzenesulfonyl)-piperidine-2-carbonyl]-amino}-propionic acid (96.0 mg, 0.177 mmol) (prepared according to the method described for the preparation of compound 17e), HOBT (29.0 mg, 0.215 mmol), 4-aminotetrahydropyran (19.6 mg, 0.194 mmol), methylene chloride (2 mL), and water (2 mL) was added EDCI (39.0 mg, 0.203 mmol) in one portion. The reaction mixture was stirred at room temperature overnight The reaction was diluted with methylene chloride, washed with water, dried over MgSO₄, filtered, and concentrated. The residue was chromatographed on silica gel eluting with ethyl acetate to afford 77.1 mg (69%) of the title compound as an amorphous white solid. mp: 78.6 °C. Rf = 0.40 (silica gel, ethyl acetate).
   LC/MS: RT 3.70 min, Area% 100, m/e 626 (M + H). ¹H NMR (300 MHz, d6-DMSO) δ 8.01 (d, J=8.2 Hz, 1H), 7.99 (brd, J=7.8 Hz, 1H), 7.94 (brs, 1H), 7.84 (d, J=7.8 Hz, 1H), 7.76 (d, J=8 Hz, 1H), 7.61 (dd, J=7.9, 7.9 Hz, 1H), 7.11 m (2H), 6.82 m (2H), 4.58 - 4.30 (m, 2H), 4.28 - 4.20 (m, 1H), 3.79 - 3.65 (m, 4H), 3.32 - 3.20 (m, 4H), 2.84 -2.64 (m, 2H), 1.93 brd (J=12.7, 1H), 1.62 - 1.00 (m, 14H).

### EXAMPLE 22

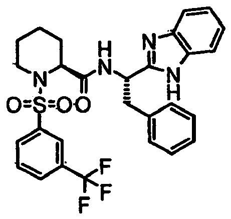

22.
   (S)-1-(3-Trifluoromethyl-benzenesulfonyl)-piperidine-2-carboxylic acid [(S)-2-(phenyl)-1-(benzimidazol-2-yl)-ethyl]-amide
   This may be prepared according to Scheme 4 above.
22a)
   [(S)-1-(Methoxy-methyl-carbamoyl)-2-phenyl-ethyl]-carbamic acid benzyl ester Isobutyl chloroformate (0.44 mL, 3.3 mmol) and N-methylmorpholine (0.73 mL, 6.7 mmol) were added to a -15 °C solution of N-carbobenzyloxy-S-phenylalanine (1.0 g, 3.3 mmol) in CH₂Cl₂ (15 mL). After stirring at -15 °C for 20 min, N,O-dimethylhydroxylamine hydrochloride (0.34 g, 3.5 mmoles) was added, and the solution was stirred for 16 h, during which time the reaction mixture warmed to room temperature. The mixture was diluted with EtOAc (200 mL), washed with saturated aqueous NaHCO₃ (200 mL) and brine (200 mL). The organic layer was dried (MgSO₄) and concentrated in vacuo to give a colorless oil. Purification of the residue by flash chromatography on silica gel eluting with 10-30% EtOAc/heptane afforded 1.0 g (90% yield) of the title compound as a colorless oil.
22b)
   ((S)-1-Benzyl-2-oxo-ethyl)-carbamic acid benzyl ester
   Lithium aluminum hydride (0.95 mL of a 1.0 M solution in THF, 0.95 mmoles) was added to a room temperature solution of [(S)-1-(Methoxy-methyl-carbamoyl)-2-phenyl-ethyl]-carbamic acid benzyl ester (0.30 g, 0.86 mmoles) in THF (10 mL). The resulting mixture was stirred at room temperature for 2 h, then was quenched by dilution with EtOAc (200 mL), and washed with 1% aqueous HCl (200 mL). The organic layer was dried (MgSO₄) and concentrated in vacuo. The residue was repeatedly diluted with CH₃CN (2x200 mL) and concentrated in vacuo to give 0.24 g (approximately quantitative yield) of ((S)-1-Benzyl-2-oxo-ethyl)-carbamic acid benzyl ester as a colorless oil, which was carried on without further purification: ES+ LC/MS m/e 284 (M+H), RT = 4.75 min.
22c)
   {(S)-1-Benzyl-2-[(E)-2-methylamino-phenylimino]-ethyl}-carbamic acid benzyl ester 2,3-Dichloro-5,6-dicyano-1,4-benzoquinone (DDQ, 0.20 g, 0.86 mmoles) was added to a room temperature solution of ((S)-1-Benzyl-2-oxo-ethyl)-carbamic acid benzyl ester (0.24 g, 0.86 mmoles) and 1,2-phenylenediamine (0.093 g, 0.86 mmoles) in CH₃CN (10 mL). The reaction mixture was stirred at room temperature for 16 h, and then concentrated in vacuo. The residue was re-dissolved in EtOAc (150 mL), and washed with saturated aqueous NaHCO₃ (3x125 mL). The organic layer was dried (MgSO₄) and concentrated in vacuo to give a brown oily solid. Purification of the residue by flash chromatography on silica gel eluting with 10-40% EtOAc/heptane afforded 0.091 g (28% yield) of {(S)-1-Benzyl-2-[(E)-2-methylamino-phenylimino]-ethyl}-carbamic acid benzyl ester as a colorless oil: ES+ LC/MS m/e 372 (M+H), RT = 3.03 min; ¹H NMR (CDCl₃) δ 9.90 (bd s, 1), 7.68 (bd s, 1), 7.21-7.33 (m, 11), 7.16 (d, 2, J=6.7 Hz), 5.64 (d, 1, J=7.2 Hz), 5.15 (dd, 1, J=15.2, 7.5 Hz), 5.07 (s, 2), 3.45 (d, 2, J=7.5 Hz).
22d)
   N-[(S)-2-Amino-3-phenyl-prop-(E)-ylidene]-N'-methyl-benzene-1,2-diamine {(S)-1-Benzyl-2-[(E)-2-methylamino-phenylimino]-ethyl}-carbamic acid benzyl ester (0.075 g, 0.20 mmol) was dissolved in absolute ethanol (7 mL) and treated with 10% palladium on carbon (0.040 g). The resulting suspension was shaken under 60-65 psi H₂ at room temperature for 16 h. The suspension was filtered and the filter cake washed with MeOH (~100 mL), and the combined filtrates concentrated in vacuo to give N-[(S)-2-Amino-3-phenyl-prop-(E)-ylidene]-N'-methyl-benzene-1,2-diamine, as a light yellow oil (0.052 g, approximately quantitative yield): ES+ LC/MS m/e 238 (M+H), RT = 3.12 min.
22e)
   (S)-1-(3-Trifluoromethyl-benzenesulfonyl)-piperidine-2-carboxylic acid {(S)-1-benzyl-2-[(E)-2-methylamino-phenylimino]-ethyl}-amide
   PyBOP (0.11 g, 0.22 mmoles) and triethylamine (0.076 mL, 0.55 mmoles) were added to a room temperature solution of (S)-1-(3-trifluoromethyl-benzenesulfonyl)-piperidine-2-carboxylic acid (0.074 g, 0.22 mmol) in CH₂Cl₂ (5 mL). After stirring at room temperature for 20 min, a solution of N-[(S)-2-amino-3-phenyl-prop-(E)-ylidene]-N'-methyl-benzene-1,2-diamine (0.052 g, 0.22 mmoles) in CH₂Cl₂ (5 mL) was added, and the resulting mixture was stirred at room temperature for 16 h. The reaction mixture was diluted with CH₂Cl₂ (100 mL) and washed with saturated aqueous NaHCO₃ (100 mL) and brine (100 mL). The organic layer was dried (MgSO₄) and concentrated in vacuo to give a yellow oil. Purification of the residue by flash chromatography on silica gel, eluting with 10-50% EtOAc/heptane afforded 0.078 g (64%) of the title compound as a colorless powder: ES+ LC/MS m/e 557 (M+H), RT = 2.92 min,; ¹H NMR CDCl₃: δ 9.99 (bd s, 1), 8.05 (s, 1), 7.94 (d, 1, J=8.0 Hz), 7.83 (d, 1, J=7.9 Hz), 7.62 (t, 1, J=7.9 Hz), 7.23-7.56 (m, 8), 7.00 (d, 1, J=7.5 Hz), 5.45 (m, 1), 4.40 (bd s, 1), 3.72 (dd, 1, J=14.5, 5.1 Hz), 3.54 (d, 1, J=14.1 Hz), 3.44 (dd, 1, J=14.5, 10.8 Hz), 2.13-2.26 (m, 2), 1.37 (m, 1), 1.22 (m, 1), 0.92-1.10 (m, 4).

### EXAMPLE 23

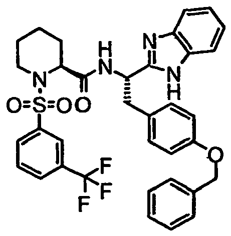

23.
   (S)-1-(3-Trifluoromethyl-benzenesulfonyl)-piperidine-2-carboxylic acid [(S)-1-(1H-benzimidazol-2-yl)-2-(4-benzyloxy-phenyl)-ethyl]-amide

This may be prepared according to Scheme 5 above.
23a)
   [(S)-1-(1H-Benzimidazol-2-yl)-2-(4-benzyloxy-phenyl)-ethyl]-carbamic acid tert-butyl ester 2-Ethoxy-1-ethoxycarbonyl-1,2,-dihydroquinoline (EEDQ, 0.17 g, 0.68 mmol) was added to a room temperature solution of (S)-3-(4-benzyloxy-phenyl)-2-tert-butoxycarbonylamino-propionic acid (0.25 g, 0.68 mmol) in DMF (3 mL). After stirring at room temperature for 20 min, a solution of 1,2-phenylenediamine (0.049 g, 0.45 mmol) in DMF (2 mL) was added, and the mixture heated at 120-140 °C for 3 days. The reaction mixture was diluted with EtOAc (100 mL) and washed with saturated NaHCO₃ (2x100 mL) and brine (100 mL). The organic layer was dried (MgSO₄) and concentrated in vacuo to give 0.20 g of a brown oil. Purification of the residue by flash chromatography on silica gel eluting with 10-50% EtOAc/heptane afforded 0.083 g (42% yield) of the title compound as a colorless powder: ES+ LC/MS m/e 444 (M+H), RT = 2.89 min; ¹H NMR (CDCl₃) δ 10.03 (bd s, 1), 7.73 (bd s, 1), 7.30-7.42 (m, 6), 7.24 (m, 2), 7.06 (d, 1, J=9.5 Hz), 6.83 (dd, 1, J=8.8, 1.0 Hz), 5.42 (bd s, 1), 5.07 (dd, 1, J=12.9, 7.9 Hz), 4.97 (s, 2), 3.32 (d, 2, J=7.3 Hz), 1.41 (s, 9).
23b)
   (S)-1-(3-tert-Butyl-benzenesulfonyl)-piperidine-2-carboxylic acid [(S)-1-(1H-benzimidazol-2-yl)-2-(4-benzyloxy-phenyl)-ethyl]-amide
   Trifluoroacetic acid (0.063 mL, 0.82 mmol) was added to a room temperature solution of [(S)-1-(1H-Benzimidazol-2-yl)-2-(4-benzyloxy-phenyl)-ethyl]-carbamic acid tert-butyl ester (0.073 g, 0.16 mmol) in CH₂Cl₂ (10 mL). The resulting solution was stirred at room temperature for 2 hours, and then concentrated in vacuo to give (S)-1-(1H-Benzimidazol-2-yl)-2-(4-benzyloxy-phenyl)-ethylammonium trifluoro-acetate as a red-brown oil. This material was re-suspended in CH₂Cl₂ (4 mL) and treated with a room temperature solution of PyBOP (0.086 g, 0.16 mmoles), triethylamine (0.080 mL, 0.58 mmol), and (S)-1-(3-Trifluoromethyl-benzenesulfonyl)-piperidine-2-carboxylic acid (0.056 g, 0.16 mmol) in CH₂Cl₂ (4 mL). The resulting mixture was stirred at room temperature for 16 h. Purification of the product by flash chromatography on silica gel eluting with 10-50% EtOAc/heptane afforded 0.075 g (69%) of the title compound as a colorless powder: ES+ LC/MS m/e 663 (M+H), 100% purity; ¹H NMR (CDCl₃) ~1:1 mixture of diastereomers δ 10.07 (bd s, 1), 8.07 (s, 1), 7.95 (d, 1, J=7.8 Hz), 7.81 (m, 1), 7.60 (m, 1), 7.24-7.42 (m, 9), 7.10 (d, 2, J=8.6 Hz), 6.88 (d, 2, J=8.6 Hz), 5.25-5.42 (m, 1), 5.04 (s, 2), 4.48 (bd s, 1), 3.32-3.84 (m, 3), 2.88 (m, 1), 1.05-1.77 (m, 6).

### EXAMPLE 24

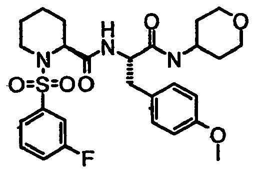

24.
   (S)-1-(3-Fluoro-benzenesulfonyl)-piperidine-2-carboxylic acid [(S)-2-(4-methoxy-phenyl)-1-(tetrahydro-pyran-4-ylcarbamoyl)-ethyl]-amide
24a)
   (S)-2-[(S)-1-tert-Butoxycarbonyl-2-(4-hydroxy-phenyl)-ethylcarbamoyl]-pjperidine-1-carboxylic acid benzyl ester
   2-1H-Benzotriazol-1-yl-1,1,3,3-tetramethyluronium tetrafluoroborate (TBTU, 4.15.g, 12.9 mmoles) was added to a room temperature solution of (S)-2-amino-3-(4-hydroxy-phenyl)-propionic acid tert-butyl ester (1.70 g, 7.17 mmoles) and 1-methylimidazole (2.60 mL, 32.7 mmoles) in 1,2-DCE (15 mL). After stirring at room temperature for 20 min, a solution of (S)-piperidine-1,2-dicarboxylic acid 1-benzyl ester (1.70 g, 6.46 mmoles) in 1,2-DCE (15 mL) was added, and the resulting mixture was stirred at room temperature for 48 h. The reaction mixture was diluted with EtOAc (200 mL) and washed with brine (3x200 mL). The aqueous layers were re-extracted with EtOAc (2x200 mL). The organic layers were dried (Na₂SO₄), combined, and concentrated in vacuo to give 4.21 g of an oil. Purification of the residue by flash chromatography on silica gel, eluting with 0-20% EtOAc/CH₂Cl₂ afforded 3.02 g (97% yield) of the title compound as a light yellow oil.
24b)
   (S)-2-[(S)-1-tert-Butoxycarbonyl-2-(4-methoxy-phenyl)-ethylcarbamoyl]-piperidine-1-carboxylic acid benzyl ester
   Methyl iodide (4.00 mL, 64.2 mmoles) was added to a room temperature solution of (S)-2-[(S)-1-tert-Butoxycarbonyl-2-(4-hydroxy-phenyl)-ethylcarbamoyl]-piperidine-1-carboxylic acid benzyl ester (1.39 g, 2.88 mmoles) and cesium carbonate (1.87 g, 5.75 mmoles) in DMF (10 mL) and the mixture stirred at room temperature for 4 days. The reaction mixture was diluted with EtOAc (200 mL) and washed with brine (3x200 mL). The aqueous layers were re-extracted with EtOAc (2x200 mL). The organic layers were dried (Na₂SO₄), combined, and concentrated in vacuo to give a yellow oil. Purification of the residue by flash chromatography, eluting with 0-10% EtOAc/CH₂Cl₂ afforded 0.71 g (50% yield) of (S)-2-[(S)-1-tert-Butoxycarbonyl-2-(4-methoxy-phenyl)-ethylcarbamoyl]-piperidine-1-carboxylic acid benzyl ester, as a colorless oil.
24c)
   (S)-3-(4-Methoxy-phenyl)-2-[((S)-piperidine-2-carbonyl)-amino]-propionic acid tert-butyl ester
   (S)-2-[(S)-1-tert-Butoxycarbonyl-2-(4-methoxy-phenyl)-ethylcarbmoyl]-piperidine-1-carboxylic acid benzyl ester (0.85 g, 1.7 mmoles) was dissolved in absolute ethanol (60 mL) and treated with 10% palladium on carbon (0.85 g) under a nitrogen atmosphere. The resulting suspension was shaken under 65-70 psi H2 at room temperature for 18 h after which it was filtered and the filter cake washed with EtOH (~200 mL), and concentrated in vacuo to give 0.51 g of a yellow oil. Purification of the residue by flash chromatography on silica gel eluting with 0-5% MeOH/CH₂Cl₂ afforded 0.26 g (42% yield) of (S)-3-(4-Methoxy-phenyl)-2-[((S)-piperidine-2-carbonyl)-amino]-propionic acid tert-butyl ester as a colorless oil: ES+ LC/MS m/e 363 (M+H), RT = 2.33 min; SiO2 TLC (5% MeOH/CH₂Cl₂) Rf=0.28.
24d)
   (S)-2-{[(S)-1-(3-Fluoro-benzenesulfonyl)-piperidine-2-carbonyl]-amino}-3-(4-methoxyphenyl)-propionic acid tert-butyl ester
   3-Fluorobenzene sulfonylchloride (0.092 g, 0.47 mmoles) was added to a room temperature solution of (S)-3-(4-Methoxy-phenyl)-2-[((S)-piperidine-2-carbonyl)-amino]-propionic acid tert-butyl ester (0.13 g, 0.36 mmoles) and triethylamine (0.25 mL, 1.8 mmoles) in 1,2-DCE (3 mL) and the resulting solution heated at 50 °C for 18 h. The reaction mixture was cooled to room temperature, diluted with brine (100 mL), and extracted with CH₂Cl₂ (3x100mL). The organic layers were dried (Na₂SO₄), combined, and concentrated in vacuo to give 0.20 g of a yellow oil. Purification of the residue by flash chromatography on silica gel eluting with 1-10% EtOAc/CH₂Cl₂ afforded 0.17 g (92% yield) of (S)-2-{[(S)-1-(3-Fluoro-benzenesulfonyl)-piperidine-2-carbonyl]-amino}-3-(4-methoxy-phenyl)-propionic acid tert-butyl ester, as a colorless oil: ES+ LC/MS m/e 465 (M-tBu), RT = 3.73 min.
24e)
   (S)-2-{[(S)-1-(3-Fluoro-benzenesulfonyl)-piperidine-2-carbonyl]-amino}-3-(4-methoxyphenyl)-propionic acid
   Trifluoroacetic acid (10 mL, 130 mmoles) was added to a room temperature solution of (S)-2-{[(S)-1-(3-Fluoro-benzenesulfonyl)-piperidine-2-carbonyl]-amino}-3-(4-methoxy-phenyl)-propionic acid tert-butyl ester (0.150 g, 0.288 mmoles) in CH₂Cl₂ (2 mL). The reaction mixture was stirred at room temperature for 3 h after which it was concentrated in vacuo to give 0.141 g of a red-brown oil. Purification of the residue by flash chromatography with 0-30% MeOH/CH₂Cl₂ afforded 0.115 g (86%) of (S)-2-{[(S)-1-(3-Fluoro-benzenesulfonyl)-piperidine-2-carbonyl]-amino}-3-(4-methoxy-phenyl)-propionic acid as a colorless powder: ES+ LC/MS m/e 456 (M+H), 419 (M-CO2H) RT = 3.08 min.
24f)
   (S)-1-(3-Fluoro-benzenesulfonyl)-piperidine-2-carboxylic acid [(S)-2-(4-methoxy-phenyl)-1-(tetrahydro-pyran-4-ylcarbamoyl)-ethyl]-amide
   2-1H-Benzotriazol-1-yl-1,1,3,3-tetramethyluronium tetrafluoroborate (TBTU, 0.072 g, 0.22 mmoles) was added to a room temperature solution of (S)-2-{[(S)-1-(3-Fluoro-benzenesulfonyl)-piperidine-2-carbonyl]-amino}-3-(4-methoxy-phenyl)-propionic acid (0.050 g, 0.108 mmoles) and 1-methylimidazole (0.050 mL, 0.63 mmoles) in 1,2-DCE (3 mL). After stirring at room temperature for 20 min, a solution of 4-aminotetrahydro-pyran (0.022 g, 0.218 mmoles) in CH₂Cl₂ (2 mL) was added. The resulting solution was stirred at room temperature for 48 hours. The reaction mixture was diluted with EtOAc (200 mL) and washed with brine (3x200 mL). The aqueous fraction was re-extracted with EtOAc (2x200 mL). The organic extract was dried (Na₂SO₄) and concentrated in vacuo to give 0.25 g of an off-white powder. Purification of the residue by flash chromatography on silica gel eluting with 1-10% MeOH/CH₂Cl₂ afforded 0.046 g (78%) of the title compound as a colorless oil: ES+ LC/MS m/e 548 (M+H), RT = 3.39 min, 90% purity; ¹H NMR (CDCl₃) δ 7.51-7.68 (m, 3), 7.30-7.37 (m, 1), 7.16 (d, 2, J=8.7Hz), 6.81-7.13 (m, 3), 6.04 (d, 1, J=7.5 Hz), 4.43 (dd, 1, J=8.0, 1.7 Hz), 4.36 (m, 1), 3.83-4.04 (m, 2), 3.80 (s, 3), 3.65 (d, 1, J=14.1 Hz), 3.40-3.52 (m, 2), 3.19 (dd, 1, J=14.1, 6.4 Hz), 2.94-3.08 (m, 1), 2.64-2.74 (m, 1), 2.07-2.22 (m, 1), 1.80-1.86 (m, 2), 1.08-1.52 (m, 8); SiO₂ TLC (5% MeOH/CH₂Cl₂) Rf= 0.35.

### EXAMPLE 25

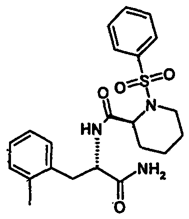

25.
   (S)-1-Benzenesulfonyl-piperidine-2-carboxylic acid ((S)-1-amido-2-o-tolyl-ethyl)-amide

This may be prepared according to Method A, shown herein above.

A Jones tube was charged with Rink amide resin (1.0 g, 1.0 mmol/g), and the resin amine was liberated by shaking with 20 % piperidine/DMF (2 mL) for ½ h. The resin was filtered, washed with DMF (5x), then re-exposed to the deprotecting solution as above. Following filtration and washing [DMF (5x), MeOH (2x), DMF (5x)], DMF was added to swell the resin followed by Fmoc-o-Me-Phe-OH (1.6 g), diisopropylcarbodiimide (0.47 mL), HOBt (0.46 g), HBTU (1.1 g), and N,N-diisopropylethylamine (1.1 mL). The tube was capped, and the reaction mixture was shaken overnight. The reagents were filtered, and the resin was washed twice with the following sequence of solvents: [DMF (4x), DMF/H₂O (2x), DMF (2x), THF (2x), THF/H₂O (2x), THF (2x), and CH₂Cl₂ (2x)]. A sample of the beads tested negative in the Kaiser test. The resin was rinsed with DMF (2x), treated employing the above deprotection protocol for ½ hour then washed as above. After swelling the resin with DMF, N-Fmoc-2(S)-piperidine-carboxylic acid (Fmoc-pip-OH) (1.4 g) was added followed by HOAt (0.54 g), HATU (1.5 g), and N,N-diisopropylethylamine (1.4 mL). The reaction mixture was shaken overnight then washed as above followed by MeOH (2x) and Et₂O (2x). A sample of the beads tested negative in the Kaiser test The polymer-supported dipeptide was washed with DMF (2x) then deprotected and washed as in the last coupling method. Suspension of the resin in dichloromethane and addition of N,N-diisopropylethylamine (0.16 mL, 0.93 mmol) was followed by addition of benzenesulfonyl chloride (0.06 mL, 0.46 mmol). The reaction mixture was shaken overnight then washed with CH₂Cl₂ (3x), THF (2x), THF/H₂O (2x), THF (3x), and CH₂Cl₂ (2x). The resin was then washed with CH₂Cl₂ (6x) then exposed to 20 % TFA/CH₂Cl₂, in the presence of 0.1 % triethylsilane for 1 hour at ambient temperature. Filtration of the cleavage solution followed by evaporation of the filtrate provided the desired product. LC-MS: RT = 3.77 minutes (100% by ELSD); MS(ES+) 385 (M-CONH₂).

### EXAMPLE 26

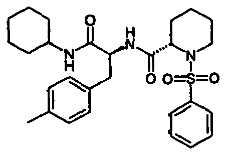

26.
   (S)-1-Benzenesulfonyl-piperidine-2-carboxylic acid ((S)-1-cyclohexylamido-2-p-tolyl-ethyl)-amide

This may be prepared according to Method B, shown herein above.

AMEBA resin (3-methoxy-4-formylphenyl linker) (0.81 g, 1.5 mmol/g) was swelled in DMF, then treated with cyclohexylamine (0.61 mL), Na(OAc)3BH (1.2 g) and AcOH (1% volume). The Jones tube was capped and several vent holes were poked in the cap with a needle. The reaction mixture was shaken overnight at room temperature. The resin was washed with DMF/H₂O (3x), DMF (2x), DMF/H₂O (2x), DMF (2x), THF (2x), THF/H₂O (2x), THF (3x), CH₂Cl₂ (2x), MeOH (2x) and Et₂O (1x) on a VacMaster vacuum box and dried. After checking a sample of the beads for consumption of the starting aldehyde (IR spectroscopy), the resin was sequentially coupled with Fmoc-p-Me-Phe-OH, deprotected, coupled with Fmoc-pip-OH, deprotected, sulfonylated, then cleaved using the conditions described in Method A. LC-MS: RT = 3.32 minutes (100% by ELSD); MS(ES+) 512 (M+H); ¹H NMR δ 1.07-1.39 (m, 10H), 1.50-1.54 (m, 1H), 1.61-1.64 (m, 4H), 1.89 (d, 1H, J = 13 Hz), 2.25 (s, 3H), 2.75 (dd, 1H, J = 9.3, 13.5 Hz), 2.87 (dd, 1H, J =5.2, 13.7 Hz), 3.14-3.12 (m, 1H), 3.46-3.48 (m, 1H), 3.58 (d, 1H, J =13.2 Hz), 4.27-4.34 (m, 1H), 4.465 (d, 1H, J = 4.5 Hz), 7.08-7.15 (m, 4H), 7.38-7.44 (m, 2H), 7.57-7.61 (m, 3H), 7.745 (d, 1H, J = 7.8 Hz), 7.86, 1H, J = 8.3 Hz).

The following compounds of Examples 27-29 were prepared according to Method B:

### EXAMPLE 27

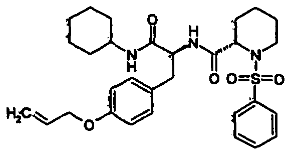

27.
   (S)-1-Benzenesulfonyl-piperidine-2-carboxylic acid [(S)-2-(4-allyloxy-phenyl)-1-cyclohexylamido-ethyl]-amide
   LC-MS: RT = 3.30 minutes (100% by ELSD); MS (ES+) 554 (M+H)

### EXAMPLE 28

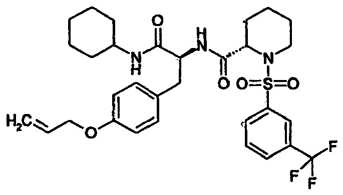

28.
   (S)-1-(3-Trifluoromethyl-benzenesulfonyl)-piperidine-2-carboxylic acid [(S)-2-(4-allyloxy-phenyl)-1-cyclohexylcarbamoyl-ethyl]-amide
   LC-MS: RT = 3.51 minutes (100% by ELSD); MS (ES+) 622 (M+H).

### EXAMPLE 29

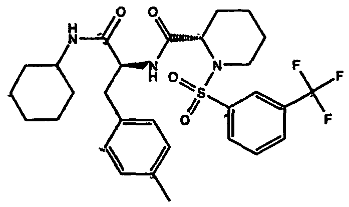

29.
   (S)-1-(3-Trifluoromethyl-benzenesulfonyl)-piperidine-2-carboxylic acid ((S)-1-cyclohexylcarbamoyl-2-p-tolyl-ethyl)-amide
   LC-MS: RT = 3.52 minutes (100% by ELSD); MS (ES+) 580 (M+H); ¹H NMR δ 0.96-1.14 (m, 7H), 1.20-1.66 (m, 8H), 1.94 (bd, 1H, J =12.7 Hz), 2.24(s, 3H), 2.84 (dd, 1H, J = 6, 13.5 Hz), 2.69 (dd, 1H, J = 8.5, 13.5 Hz), 3.18-3.25 (m, 1H), 3.43-3.45 (m, 1H), 3.66 (bd, 1H, J = 12.5 Hz), 4.27 (dd, 1H, J = 8.3, 14.3 Hz), 4.56 (d, 1H, J = 0.5 Hz), 7.10 (m, 4H), 7.56-7.61 (m, 1H), 7.70-7.76 (m, 2H), 7.94 (s, 1H), 7.96-8.01 (m, 2H).

The synthesis of the following Example illustrates the use of Method C.

### EXAMPLE 30

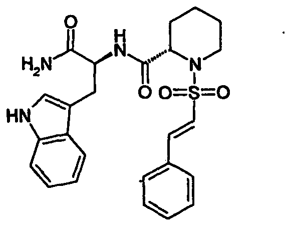

30.
   (S)-1-((E)-2-Phenyl-ethenesulfonyl)-piperidine-2-carboxylic acid [(S)-1-amido-2-(1H-indol-3-yl)-ethyl]-amide
   A sample of Rink amide resin (0.06 g, 1 mmol/g) was loaded with Fmoc-Trp(Boc)-OH and Fmoc-pip-OH using the conditions in Method A. After deprotection and washing of the dipeptide, dichloroethane was added followed by N,N-diisopropylethylamine (0.1 mL, 0.44 mmol). The resin was shaken for 5 minutes then treated with β-styrenesulfonyl chloride (0.06 g). The resin was placed in a FlexChem^{®} rotational oven (Robbins) and rotated at 45 C overnight. After allowing it to cool to ambient temperature, the resin was washed with dichloroethane (2x), CH₂Cl₂ (3x), THF (2x), THF/H₂O (2x), THF (3x), and CH₂Cl₂(3x). The product was cleaved from the resin as in Method A. LC-MS: RT = 3.28 minutes (100% by ELSD); MS(ES+) 436 (M-CONH2).

The synthesis of the following Example illustrates the use of Method D.

### EXAMPLE 31

31.
   (S)-1-(4,5-Dibromo-thiophene-2-sulfonyl)-piperidine-2-carboxylic acid [(S)-1-amido-2-(1H-indol-3-yl)-ethyl]-amide

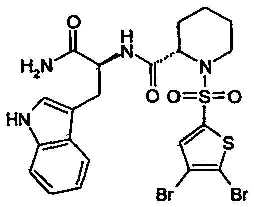

The Rink resin-supported dipeptide (0.033 g), prepared and deprotected according to Method A, was placed in a microkan^{®} (Irori) with a radiofrequency-encoded tag. The loaded kan was placed in a test tube (13 x 100 mm), and CH₂Cl₂ (1.2 mL) was added followed by N,N-diisopropylethylamine (0.06 mL) and 4,5-dibromothiophene-2-sulfonylchloride (0.06 g). The reaction mixture was shaken overnight then washed with CH₂Cl₂ (3x), THF (2x), THF/H₂O (2x), THF (2x), CH₂Cl₂ (2x), MeOH (2x), and Et₂O (2x). The microkan^{®} was dried under a stream of nitrogen then cleaved with 20% TFA/CH₂Cl₂ in the presence of triethylsilane (0.1%) and isolated as in Method A. LC-MS: RT = 3.50 minutes (100% by ELSD); MS(ES+) 574 (M-CONH₂); ¹H NMR δ 1.11-1.47 (m, 3H), 1.49-1.54 (m, 3H), 1.99 (bd, 1H, J = 13.3 Hz), 2.94 (dd, 1H, J = 7.7, 14.5 Hz), 3.09 (dd, 1H, J = 5.7, 14.5 Hz), 3.30-3.28 (m, 1H), 3:60-3.56 (m, 1H), 4.40 (q, 1H, J = 7.8, 14 Hz); 4.55 (d, 1H, J = 4.5 Hz); 6.95-7.07 (m, 3H); 7.15 (d, 1H, J = 7.7 Hz), 7.31 (d, 1H, J = 8 Hz), 7.39 (bs, 1H), 7.59 (d, 1H, J = 15 Hz), 8.01 (d, 1H, J = 8 Hz), 10.80 (s, 1H).

The following Example illustrates the use of Method E, as shown hereinabove.

### EXAMPLE 32

35. 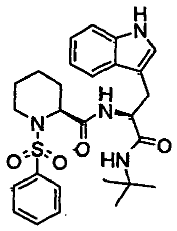
32.
   (S)-1-Benzenesulfonyl-piperidine-2-carboxylic acid ((S)-1-tert-butylamido-2-(1H-indol-3-yl)-ethyl]-amide
   A peptide synthesis vessel was charged with Marshall resin (hydroxy-thiophenol linker, Wang resin) (6 g, 1.34 mmol/g) and DMF (60 mL) was added followed by Boc-Trp-OH-(5 g), HOBt (2.2 g), diisopropylcarbodiimide (2.6 mL), and 4-(dimethylamino)pyridine (0.1 g). The reaction mixture was shaken at room temperature for 24 h, and then the resin was washed with DMF (3x), THF (3x), CH₂Cl₂ (3x), and Et₂O (3x) and dried in vacuo overnight. The dried resin was rinsed a few times with CH₂Cl₂ then treated with 50% trifluoroacetic acid in CH₂Cl₂ (80 mL) in the presence of 1 mg indole. After shaking for 1.5 minutes, the cleavage solution was removed and fresh cleavage solution (80 mL) was added. The resin was shaken at ambient temperature for ½ h, drained, then washed with CH₂Cl₂(4x), 5% N,N-diisopropylethylamine/CH₂Cl₂ (2x, 2 minute exposure each), and CH₂Cl₂ (13 x). CH₂Cl₂ (60 mL), PhSO₂-pip-OH (4.8 g), and diisopropylcarbodiimide (2.8 mL) were added and the reaction mixture was shaken at room temperature for 60 h then washed using the above wash sequence. The resin was then re-exposed to the loading conditions and washed as before. A 100 mg sample of the resin was placed in a Quest^{®} 210 reaction vessel, and pyridine (1 mL), N,N-diisopropylethylamine (0.09 mL), and t-butylamine (0.14 mL) were added. The reaction mixture was agitated at room temperature for 24 h then filtered and concentrated. The desired product was obtained after SCX chromatography (Varian^{®} BondElut^{®}, MeOH then 2M NH₃/MeOH) and solvent evaporation. LC-MS: RT = 1.92 minutes (70% by ELSD); MS(ES+) 511 (M+H).

The synthesis of Examples 33-35 were achieved by the use of Method F, as shown hereinabove.

### EXAMPLE 33

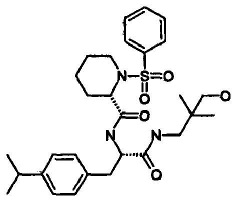

33.
   (S)-1-Benzenesulfonyl-piperidine-2-carboxylic acid [(S)-1-(3-hydroxy-2,2-dimethyl-propylamido)-2-(4-isopropyl-phenyl)-ethyl]-amide
   Wang resin (6 g, 10.2 mmol) was swelled in anhydrous DMF (60 ml) for 10 minutes in a peptide vessel, then treated with Fmoc-L-Phe(4-i-Pr)-OH (3 eq), diisopropylcarbodiimide (3 eq) and 4-(dimethylamino)pyridine (0.3 eq). The mixture was shaken overnight at room temperature. The resin was filtered and then washed successively with DMF (3x), 20% water/DMF (3x), DMF (3x), THF (3x), and CH₂Cl₂ (3x) and dried in vacuo. A 3 g (5.1 mmol) aliquot of this resin was transferred to a Jones tube, then treated with 20% piperidine/DMF (30 mL) for approximately 1 hour for Fmoc removal, washed as before and dried in vacuo. The resin was then swelled in anhydrous DMF (30 ml) for 10 minutes in a Jones tube then treated with PhSO₂-pip-OH (3 eq), HOBt (3 eq), HBTU (3 eq), diisopropylcarbodiimide (3 eq), and N,N-diisopropylethylamine(6 eq). The mixture was vortexed overnight, washed as before, and dried in vacuo. The resin was cleaved with 50%TFA in CH₂Cl₂ (30 ml) at room temperature for 1 hour. The mixture was filtered, the resin was rinsed with CH₂Cl₂ three times and the combined filtrate was concentrated and dried in vacuo overnight. Tetrafluorophenol resin (Wang resin, tetrafluorophenoxy linker, 1.5g, 1.5 mmol) was swelled in anhydrous DMF (15 ml) for 10 minutes in a Jones tube and was then treated with a solution of the above prepared carboxylic acid residue in anhydrous DMF (5 ml) and diisopropylcarbodiimide (2 eq). The mixture was vortexed overnight at room temperature, washed as above, and dried in vacuo. An aliquot of this loaded resin (0.5 g, 0.5 mmol) was placed in a Jones tube then suspended in dichloroethane (5 mL) and treated with 3-amino-2, 2-dimethyl-1-propanol (0.4 mmol) and shaken overnight. The mixture was filtered, the resin was rinsed with dichloroethane three times and the combined filtrate was concentrated and purified by reverse phase HPLC. LC-MS (of diastereomeric mixture): RT = 3.40 minutes (100% by ELSD); MS (ES+) 544 (M+H); ¹H NMR (CDCl₃) (as a ca. 3:2 mixture of diastereomers) δ 0.70 (s, minor) and 0.84 (s, major, 3H), 0.78 (s, minor) and 0.86 (s, major) (3H), 1.20 (d, 3H, J = 7 Hz), 1.22 (d, 3H, J = 7 Hz), 0.82-1.52 (series of m, 5H), 3.00-3.86 (series of m, 10H), 4.31 (m, major) and 4.49 (m, minor) (1H), 4.52 (ddd, minor, J = 8, 7.5, 7 Hz) and 4.74 (ddd, major, J = 10, 8, 5.5 Hz) (1H), 6.31 (t, minor) and 6.91 (t, major, J = 6 Hz) (1H), 6.73 (d, J = 8Hz, major) and 6.94 (d, minor, J = 8 Hz) (1H), 7.14-7.20 (m, 4H), 7.50-7.66 (m, 3H), 7.80 (m, major) and 7.85 (m, minor) (2H).

### EXAMPLE 34

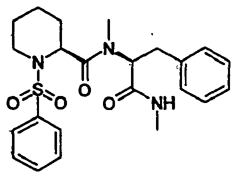

34.
   (S)-1-Benzenesulfonyl-piperidine-2-carboxylic acid methyl-((S)-1-methylamido-2-phenylethyl)-amide
   LC-MS: RT = 3.29 minutes (100% by ELSD); MS (ES+) 413 (M+H)

### EXAMPLE 35

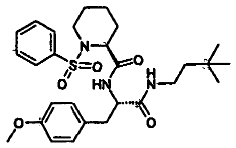

35.
   (S)-1-Benzenesulfonyl-piperidine-2-carboxylic acid [(S)-1-(3,3-dimethyl-butylamido)-2-(4-methoxy-phenyl)-ethyl]-amide
   LC-MS: RT = 3.46 minutes (100% by ELSD); MS (ES+) 530-(M+H); ¹H NMR (CDCl₃) (isolated as a 47:53 mixture of diastereomers) δ 0.89 (2s, 9H), 1.10-1.19 (m, 2H), 1.21-1.50 (m, 5H), 2.19-2.08 (m, 1H), 2.42-2.50 (m, 0.5H), 2.88-3.26 (m, 4.6H), 3.62 (bd, 0.5 H, J = 14Hz), 3.79 and 3.77 (s, 3H), 3.85-3.90 (bd, 0.5H, J = 14Hz); 4.37-4.38 (m, 0.5H), 4.39-4.46 (m, 0.5H), 4.48-4.50 (m, 0.5H), 4.56-4.64 (m, 0.5H), 5.51 and 6.03 (t, 1H, J = 5 Hz), 6.81 and 7.03 (d, 1H, J = 8 Hz), 6.84 (m, 2H) 7.13 (2 d, 2H, J = 8.5 Hz), 7.64-7.51 (m, 3H), 7.82 (d, 1H, J = 7.2 Hz), 7.86 (d, 1H, J = 7 Hz).

### EXAMPLE 36

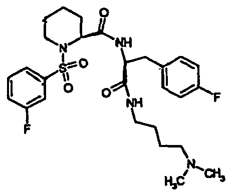

(S)-1-(3-Fluoro-benzenesulfonyl)-piperidine-2-carboxylic acid [(S)-1-(4-dimethylamino-butylcarbamoyl)-2-(4-fluoro-phenyl)-ethyl]-amide

The title compound is prepared employing the procedure described for Example 4.

### EXAMPLE 37

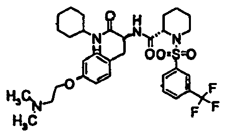

(S)-1-(3-Trifluoromethyl-benzenesulfonyl)-piperidine-2-carboxylic acid {(S)-1-cyclohexylcarbamoyl-2-[4-(2-dimethylamino-ethoxy)-phenyl]-ethyl}-amide

The title compound is prepared employing the procedure described for Example 17.

### EXAMPLE 38

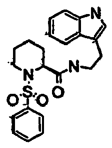

(S)-1-Benzenesulfonyl-piperidine-2-carboxylic acid [1-carbamoyl-2-(1H-indol-3-yl)-ethyl]-amide

The title compound is prepared employing the procedure described for Example 7.

### EXAMPLE 39

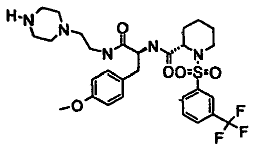

(S)-1-(3-Trifluoromethyl-benzenesulfonyl)-piperidine-2-carboxylic acid [(S)-2-(4-methoxyphenyl)-1-(2-piperain-1-yl-ethylcarbamoyl)-ethyl]-amide

The title compound is prepared employing the procedure described for Example 4.

### EXAMPLE 40

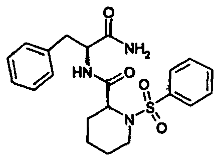

(S)-1-Benzenesulfonyl-piperidine-2-carboxylic acid ((S)-1-carbamoyl-2-phenyl-ethyl)-amide

The title compound is prepared employing the procedure described for Example 7.

### EXAMPLE 41

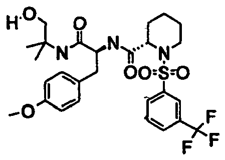

(S)-1-(3-Trifluoromethyl-benzenesulfonyl)-piperidine-2-carboxylic acid[(S)-1-(2-hydroxy-1,1-dimethyl-ethylcarbamoyl)-2-(4-methoxy-phenyl)-ethyl]-amide.

The title compound is prepared employing the procedure described for Example 4.

### EXAMPLE 42

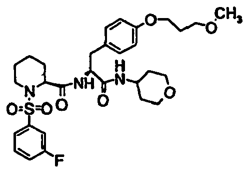

(S)-1-(3-Fluoro-benzenesulfonyl)-piperidine-2-carboxylic acid [(S)-2[4-(3-methoxy-propoxy)-phenyl]-1-(tetrahydro-pyran-4-ylcarbamoyl)-ethyl]-amide

The title compound is prepared employing the procedure described for Example 17.

### EXAMPLE 43

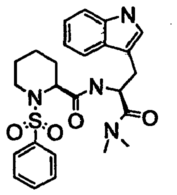

(S)1-Benzenesulfonyl-piperidine-2-carboxylic acid [(S)-1-dimethylcarbamoyl-2-(1H-indol-3-yl)-ethyl]-amide

The title compound is prepared employing the procedure described for Example 4.

### EXAMPLE 44

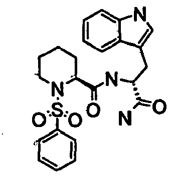

(S)-1-Benzenesulfonyl-piperidine-2-carboxylic acid [(R)-1-carbamoyl-2-(1H-indol-3-yl)-ethyl]-amide

The title compound is prepared employing the procedure described for Example 7.

### EXAMPLE 45

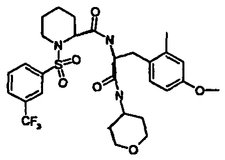

(S)-1-(3-Trifluoromethyl-benzenesulfonyl)-piperidine-2-carboxylic acid [(S)-2-(2-methyl-4-methoxy-phenyl)-1-(tetrahydro-pyran-4-ylcarbamoyl)-ethyl]-amide

The title compound is prepared employing the procedure described for Example 4.

### EXAMPLE 46

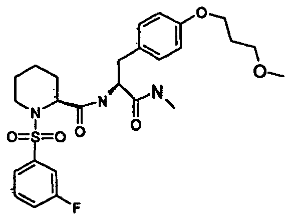

(S)-1-(3-Fluoro-benzenesulfonyl)-piperidine-2-carboxylic acid {(S)-2-[4-(3-methoxy-propoxy)-phenyl]-1-methylcarbamoyl-ethyl}-amide

To (S)-2-{[(S)-1-(3-fluoro-benzenesulfonyl)-piperidine-2-carbonyl]-amino}-3-[4-(3-methoxy-propoxy)-phenyl]-propionic acid (98mg, 0.19mmol) in dichloromethane (3mL) was added, a 2.0M methyl amine solution in THF (97µL, 1.9mmol), PyBoP (87mg, 0.19mmol) at room temperature. Upon reaction completion water was added. The mixture was extracted with ethyl acetate. The organic layer was dried over magnesium sulfate and concentrated. The residue was chromatographed on silica gel (dichloromethane/ethyl acetate, 80/20 as eluant) to produce 35 (34%) mg of (S)-1-(3-fluoro-benzenesulfonyl)-piperidine-2-carboxylic acid {(S)-2-[4-(3-methoxy-propoxy)-phenyl]-1-methylcarbamoyl-ethyl}-amide. LC/MS rt = 3.95,536 m/e (M+H). ¹H NMR (300MHz, dmso-d6) 7.93 (d, J=8.2Hz, 1H), 7.85 (m, 1H), 7.48-7.37 (m, 4H), 7.11 (d, J= 8.3Hz, 2H), 6.83 (d, J= 8.5Hz), 2H), 4.51 (d, J= 4.5Hz, 1H), 4.25 (m, 1H), 3.95 (t, J=6.5Hz, 2H), 3.61 (m, 1H), 3.42 (t, J= 6.2Hz, 2H), 3.30-3.21 (m, 5H), 3.15 (m, 1H), 2.90-2.83 (m, 1H), 2.73-2.66 (m, 1H), 2.54 (d, J= 4.8Hz, 3H), 1.95-1.97 (m, 2H), 1.50-1.25 (m, 2H), 150-1.05 (m, 2H).

### EXAMPLE 47

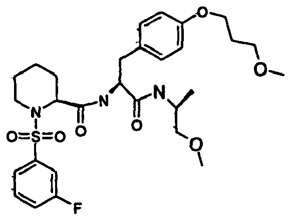

(S)-1-(3-Fluoro-benzenesulfonyl)-piperidine-2-carboxylic acid {(S)-1-((S)-2-methoxy-1-methyl-ethylcarbamoyl)-2-[4-(3-methoxy-propoxy)-phenyl]-ethyl}-amide

To (S)-2-{[(S)-1-(3-fluoro-benzenesulfonyl)-piperidine-2-carbonyl]-amino}-3-[4-(3-methoxy-propoxy)-phenyl]-propionic acid (98mg, 0.19mmol) in dichloromethane (2mL) was added 2-methoxy-1-(S)-methyl ethyl amine (19mg, 0.19mmol), 1-hydroxybenzotriazole hydrate (36mg, 0.27mol) and 1-(3-dimethylaminopropyl)-3-ethylcarbodiimide hydrochloride (47mg, 0.25mmol) at room temperature. Upon reaction completion water was added. The mixture was extracted with ethyl acetate. The organic layer was dried over magnesium sulfate and concentrated. The residue was chromatographed on silica gel (dichloromethane/ethyl acetate, 80/20 as eluant) to produce 87 (77%) mg of (S)-1-(3-fluoro-benzenesulfonyl)-piperidine-2-carboxylic acid {(S)-1-((S)-2-methoxy-1-methyl-ethylcarbamoyl)-2-[4-(3-methoxy-propoxy)-phenyl]-ethyl}-amide. LC/MS rt = 3.95,536 m/e (M+H). ¹H NMR (300MHz, dmso-d6) 7.90 (d, J=8Hz, 1H), 7.76 (d, J=8Hz, 1H), 7.48-7.37 (m, 4H), 7.13(d, J=8.5Hz, 2H), 6.83 (d, J=8.5Hz, 2H), 4.50 (m, 1H), 4.28 (m, 1H), 3.96 (t, J=6.4Hz, 2H), 3.79 (m, 1H), 3.63 (m, 1H), 3.42 (t, J=6.2Hz, 2H), 3.30-3.21 (m, 9H), 3.17-3.05 (m, 2H), 3.90-3.82 (m, 1H), 3.75-3.65 (m, 1H), 1.98-1.85 (m, 2H), 1.45-1.10 (m, 4H), 1.00 (d, J=6.7Hz, 3H).

### EXAMPLE 48

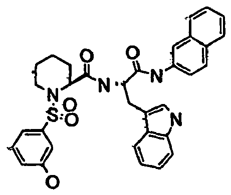

(S)-1-(3-Hydroxy-benzenesulfonyl)-piperidine-2-carboxylic acid [(S)-2-(1H-indol-3-yl)-1-(naphthalen-2-ylcarbamoyl)-ethyl]-amide

The title compound is prepared employing the procedure described for Example 4.

**TABLE 3**

| Compound | Compound Number | Method of preparation | Reaction vessel | LC | MS |
|---|---|---|---|---|---|
| (S)-1-Benzenesulfonyl-piperidine-2-carboxylic acid [(S)-1-(2,2-dimethyl-propylamido)-2-phenyl-ethyl]-amide | 49 | Method E | Jones tube | 3.17 (100% ELSD) | 486 (M+H)⁺ |
| (4-{(S)-2-[((S)-1-Benzenesulfonyl-piperidine-2-carbonyl)-amino]-2-amido-ethyl}-benzyl)-phosphonic acid diethyl ester | 50 | Method A | Jones tube | 3.09 (100% ELSD) | 566 (M+H)⁺ |
| (S)-1-Benzenesulfonyl-piperidine-2-carboxylic acid [(S)-2-(4-acetylamino-phenyl)-1-amido-ethyl]-amide | 51 | Method A | Jones tube | 2.83 (100% ELSD) | 473 (M+H)⁺ |
| (S)-1-Benzenesulfonyl-piperidine-2-carboxylic acid [(S)-1-amido-2-(4-hydroxymethyl-phenyl)-ethyl]-amide | 52 | Method A | Jones tube | 2.75 (100% ELSD) | 428-(M-OH)⁺ |
| (S)-1-(Naphthalene-2-sulfonyl)-piperidine-2-carboxylic acid [1-amido-2-(1H-indol-3-yl)-ethyl]-amide | 53 | Method A | Jones tube | 3.35 (100% ELSD) | 460 (M-CONH₂)⁺ |
| (S)-1-(3-Trifluoromethyl-benzenesulfonyl)-piperidine-2-carboxylic acid [(S)-1-(cyclohexylmethyl-amido)-2-(4-methylphenyl)-ethyl]-amide | 54 | Method B | Jonestube | 3.99 (100% ELSD) | 594 (M+H)⁺ |
| (S)-1-(3-Trifluoromethyl-benzenesulfonyl)-piperidine-2-carboxylic acid [(S)-1-(4-amino-butylamido)-2(4-methylphenyl)-ethyl]-amide | 55 | Method B | Jones tube | 3.24 (100% ELSD) | 569 (M+H)⁺ |
| (S)-1-Benzenesulfonyl-piperidine-2-carboxylic acid [(S)-1-amido-2-(1H-indol-3-yl)-ethyl]-amide | 56 | Method A | Quest 210 (Argonaut) | 1.64 ELSD) | 455 (M+H)⁺ |
| (S)-1-Benzenesulfonyl-piperidine-2-carboxylic acid ((S)-1-amido-2-pyridin-4-yl-ethyl)-amide | 57 | Method A | Quest 210 (Argonaut) (100% | 2.59 ELSD) | 417 (M+H)⁺ |
| (S)-1-Benzenesulfonylpiperidine-2-carboxylic acid ((S)-1-amido-2-cyclohexyl-ethyl)-amide | 58 | Method A | Quest 210 (Argonaut) | 3.50 ELSD) | 377 (M-CONH₂)⁺ |
| (S)-1-Benzenesulfonyl-piperidine-2-carboxylic acid {(S)-2-phenyl-1-[(thiophen-2-ylmethyl)-amido]-ethyl}-amide | 59 | Method A | Quest 210 (Argonaut) | 3.05 (100% ELSD) | 512 (M+H)⁺ |

## Claims

1. Compounds, including enantiomers, stereoisomers, rotamers and tautomers of said compounds and pharmaceutically acceptable salts, or solvates thereof, said compounds having the general structure shown in Formula I: 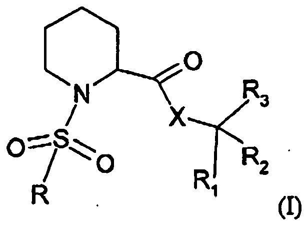
wherein: R is fluoroalkyl of the formula CₙHₓF_{y} wherein n is an integer from 1 to 6, x is an integer from 0 to 12, y is an integer from 1 to 13 and the sum of x and y is 2n+1, aryl, aryl C₁₋₆ alkyl, aryl C₂₋₄ alkenyl, wherein aryl, is selected from phenyl, biphenyl and naphthyl, heteroaryl or heteroaryl C₁₋₄ alkyl, wherein heteroaryl is selected from pyridyl, thiophenyl and benzothiophenyl, and heterocyclyl selected from piperidinyl and piperazinyl,
R₁ is hydrogen or C₁₋₆ alkyl;
R₂ is CONHR₄, benzimidazol-2-yl, CR₅R₆OH, or CR₇R₈NHR₉;
R₃ is aryl, aryl C₁₄ alkyl or aryl C₂₋₄ alkenyl, wherein aryl is selected from phenyl, biphenyl, naphthyl, bisarylmethyl, heteroaryl and heteroaryl C₁₋₄ alkyl, wherein heteroaryl is selected from indolyl, thiophenyl, benzothiophenyl, furanyl, benzofuranyl, 1,3-benzodioxolyl, 1, 4-benzodioxanyl and C₅₋₇ cycloalkyl C₁ alkyl;
X is NR₁₀ or CH₂;
wherein:
R₄ is selected from the group consisting of hydrogen, C₁₋₁₀ alkyl, C₂₋₁₀ alkenyl, C₃₋₈ cycloalkyl, C₈₋₁₂ bicycloalkyl, C₁₀₋₁₄ tricycloalkyl, C₃₋₈ cycloalkyl C₁₋₆ alkyl, C₈₋₁₂ bicycloalkyl C₁₋₆ alkyl, C₈₋₁₂ bicycloalkylamino- C₁₋₆ alkyl, C₈₋₁₂ tricycloalkylamino-C₁₋₆ alkyl, C₃₋₈ cycloalkenyl, C₃₋₈ cycloalkenyl C₁₋₆ alkyl, fluoroalkyl of the formula CₙHₓF_{y} wherein n is an integer from 1 to 20, x is an integer from 0 to 40, y is an integer from 1 to 41 and the sum of x and y is 2n+1, amino C₂₋₂₀ alkyl, mono or dialkylamino C₂₋₂₀ alkyl, mono-, di, or trihydroxy C₂₋₂₀ alkyl, C₁₋₆ alkoxy- C₂₋₂₀ alkyl, C₁₋₂₀ thioalkyl, C₁₋₆ alkyl-thio-C₂₋₂₀ alkyl, hydroxy-C₂₋₆ -alkoxy-C₂₋₆ alkyl, C₁₋₆ alkyl-bis-(hydroxy-C₁₋₆ alkyl), aryl, aryl C₁₋₁₀ alkyl, aryl C₃₋₄ alkenyl, wherein aryl is selected from phenyl, biphenyl, indanyl and naphthyl, heteroaryl or heteroaryl C₁₋₁₀ alkyl, wherein heteroaryl is selected from pyridyl, thiophenyl, furanyl, benzofufanyl, benzothiophenyl, pyrrolidinyl, and indolyl; heterocycloalkyl or heterocycloalkyl C₁₋₁₀ alkyl wherein heterocycloalkyl is selected from tetrahydrofuranyl, tetrahydropyranyl, morpholinyl, piperidinyl, pyrrolidinyl, tetrahydrothiophenyl, piperazinyl, dioxolanyl, sulfolanyl and 1,1-dioxotetrahydrothiopyranyl; heterobicyclyl of the formula: 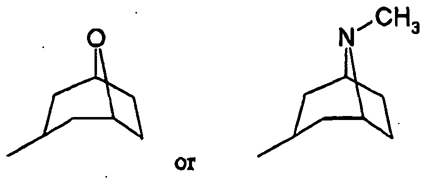 comprising C₅₋₈ cycloalkyl, C₈₋₁₀bicycloalkyl, and/or heterocyclyl, optionally substituted with one or more substituents selected from the group consisting of C₁₋₁₀ alkyl, C₁₋₁₀ alkoxy, hydroxy, hydroxy C₁₋₁₀ alkoxy, hydroxy C₁₋₁₀ alkyl, C₁₋₁₀ acyloxy, amino, C₁₋₁₀ alkylcarbonylamino, hydroxy C₁₋₂₀ alkylcarbonylamino, amino C₂₋₁₀ alkoxy, mono-C₁₋₆ -alkylamino-C₂₋₁₀-alkoxy, di-C₁₋₆-alkylamino-C₂₋₁₀-alkoxy, mono-(C₁₋₁₀ alkyl)amino, di-(C₁₋₁₀ alkyl)amino, amino C₁₋₁₀ alkylcarbonylamino, C₁₋₁₀ alkylaminocarbonyl, hydroxy C₁₋₁₀ alkylaminocarbonyl, amino C₂₋₁₀ alkylaminocarbonyl, mono or dialkylamino C₁₋₁₀ alkylaminocarbonyl, oxo, optionally substituted aryl and optionally substituted heteroaryl, wherein aryl is selected from phenyl, naphthyl and biphenyl, and heteroaryl is selected from pyridyl and thiophenyl; and
wherein: the aryl or heteroaryl groups of R are optionally substituted with one or more substituents selected from the group consisting of fluorine, chlorine, bromine, C₁₋₂₀ alkyl, fluoroalkyl or fluoroalkoxy of the formula CₙHₓF_{y} or OCₙHₓF_{y} wherein n is an integer from 1 to 4, x is an integer from 0 to 8, y is an integer from 1 to 9 and the sum of x and y is 2n+1, C₁₋₂₀ alkoxy, C₁₋₂₀ alkenyloxy, C₁₋₁₀-alkoxy-C₁₋₁₀-alkoxy, hydroxy, hydroxy C₁₋₂₀ alkoxy, hydroxy C₁₋₂₀ alkyl, C₁₋₂₀ acyloxy, hydroxy C₁₋₂₀ acyl, nitro, amino, aminosulfonyl, C₁₋₂₀ alkylcarbonylamino, hydroxy C₁₋₂₀ alkylcarbonylamino, amino C₂₋₂₀ alkoxy, amino C₁₋₂₀ alkylcarbonylamino, C₁₋₂₀ alkylaminocarbonyl, hydroxy C₁₋₂₀ alkylaminocarbonyl, amino C₁₋₂₀ alkylaminocarbonyl, mono or dialkylamino C₁₋₂₀ alkylaminocarbonyl, optionally substituted aryl or heteroarylsulfonyl, aminosulfonyl, mono- or di(C₁₋₆)alkylaminosulfonyl, optionally substituted phenyl or thiophenyl, optionally substituted phenyl or thiophenyl C₁₋₁₀ alkyloxy, and cyano;
wherein: the phenyl or thiophenyl substituent on the aryl or heteroaryl group of R is optionally substituted with one or more substituents selected from the group consisting of fluorine, chlorine, bromine, C₁₋₅ alkyl, fluoroalkyl or fluoroalkoxy of the formula CₙHₓF_{y} or OCₙHₓF_{y} wherein n is an integer from 1 to 4, x is an integer from 0 to 8, y is an integer from 1 to 9 and the sum of x and y is 2n+1, C₁₋₁₀ alkoxy, C₁₋₁₀ alkenyloxy, C₁₋₅ alkoxy-C₁₋₅ alkoxy, hydroxy, hydroxy C₁₋₁₀ alkoxy, hydroxy C₁₋₁₀ alkyl, hydroxy C₁₋₂₀-alkyloxo, nitro, amino, aminosulfonyl, C₁₋₁₀ alkylcarbonylamino, hydroxy -C₁₋₁₀ alkylcarbonylamino, amino C₂₋₁₀ alkoxy, amino C₁₋₁₀ alkylcarbonylamino, C₁₋₁₀ alkylaminocarbonyl, hydroxy C₁₋₁₀ alkylaminocarbonyl, amino C₁₋₁₀ alkylaminocarbonyl, mono or dialkylamino C₁₋₁₀ alkylaminocarbonyl, optionally substituted aryl C₁₋₁₀ alkyloxy, and cyano;
wherein: the aryl or heteroaryl of R₃ is optionally substituted with one or more substituents selected from the following: C₃₋₈ cycloalkyloxy, C₁₋₁₀ alkoxy, C₁₋₁₀ alkyl, C₂₋₁₀ alkenyl, C₃₋₁₀ alkenyloxy, mono- (C₁₋₆-alkyl)amino-C₂₋₆-alkyloxy, di-(C₁₋₆ alkyl)amino-C₂₋₆-alkyloxy, amino-C₂₋₆alkloxy, heterocyclyl-C₂₋₆alkyloxy, aryl-C₂₋₆-alkyloxy, heteroaryl-C₂₋₆-alkyloxy, fluoroalkyl or fluoroalkoxy of the formula CₙHₓF_{y} or OCₙHₓF_{y} wherein n is an integer from 1 to 4, x is an integer from 0 to 8, y is an integer from 1 to 9 and the sum of x and y is 2n+1, C₁₋₆-alkoxy-C₂₋₆-alkoxy, aryl-C₁₋₆-alkyloxy, hydroxy, chloro, fluoro, bromo, hydroxy-C₁₋₆-alkoxy, hydroxy-C₁₋₁₀ alkyl, C₁₋₆ alkyloxo, cyano, amino, 4-aryloxo, di-C₁₋₅ alkylphosphityl-C₁₋₅ alkyl, C₁₋₆ alkyloxoamino, C₁₋₆ alkyloxo(C₁₋₆ alkyl)amino, C₁₋₁₀alkylsulfonyl, arylsulfonyl, carboxy, C₁₋₂₀ carboalkoxy, dialkylamino-C₁₋₁₀ alkyl, monoalkylamino-C₁₋₁₀ alkyl, and amino-C₁₋₁₀ alkyl;
wherein: the heterocycloalkyl substituent group attached to the alkyloxy group of R₃ is selected from the following: pyrrolidinyl, morpholinyl, piperidinyl, and piperazinyl;
wherein: the heteroaryl substituent group attached to the alkyloxy group of R₃ is selected from pyridinyl, and pyrrolidinyl;
wherein: the aryl of the arylsulfonyl substituent on the aryl of R₃ is selected from phenyl, indolyl, thiophenyl, and furanyl;
wherein: the aryl or heteroaryl groups of R₄ are optionally substituted with one or more substituents selected from the following: mono- or di-(C₁₋₆ alkyl)amino, mono- or di (C₁₋₆)alkylamino-C₂₋₆ alkyl, amino-C₁₋₆ alkyl, heterocycloalkyl, amino, C₁₋₂₀ alkoxy, chloro, fluoro, bromo, C₁₋₁₀ alkyl, aryl and heteroaryl;
wherein: the heterocycloalkyl group attached to the aryl of R₄ is selected from the following: morpholinyl, pyrrolidinyl, piperazinyl, and piperidinyl;
wherein: the heteroaryl group attached to the aryl of R₄ is selected from pyridyl, pyrrolyl, and furanyl;
R₅, R₆ = independently H, or C₁₋₆ alkyl;
R₇, R₈ = independently H, or C₁₋₆ alkyl;
R₉ = di-(C₁₋₅ alkyl)amino-C₂₋₄ alkyl; mono(C₁₋₅ alkyl)amino-C₂₋₄ alkyl; amino-C₂₋₄ alkyl, or heterocycloalkyl methyl in which heterocycloalkyl is selected from the group consisting of thiophenyl, furanyl, pyrrolyl, and pyridinyl; and
R₁₀ = H, or C₁₋₆ alkyl, except the compound wherein R is a phenyl para-substituted by a methyl group, R₁ is hydrogen, R₂ is CR5R6OH, R₃ is phenylmethyl, X is NR₁₀, R₅, R₆ and R₁₀ are hydrogen.

2. The compound according to claim 1, wherein:
R is is selected from the group consisting of aryl, aryl C₁₋₆ alkyl, aryl C₂₋₄ alkenyl, wherein aryl is selected from phenyl, biphenyl or naphthyl; heteroaryl or heteroaryl C₁₋₄ alkyl, wherein heteroaryl is selected from pyridyl, thiophenyl or benzothiophenyl; and heterocyclyl selected from piperidinyl or piperazinyl;
R₁ is hydrogen or methyl;
R₂ is CONHR₄, benzimidazol-2-yl, CR₅R₆OH, or CR₇R₈NHR₉;
R₃ is selected from the group consisting of phenyl and aryl C₁₋₄ alkyl or aryl C₂₋₄ alkenyl, wherein aryl is selected from phenyl, biphenyl, naphthyl or bisarylmethyl, and heteroaryl or heteroaryl C₁₋₄ alkyl, wherein heteroaryl is selected from indolyl and thiophenyl.
X is NR₁₀ or CH₂; wherein R₄ is selected from the group consisting of hydrogen, C₁₋₁₀ alkyl, C₂₋₁₀ alkenyl, C₃₋₈ cycloalkyl, C₈₋₁₂ bicycloalkyl, C₁₀₋₁₄ tricycloalkyl, C₃₋₈ cycloalkyl-C₁₋₆ alkyl, C₈₋₁₂ bicycloalkylC₁₋₆ alkyl, C₈₋₁₂ bicycloalkylamino- C₁₋₆ alkyl, C₈₋₁₂ tricycloalkylamino-C₁₋₆ alkyl, C₃₋₈ cycloalkenyl, C₃₋₈ cycloalkenyl C₁₋₆ alkyl, fluoroalkyl of the formula CₙHₓF_{y} wherein n is an integer from 1 to 10, x is an integer from 0 to 20, y is an integer from 1 to 21 and the sum of x and y is 2n+1, amino C₂₋₁₀ alkyl, mono or dialkylamino C₂₋₁₀ alkyl, mono-, di, or trihydroxy C₂₋₁₀ alkyl, C₁₋₆-alkoxy-C₂₋₁₀ alkyl, C₁₋₁₀ thioalkyl, C₁₋₆alkyl-thio-C₂₋₁₀alkyl, hydroxy-C₂₋₆-alkoxy-C₂₋₆alkyl, C₁₋₆alkyl-bis-(hydroxy-C₁₋₆alkyl), aryl, aryl C₁₋₆ alkyl, and aryl C₃₋₄ alkenyl, wherein aryl is selected from phenyl, biphenyl, indanyl and naphthyl, heteroaryl or heteroaryl C₁₋₁₀ alkyl, wherein heteroaryl is selected from pyridyl, thiophenyl, furanyl, benxofuranyl, benzothiophenyl, pyrrolidinyland indolyl; heterocycloalkyl or heterocycloalkyl C₁₋₁₀ alkyl wherein hetexocycloalkyl is selected from tetrahydrofuranyl, tetrahydropyranyl, morpholinyl, piperidinyl, pyrrolidinyl, piperazinyl, dioxolanyl, sulfolanyl and 1,1-dioxotetrahydrothiopyranyl; wherein C₅₋₈ cycloalkyl, C₈₋₁₂ bicycloalkyl, or heterocycloalkyl is optionally substituted with one or more substituents selected from the group consisting of C₁₋₆ alkyl, C₁₋₆ alkoxy, hydroxy, hydroxy C₁₋₆ alkoxy, hydroxy C₁₋₆ alkyl, amino, C₁₋₆ alkylcarbonylamino, hydroxy C₁₋₆ alkylcarbonylamino, amino C₂₋₆ alkoxy, mono-C₁₋₆-alkylamino-C₂₋₆-alkoxy, di-C₁₋₆-alkylamino-C₂₋₆-alkoxy, mono-(C₁₋₆ alkyl)amino, di-(C₁₋₆ alkyl)amino, amino C₁₋₆ alkylcarbonylamino, C₁₋₆ alkylaminocarbonyl, hydroxy C₁₋₆ alkylaminocarbonyl, amino C₂₋₆ alkylaminocarbonyl, mono or dialkylamino C₁₋₆ alkylaminocarbonyl, oxo, optionally substituted aryl and optionally substituted heteroaryl; wherein aryl is selected from phenyl, naphthyl and biphenyl and heteroaryl is selected from pyridyl and thiophenyl;
wherein: the aryl or heteroaryl- groups of R are optionally substituted with one or more substituents selected from the group consisting of fluorine, chlorine, bromine, C₁₋₁₀ alkyl, fluoroalkyl or fluoroalkoxy of the formula CₙHₓF_{y} or OCₙHₓF_{y} wherein n is an integer from 1 to 4, x is an integer from 0 to 8, y is an integer from 1 to 9 and the sum of x and y is 2n+1, C₁. ₁₀ alkoxy, C₁₋₁₀ alkenyloxy, C₁₋₁₀ -alkoxy- C₁₋₁₀ -alkoxy, hydroxy, hydroxy C₁₋₁₀ alkoxy, hydroxy C₁₋₁₀ alkyl, hydroxy C₁₋₁₀ acyl, nitro, amino, aminosulfonyl, C₁₋₁₀ alkylcarbonylamino, hydroxy C₁₋₁₀ alkylcarbonylamino, amino C₂₋₁₀ alkoxy, amino C₁₋₁₀ alkylcarbonylamino, C₁₋₁₀ alkylaminocarbonyl, hydroxy C₁₋₁₀ alkylaminocarbonyl, amino C₁₋₁₀ alkylaminocarbonyl, mono or dialkylamino C₁₋₁₀ alkylaminocarbonyl, optionally substituted aryl or heteroarylsulfonyl, aminosulfonyl, mono- or di(C₁₋₆)alkylaminosulfonyl. optionally substituted phenyl or thiophenyl, optionally substituted phenyl or thiophenyl C₁₋₁₀ alkyloxy, and cyano;
wherein the phenyl or thiophenyl substituent on the aryl or heteroaryl group of R is optionally substituted with one or more substituents selected from the group consisting of fluorine, chlorine, bromine, C₁₋₅ alkyl, CF₃, OCF₃, C₁₋₆ alkoxy, C₁₋₆ alkenyloxy, C₁₋₅ alkoxy-C₁₋₅ alkoxy, hydroxy, hydroxy C₁₋₁₀ alkoxy, hydroxy C₁₋₁₀ alkyl, nitro, amino, aminosulfonyl, C₁₋₁₀ alkylcarbonylamino, hydroxy C₁₋₆ alkylcarbonylamino, amino C₂₋₆ alkoxy, amino C₁₋₆ alkylcarbonylamino, C₁₋₆ alkylaminocarbonyl, hydroxy C₁₋₆ alkylaminocarbonyl, amino C₁₋₆ alkylaminocarbonyl, mono or dialkylamino C₁₋₆ alkylaminocarbonyl, optionally substituted aryl C₁₋₆ alkyloxy, and cyano;
wherein:
the aryl or heteroaryl of R₃ is optionally substituted with one or more substituents selected from the following: C₃₋₆ cycloalkyloxy, C₁₋₆ alkoxy, C₁₋₆ alkyl, C₂₋₆ alkenyl, C₃₋₆ alkenyloxy, mono- (C₁₋₆ alkyl)amino-C₂₋₆ alkyloxy, di-(C₁₋₆ alkyl)amino-C₂₋₆ alkyloxy, amino-C₂₋₆ alkyloxy, heterocyclyl- C₂₋₆ alkyloxy, aryl- C₂₋₆ alkyloxy, heteroaryl- C₂₋₆ alkyloxy, fluoroalkyl or fluoroalkoxy of the formula CₙHₓF_{y} or OCₙHₓF_{y} wherein n is an integer from 1 to 4, x is an integer from 0 to 8, y is an integer from 1 to 9 and the sum of x and y is 2n+1, C₁₋₆alkoxy - C₂₋₆ alkyloxy, aryl- C₁₋₆ alkoxy, hydroxy, chloro, fluoro, bromo, hydroxy- C₁₋₆ alkoxy, hydroxy-C₁₋₁₀ alkyl, C₁₋₆ alkyloxo, cyano, amino, 4-aryloxo, C₁₋₆ alkyloxoamino, C₁₋₆ alkyloxo(C₁₋₆ alkyl)amino, carboxy, dialkylamino- C₁₋₆ alkyl, monoalkylamino- C₁₋₆ alkyl, and amino- C₁₋₆ alkyl;
wherein: the heterocyclyl substituent group attached to the alkyloxy group of R₃ is selected from the following: pyrrolidinyl, morpholinyl, piperidinyl, and piperazinyl;
wherein: the heteroaryl substituent group attached to the alkyloxy group of R₃ is selected from pyridinyl, and pyrrolidinyl;
wherein:
the aryl or heteroaryl groups of R₄ are optionally substituted with one or more substituents selected from the following: mono- or di-(C₁₋₆ alkyl)amino,
mono- or di (C₁₋₆)akylamino-C₂₋₆-alkyl, amino- C₁₋₆ alkyl,
heterocyclyl, amino, C₁₋₁₀ alkoxy, chloro, fluoro, bromo, C₁₋₁₀ alkyl, aryl and heteroaryl;
wherein the heterocyclyl group attached to the aryl of R₄ is selected from the following: morpholinyl, pyrrolidinyl, piperazinyl, piperidinyl;
wherein the heteroaryl group attached to the aryl of R₄ is selected from pyridyl, pyrrolyl, and furanyl;
R₅, R₆ = H;
R₇, R₈ = H;
R₉ = di-(C₁₋₅ alkyl)amino-C₂₋₄ alkyl; mono(C₁₋₅ alkyl)amino-C₂₋₄ alkyl; amino-C₂₋₄ alkyl, heterocycloalkyl methyl in which heterocycloalkyl is selected from group consisting of thiophenyl, furanyl, pyrrolyl, pyridinyl; and
R₁₀ = H, CH₃, except the compound wherein R is a phenyl para-substituted by a methyl group, R₁ is hydrogen, R₂ is CR5R60H, R₃ is phenylmethyl, X is NR₁₀, R₅, R₆ and R₁₀ are hydrogen.

3. The compound according to claim 2, wherein:
R is aryl selected from phenyl, biphenyl and naphthyl, or heteroaryl selected from pyridyl and thiophenyl,
R₁ is hydrogen or methyl,
R₂ is CONHR_{4.}
R₃ is aryl-C₁ alkyl wherein aryl is selected from phenyl, biphenyl and naphthyl, or heteroaryl-C₁ alkyl wherein heteroaryl is selected from indolyl and thiophenyl,
X is NR₁₀, wherein R₄ is selected from the group consisting of hydrogen, C₁₋₁₀ alkyl, C₂₋₁₀. alkenyl, C₃₋₈ cycloalkyl, C₈₋₁₂ bicycloalkyl, C₁₀₋₁₄ tricycloalkyl, C₃₋₈ cycloalkyl-C₁₋₆ alkyl, C₈₋₁₂ bicycloalkylC₁₋₆, alkyl, C₈₋₁₂ bicycloamino-C₁₋₆ alkyl, C₈₋₁₂ tricycloamino-C₁₋₆ alkyl, C₃₋₈ cycloalkenyl, C₃₋₈ cycloalkenyl C₁₋₆ alkyl, fluoroalkyl of the formula CₙHₓF_{y} wherein n is an integer from 1 to 6, x is an integer from 0 to 12, y is an integer from 1 to 13 and the sum ofx and y is 2n+1, amino C₂₋₈ alkyl, mono or dialkylamino C₂₋₈ alkyl, mono-, di-, or trihydroxy C₂₋₁₀ alkyl, C₁₋₆ -alkoxy-C₂₋₁₀ alkyl, C₁₋₆ thioalkyl, C₁₋₆ alkyl-thio-C₂₋₆ alkyl, hydroxy- C₂₋₆-alkoxy- C₂₋₆ alkyl, C₁₋₆ alkyl-bis-(hydroxy-C₁₋₆ alkyl), aryl, aryl C₁₋₆alkyl, wherein aryl is selected from phenyl, biphenyl, and naphthyl, heteroaryl or heteroaryl C₁₋₆ -alkyl, wherein heteroaryl is selected from pyridyl, thiophenyl, heterocycloalkyl or heterocycloalkyl C₁₋₆alkyl wherein heterocycloalkyl is selected from tetrahydrofuranyl, tetrahydropyranyl, morpholinyl, piperidinyl, pyrrolidinyl, and piperazinyl;
wherein: the aryl or heteroaryl groups of R are optionally substituted with one or more substituents selected from the group consisting of fluorine, chlorine, bromine, C₁₋₁₀ alkyl, CF₃, OCF₃, C₁₋₁₀ alkoxy, C₁₋₁₀ alkenyloxy, C₁₋₅ alkoxy- C₁₋₁₀ -alkoxy, hydroxy, hydroxy C₁₋₁₀ alkoxy, hydroxy C₁₋₁₀ alkyl. nitro, amino, C₁₋₁₀ alkylcarbonylamino, hydroxy C₁₋₁₀ alkylcarbonylamino, amino C₂₋₁₀ alkoxy, amino C₁₋₁₀ alkylcarbonylamino, optionally substituted phenyl or thiophenyl, optionally substituted phenyl or thiophenyl C₁₋₁₀ alkyloxy, and cyano;
wherein the phenyl or thiophenyl substituent on the aryl or heteroaryl group of R is optionally substituted with one or more substituents selected from the group consisting of fluorine, chlorine, bromine, C₁₋₅ alkyl, CF₃, OCF₃, C₁₋₆alkoxy, C₁₋₆ alkenyloxy, C₁₋₅ alkoxy-C₁₋₅ alkoxy, hydroxy, hydroxy C₁₋₁₀ alkoxy, hydroxy C₁₋₁₀ alkyl, nitro, optionally substituted aryl C₁₋₆ alkyloxy, and cyano;
wherein: the aryl or heteroaryl of R₃ is optionally substituted with one or more substituents selected from: C₃₋₆ cycloalkyloxy, C₁₋₆ alkoxy, C₁₋₆ alkyl, C₂₋₆ alkenyl, C₃₋₆ alkenyloxy, mono- (C₁₋₆ alkyl)amino- C₂₋₆ alkyloxy, di-(C₁₋₆ alkyl)amino- C₂₋₆ -alkyloxy, amino- C₂₋₆ alkyloxy, heterocyclyl- C₂₋₆ alkyloxy, aryl- C₂₋₆ -alkyloxy, heteroaryl- C₂₋₆ -alkyloxy, fluoroalkyl or fluoroallcoxy of the formula CₙHₓF_{y} or OCₙH_{X}F_{y} wherein n is an integer from 1 to 4, x is an integer from 0 to 8, y is an integer from 1 to 9 and the sum of x and y is 2n+1, C₁₋₆ -alkoxy- C₂₋₆ -alkoxy, aryl- C₁₋₆ -alkyloxy, hydroxy, chloro, fluoro, bromo, nitro, hydroxy-C₁₋₆ -alkoxy, hydroxy- C₁₋₆, C₁₋₆ alkyloxo, cyano, amino, 4-aryloxo, C₁₋₆ alkyloxoamino, carboxy, dialkylamino- C₁₋₆ alkyl, monoalkylamino- C₁₋₆ -alkyl, and amino- C₁₋₆ alkyl; wherein: the heterocycloalkyl substituent group attached to the alkyloxy group of R₃ is selected from: pyrrolidinyl, morpholinyl, and piperidinyl;
wherein: the heteroaryl substituent group attached to the alkyloxy group of R₃ is selected from pyridinyl, and pyrrolidinyl;
wherein: the aryl or heteroaryl groups of R₄ are optionally substituted with one or more substituents selected from the following: mono- or di-(C₁₋₆ -alkyl)amino, mono- or di (C₁-₆)alkamino-C₂₋₆-alkyl, amino- C₁₋₆ -alkyl,
heterocycloalkyl, amino, C₁₋₆ alkoxy, chloro, fluoro, bromo, C₁₋₆ -alkyl, aryl and heteroaryl; wherein the heterocycloalkyl group attached to the aryl of R₄ is selected from the following: morpholinyl, pyrrolidinyl, piperazinyl, and piperidinyl; and
wherein the heteroaryl group attached to the aryl of R₄ is selected from, pyridyl and pyrrolyl.

4. The compound according to claim 3, wherein:
R is aryl, wherein aryl is selected from phenyl, and naphthyl,
R₁ is hydrogen or methyl ,
R₂ is CONHR₄,
R₃ is aryl-C₁-alkyl, wherein aryl is selected from phenyl, indolyl, and thiophenyl;
X is NH wherein R₄, is selected from the group consisting ofhydrogen, C₁₋₁₀ alkyl, C₃₋₆ cycloalkyl, adamantyl, adamantyl-C₁-alkyl, 2-adamantylaminoethyl, C₃₋₆ -cycloalkyl-C₁ alkyl, 3-propenyl, trifluoromethyl-C₁-alkyl, 4-amino-n-butyl, 4-(methylamino)-n-butyl, 4-(dimethylamino)-n-butyl; 4-diethylaminobutyl, 2-hydroxyethyl, 2-(2-hydroxyethoxy)ethyl, 3-hydroxypropyl, 2-methoxyethylaryl, wherein aryl is selected from phenyl and naphthyl; aryl C₁-alkyl, wherein aryl is selected from phenyl, and biphenyl, heteroaryl C₁-alkyl, wherein heteroaryl is selected from 2-thiophenyl and 2-pyridinyl, heterocycloalkyl, wherein heterocycloalkyl is selected from 4-tetrahydropyranyl, 4-piperidinyl, 2,2,6,6-tetramethylpiperidinyl and 1-ethyl-4-piperidinyl, heterocycloalkyl-C₁-alkyl, wherein heterocycloalkyl is selected from 4-tetrahydropyranyl and 2-tetrahydrofuranyl, 2- heterocycloalkyl-C₁-alkyl, wherein heterocycloalkyl is selected from 4-tetrahydropyranyl and 1-piperazinyl;
wherein: the phenyl of R is optionally substituted with one or two substituents in the 3-, 4-, or 5-positions selected from the group consisting of fluorine, chlorine, bromine, trifluoromethyl, C₁₋₇ alkyloxy, C₁₋₇ alkenyloxy, C₁₋₈ alkyl, and C₂₋₃ hydroxypropyl; the naphthyl of R is optionally substituted with one of the substituents selected from chlorine, fluorine, and dimethylamino; the phenyl of R₃ is optionally substituted with one or two substituents selected from the group consisting of C₁₋₅ alkyl, C₁₋₅ alkyloxy, 2-dimethylaminoethoxy, 2-pyrrolidino-ethoxy, 3-fluoropropyloxy, 3-methoxypropyloxy, cyclopentoxy, benzyloxy, hydroxy, chloro, fluoro, bromo, trifluoromethyl, 1-hydroxyethyl, acetyl, allyloxy, cyano, amino, 4-benzoyl, and acetoxy; the phenyl of R₄ is optionally substituted with one or two substituents selected from the group consisting of 4-dimethylamino, 4-methylamino, 4-amino, methoxy, ethoxy, chloro, 4-morpholino, fluoro, and methyl.

5. The compound according to claim 1, selected from:
(S)-1-(3-Trifluoromethyl-benzenesulfonyl)-piperidine-2-carboxylic acid [(S)-1-(4-dimethylamino-phenylcarbamoyl)-2-(4-methoxy-phenyl)-ethyl]-amide;
(S)-1-(3-Trifluoromethyl-benzenesulfonyl)-piperidine-2-carboxylic acid [(S)-2-(4-acetylphenyl)-1-(4-ethoxy-phenylcarbamoyl)-ethyl]-amide;
(S)-1-(3- Trifluoromethyl-benzenesulfonyl)-piperidine-2-carboxylic acid [(S)-1-(4-morpholin-4-yl-phenylcarbamoyl)-2-(4-trifluoromethyl-phenyl)-ethyl]-amide;
(S)-1-(3-Trifluoromethyl-benzenesulfonyl)-piperidine-2-carboxylic, acid [(S)-2-(4-methoxyphenyl)-1-(tetrahydro-pyran-4-ylcarbamoyl)-ethyl]-amide;
(S)-1-(3-Trifluoromethyl-benzenesulfonyl)-piperidine-2-carboxylic acid [(S)-2-(2-methyl-4-methoxy-phenyl)-1-(tetrahydro-pyran-4-ylcarbamoyl)-ethyl]-amide;
(S)-1-(3-Trifluoromethyl-benzenesulfonyl)-piperidine-2-carboxylic acid [(S)-2-(3,4-dimethoxy-phenyl)-1-(tetrahydro-pyran-4-yl-carbamoyl)-ethyl]-amide;
(S)-1-(3-Trifluoromethyl-benzenesulfonyl)-piperidine-2-carboxylic acid [(S)-2-(4-methoxyphenyl)-1-(4-morpholin-4-yl-phenylcarbamoyl)-ethyl]-amide;
(S)-1-(3-Methoxy-benzenesulfonyl)-piperidine-2-carboxylic acid ((S)-1-carbamoyl-2-phenylethyl)-amide;
(S)-1-(3-Hydroxy-benzenesulfonyl)-piperidine-2-carboxylic acid ((S)-1-carbamoyl-2-phenylethyl)-amide;
(S)-1-(3-Propoxy-benzenesulfonyl)-piperidine-2-carboxylic acid ((S)-1-carbamoyl-2-phenylethyl)-amide;
(S)-1-(3-Trifluoromethyl-benzenesulfonyl)-piperidine-2-carboxylic acid [(S)-2-[4-(1-hydroxy-ethyl)-phenyl]-1-(4-morpholin-4-yl-phenylcarbamoyl)-ethyl]-amide;
(S)-1-(3-Trifluoromethyl-benzenesulfonyl)-piperidine-2-carboxylic acid {(S)-1-(4-ethoxy-phenylcarbamoyl)-2-[4-(1-hydroxy-ethyl)-phenyl]-ethyl}-amide;
(S)-1-(3-Methoxy-benzenesulfonyl)-piperidine-2-carboxylic acid [(S)-2-(indol-3-yl)-1-(naphthalen-2-ylcarbamoyl)-ethyl]-amide;
(S)-1-(3-Trifluoromethyl-benzenesulfonyl)-piperidine-2-carboxylic acid {(S)-1-(4-dimethylamino-phenylcarbamoyl)-2-[4-(1-hydroxy-ethyl)-phenyl]-ethyl}-amide;
(S)-1-(3,5-Dichloro-benzenesulfonyl)-piperidine-2-carboxylic acid [(S)-2-(1H-indol-3-yl)-1-methylcarbamoyl-ethyl]-amide;
(S)-1-(3-Trifluoromethyl-benzenesulfonyl)-piperidine-2-carboxylic acid [(S)-2-(4-acetylphenyl)-1-(4-morpholin-4-yl-phenylcarbamoyl)-ethyl]-amide;
(S)-1-(3-Fluoro-benzenesulfonyl)-piperidine-2-carboxylic acid [(S)-2-(4-cyclopentyloxy-phenyl)-1-methyl-1-(tetrahydro-pyran-4-ylcarbamoyl)-ethyl]-amide;
(S)-1-(3-Fluoro-benzenesulfonyl)-piperidine-2-carboxylic acid [(S)-2-(4-isopropoxy-phenyl)-1-(2,2,6,6-tetramethyl-piperidin-4-ylcarbamoyl)-ethyl]-amide;
(S)-1-(3-Fluoro-benzenesulfonyl)-(2S)-piperidine-2-carboxylic acid [(S)-2-[4-(3-fluoro-propoxy)-phenyl]-1-(2,2,6,6-tetramethyl-piperidin-4-ylcarbamoyl)-ethyl]-amide;
(S)=1-(3-Fluoro-benzenesulfonyl)-piperidine-2-carboxylic acid [(S)-2-[4-(3-fluoro-propoxy)-phenyl]-1-((S)-2-methoxy-1-methyl-ethylcarbamoyl)-ethyl]-amide;
(S)-1-(3-Trifluoromethyl-benzenesulfonyl)-piperidine-2-carboxylic acid [(S)-2-(4-isopropoxy-phenyl)-1-methylcarbamoyl-ethyl]-amide;
(S)-1-(3-Trifluoromethyl-benzenesulfonyl)-piperidine-2-carboxylic acid [(S)-2-(4-isopropoxy-phenyl)-1-(tetrahydro-pyran-4-ylcarbamoyl)-ethyl]-amide;
(S)-1-(3-Trifluoromethyl-benzenesulfonyl)-piperidine-2-carboxylic acid [(S)-2-(phenyl)-1-(benzimidazol-2-yl)-ethyl]-amide;
(S)-1-(3-Trifluoromethyl-benzenesulfonyl)-piperidine-2-carboxylic acid [(S)-2-(4-(benzyloxy)phenyl)-1-(benzimidazol-2-yl)-ethyl]-amide;
(S)-1-(3-Fluoro-benzenesulfonyl)-piperidine-2-carboxylic acid [(S)-2-(4-methoxy-phenyl)-1-(tetrahydropyran-4-yl-carbamoyl)-ethyl]-amide;
(S)-1-Benzenesulfonyl-piperidine-2-carboxylic acid ((S)-1-amido-2-(2-methylphenyl)-ethyl)-amide;
(S)-1-Benzenesulfonyl-piperidine-2-carboxylic acid ((S)-1-cyclohexylamido-2-(4-methylphenyl)-ethyl)-amide;
(S)-1-Benzenesulfonyl-piperidine-2-carboxylic acid [(S)-2-(4-allyloxy-phenyl)-1-cyclohexylamido-ethyl]-amide;
(S)-1-(3-Trifluoromethyl-benzenesulfonyl)-piperidine-2-carboxylic acid [(S)-2-(4-allyloxy-phenyl)-1-cyclohexylcarbamoyl-ethyl]-amide;
(S)-1-(3-Trifluoromethyl-benzenesulfonyl)-piperidine-2-carboxylic acid ((S)-1-cyclohexylamido-2-(4-methylphenyl)-ethyl)-amide;
(S)-1-((E)-2-Phenyl-ethenesulfonyl)-piperidine-2-carboxylic acid [(S)-1-amido-2-(1H-indol-3-yl)-ethyl]-amide;
(S)-1-(4,5-Dibromo-thiophene-2-sulfonyl)-piperidine-2-carboxylic acid [(S)-1-amido-2-(1H-indol-3-yl)-ethyl]-amide;
(S)-1-Benzenesulfonyl-piperidine-2-carboxylic acid [(S)-1-tert-butylamido-2-(1H-indol-3-yl)-ethyl]-amide;
(S)-1-Benzenesulfonyl-piperidine-2-carboxylic acid [(S)-1-(3-hydroxy-2,2-dimethyl-propylamido)-2-(4-isopropyl-phenyl)-ethyl]-amide;
(S)-1-Benzenesulfonyl-piperidine-2-carboxylic acid methyl-((S)-1-methylamido-2-(phenyl)-ethyl)-amide;
(S)-1-Benzenesulfonyl-piperidine-2-carboxylic acid [(S)-1-(3,3-dimethyl-butylamido)-2-(4-methoxyphenyl)-ethyl]-amide;
(S)-1-Benzenesulfonyl-piperidine-2-carboxylic acid [(S)-1-(2,2-dimethyl-propylamido)-2-(phenyl)-ethyl]-amide;
(4-{(S)-2-[((S)-1-Benzenesulfonyl-piperidine-2-carbonyl)-amino]-2-amido-ethyl}-benzyl)-phosphonic acid diethyl ester;
(S)-1-Benzenesulfonyl-piperidine-2-carboxylic acid [(S)-2-(4-(acetylamino)-phenyl)-1-amido-ethyl]-amide;
(S)-1-Benzenesulfonyl-piperidine-2-carboxylic acid [(S)-1-amido-2-(4-(hydroxymethyl)-phenyl)-ethyl]-amide;
(S)-1-(Naphthalene-2-sulfonyl)-piperidine-2-carboxylic acid [(S)-1-amido-2-(1H-indol-3-yl)-ethyl]-amide;
(S)-1-(3-Trifluoromethyl-benzenesulfonyl)-piperidine-2-carboxylic acid [(S)-1-(cyclohexylmethyl-amido)-2-(4-methylphenyl)ethyl]-amide;
(S)-1-(3-Trifluoromethyl-benzenesulfonyl)-piperidine-2-carboxylic acid [(S)-1-(4-amino-butylamido)-2-(4-methylphenyl)-ethyl]-amide;
(S)-1-Benzenesulfonyl-piperidine-2-carboxylic acid [(S)-1-amido-2-(1H-indol-3-yl)-ethyl]-amide;
(S)-1-Benzenesulfonyl-piperidine-2-carboxylic acid ((S)-1-amido-2-(pyridin-4-yl)-ethyl)-amide;
(S)-1-Benzenesulfonyl-piperidine-2-carboxylic acid ((S)-1-amido-2-cyclohexyl-ethyl)-amide;
(S)-1-Benzenesulfonyl-piperidine-2-carboxylic acid {(S)-2-phenyl-1-[(thiophen-2-ylmethyl)-amido]-ethyl}-amide;
(S)-1-(3-Fluoro-benzenesulfonyl)-piperidine-2-carboxylic acid [(S)-1-(4-dimethylamino-butylcarbamoyl)-2-(4-fluoro-phenyl)-ethyl]-amide;
(S)-1-(3-Fluoro-benzenesulfonyl)-piperidine-2-carboxylic acid [(S)-2-[4-(3-methoxy-propoxy)-phenyl]-1-(tetrahydro-pyran-4-ylcarbamoyl)-ethyl]-amide;
(S)-1-(3-Trifluoromethyl-benzenesulfonyl)-piperidine-2-carboxylic acid {(S)-1-cyclohexylcarbamoyl-2-[4-(2-dimethylamino-ethoxy)-phenyl]-ethyl}-amide;
(S)-1-(3-Trifluoromethyl-benzenesulfonyl)-piperidine-2-carboxylic acid [(S)-2-(4-methoxyphenyl)-1-(2-piperazin-1-yl-ethylcarbamoyl)-ethyl]-amide;
(S)-1-(3-Trifluoromethyl-benzenesulfonyl)-piperidine-2-carboxylic acid [(S)-1-(2-hydroxy-1,1-dimethyl-ethylcarbamoyl)-2-(4-methoxy-phenyl)-ethyl]-amide;
(S)-1-Benzenesulfonyl-piperidine-2-carboxylic acid ((S)-1-carbamoyl-2-phenyl-ethyl)-amide;
(S)-1-(3-Fluoro-benzenesulfonyl)-piperidine-2-carboxylic acid {(S)-2-[4-(3-methoxy-propoxy)-phenyl]-1-methylcarbamoyl-ethyl}-amide;
(S)-1-(3-Fluoro-benzenesulfonyl)-piperidine-2-carboxylic acid {(S)-1-((S)-2-methoxy-1-methyl-ethylcarbamoyl)-2-[4-(3-methoxy-propoxy)-phenyl]-ethyl}-amide; and
(S)-1-(3-Hydroxy-benzenesulfonyl)-piperidine-2-carboxylic acid [(S)-2-(1H-indol-3-yl)-1-(naphthalen-2-ylcarbamoyl)-ethyl]-amide.

6. A pharmaceutical composition composing a compound, including enantiomers, stereoisomers, rotamers and tautomers of said compound and pharmaceutically acceptable salts, or solvates thereof, having the general structure shown in formula I as defined in claim 1.

7. The pharmaceutical composition according to claim 6, wherein said compound of formula I is as defined in claim 2.

8. The pharmaceutical composition according to claim 7, wherein said compound of formula I is as defined in claim 3.

9. The pharmaceutical composition according to claim 8, wherein said compound or formula I is as defined in claim 4.

10. A pharmaceutical composition according to claim 7, wherein the compound is selected from:
(S)-1-(3-Trifluoromethyl-benzenesulfonyl)-piperidine-2-carboxylic acid [(S)-1-(4-dimethylamino-phenylcarbamoyl)-2-(4-methoxy-phenyl)-ethyl]-amide;
(S)-1-(3-Trifluoromethyl-benzenesulfonyl)-piperidine-2-carboxylic acid [(S)-2-(4-acetylphenyl)-1-(4-ethoxy-phenylcarbamoyl)-ethyl]-amide;
(S)-1-(3-Trifluoromethyl-benzenesulfonyl)-piperidine-2-carboxylic acid [(S)-1-(4-morpholin-4-yl-phenylcarbamoyl)-2-(4-trifluoromethyl-phenyl)-ethyl]-amide;
(S)-1-(3-Trifluoromethyl-benzenesulfonyl)-piperidine-2-carboxylic acid [(S)-2-(4-methoxyphenyl)-1-(tetrahydro-pyran-4-ylcarbamoyl)-ethyl]-amide;
(S)-1-(3-Trifluoromethyl-benzenesulfonyl)-piperidine-2-carboxylic acid [(S)-2-(2-methyl-4-methoxy-phenyl)-1-(tetrahydro-pyran-4-ylcarbamoyl)-ethyl]-amide;
(S)-1-(3-Trifluoromethyl-benzenesulfonyl)-piperidine-2-carboxylic acid [(S)-2-(3,4-dimethoxy-phenyl)-1-(tetrahydro-pyran-4-yl-carbamoyl)-ethyl]-amide;
(S)-1-(3-Trifluoromethyl-benzenesulfonyl)-piperidine-2-carboxylic acid [(S)-2-(4-methoxyphenyl)-1-(4-morpholin-4-yl-phenylcarbamoyl)-ethyl]-amide;
(S)-1-(3-Methoxy-benzenesulfonyl)-piperidine-2-carboxylic acid ((S)1-carbamoyl-2-phenylethyl)-amide;
(S)-1-(3-Hydroxy-benzenesulfonyl)-piperidine-2-carboxylic acid ((S)-1-carbamoyl-2-phenylethyl)-amide;
(S)-1-(3-Propoxy-benzenesulfonyl)-piperidine-2-carboxylic acid ((S)-1-carbamoyl-2-phenylethyl)-amide;
(S)-1-(3-Trifluoromethyl-benzenesulfonyl)-piperidine-2-carboxylic acid [(S)-2-[4-(1-hydroxy-ethyl)-phenyl]-1-(4-morpholin-4-yl-phenylcarbamoyl)-ethyl]-amide;
(S)-1-(3-Trifluoromethyl-benzenesulfonyl)-piperidine-2-carboxylic acid {(S)-1-(4-ethoxy-phenylcarbamoyl)-2-[4-(1-hydroxy-ethyl)-phenyl]-ethyl}-amide;
(S)-1-(3-Methoxy-benzenesulfonyl)-piperidine-2-carboxylic acid [(S)-2-(indol-3-yl)-1-(naphthalen-2-ylcarbamoyl)-ethyl]-amide;
(S)-1-(3-Trifluoromethyl-benzenesulfonyl)-piperidine-2-carboxylic acid {(S)-1-(4-dimethylamino-phenylcarbamoyl)-2-[4-(1-hydroxy-ethyl)-phenyl]-ethyl}-amide;
(S)-1-(3,5-Dichloro-benzenesulfonyl)-piperidine-2-carboxylic acid [(S)-2-(1H-indol-3-yl)-1-methylcarbamoyl-ethyl]-amide;
(S)-1-(3-Trifluoromethyl-benzenesulfonyl)-piperidine-2-carboxylic acid [(S)-2-(4-acetylphenyl)-1-(4-morpholin-4-yl-phenylcarbamoyl)-ethyl]-amide;
(S)-1-(3-Fluoro-benzenesulfonyl)-piperidine-2-carboxylic acid [(S)-2-(4-cyclopentyloxy-phenyl)-1-methyl-1-(tetrahydro-pyran-4-ylcarbamoyl)-ethyl]-amide;
(S)-1-(3-Fluoro-benzenesulfonyl)-piperidine-2-carboxylic acid [(S)-2-(4-isopropoxy-phenyl)-1 -(2,2,6,6-teunnethyl-piperidin-4-yloarbalnoyl)-ethyl],anfldej
(S)-1-(3-Fluoro-benzenesulfonyl)-(2S)-piperidine-2-carboxylic acid [(S)-2-[4-(3-fluoro-propoxy)-phenyl]-1-(2,2,6,6-tetramethyl-piperidin-4-ylcarbamoyl)-ethyl]-amide;
(S)-1-(3-Fluoro-benzenesulfonyl)-piperidine-2-carboxylic acid [(S)-2-[4-(3-fluoro-propoxy)-phenyl]-1-((S)-2-methoxy-1-methyl-ethylcarbamoyl)-ethyl]-amide;
(S)-1-(3-Trifluoromethyl-benzonesulfonyl)-piperidine-2-carboxylic acid [(S)-2-(4-isopropoxy-phenyl)-1-methylcarbamoyl-ethyl]-amide;
(S)-1-(3-Trifluoromethyl-benzenesulfonyl)-piperidine-2-carboxylic acid [(S)-2-(4-isopropoxy-phenyl)-1-(tetrahydro-pyran-4-ylcarbamoyl)-ethyl]-amide;
(S)-1-(3-Trifluoromethyl-benzenesulfonyl)-piperidine-2-carboxylic acid [(S)-2-(phenyl)-1-(benzimidazol-2-yl)-ethyl]-amide;
(S)-1-(3-Trifluoromethyl-benzenesulfonyl)-piperidine-2-carboxylic acid [(S)-2-(4-(benzyloxy)phenyl)-1-(benzimidazol-2-yl)-ethyl]-amide;
(S)-1-(3-Fluoro-benzenesulfonyl)-piperidine-2-carboxylic acid [(S)-2-(4-methoxy-phenyl)-1-(tetrahydropyran-4-yl-carbamoyl)-ethyl]-amide;
(S)-1-Benzenesulfonyl-piperidine-2-carboxylic acid ((S)-1-amido-2-(2-methylphenyl)-ethyl)-amide;
(S)-1-Benzenesulfonyl-piperidine-2-carboxylic acid ((S)-1-cyclohexylamido-2-(4-methylphenyl)-ethyl)-amide;
(S)-1-Benzenesulfonyl-piperidine-2-carboxylic acid [(S)-2-(4-allyloxy-phenyl)-1-cyclohexylamido-ethyl]-amide;
(S)-1-(3-Trifluoromethyl-benzenesulfonyl)-piperidine-2-carboxylic acid [(S)-2-(4-allyloxy-phenyl)-1-cyclohexylcarbamoyl-ethyl]-amide;
(S)-1-(3-Trifluoromethyl-benzenesulfonyl)-piperidine-2-carboxylic acid ((S)-1-cyclphexylamido-2-(4-methylphenyl)-ethyl)-amide;
(S)-1-((E)-2-Phenyl-ethenesulfonyl)-piperidine-2-carboxylic acid [(S)-1-amido-2-(1H-indol-3-yl)-ethyl]-amide;
(S)-1-(4,5-Dibromo-thiophene-2-sulfonyl)-piperidine-2-carboxylic acid [(S)-1-amido-2-(1H-indol-3-yl)-ethyl]-amide;
(S)-1-Benzenesulfonyl-piperidine-2-carboxylic acid (S)-1-tert-butylamido-2-(1H-indol-3-yl)-ethyl]-amide;
(S)-1-Henzenesulfonyl-piperidine-2-carboxylic acid [(S)-1-(3-hydroxy-2,2-dimethyl-propylamido)-2-(4-isopropyl-phenyl)-ethyl]-amide;
(S)-1-Benzenesulfonyl-piperidine-2-carboxylic acid methyl-((S)-1-methylamido-2-(phenyl)-ethyl)-amide;
(S)-1-Benzenesulfonyl-piperidine-2-carboxylic acid [(S)-1-(3,3-dimethyl-butylamido)-2-(4-methoxyphenyl)-ethyl]-amide;
(S)-1-Benzenesulfonyl-piperidine-2-carboxylic acid [(S)-1-(2,2-dimethyl-propylamido)-2-(phenyl)-ethyl]-amide;
(4-{(S)-2-[((S)-1-Benzenesulfonyl-piperidine-2-carbonyl)-amino]-2-amido-ethyl}-benzyl)-phosphonic acid diethyl ester;
(S)-1-Benzenesulfonyl-piperidine-2-carboxylic acid [(S)-2-(4-(acetylamino)-phenyl)-1-amido-ethyl]-amide;
(S)-1-Benzenesulfonyl-piperidine-2-carboxylic acid [(S)-1-amido-2-(4-(hydroxymethyl)-phenyl)-ethyl]-amide;
(S)-1-(Naphthalene-2-sulfonyl)-piperidine-2-carboxylic acid [(S)-1-amido-2-(1H-indol-3-yl)-ethyl]-amide;
(S)-1-(3-Trifluoromethyl-benzenesulfonyl)-piperidine-2-carboxylic acid [(S)-1-(cyclohexylmethyl-amido)-2-(4-methylphenyl)ethyl]-amide;
(S)-1-(3-Trifluoromethyl-benzenesulfonyl)-piperidine-2-carboxylic acid [(S)-1-(4-amino-butylamido)-2-(4-methylphenyl)-ethyl]-amide;
(S)-1-Benzesulfonyl-piperidine-2-carboxylic acid [(S)-1-amido-2-(1H-indol-3-yl)-ethyl]-amide;
(S)-1-Benzenesulfonyl-piperidine-2-carboxylic acid ((S)-1-anudo-2-(pyridin-4-yl)-ethyl)-amide;
(S)-1-Benzenesulfonyl-piperidine-2-carboxylic acid ((S)-1-amido-2-cyclohexyl-ethyl)-amide;
(S)-1-Benzenesulfonyl-piperidine-2-carboxylic acid {(S)-2-phenyl-1-[(thiophen-2-ylmethyl)-amido]-ethyl}-amide;
(S)-1-(3-Fluoro-benzenesulfonyl)-piperidine-2-carboxylic acid [(S)-1-(4-dimethylamino-butylcarbamoyl)-2-(4-fluoro-phenyl)-ethyl]-amide;
(S)-1-(3-Fluoro-benzenesulfonyl)-piperidine-2-carboxylic acid [(S)-2-[4-(3-methoxy-pxopoxy)-phenyl]-1-(tetrahydro-pyran-4-ylcarbamoyl)-ethyl]-amide;
(S)-1-(3-Trifluoromethyl-benzenesulfonyl)-piperidine-2-carboxylic acid {(S)-1-cyclohexylcarbamoyl-2-[4-(2-dimethylamino-ethoxy)-phenyl]-ethyl}-amide;
(S)-1-(3-Trifluoromethyl-benzenesulfonyl)-piperidine-2-carboxylic acid [(S)-2-(4-methoxyphenyl)-1-(2-piperazin-1-yl-ethylcarbamoyl)-ethyl]-amide;
(S)-1-(3-Trifluoromethyl-benzenesulfonyl)-piperidine-2-carboxylic acid [(S)-1-(2-hydroxy-1,1-dimethyl-ethylcarbamoyl)-2-(4-methoxy-phenyl)-ethyl]-amide;
(S)-1-Benzenesulfonyl-piperidine-2-carboxylic acid ((S)-1-carbamoyl-2-phenyl-ethyl)-amide;
(S)-1-(3-Fluoro-benzenesulfonyl)-piperidine-2-carboxylic acid {(S)-2-[4-(3-methoxy-propoxy)-phenyl]-1-methylcarbamoyl-ethyl}-amide;
(S)-1-(3-Fluoro-benzenesulfonyl)-piperidine-2-carboxylic acid {(S)-1-((S)-2-methoxy-1-methyl-ethylcarbamoyl)-2-[4-(3-methoxy-propoxy)-phenyl]-ethyl}-amide; and
(S)-1-(3-Hydroxy-benzenesulfonyl)-piperidine-2-carboxylic acid [(S)-2-(1H-indol-3-yl)-1-(naphthalen-2-ylcarbamoyl)-ethyl]-amide.

11. A compound having the general structure shown in formula I as defined, in claim 1, including enantiomers, stereoisomers, rotamers and tautomers of said compound and pharmaceutically acceptable salts, or solvates thereof, for the preparation of a medicament for treating a disorder involving neurological damage,

12. The compound according to claim 11, wherein the disorder is Parkinson's disease.

13. The compound according to claim 11, wherein the disorder is multiple sclerosis.

14. The compounds according to claim 11, wherein the disorder is Alzheimer's disease.

15. The compound according to claim 11, wherein the disorder is stroke.

16. The compound according to claim 11, wherein the disorder is spinal cord injury.

17. The compound according to claim 11, wherein said compound is as defined in claim 2.

18. The compound according to claim 11, wherein said compound is as defined in claim 3.

19. The compound according to claim 11, wherein said compound is as defined in claim 4.

20. The compound according to claim 11, wherein said compound is selected from
(S)-1-(3-Trifluoromethyl-benzenesulfonyl)-piperidine-2-carboxylic acid [(S)-1-(4-dimethylamino-phenylcarbamoyl)-2-(4-methoxy-phenyl)-ethyl]-amide;
(S)-1-(3-Trifluoromethyl-benzenesulfonyl)-piperidine-2-carboxylic acid [(S)-2-(4-acetylphenyl)-l-(4-ethoxy-phenylcarbamoyl)-ethyl]-amide;
(S)-1-(3-Trifluoromethyl-benzenesulfonyl)-piperidine-2-carboxylic acid [(S)-1-(4-morpholin-4-yl-phenylcarbamoyl)-2-(4-trifluoromethyl-phenyl)-ethyl]-amide;
(S)-1-(3-Trifluoromethyl-benzenesulfonyl)-piperidine-2-carboxylic acid [(S)-2-(4-methoxyphenyl)-1-(tetrahydro-pyran-4-ylcarbamoyl)-ethyl]-amide;
(S)-1-(3-Trifluoromethyl-benzenesulfonyl)-piperidine-2-carboxylic acid [(S)-2-(2-methyl-4-methoxy-phenyl)-1-(tetrahydro-pyran-4-ylcarbamoyl)-ethyl]-amide;
(S)-1-(3-Trifluoromethyl-benzenesulfonyl)-piperidine-2-carboxylic acid [(S)-2-(3,4-dimethoxy-phenyl)-1-(tetrahydro-pyran-4-yl-carbamoyl)-ethyl]-amide;
(S)-1-(3-Trifluoromethyl-benzenesulfonyl)-piperidine-2-carboxylic acid [(S)-2-(4-methoxyphenyl)-1-(4-morpholin-4-yl-phenylcarbamoyl)-ethyl]-amide;
(S)-1-(3-Methoxy-benzenesulfonyl)-piperidine-2-carboxylic acid ((S)1-carbamoyl-2-phenylethyl)-amide;
(S)-1-(3-Hydroxy-benzenesulfonyl)-piperidine-2-carboxylic acid ((S)-1-carbamoyl-2-phenylethyl)-amide;
(S)-1-(3-Propoxy-benzenesulfonyl)-piperidine-2-carboxylic acid ((S)-1-carbamoyl-2-phenylethyl)-amide;
(S)-1-(3-Trifluoromethyl-benzenesulfonyl)-piperidine-2-carboxylic acid [(S)-2-[4-(1-hydroxy-ethyl)-phenyl]-1-(4-morpholin-4-yl-phenylcarbamoyl)-ethyl]-amide;
(S)-1-(3-Trifluoromethyl-benzenesulfonyl)-piperidine-2-carboxylic acid {(S)-1-(4-ethoxy-phenylcarbamoyl)-2-[4-(1-hydroxy-ethyl)-phenyl]-ethyl]-amide;
(S)-1-(3-Methoxy-benzenesulfonyl)-piperidine-2-carboxylic acid [(S)-2-(indol-3-yl)-1-(naphthalen-2-ylcarbamoyl)-ethyl]-amide;
(S)-1-(3-Trifluoromethyl-benzenesulfonyl)-piperidine-2-carboxylic acid {(S)-1-(4-dimethylamino-phenylcarbamoyl)-2-[4-(1-hydroxy-ethyl)-phenyl]-ethyl}-amide;
(S)-1-(3,5-Dichloro-benzenesulfonyl)-piperidine-2-carboxylic acid [(S)-2-(1H-indol-3-yl)-1-methylcarbamoyl-ethyl]-amide;
(S)-1-(3-Trifluoromethyl-benzenesulfonyl)-piperidine-2-carboxylic acid [(S)-2-(4-acetylphenyl)-1-(4-morpholin-4-yl-phenylcarbamoyl)-ethyl]-amide;
(S)-1-(3-Fluoro-benzenesulfonyl)-piperidine-2-carboxylic acid [(S)-2-(4-cyclopentyloxy-phenyl)-1-methyl-1-(tetrahydro-pyran-4-ylcarbamoyl)-ethyl]-amide;
(S)-1-(3-Fluoro-benzenesulfonyl)-piperidine-2-carboxylic acid [(S)-2-(4-isopropoxy-phenyl)-1-(2,2,6,6-tetramethyl-piperidin-4-ylcarbamoyl)-ethyl]-amide;
(S)-1-(3-Fluoro-benzenesulfonyl)-(2S)-piperidine-2-carboxylic acid [(S)-2-[4-(3-fluoro-propoxy)-phenyl]-1-(2,2,6,6-tetramethyl-piperidin-4-ylcarbamoyl)-ethyl]-amide;
(S)-1-(3-Fluoro-benzenesulfonyl)-piperidine-2-carboxylic acid [(S)-2-[4-(3-fluoro-propoxy)-phenyl]-1-((S)-2-methoxy-1-methyl-ethylcarbamoyl)-ethyl]-amide;
(S)-1-(3-Trifluoromethyl-benzenesulfonyl)-piperidine-2-carboxylic acid [(S)-2-(4-isopropoxy-phenyl)-1-methylcarbamoyl-ethyl]-anaide;
(S)-1-(3-Trifluoromethyl-benzenesulfonyl)-piperidine-2-carboxylic acid [(S)-2-(4-isopropoxy-phenyl)-1-(tetrahydro-pyran-4-ylcarbamoyl)-ethyl]-amide;
(S)-1-(3-Trifluoromethyl-benzenesulfionyl)-piperidine-2-carboxylic acid [(S)-2-(phenyl)-1-(benzimidazol-2-yl)-ethyl]-amide;
(S)-1-(3-Trifluoromethyl-benzenesulfonyl)-piperidine-2-carboxylac acid [(S)-2-(4-(benxyloxy)phenyl)-1-(benzimidazol-2-yl)-ethyl]-amide;
(S)-1-(3-Fluoro-benzenesulfonyl)-piperidine-2-carboxylic acid [(S)-2-(4-methoxy-phenyl)-1-(tetrahydropyran-4-yl-carbamoyl)-ethyl]-amide;
(S)-1-Benzenesulfonyl-piperidine-2-carboxylic acid ((S)-1-amido-2-(2-methylphenyl)-ethyl)-amide;
(S)-1-Benzenesulfonyl-piperidine-2-carboxylic acid ((S)-1-cyclohexylamido-2-(4-methylphenyl)-ethyl)-amide;
(S)-1-Benzenesulfonyl-piperidine-2-carboxylic acid [(S)-2-(4-allyloxy-phenyl)-1-cyclohexylamido-ethyl]-amide;
(S)-1-(3-Trifluoromethyl-benzenesulfonyl)-piperidine-2-carboxylic acid [(S)-2-(4-allyloxy-phenyl)-1-cyclohexylcarbamoyl-ethyl]-amide;
(S)-1-(3-Trifluoromethyl-benzenesulfonyl)-piperidine-2-carboxylic acid ((S)-1-cyclohexylamido-2-(4-methylphenyl)-ethyl)-amide;
(S)-1-((E)-2-Phenyl-ethenesulfonyl)-piperidine-2-carboxylic acid [(S)-1-amido-2-(1H-indol-3-yl)-ethyl]-amide;
(S)-1-(4,5-Dibromo-thiophene-2-sulfonyl)-piperidine-2-carboxylic acid [(S)-1-amido-2-(1H-indol-3-yl)-ethyl]-amide;
(S)-1-Benzonesulfonyl-piperidine-2-carboxylic acid [(S)-1-tert-butylamido-2-(1H-indol-3-yl)-ethyl]-amide;
(S)-1-Benxenesulfonyl-piperidine-2-carboxylic acid [(S)-1-(3-hydroxy-2,2-dimethyl-propylamido)-2-(4-isopropyl-phenyl)-ethyl]-amide;
(S)-1-Benzenesulfonyl-piperidine-2-carboxylic acid methyl-((S)-1-methylamido-2-(phenyl)-ethyl)-amide;
(S)-1-Benzenesulfonyl-piperidine-2-carboxylic acid [(S)-1-(3,3-dimethyl-butylamido)-2-(4-methoxyphenyl)-ethyl]-amide;
(S).I .Benzenesuvbnyl-piperidine-2-carboxylic acid [(S)-1-(2,2-dimethyl-propylamido)-2-(phenyl)-ethyl]-amide;
(4-{(S)-2-[((S)-1-Benzenesulfonyl-piperidine-2-carbonyl)-amino]-2-amido-ethyl} -benzyl)-phosphonic acid diethyl ester;
(S)-1-Benzenesulfonyl-piperidine-2-carboxylic acid -[(8)-2-(4-(acetylamino)-phenyl)-1-amido-ethyl]-amide;
(S)-1-Benzenesulfonyl-piperidine-2-carboxylic acid [(S)-1-amido-2-(4-(hydroxymethyl)-phenyl)-ethyl]-amide;
(S)-1-(Naphthalene-2-sulfonyl)-piperidine-2-carboxylic acid [(S)-1-amido-2-(1H-indol-3-yl)-ethyl]-amide;
(S)-1-(3-Trifluoromethyl-benzenesulfonyl)-piperidine-2-carboxylic acid [(S)-1-(cyclohexylmethyl-amido)-2-(4-methylphenyl)ethyl]-amide;
(S)-1-(3-Trifluoromethyl-benzenesulfonyl)-piperidine-2-carboxylic acid [(S)-1-(4-amino-butylamido)-2-(4-methylphenyl)-ethyl]-amide;
(S)-1-Benzenesulfonyl-piperidine-2-carboxylic acid [(S)-1-amido-2-(1H-indol-3-yl)-ethyl]-amide;
(S)-1-Benzenesulfonyl-piperidine-2-carboxylic acid ((S)-1-amido-2-(pyridin-4-yl)-ethyl)-amide;
(S)-1-Benzenesulfonyl-piperidine-2-carboxylic acid ((S)-1-annido-2-cyclohexyl-ethyl)-amide;
(S)-1-Benzenesulfonyl-piperidine-2-carboxylic acid {(S)-2 phenyl-1-[(thiophen-2-ylmethyl)-amido]-ethyl}-amide;
(S)-1-(3-Fluoro-benzenesulfonyl)-piperidine-2-carboxylic acid [(S)-1-(4-dimethylamino-butylcarbamoyl)-2-(4-fluoro-phenyl)-ethyl]-amide;
(S)-1-(3-Fluoro-benzenesulfonyl)-piperidine-2-carboxylic acid [(S)-2-[4-(3-methoxy-propoxy)-phenyl]-1-(tetrahydro-pyran-4-ylcarbamoyl)-ethyl]-amide;
(S)-1-(3-Trifluoromethyl-benzenesulfonyl)-piperidine-2-carboxylic acid {(S)-1-cyclohexylcarbamoyl-2-[4-(2-dimethylamino-ethoxy)-phenyl]-ethyl}-amide;
(S)-l-(3-Trifluoromethyl-benzenesulfonyl)-piperidine-2-carboxylic acid [(S)-2-(4-methoxyphenyl)-1-(2-piperazin-1-yl-ethylcarbamoyl)-ethyl]-amide;
(S)-1-(3- Trifluoromethyl-benzenesulfonyl)-piperidine-2-carboxylic acid [(S)-1-(2-hydroxy-1,1-dimethyl-ethylcarbamoyl)-2-(4-methoxy-phenyl)-ethyl]-amide;
(S)-1-Benzenesulfonyl-piperidine-2-carboxylic acid ((S)-1-carbamoyl-2-phenyl-ethyl)-amide;
(S)-1-(3-Fluoro-benzenesulfonyl)-piperidine-2-carboxylic acid {(S)-2-[4-(3-methoxy-propoxy)-phenyl]-1-methylcarbamoyl-ethyl}-anude;
(S)-1-(3-Fluoro-benzenesulfonyl)-piperidine-2-caxboxylic acid {(S)-1-((S)-2-methoxy-1-methyl-ethylcarbamoyl)-2-[4-(3-methoxy-propoxy)-phenyl]-ethyl}-amide; and
(S)-1-(3-Hydroxy-benzenesulfonyl)-piperidine-2-carboxylic acid [(S)-2-(1H-indol-3-yl)-1-(naphthalen-2-ylcarbamoyl)-ethyl]-amide.

## Patentansprüche

1. Verbindungen, einschließlich Enantiomere, Stereomere, Rotamere und Tautomere dieser Verwindungen und pharmazeutisch unbedenkliche Salze oder solvates davon, wobei die Verbindungen die allgemeine Struktur der allgemeinen Formel I: 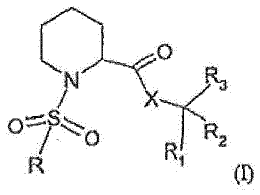 mit den folgenden Bedeutungen aufweiten:
R bedeutet Fluoralkyl der Formel CₙHₓF_{y}, wobei n eine ganze Zahl von 1 bis 6, x eine ganze Zahl von 0 bis 12 bedeutet, y eine ganze Zahl von 1 bis 13 bedeutet und die Summe von x und y 2n+1 beträgt, Acryl, Aryl-C₁₋₆-alkyl, Aryl-C₂₋₄-alkenyl, wobei Aryl ausgewählt ist aus der Reihe Phenyl, Biphenyl und Naphthyl, Heteroaryl oder Heteroaryl-C₁₋₄-alkyl, wobei Heteroaryl aus der Reihe Pyridyl, Thiophenyl und Benzothiophenyl ausgewählt ist und Heterocyclyl aus der Reihe Piperidinyl und Piperazinyl ausgewählt ist,
R₁ bedeutet Wasserstoff oder C₁₋₆-Alkyl;
R₂ bedeutet CONHR₄, Benzimidazol-2-yl, CR₅R₆OH oder CR₇R₈NHR₉;
R₃ bedeutet Aryl, Aryl-C₁₋₄-alkyl oder Aryl-C₂₋₄-alkenyl, wobei Aryl ausgewählt ist aus der Reihe Phenyl, Biphenyl, Naphthyl, Bisarylmethyl, Heteroaryl und Heteroaryl-C₁-₄-alkyl, wobei Heteroaryl aus der Reihe Indolyl, Thiophenyl, Benzothiophenyl, Furanyl, Benzofuranyl, 1,3-Benzodioxolyl, 1,4-Benzodioxanyl und C₅₋₇-Cycloalkyl-C₁-alkyl ausgewählt ist;
X bedeutet NR₁₀ oder CH₂;
wobei:
R₄ ausgewählt ist aus der Gruppe bestehend aus Wasserstoff, C₁₋₁₀-Alkyl, C₂₋₁₀-Alkenyl, C₃₋₈-Cycloalkyl, C₈₋₁₂-Bicycloalkyl, C₁₀₋₁₄-Tricycloalkyl-, C₃₋₈-Cycloalkyl-C₁₋₆-alkyl, C₈₋₁₂-Bicycloalkyl-C₁₋₆-alkyl, C₈₋₁₂-Bicycloalkylamino-C₁₋₆-alkyl, C₈₋₁₂-Tricycloalkylamino-C₁₋₆-alkyl, C₃₋₈-Cycloalkenyl, C₃₋₈-Cycloalkenyl-C₁₋₆-alkyl, Fluoralkyl der Formel CₙHₓF_{y}, wobei n eine ganze Zahl von 1 bis 20 bedeutet, x eine ganze Zahl von 0 bis 40 bedeutet, y eine ganze Zahl von 1 bis 41 bedeutet und die Summe von x und y 2n+1 beträgt, Amino-C₂₋₂₀-alkyl, Mono- oder Dialkylamino-C₂₋₂₀-alkyl, Mono-, Di- oder Trihydroxy-C₂₋₂₀-alkyl, C₁₋₆-Alkoxy-C₂₋₂₀-alkyl, C₁₋₂₀-Thioalkyl, C₁₋₆-Alkylthio-C₂₋₂₀-alkyl, Hydroxy-C₂₋₆-alkoxy-C₂₋₆-alkyl, C₁₋₆-Alkylbis-(hydroxy-C₁₋₆-alkyl), Aryl, Aryl-C₁₋₁₀-alkyl, Aryl-C₄₋₃-alkenyl, wobei Aryl ausgewählt ist aus der Reihe Phenyl, Biphenyl, Indanyl und Naphthyl, Heteroaryl oder Heteroaryl-C₁₋₁₀-alkyl, wobei Heteroaryl aus der Reihe Pyridyl, Thiophenyl, Furanyl, Benzofuranyl, Benzothiophenyl, Pyrrolidinyl und Indolyl ausgewählt ist; Heterocycloalkyl oder Heterocyclo-alkyl-C₁₋₁₀-alkyl, wobei Heterocycloalkyl aus der Reihe Tetrahydrofuranyl, Tetrahydropyranyl, Morpholinyl, Piperidinyl, Pyrrolidinyl, Tetrahydrothiophenyl, Piperazinyl, Dioxolanyl, Sulfolanyl und 1,1-Dioxotetrahydrothiopyranyl ausgewählt ist; Heterobicyclyl der Formel; 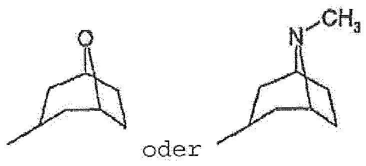 umfassend C₅₋₈-Cycloalkyl, C₈-₁₂-Bicycloalkyl und/oder Heterocyclyl, gegebenenfalls substituiert durch einen oder mehrere Substituenten, ausgewählt aus der Gruppe bestehend aus C₁₋₁₀-Alkyl, C₁₋₁₀-Alkoxy, Hydroxy, Hydroxy-C₁₋₁₀-alkoxy, Hydroxy-C₁₋₁₀-alkyl, C₁₋₁₀-Acyloxy, Amine, C₁₋₁₀-Alkylcarbonyl-amino, Hydroxy-C₁₋₂₀-alkylcarbonylamino, Amino-C₂₋₁₀-alkoxy, Mono-C₁₋₆-alkylamino-C₂₋₁₀-alkoxy, Di-C-₁₋₆-alkylamino-C₂-₁₀-alkoxy, Mono-(C₁₋₁₀-alkyl)amino, Di-(C₁₋₁₀-alkyl) amino, Amino-C₁₋₁₀-alkylcarbonylamino, C₁₋₁₀-Alkylaminocarbonyl, Hydroxy-C₁₋₁₀-alkylamino-carbonyl, Amino-C₂₋₁₀-alkylaminocarbonyl, Mono- oder Dialkylamino-C₁₋₁₀-alkylaminocarbonyl, Oxo, gegebenenfalls substituiertes Aryl und gegebenenfalls substituiertes Heteroaryl, wobei Aryl aus der Reihe Phenyl, Naphthyl unde Biphenyl ausgewählt ist und Heteroaryl aus der Reihe Pyridyl und Thiophenyl ausgewählt ist; und
wobei: die Acryl- oder Heteroarylgruppen von R gegebenenfalls durch einen oder mehrere Substituenten aus der folgenden Gruppe substituiert sind: Fluor, Chlor, Brom, C₁₋₂₀-Alkyl, Fluoralkyl oder Fluoralkoxy der Formel CₙHₓF_{y} oder OCₙHₓF_{y}, wobei n eine ganze Zahl von 1 bis 4 bedeutet, x eine ganze Zahl von 0 bis 8 bedeutet, y eine ganze Zahl von 1 bis 9 bedeutet und die Summe von x und y 2n+1 beträgt, C₁₋₂₀-Alkoxy, C₁₋₂₀-Alkenyloxy, C₁₋₁₀-Alkoxy-C₁₋₁₀-alkoxy, Hydroxy, Hydroxy-C₁₋₂₀-alkoxy, Hydroxy-C₁₋₂₀-alkyl, C₁₋₂₀-Acyloxy, Hydroxy-C₁₋₂₀-acyl, Nitro, Amino, Aminosulfonyl, C₁₋₂₀-Alkylcarbonylamino, Hydroxy- C₁₋₂₀-alkylcarbonylamino, Amino-C₂₋₂₀-alkoxy, Amino-C₁₋₂₀-alkylcarbonylamino, C₁₋₂₀-Alkylaminocarbonyl, Hydroxy-C₁₋₂₀-alkylaminocarbonyl, Amino-C₁₋₂₀-alkylaminocarbonyl, Mono- oder Dialkylamino-C₁₋₂₀-alkylaminocarbonyl, gegebenenfalls substituiertes Aryl- oder Heteroarylsulfonyl, Aminosulfonyl, Mono- oder Di(C₁₋₆)alkylaminosulfonyl, gegebenenfalls substituiertes Phenyl oder Thiophenyl, gegebenenfalls substituiertes Phenyl-oder Thiophenyl-C₁₋₁₀-alkyloxy und Cyano;
wobei: der Phenyl- oder Thiophenylsubstituent an der Aryl- oder Heteroarylgruppe von R gegebenenfalls durch einen oder mehrere Substituenten aus der folgenden Gruppe substituiert ist: Fluor, color, Brom, C₁₋₅-Alkyl, Fluoralkyl oder Fluoralkoxy der Formel CₙHₓF_{y} oder OCₙHₓF_{y}, wobei n eine ganze Zahl von 1 bis 4 bedeutet, x eine ganze Zahl von 0 bis 8 bedeuten, y eine ganze Zahl von 1 bis 9 bedeutet und die Summe von x und y 2n+1 beträgt, C₁₋₁₀-Alkoxy, C₁₋₁₀-Alkenyloxy, C₁₋₅-Alkoxy-C₁₋₅-alkoxy, Hydroxy, Hydroxy-C₁₋₁₀-alkoxy, Hydroxy-C₁₋₁₀-alkyl, Hydroxy-C₁₋₂₀-alkyloxo, Nitro, Amine, Aminosulfonyl, C₁₋₁₀-Alkylcarbonylamino, Hydroxy-C₁₋₁₀-alkylcarbonylamino, Amino-C₂₋₁₀-alkoxy, Amino-C₁₋₁₀-alkylcarbonylamino, C₁₋₁₀-Alkylaminocarbonyl, Hydroxy-C₁-₁₀-alkylaminocarbonyl, Amino-C₁₋₁₀-alkylaminocarbonyl, Mono- oder Dialkylamino-C₁₋₁₀-alkylaminocarbonyl, gegebenenfalls substituiertes Aryl-C₁₋₁₀-alkyloxy, und Cyano;
wobei: das Aryl oder Heteroaryl von R₃ gegebenenfalls durch einen oder mehr Substituenten aus der folgenden Reihe substituiert ist: C₃₋₈-Cycloalkyloxy, C₁₋₁₀-Alkoxy, C₁₋₁₀-Alkyl, C₂₋₁₀ -Alkenyl, C-₃₋₁₀-Alkenyloxy, Mono-(C₁₋₆-alkyl)amino-C₂₋₆-alkyloxy, Di-(C₁₋₆-alkyl)amino-C₂₋₆-alkyloxy, Amino-C₂₋₆-alkyloxy, Heterocyclyl-C₂₋₆-alkyloxy, Aryl-C₂₋₆-alkyloxy, Heteroaryl-C₂₋₆-alkyloxy, Fluoralkyl oder Fluoralkoxy der Formel CₙHₓF_{y} oder OCₙHₓF_{y}, wobei n eine ganze Zahl von 1 bis 4 bedeutet, x eine ganze Zahl von 0 bis 8 bedeutet, y eine ganze Zahl von 1 bis 9 bedeutet und die Summe von x und y 2n+1 beträgt, C₁₋₀Alkoxy-C₂₋₆-alkoxy, Aryl-C₁-₆-alkyloxy, Hydroxy, chlor, Fluor, Brom, Hydroxy-C₁₋₆-alkoxy, Hydroxy-C₁₋₁₀-alkyl, C₁₋₆-Alkyloxo, Cyano, Amino, 4-Aryloxo, Di-C₁₋₅-alkylphosphityl-C₁-₅-alkyl, C₁₋₆-Alkyloxoamino, C₁₋₆-Alkyloxo(C₁₋₆-alkyl)amino, C₁₋₁₀-Alkylsulfonyl, Arylsulfonyl, Carboxy, C₁₋₂₀-Carboalkoxy, Dialkylamino-C₁₋₁₀-alkyl, Monoalkylamino-C₁₋₁₀-alkyl und Amino-C₁₋₁₀-alkyl;
wobei: die Heterocycloalkyl-Substituentengruppe, die an die Alkyloxygruppe von R₃ gebunden ist, aus der folgenden Reihe ausgewählt ist: Pyrrolidinyl, Morpholinyl, Piperidinyl und Piperazinyl;
wobei: die Heteroarylsubstituentengruppe, die an die Alkyloxygruppe von R₃ gebunden ist, aus der Reihe Pyridinyl und Pyrrolidinyl ausgewählt ist;
wobei: das Acryl des Arylsulfonylsubstituenten am Acryl von R₃ aus der Reihe Phenyl, Indolyl, Thiophenyl und Furanyl ausgewählt ist;
wobei: die Acryl- oder Heteroarylgruppen von R₄ gegebenenfalls durch einen oder mehrere Substituenten aus der folgenden Reihe substituiert sind: Mono- oder Di(C₁₋₆-alkyl)amino, Mono- oder Di(C₁₋₆)alkylamino-C₂₋₆-alkyl, Amino-C₁₋₆-alkyl, Heterocycloalkyl, Amino, C₁₋₂₀-Alkoxy, Chlor, Fluor, Brom, C₁₋₁₀-Alkyl, Aryl und Heteroaryl;
wobei: die Heterocycloalkylgruppe, die an das Acryl von R₄ gebunden ist, aus der folgenden Reihe ausgewählt ist: Morpholinyl, Pyrrolidinyl, Piperazinyl und Piperidinyl;
wobei: die Heteroarylgruppe, die an das Aryl von R₄ gebunden ist, aus der Reihe Pyridyl, Pyrrolyl und Furanyl ausgewählt ist;
R₅, R₆ = unabhängig H oder C₁₋₆-Alkyl;
R₇, R₈ = unabhängig H oder C₁₋₆-Alkyl;
R₉ = Di-(C₁₋₅-alkyl)amino-C₂₋₄-alkyl, Mono(C₁₋₅-alkyl)amino-C₃₋₄-alkyl; Amino-C₂₋₄-alkyl oder Heterocycloalkylmethyl, wobei Heterocycloalkyl aus der Gruppe bestehend aus Thiophenyl, Furanyl, Pyrrolyl und Pyridinyl ausgewählt ist; und
R₁₀ = H oder C₁₋₆-Alkyl, mit Aufnahme der Verbindung, in der R ein durch eine Methylgruppe in para-Stellung substituiertes Phenyl bedeutet, R₁ Wasserstoff bedeutet, R₂ CR₅R₆OH bedeutet, R₃ Phenylmethyl bedeutet, X NR₁₀ bedeutet, R₅, R₆ und R₁₀ Wasserstoff bedeuten.

2. Verbindung nach Anspruch 1, mit den folgenden Bedeutungen:
R stammt aus der Gruppe bestehend aus Aryl, Aryl-C₁₋₆-alkyl, Aryl-C₂₋₄-alkenyl, wobei Acryl aus der Gruppe Phenyl, Biphenyl oder Naphthyl ausgewählt ist; Heteroaryl oder Heteroaryl-C₁₋₄-alkyl, wobei Heteroaryl aus der Reihe Pyridyl, Thiophenyl oder Benzothiophenyl ausgewählt ist; und Heterocyclyl, das aus der Reihe Piperidinyl oder Piperazinyl ausgewählt ist;
R₁ bedeutet Wasserstoff oder Methyl;
R₂ bedeutet CONHR₄, Benzimidazol-2-yl, CR₅R₆OH oder CR₇R₈NHR₉;
R₃ stammt aus der Gruppe bestehend aus Phenyl und Aryl-C₁₋₄-alkyl oder Aryl-C₂₋₄-alkenyl, wobei Aryl ausgewählt ist aus der Reihe Phenyl, Biphenyl, Naphthyl oder Bisarylmethyl, und Heteroaryl oder Heteroaryl-C₁₋₄-alkyl, wobei Heteroaryl aus der Reihe Indolyl und Thiophenyl, ausgewählt ist;
X bedeuten NR₁₀ oder CH₂; wobei R₄ ausgewählt ist aus der Gruppe bestehend aus Wasserstoff, C₁₋₁₀-Alkyl, C₂₋₁₀-Alkenyl, C₃₋₈-Cycloalkyl, C₈₋₁₂-Bicycloalkyl, C₁₀₋₁₄-Tricycloalkyl, C₃₋₈-Cycloalkyl-C₁₋₆-alkyl, C₈₋₁₂-Bicycloalkyl-C₁₋₆-alkyl, C₈₋₁₂-Bicycloalkylamino-C₁₋₆-alkyl, C₈₋₁₂-Tricycloalkylamino-C₁₋₆-alkyl, C₃₋₈-Cycloalkenyl, C₃₋₈-Cycloalkenyl-C₁₋₆-alkyl, Fluoralkyl der Formel CₙHₓF_{y}, in der n eine ganze Zahl von 1 bis 10 bedeutet, x eine ganze Zahl von 0 bits 20 bedeutet, y eine ganze Zahl von 1 bis 21 bedeutet, und die Summe von x und y 2n+1 beträgt, Amino-C₂₋₁₀-alkyl, Mono- oder Dialkylamino-C₂₋₁₀-alkyl, Mono-, Di- oder Trihydroxy-C₂₋₁₀-alkyl, C₁₋₆-Alkoxy-C₂₋₁₀-alkyl, C₁₋₁₀-Thioalkyl, C₁₋₆-Alkylthio-C₂₋₁₀-alkyl, Hydroxy-C₂₋₆-alkoxy-C₂₋₆-alkyl, C₁₋₆-Alkylbis-(hydroxy-C₁₋₆-alkyl), Acryl, Aryl-C₁₋₆-alkyl und Aryl-C₃₋₄-alkenyl, wobei Acryl ausgewählt ist aus der Reihe Phenyl, Biphenyl, Indanyl und Naphthyl; Heteroaryl oder Heteroaryl-C₁₋₁₀-alkyl, wobei Heteroaryl aus der Reihe Pyridyl; Thiophenyl, Furanyl, Benzofuranyl Reihe Pyridyl, Thiophenyl, Furanyl, Benzofuranyl, Benzothiophenyl, Pyrrolidinyl und Indolyl ausgewählt ist; Heterocycloalkyl oder Heterocycloalkyl-C₁₋₁₀-alkyl, wobei Heterocycloalkyl aus der Reihe Tetrahydrofuranyl, Tetrahydropyranyl, Morpholinyl, Piperidinyl, Pyrrolidinyl, Piperazinyl, Dioxolanyl, Sulfolanyl und 1,1-Dioxotetrahydrothiopyranyl ausgewählt ist; wobei C₅₋₈-Cycloalkyl, C₈₋₁₂-Bicycloalkyl oder Heterocyclyl gegebenenfalls durch einen oder mehr Substituenten aus der Gruppe bestehend aus C₁₋₆-Alkyl, C₁₋₆-Alkoxy, Hydroxy, Hydroxy-C₁₋₆-alkoxy, Hydroxy-C₁₋₆-alkyl, Amino, C₁₋₆Alkylcarbonylamino, Hydroxy-C₁₋₆-alkylcarbonylamino, Amino-C₂₋₆-alkoxy, Mono-C₁₋₆-alkylamino-C₂₋₆-alkoxy, Di-C₁₋₆-alkylamino-C₂₋₆-alkoxy, Mono-(C₁₋₆-alkyl)amino, Di-(C₁₋₆-alkyl)amino, Amino-C₁₋₆-alkylcarbonylamino, C₁₋₆-alkylaminocarbonyl, Hydroxy-C₁₋₆-alkylamino-carboxyl, Amino-C₂₋₆-alkylaminocarbonyl, Mono- oder Dialkylamino-C₁₋₆-alkylaminocarbonyl, Oxo, gegebenenfalls substituiertes Aryl und gegebenenfalls substituiertes Heteroaryl substituiert ist; wobei Aryl aus der Reihe Phenyl, Naphthyl und Biphenyl ausgewählt ist und Heteroaryl aus der Reihe Pyridyl und Thiophenyl ausgewählt ist;
wobei: die Acryl- oder Heteroarylgruppen von R gegebenenfalls durch einen oder mehrere Substituenten aus der folgenden Gruppe ausgewählt sind: Fluor, Chlor, Rom, C₁₋₁₀-Alkyl, Fluoralkyl oder Fluoralkoxy der Formel CₙHₓF_{y} oder OCₙHₓF_{y}, wobei n eine ganze Zahl von 1 bis 4 bedeutet, x eine ganze Zahl von 0 bis 8 bedeutet, y eine ganze Zahl von 1 bis 9 bedeutet und die Summe von x und y 2n+1 beträgt, C₁₋₁₀-Alkoxy, C₁₋₁₀-Alkenyloxy, C₁₋₁₀-Alkoxy-C₁₋₁₀-alkoxy, Hydroxy, Hydroxy-C₁₋₁₀-alkoxy, Hydroxy-C₁₋₁₀-alkyl, Hydroxy-C₁₋₁₀-acyl, Nitro, Amino, Aminosulfonyl, C₁₋₁₀-Alkylcarbonylamino, Hydroxy-C₁₋₁₀-alkylcarbonylamino, Amino-C₂₋₁₀-alkoxy, Amino-C₁₋₁₀-alkylcarbonylamino, C₁₋₁₀-Alkylaminocarbonyl, Hydroxy-C₁₋₁₀-alkylaminocarbonyl, Amino-C₁₋₁₀-alkylaminocarbonyl, Mono- oder Dialkylamino-C₁₋₁₀-alkylaminocarbonyl, gegebenenfalls substituiertes Aryl- oder Heteroarylsulfonyl, Aminosulfonyl, Mono- oder Di(C₁₋₆)alkylaminosulfonyl, gegebenenfalls substituiertes Phenyl oder Thiophenyl, gegebenenfalls substituiertes Phenyl- oder Thiophenyl-C₁₋₇-alkyloxy und Cyano;
wobei der Phenyl- oder Thiophenylsubstituent an der Aryl- oder Heteroarylgruppe von R gegebenenfalls durch einen oder mehrere Substituenten aus der folgenden Gruppe substituiert ist: Fluor, Chlor, Brom, C₁₋₅-Alkyl, CF₃, OCF₃, C₁₋₆-Alkoxy, C₁₋₆-Alkenyloxy, C₁₋₅-Alkoxy-C₁₋₉-alkoxy, Hydroxy, Hydroxy-C₁₋₁₀-alkoxy, Hydroxy-C₂₋₁₀-alkyl, Nitro, Amino, Aminosulfonyl, C₁₋₁₀-Alkylcarbonylamino, Hydroxy-C₁₋₆-alkylcarbonylamino, Amino-C₂₋₆-alkoxy, Amino-C₁-,₅-alkylcarhonylamino, C₁₋₆-Alkylamino-carbonyl, Hydroxy-C₁₋₆-alkylaminocarbonyl, Amine-C₁₋₆-alkylaminocarbonyl, Mono- oder Dialkylamin-C₁₋₆-alkylaminocarbonyl, gegebenenfalls substituiertes Aryl-C₁₋₆-alkyloxy und Cyano; wobei das Aryl oder Heteroaryl von R₃ gegebenenfalls durch einen oder mehr Substituenten aus der folgenden Reihe substituiert ist: C₃₋₆-Cycloalkyloxy, C₁₋₆-Alkoxy, C₁₋₆-Alkyl, C₂₋₆-Alkenyl, C₃₋₆-Alkenyloxy, Mono-(C₁₋₆-alkyl)amino-C₂₋₆-alkyloxy, Di-(C₁₋₆-alkyl)amino-C₂₋₆-alkyloxy, Amino-C₂₋₆alkyloxy, Heterocyclyl-C₂₋₆-alkyloxy, Aryl-C₂₋₁₃-alkyloxy, Heteroaryl-C₂₋₆-alkyloxy, Fluoralkyl oder Fluoralkoxy der Formel CₙHₓF_{y} oder OCₙHₓF_{y}, wobei n eine ganze Zahl von 1 bis 4 bedeutet, x eine ganze Zahl von 0 bis 8 bedeutet, y eine ganze Zahl von 1 bis 9 bedeutet, und die Summe von x und y 2n+1 beträgt, C₁₋₆-Alkoxy-C₂₋₆-alkyloxy, Aryl-C₁₋₆-alkoxy, Hydroxy, Chlor, Fluor, Brom, Hydroxy-C₁₋₆-alkoxy, Hydroxy-C₁₋₁₀-alkyl, C₁₋₆-Alkyloxo, Cyano, Amine, 4-Aryloxo, C₁₋₆-Alkyloxoamino, C₁₋₆-Alkyloxo(C₁₋₆-alkyl)amino, Carboxy, Dialkylamino-C₁₋₆-alkyl, Monoalkylamino-C₁₋₆-alkyl und Amina-C₁₋₆-alkyl; wobei: die Heterocyclyl-Substituentengruppe, die an die Alkyloxygruppe von R₃ gebunden ist, aus der folgenden Reihe ausgewählt ist: Pyrrolidinyl, Morpholinyl, Piperidinyl und Piperazinyl; wobei: die Heteroaryl-Substituentengruppe, die an die Alkyloxygruppe von R₃ gebunden ist, aus der Reihe Pyridinyl und Pyrrolidinyl ausgewählt ist; wobei: die Aryl- oder Heteroarylgruppen von R₄ gegebenenfalls durch einen oder mehr Substituenten aus der folgenden Reihe substituiert sind: Mono-oder Di(C₁₋₅-alkyl)amino, Mono- oder Di(C₁₋₆)alkylamino-C₂₋₆-alkyl, Amino-C₁₋₆-alkyl, Heterocyclyl, Amine, C₁₋₁₀-Alkoxy, Chlor, Fluor, Brom, C₁₋₁₀-Alkyl, Aryl und Heteroaryl; wobei die Heterocyclylgruppe, die an das Aryl von R₄ gebunden ist, aus der folgenden Reihe ausgewählt ist: Morpholinyl, Pyrrolidinyl, Piperazinyl und Piperidinyl; wobei die Heteroarylgruppe, die an was Aryl von R₄ gebunden ist, aus der Reihe Pyridyl, Pyrrolyl and Furanyl ausgewählt ist;
R₅, R₆ = H;
R₇, R₈ = H;
R₉ = Di-(C₁₋₅-alkyl)amino-C₂₋₁₀-alkyl; Mono (C₁₋₅ -alkyl)amino-C₂₋₄-alkyl; Amino-C₂₋₄-alkyl, Heterocycloalkylmethyl, in dem das Heterocycloalkyl aus der Gruppe Thiophenyl, Furanyl, Pyrrolyl, Pyridinyl ausgewählt ist; und
R₁₀ = H, CH₃, mit Aufnahme der Verbindung, in der R ein Phenyl, das in para-Stellung durch eine Methylgruppe substituiert ist, bedeutet, R₁ Wasserstoff bedeutet, R₂ CR₅R₆OH bedeutet, R₃ Phenylethyl bedeutet, X NR₁₀ bedeutet, R₅, R₆ und R₁₀ Wasserstoff bedeuten.

3. verbindung nach Anspruch 2, mit den folgenden Bedeutungen:
R bedeutet Aryl aus der Reihe Phenyl, Biphenyl und Naphthyl, oder Heteroaryl aus der Reihe Pyridyl und Thiophenyl,
R₁ bedeutet Wasserstoff oder Methyl;
R₂ bedeutet CONHR₄,
R3 bedeutet Aryl-C₁-alkyl, wobei Aryl aus der Reihe Phenyl, Biphenyl und Naphthyl ausgewählt ist, oder Heteroaryl-C₁-lkyl, wobei Heteroaryl aus der Reihe Indolyl und Thiophenyl ausgewählt ist,
X bedeutet NR₁₀ wobei R₄ ausgewählt ist aus der Gruppe bestehend aus Wasserstoff, C₁₋₁₀-Alkyl, C₂₋₁₀-Alkenyl, C₃₋₈-Cycloalkyl, C₈₋₁₂-Bicycloalkyl, C₁₀₋₁₄-Tricycloalkyl, C₃₋₈-Cycloalkyl-C₁₋₆-alkyl, C₈₋₁₂-Bicycloalkyl-C₁₋₆-alkyl, C₈₋₁₂-Bicycloamino-C₁₋₆-alkyl, C₈₋₁₂-Tricycloalkylamino-C₁₋₆-alkyl, C₃₋₈-Cycloalkenyl, C₃₋₈-Cycloalkenyl-C₁₋₆alkyl, Fluoralkyl der Formel CₙHₓF_{y}, wobei n eine ganze Zahl von 1 bis 6 bedeutet, x eine ganze Zahl von 0 bis 12 bedeutet, x eine ganze Zahl von 1 bis 13 bedeutet und die Summe von x und y 2n+1 beträgt, Amino-C₂₋₈-alkyl, Mono- oder Dialkylamino-C₂₋₈-alkyl, Mono-, Di- oder Trihydroxy-C₂₋₁₀-alkyl, C₁₋₆-Alkoxy-C₂₋₁₀-alkyl, C₁₋₆-Thioalkyl, C₁₋₆-Alkylthio-C₂₋₆-alkyl, Hydroxy-C₂₋₆-alkoxy-C₂₋₆-alkyl, C₁₋₆-Alkylbis-(hydroxy-C₁-C₆-alkyl), Aryl, Aryl-C₁₋₆-alkyl, wobei Aryl ausgewählt ist aus der Reihe Phenyl, Biphenyl und Naphthyl, Heteroaryl oder Heteroaryl-C₁₋₆-alkyl, wobei Heteroaryl ausgewählt ist aus der Reihe Pyridyl, Thiophenyl, Heterocycloalkyl oder Heterocycloalkyl-C₁₋₆-alkyl, wobei Heterocycloalkyl ausgewählt ist aus der Reihe Tetrahydrofuranyl, Tetrahydropyranyl, Morpholinyl, Piperidinyl, Pyrrolidinyl und Piperazinyl;
wobei die Aryl- oder Heteroarylgruppen von R gegebenenfalls durch einen oder mehr Substituenten substituiert sind, die aus der folgenden Gruppe ausgewählt sind: Fluor, Chlor, Brom, C₁₋₁₀-Alkyl, CF₃, OCF₃, C₁₋₁₀-Alkoxy, C₁₋₁₀-Alkenyloxy, C₁₋₅-Alkoxy-C₁₋₁₀-alkoxy, Hydroxy, Hydroxy-C₁₋₁₀-alkoxy, Hydroxy-C₁₋₁₀-alkyl, Nitro, Amino, C₁₋₁₀-Alkylcarbonylamino, Hydroxy-C₁₋₁₀-alkylcarbonylamino, Amino-C₂₋₁₀-alkoxy, Amino-C₁₋₁₀-alkylcarbonylamino, gegebenenfalls substituiertes Phenyl oder Thiophenyl, gegebenenfalls substituiertes Phenyl- oder Thiophenyl-C₁₋₁₀-alkyloxy, und Cyano;
wobei der Phenyl- oder Thiophenylsubstituent an der Acryl- oder Heteroarylgrtippe von R gegebenenfalls substituiert ist durch einen oder mehr Substituenten, die aus der folgenden Gruppe ausgewählt sind: Fluor, Chlor, Brom, C₁₋₅-Alkyl, CF₃, OCF₃, C₁₋₆-Alkoxy, C₁₋₆-Alkenyloxy, C₁₋₅-Alkoxy-C₁₋₅-alkoxy, Hydroxy, Hydroxy-C₁₋₁₀-alkoxy, Hydroxy-C₁₋₁₀-alkyl, Nitro, gegebenenfalls substituiertes Aryl-C₁₋₆-alkyloxy, und Cyano;
wobei das Aryl oder Heteroaryl von R₃ gegebenenfalls durch einen oder mehr Substituenten aus der folgenden Reihe substituiert ist: C₃₋₆-Cycloalkyloxy, C₁₋₆-Alkoxy, C₁₋₆-Alkyl, C₂₋₆-Alkenyl, C₃₋₆-Alkenyloxy, Mono-(C₁₋₆-alkyl)amino-C₂-₆-alkyloxy, Di-(C₁₋₆-alkyl)amino-C₂₋₆-alkyloxy, Amino-C₂₋₆-alkyloxy, Heterocyclyl-C₂₋₆-alkyloxy, Aryl-C₂₋₆-alkyloxy, Heteroaryl-C₂₋₆-alkyloxy, Fluoralkyl oder Fluoralkoxy der Formel CₙHₓF_{y} oder OCₙHₓF_{y}, wobei n eine ganze Zahl von 1 bis 4 bedeutet, x eine ganze Zahl von 0 bis 8 bedeutet, y eine ganze Zahl von 1 bis 9 bedeutet und die Summe von x und y 2n+1 beträgt, C₁₋₆-Alkoxy-C₂₋₆-alkoxy, Aryl-C₁₋₆-alkyloxy, Hydroxy, Chlor, Fluor, Brom, Nitro, Hydroxy-C₁₋₆-alkoxy, Hydroxy-C₁₋₆-alkyl, C₁₋₆-Alkyloxo, Cyano, Amine, 4-Aryloxo, C₂₋₆-Alkyloxoamino, Carboxy, Dialkylamino-C₁₋₆-alkyl, Monoalkylamino-C₁₋₆-alkyl und Amino-C₁₋₆-alkyl; wobei die Heterocycloalkyl-Substituentengruppe, die an die Alkyloxygruppe von R₃ gebunden ist, aus der Reihe Pyrrolidinyl, Morpholinyl und Piperidinyl ausgewählt ist;
wobei die Heterocycloalkyl-Substituentengruppe, die an die Alkyloxygruppe von R₃ gebunden ist, aus der Reihe Pyridinyl und Pyrrolidinyl ausgewählt ist;
wobei die Aryl- oder Heteroarylgruppen von R₄ gegebenenfalls durch einen oder mehr Substituenten substituiert sind, die aus der folgenden Reihe ausgewählt sind: Mono- oder Di(C₁₋₆-alkyl)amino, Mono-oder Di(C₁₋₆)alkylamino-C₂₋₆-alkyl, Amino-C₁₋₆-alkyl, Heterocycloalkyl, Amine, C₁₋₆-Alkoxy, Chlor, Fluor, Brom, C₁₋₆-Alkyl, Aryl und Heteroaryl;
wobei die Heterocycloalkylgruppe, die an das Aryl von R₄ gebunden ist, aus der folgenden Reihe ausgewählt ist: Morpholinyl, Pyrrolidinyl, Piperazinyl und Piperidinyl; und
wobei die Heteroarylgruppe, die an das Aryl von R₄ gebunden ist, aus der Reihe Pyridyl und Pyrrolyl ausgewählt ist.

4. Verbindung nach Anspruch 3, mit den folgenden Bedeutungen;
R bedeutet Acryl, wobei Aryl aus der Reihe Phenyl und Naphthyl ausgewählt ist,
R₁ bedeutet Wasserstoff oder Methyl,
R₂ bedeutet CONHR₄,
R₃ bedeutet Aryl-C₁-alkyl, wobei Aryl aus der Reihe Phenyl, Indolyl und Thiophenyl ausgewählt ist;
X bedeutet NH wobei R₄ ausgewählt ist aus der Gruppe bestehend aus Wasserstoff, C₁₋₁₀-Alkyl, C₃₋₆-Cycloalkyl, Adamantyl, Adamantyl-C₁-alkyl, 2-Adamantylaminoethyl, C₃₋₆-Cycloalkyl-C₁-alkyl, 3-Propenyl, Trifluormethyl-C₁-alkyl, 4-Amino-n-butyl, 4-(Methylamino)-n-butyl, 4-(Dimethylamino)-n-butyl, 4-Diethylaminobutyl, 2-Hydroxyethyl, 2-(2-Hydroxyethoxy)ethyl, 3-Hydroxypropyl, 2-Methoxyethylaryl, wobei Aryl ausgewählt ist aus der Reihe Phenyl und Naphthyl; Aryl-C₁-alkyl, wobei Aryl ausgewählt ist aus der Reihe Phenyl, und Biphenyl, Heteroaryl-C₁-alkyl, wobei Heteroaryl ausgewählt ist aus der Reihe 2-Thiophenyl und 2-Pyridinyl, Heterocycloalkyl, wobei Heterocycloalkyl ausgewählt ist aus der Reihe 4-Tetrahydropyranyl, 4-Piperidinyl, 2,2,6,6-Tetramethylpiperidinyl und 1-Ethyl-4-piperidinyl, Heterocycloalkyl-C₁-alkyl, wobei Heterocycloalkyl ausgewählt ist aus der Reihe 4-Tetrahydropyranyl und 2-Tetrahydrofuranyl, 2-Heterocycloalkyl-C₂-alkyl, wobei Heterocycloalkyl ausgewählt ist aus der Reihe 4-Tetrahydropyranyl und 1-Piperazinyl; wobei das Phenyl von R gegebenenfalls in 3-, 4-oder 5-position durch einen oder zwei Substituenten substituiert ist, die aus der Gruppe bestehend aus Fluor, Chlor, Brom, Trifluormethyl, C₁₋₇-Alkyloxy, C₁₋₇-Alkenyloxy, C₁₋₈-Alkyl und C₂₋₃-Hydroxypropyl ausgewählt sind; das Naphthyl von R gegebenenfalls durch einen der Substituenten, die aus der Reihe Chlor, Fluor und Dimethylamin ausgewählt sind, substituiert ist; das Phenyl von R₃ gegebenenfalls durch einen oder zwei Substituenten aus der Gruppe bestehend aus C₁₋₅-Alkyl, C₁₋₅-Alkyloxy, 2-Dimethylaminoethoxy, 2-Pyrrolidinethoxy, 3-Fluorpropyloxy, 3- Methoxypropyloxy, Cyclopentoxy, Benzyloxy, Hydroxy, Chlor, Fluor, Brom, Trifluormethyl, 1-Hydroxymethyl, Acetyl, Allyloxy, Cyano, Amine, 4-Benzoyl und Acetoxy substituiert ist; das Phenyl von R₄ gegebenenfalls durch einen oder zwei Substituenten substituiert ist, die aus der Gruppe bestehend aus 4-Dimethylamino, 4-Methylamino, 4-Amino, Methoxy, Ethoxy, Chlor, 4-Morpholino, Fluor und Methyl ausgewählt sind.

5. Verbindung nach Anspruch 1, ausgewählt aus der Reihe:
(S)-1-(3-Trifluormethylbenzolsulfonyl)piperidin-2-carbonsäure-[(S)-1-(4-dimethylaminophenylcarbamoyl)-2-(4-methoxyphenyl)ethyl]amid;
(S)-1-(3-Trifluormethylbenzolsulfonyl)piperidin-2-carbonsäure-[(S)-2-(4-acetylphenyl)-1-(4-ethoxyphenylcarbamoyl)ethyl]amid;
(S)-1-(3-Trifluormethylbenzolsulfonyl)piperidin-2-carbonsäure-[(S)-1-(4-morpholin-4-ylphenylcarbamoyl)-2 -(4-trifluormethylphenyl)ethyl]amid;
(S)-1-(3-Trifluormethylbenzolsulfonyl)piperidin-2-carbonsäure[(S)-2-(4-methoxyphenyl)-1-(tetrahydropyran-4-ylcarbamoyl)ethyl]amid;
(S)-1-(3-Trifluormethylbenzolsulfonyl)piperidin-2-carbonsäure[(S)-2-(2-methyl-4-methoxyphenyl)-1-(tetrahydropyran-4-ylcarbamoyl)ethyl]amid;
(S)-1-(3-Trifluormethylbenzolsulfonyl)piperidin-2-carbonsäure[(S)-2-(3,4-dimethoxyphenyl)-1-(tetrahydropyran-4-ylcarbamoyl)ethyl]amid;
(S)-1-(3-Trifluormethylbenzolsulfonyl)piperidin-2-carbonsäure[(S)-2-(4-methoxyphenyl)-1-(4-morpholin-4-ylphenylcarbamoyl)ethyl]amid;
(S)-1-(3-Methoxybenzolsulfonyl)piperidin-2-carbonsäure((S)-1-carbamoyl-2-phenylethyl)amid;
(S)-1-(3-Hydroxybenzolsulfonyl)piperidin-2-carbonsäure((S)-1-carbamoyl-2-phenylethyl)amid;
(S)-1-(3-Propoxybenzolsulfonyl)piperidin-2-carbonsäure((S)-1-carbamoyl-2-phenylethyl)amid;
(S)-1-(3-Trifluormethylbenzolsulfonyl)piperidin-2-carbonsäure[(S)-2-[4-(1-hydroxyethyl)phenyl]-1-(4-morpholin-4-ylphenylcarbamoyl)ethyl]amid;
(S)-1-(3-Trifluormethylbenzolsulfonyl)piperidin-2-carbonsäure{(S)-1-(4-ethoxyphenylcarbamoyl)-2-[4-(1-hydroxyethyl)phenyl]ethyl}amid;
(S)-1-(3-Methoxybenzolsulfonyl)piperidin-2-carbonsäure[(S)-2-(indol-3-yl)-1-(naphthalin-2-ylcarbamoyl)ethyl)amid;
(S)-1-(3-Trifluormethylbenzolsulfonyl)piperidin-2-carbonsäure{(S)-1-(4-dimethylaminophenylcarbamoyl)-2-[4-(1-hydroxyethyl)phenyl]ethyl}amid;
(S)-1-(3,5-Dichlorbenzolsulfonyl)piperidin-2-carbonsäure[(S)-2-(1H-indol-3-yl)-1-(methylcarbamoylethyl]amid;
(S)-1-(3-Trifluormethylbenzolsulfonyl)piperidin-2-carbonsäure[(S)-2-(4-acetylphenyl)-1-(4-morpholin-4-ylphenylcarbamoyl)ethyl]amid;
(S)-1-(3-Fluorbenzolsulfonyl)piperidin-2-carbonsäure[(S)-2-(4-cyclopentyloxyphenyl)-1-methyl-1-(tetrahydropyran-4-ylcarbamoyl)ethyl]amid;
(S)-1-(3-Fluorbenzolsulfonyl)piperidin-2-carbonsäure[(S)-2-(4-isopropoxyphenyl)-1-(2,2,6,6-tetramethylpiperidin-4-ylcarbamoyl)ethyl]amid;
(S)-1-(3-Fluorbenzolsulfonyl)-(2S)piperidin-2-carbonsäure[(S)-2-[4-(3-fluorpropoxy)phenyl]-1-(2,2,6,6-tetramethylpiperidin-4-ylcarbamoyl)ethyl]amid;
(S)-1-(3-Fluorbenzolsulfonyl)piperidin-2-carbonsäure[(S)-2-[4-(3-fluorpropoxyphenyl]-1-((S)-2-methoxy-1-methylethylcarbamoyl)ethyl]amid;
(S)-1-(3-Trifluormethylbenzolsulfonyl)piperidin-2-carbonsäure[(S)-2-(4-isopropoxyphenyl)-1-methylcarbamoylethyl]amid;
(S)-1-(3-Trifluormethylbenzolsulfonyl)piperidin-2-carbonsäure[(S)-2-(4-isopropoxyphenyl)-1-(tetrahydropyran-4-ylcarbamoyl)ethyl]amid;
(S)-1-(3-Trifluormethylbenzolsulfonyl)piperidin-2-carbonsäure[(S)-2-(phenyl)-1-(benzimidazol-2-yl)ethyl]amid;
(S)-1-(3-Trifluormethylbenzolsulfonyl)piperidin-2-carbonsäure[(S)-2-(4-(benzyloxy)phenyl)-1-(benzimidazol-2-yl)ethyl]amid;
(S)-1-(3-Fluorbenzolsulfonyl)piperidin-2-carbonsäure[(S)-2-(4-methoxyphenyl)-1-(tetrahydropyran-4-ylcarbamoyl)ethyl]amid;
(S)-1-Benzolsulfonylpiperidin-2-carbonsäure((S)-1-amido-2-(2-methylphenyl)ethyl)amid;
(S)-1-Benzolsulfonylpiperidin-2-carbonsäure((S)-1-cyclohexylamido-2-(4-methylphenyl)ethyl)amid;
(S)-1-Benzolsulfonylpiperidin-2-carbonsäure[(S)-2-(4-allyloxyphenyl)-1-cyclohexylamidoethyl]amid;
(S)-1-(3-Trifluormethylbenzolsulfonyl)piperidin-2-carbonsäure[(S)-2-(4-allyloxyphenyl)-1-cyclohexylcarbamoylethyl]amid;
(S)-1-3-Trifluormethylbenzolsulfonyl)piperidin-2-carbonsäure((s)-1-cyclohexylamido-2-(4-methylphenyl)ethyl)amid;
(S)-1-((E)-2-Phenylethensulfonyl)piperidin-2-carbonsäure[(S)-1-amido-2-(1H-indol-3-yl)ethyl]amid;
(S)-1-(4,5-Dibromthiophen-2-sulfonyl)piperidin-2-carbonsäure[(S)-1-amido-2-(1H-indol-3-yl)ethyl]amid;
(S)-1-Benzolsulfonylpiperidin-2-carbonsäure[(S)-1-tert.-butylamido-2-(1H-indol-3-yl)ethyl]amid;
(S)-1-Benzolsulfonylpiperidin-2-carbonsäure[(S)-1-(3-hydroxy-2,2-dimethylpropylamido)-2-(4-isopropylphenyl)ethyl]amid;
(S)-1-Benzolsulfonylpiperidin-2-carbonsäure((S)-1-methylamido-2-(phenyl)ethyl)amid;
(S)-1-Benzolsulfonylpiperidin-2-carbonsäure[(S)-1-(3,3-dimethylbutylamido)-2-(4-methoxyphenyl)ethyl]amid;
(S)-1-Benzolsulfonylpiperidin-2-carbonsäure[(S)-1-(2,2-dimethylpropylamido)-2-(phenyl)ethyl]amid;
(4-{(S)-2-[((S)-1-Benzolsulfonylpiperidin-2-carbonyl)amino]-2-amidoethyl}benzyl)phosphonsäurediethylester;
(S)-1-Benzolsulfonylpiperidin-2-carbonsäure[(S)-2-(4(acetylamino)phenyl)-1-amidoethyl]amid;
(S)-1-Benzolsulfonylpiperidin-2-carbonsäure[(S)-1-amido-2-(4-(hydroxymethyl)phenyl)ethyl]amid;
(S)-1-(Naphthalin-2-sulfonyl)piperidin-2-carbonsäure[(S)-1-amido-2-1H-indol-3-yl)ethyl]amid;
(S)-1-(3-Trifluormethylbenzolsulfonyl)piperidin-2-carbonsäure(S)-1-(cyclohexylmethylamido)-2-(4-methylphenyl)ethyl]amid;
(S)-1-(3-Trifluormethylbenzolsulfonyl)piperidin-2-carbonsäure((S)-1-(4-aminobutylamido)-2-(4-methylphenyl)ethyl]amid;
(S)-1-Benzolsulfonylpiperidin-2-carbonsäure[(S)-1-amido-2-(1H-indol-3-yl)ethyl]amid;
(S)-1-Benzolsulfonylpiperidin-2-carbonsäure((S)-1-amido-2-(pyridin-4-yl)ethyl)amid;
(S)-1-Benzolsulfonylpiperidin-2-carbonsäure((S)-1-amido-2-cyclohexylethyl)amid;
(S)-1-Benzolsulfonylpiperidin-2-carbonsäure{(S)-2-phenyl-1-[(thiophen-2-ylmethyl)amido]ethyl}amid;
(S)-1-(3-Fluorbenzolsulfonyl)piperidin-2-carbonsäure[(S)-1-(4-dimethylaminobutylcarbamoyl)-2-(4-fluorphenyl)ethyl]amid;
(S)-1-(3-Fluorbenzolsulfonyl)piperidin-2-carbonsäure[(S)-2-[4-(3-methoxyproyoxy)phenyl]-1-(tetrahydropyran-4-ylcarbamoyl)ethyl]amid;
(S)-1-(3-Trifluormethylbenzolsulfonyl)piperidin-2-carbonsäure{(S)-1-(cyclohexylcarbamoyl)-2-[4-(2-dimethylaminoethoxy)phenyl]ethyl)amid;
(S)-1-(3-Trifluormethylbenzolsulfonyl)piperidin-2-carbonsäure[(S)-2-(4-methoxyphenyl)-1-(2-piperazin-1-ylethylcarbamoyl)ethyl]amid;
(S)-1-(3-Trifluormethylbenzolsulfonyl)piperidin-2-carbonsäure[(S)-1-(2-hydroxy-1,1-dimethylethylcarbamoyl)-2-(4-methoxyphenyl)ethyl]amid;
(S)-1-Benzolsulfonylpiperidin-2-carbonsäure((S)-1-carbamoyl-2-phenylethyl)amid;
(S)-1-(3-Fluorbenzolsulfonyl)piperidin-2-carbonsäure{(S)-2-[4-(3-methoxypropoxy)phenyl]-1-methylcarbamoylethyl}amid;
(S)-1-(3-Fluorbenzolsulfonyl)piperidin-2-carbonsäure{(S)-1-((S)-2-methoxy-1-methylethylcarbamoyl)-2-[4-(3-methoxypropoxy)phenyl]ethyl}amid; und
(S)-1-(3-Hydroxybenzolsulfonyl)piperidin-2-carbonsäure[(S)-2-(1H-indol-3-yl)-1-(naphthalin-2-ylcarbamoyl)ethyl]amid.

6. Pharmazeutische Zusammensetzung, umfassend eine Verbindung, einschließlich der Enantiomere, Stereomere, Rotamere und Tautomere von dieser Verbindung, sowie pharmazeutisch annehmbare Salze oder Solvate davon, mit der allgemeinen Struktur gemäß Formel 1 wie in Anspruch 1 definiert.

7. Pharmazeutische Zusammensetzung nach Aspruch 6, wobei die Verbindung der Formel I wie in Anspruch 2 definiert ist.

8. Pharmazeutische Zusammensetzung nach Anspruch 7, wobei die Verbindung der Formel I wie in Anspruch 3 definiert ist.

9. Pharmazeutische Zusammensetzung nach Anspruch 8, wobei die Verbindung der Formel I wie in Anspruch 4 definiert ist.

10. Pharmazeutische Zusammensetzung nach Anspruch 7, wobei die Verbindung aus der folgenden Reihe ausgewählt ist:
(S)-1-3-Trifluormethylbenzolsulfonyl)piperidin-2-carbonsäure-[(S)-1-(4-dimethylaminophenylcarbamoyl)-2-(4-methoxyphenyl)ethyl]amid;
(S)-1-(3-Trifluormethylbenzolsulfonyl)piperidin-2-carbonsäure-[(S)-2-(4-acetylphenyl)-1-(4-ethoxyphenylcarbamoyl)ethyl]amid;
(S)-1-(3-Trifluormethylbenzolsulfonyl)piperidin-2-carbonsäure-[(S)-1-(4-morpholin-4-ylphenylcarbamoyl)-2 -(4-trifluormethylphenyl)ethyl]amid;
(S)-1-(3-Trifluormethylbenzolsulfonyl)piperidin-2-carbonsäure[(S)-2-(4-methoxyphenyl)-1-(tetrahydropyran-4-ylcarbamoyl)ethyl]amid;
(S)-1-(3-Trifluormethylbenzolsulfonyl)piperidin-2-carbonsäure[(S)-2-(2-methyl-4-methoxyphenyl)-1-(tetrahydropyran-4-ylcarbamoyl)ethyl]amid;
(S)-1-(3-Trifluormethylbenzolsulfonyl)piperidin-2-carbonsäure[(S)-2-3,4-dimethoxyphenyl)-1-(tetrahydropyran-4-ylcarbamoyl)ethyl]amid;
(S)-1-(3-Trifluormethylbenzolsulfonyl)piperidin-2-carbonsäure[(S)-2-(4-methoxyphenyl)-1-(4-morpholin-4-ylphenylcarbamoyl)ethyl]amid;
(S)-1-(3-Methoxybenzolsulfonyl)piperidin-2-carbonsäure((S)-1-carbamoyl-2-phenylethyl)amid;
(S)-1-(3-Hydroxybenzolsulfonyl)piperidin-2-carbonsäure((S)-1-carbamoyl-2-phenylethyl)amid;
(S)-1-(3-Propoxybenzolsulfonyl)piperidin-2-carbonsäure((S)-1-carbamoyl-2-phenylethyl)amid;
(S)-1-(3-Trifluormethylbenzolsulfonyl)piperidin-2-carbonsäure[(S)-2-[4-(1-hydroxyethyl)phenyl]-1-(4-morpholin-4-ylphenylcarbamoyl)ethyl]amid;
(S)-1-(3-Trifluormethylbenzolsulfonyl)piperidin-2-carbonsäure{(S)-1-(4-ethoxyphenylcarbamoyl)-2-[4-(1-hydroxyethyl)phenyl]ethyl}amid;
(S)-1-(3-Methoxybenzolsulfonyl)piperidin-2-carbonsäure[(S)-2-(indol-3-yl)-1-(naphthalin-2-ylcarbamoyl)ethyl)amid;
(S)-1-(3-Trifluormethylbenzolsulfonyl)piperidin-2-carbonsäure{(S)-1-(4-dimethylaminophenylcarbamoyl)-2-[4-(1-hydroxyethyl)phenyl]ethyl}amid;
(S)-1-(3,5-Dichlorbenzolsulfonyl)piperidin-2-carbonsäure[(S)-2-(1H-indol-3-yl)-1-(methylcarbamoylethyl]amid;
(S)-1-(3-Trifluormethylbenzolsulfonyl)piperidin-2-carbonsäure[(S)-2-(4-acetylphenyl-1-(4-morpholin-4-ylphenylcarbamoyl)ethyl]amid;
(S)-1-(3-Fluorbenzolsulfonyl)piperidin-2-carbonsäure[(S)-2-(4-cyclopentyloxyphenyl)-1-methyl-1-(tetrahydropyran-4-ylcarbamoyl)ethyl]amid;
(S)-1-(3-Fluorbenzolsulfonyl)piperidin-2-carbonsäure[(S)-2-(4-isopropoxyphenyl)-1-(2,2,6,6-tetramethylpiperidin-4-ylcarbamoyl)ethyl]amid;
(S)-1-(3-Fluorbenzolsulfonyl)-(2S)piperidin-2-carbonsäure[(S)-2-[4-(3-fluorpropoxy)phenyl]-1-(2,2,6,6-tetramethylpiperidin-4-ylcarbamoyl)ethyl]amid;
(S)-1-(3-Fluorbenzolsulfonyl)piperidin-2-carbonsäure[(S)-2-[4-(3-fluorpropoxyphenyl]-1-(S)-2-methoxy-1-methylethylcarbamoyl)ethyl]amid;
(S)-1-(3-Trifluormethylbenzolsulfonyl)piperidin-2-carbonsäure[(S)-2-(4-isopropoxyphenyl)-1-methylcarbamoylethyl]amid;
(S)-1-(3-Trifluormethylbenzolsulfonyl)piperidin-2-carbonsäure[(S)-2-(4-isopropoxyphenyl)-1-(tetrahydropyran-4-ylcarbamoyl)ethyl]amid;
(S)-1-(3-Trifluormethylbenzolsulfonyl)piperidin-2-carbonsäure[(S)-2-(phenyl)-1-(benzimidazol-2-yl)ethyl]amid;
(S)-1-(3-Trifluormethylbenzolsulfonyl)piperidin-2-carbonsäure[(S)-2-(4-(benzyloxy)phenyl)-1-(benzimidazol-2-yl)ethyl]amid;
(S)-1-(3-Fluorbenzolsulfonyl)piperidin-2-carbonsäure[(S)-2-(4-methoxyphenyl)-1-(tetrahydropyran-4-ylcarbamoyl)ethyl]amid;
(S)-1-Benzolsulfonylpiperidin-2-carbonsäure((S)-1-amido-2-(2-methylphenyl)ethyl)amid;
(S)-1-Benzolsulfonylpiperidin-2-carbonsäure((S)-1-cyclohexylamido-2-(4-methylphenyl)ethyl)amid;
(S)-1-Benzolsulfonylpiperidin-2-carbonsäure[(S)-2-(4-allyloxyphenyl)-1-cyclohexylamidoethyl]amid;
(S)-1-(3-Trifluormethylbenzolsulfonyl)piperidin-2-carbonsäure[(S)-2-(4-allyloxyphenyl)-1-cyclohexylcarbamoylethyl]amid;
(S)-1-(3-Trifluormethylbenzolsulfonyl)piperidin-2-carbonsäure((S)-1-cyclohexylamido-2-(4-methylphenyl)ethyl)amid;
(S)-1-((E)-2-Phenylethensulfonyl)piperidin-2-carbonsäure[(S)-1-amido-2-(1H-indol-3-yl)ethyl]amid;
(S)-1-(4,5-Dibromthiophen-2-sulfonyl)piperidin-2-carbonsäure[(S)-1-amido-2-(1H-indol-3-yl)ethyl]amid;
(S)-Benzolsulfonylpiperidin-2-carbonsäure[(S)-1-tert.-butylamido-2-(1H-indol-3-yl)ethyl]amid;
(S)-1-Benzolsulfonylpiperidin-2-carbonsäure[(S)-1-(3-hydroxy-2,2-dimethylpropylamido)-2-(4-isopropylphenyl)ethyl]amid;
(S)-1-Benzolsulfonylpiperidin-2-carbonsäuremethyl-((S)-1-methylamido-2-(phenyl)ethyl)amid;
(S)-1-Benzolsulfonylpiperidin-2-carbonsäure[(S)-1-(3,3-dimethylbutylamido)-2-(4-methoxyphenyl)ethyl]amid;
(S)-1-Benzolsulfonylpiperidin-2-carbonsäure[(S)-1-(2,2-simethylpropylamido)-2-(phenyl)ethyl]amid;
(4-{(S)-2-[((S)-1-Benzolsulfonylpiperidin-2-carbonyl)amino]-2-amidoethyl}benzyl)phosphonsäure-diethylester;
(S)-1-Benzolsulfonylpiperidin-2-carbonsäure[(S)-2-(4(acetylamino)phenyl)-1-amidoethyl]amid;
(S)-1-Benzolsulfonylpiperidin-2-carbonsäure[(S)-1-amido-2-(4-(hydroxymethyl)phenyl)ethyl]amid;
(S)-1-(Naphthalin-2-sulfonyl)piperidin-2-carbonsäure[(S)-1-amido-2-(1H-indol-3-yl)ethyl]amid;
(S)-1-(3-Trifluormethylbenzolsulfonyl)piperidin-2-carbonsäure((S)-1-(cyclohexylmethylamido)-2-(4-methylphenyl)ethyl]amid;
(S)-1-(3-Trifluormethylbenzolsulfonyl)piperidin-2-carbonsäure((S)-1-(4-aminobutylamido)-2-(4-methylphenyl)ethyl]amid;
(S)-1-Benzolsulfonylpiperidin-2-carbonsäure[(S)-1-amido-2-(1H-indol-3-yl)ethyl]amid;
(S)-1-Benzolsulfonylpiperidin-2-carbonsäure((S)-1-amido-2-(pyridin-4-yl)ethyl)amid;
(S)-1-Benzolsulfonylpiperidin-2-carbonsäure((S)-1-amido-2-cyclohexylethyl)amid;
(S)-1-Benzolsulfonylpiperidin-2-carbonsäur{(S)-2-phenyl-1-[(thiophen-2-ylmethyl)amido]ethyl}amid;
(S)-1-(3-Fluorbenzolsulfonyl)piperidin-2-carbonsäure[(S)-1-(4-dimethylaminobutylcarbamoyl)-2-(4-fluorphenyl)ethyl]amid;
(S)-1-(3-Fluorbenzolsulfonyl)piperidin-2-carbonsäure[(S)-2-[4-(3-methoxypropoxy)phenyl-1-(tetrahydropyran-4-ylcarbamoyl)ethyl]amid;
(S)-1-(3-Trifluormethylbenzolsulfonyl)piperidin-2-carbonsäure{(S)-1-(cyclohexylcarbamoyl)-2-[4-(2-dimethylaminoethoxy)phenyl]ethyl}amid;
(S)-1-(3-Trifluormethylbenzolsulfonyl)piperidin-2-carbonsäure[(S)-2-(4-methoxyphenyl)-1-(2-piperazin-1-ylethylcarbamoyl)ethyl]amid;
(S)-1-(3-Trifluormethylbenzolsulfonyl)piperidin-2-carbonsäure[(S)-1-(2-hydroxy-1,1-dimethylethylcarbamoyl)-2-(4-methoxyphenyl)ethyl]amid;
(S)-1-Benzolsulfonylpiperidin-2-carbonsäure((S)-1-carbamoyl-2-phenylethyl)amid;
(S)-1-(3-Fluorbenzolsulfonyl)piperidin-2-carbonsäure{(S)-2-[4-(3-methoxypropoxy)phenyl]-1-methylcarbamoylethyl}amid;
(S)-1-(3-Fluorbenzolsulfonyl)piperidin-2-carbonsäure{(S)-1-((S)-2-methoxy-1-methylethyl-carbamoyl)-2-[4-(3-methoxypropoxy)phenyl]ethyl}amid; und
(S)-1-(3-Hydroxybenzolsulfonyl)piperidin-2-carbonsäure[(S)-2-(1H-indol-3-yl)-1-(naphthalin-2-ylcarbamoyl)ethyl]amid.

11. Verbindung der allgemeinen Struktur gemäß Formel I wie in Anspruch 1 definiert, einschließlich Enantiomere, Stereomere, Rotamere und Tautomere der Verbindung und pharmazeutisch annehmbare Salze oder Solvate davon, für die Herstellung eines Arzneimittels zur Behandlung einer Krankheit, die Nervenschädigung beeinhaltet.

12. Verbindung nach Anspruch 11, wobei es sich bei der Krankheit um Morbus Parkinson handelt.

13. Verbindung nach Anspruch 11, wobei es sich bei der Krankheit um multiple Sklerose handelt.

14. Verbindung nach Anspruch 11, wobei es sich bei der Krankheit um Alzheimer-Krankheit handelt.

15. Verbindung nach Anspruch 11, wobei es sich bei der Krankheit um Schlaganfall handelt.

16. Verbindung nach Anspruch 11, wobei es sich bei der Krankheit um Rückenmarksverletzung handelt.

17. Verbindung nach Anspruch 11, wobei die Verbindung wie in Anspruch 2 definiert ist.

18. Verbindung nach Anspruch 11, wobei die Verbindung wie in Anspruch 3 definiert ist.

19. Verbindung nach Anspruch 11, wobei die Verbindung wie in Anspruch 4 definiert ist.

20. Verbindung nach Anspruch 11, wobei die Verbindung aus der folgenden Reihe stammt:
(S)-1-(3-Trifluormethylbenzolsulfonyl)piperidin-2-carbonsäure-[(S)-1-(4-dimethylaminophenylcarbamoyl)-2-(4-methoxyphenyl)ethyl]amid;
(S)-1-(3-Trifluormethylbenzolsulfonyl)piperidin-2-carbonsäure-[(S)-2-(4-acetylphenyl)-1-(4-ethoxyphenylcarbamoyl)ethyl]amid;
(S)-1-(3-Trifluormethylbenzolsulfonyl)piperidin-2-carbonsäure-[(S)-1-(4-morpholin-4-ylphenylcarbamoyl)-2 -(4-trifluormethylphenyl)ethyl]amid;
(S)-1-(3-Trifluormethylbenzolsulfonyl)piperidin-2-carbonsäure[(S)-2-(4-methoxyphenyl)-1-(tetrahydropyran-4-ylcarbamoyl)ethyl]amid;
(S)-1-(3-Trifluormethylbenzolsulfonyl)piperidin-2-carbonsäure[(S)-2-(2-methyl-4-methoxyphenyl)-1-(tetrahydropyran-4-ylcarbamoyl)ethyl]amid;
(S)-1-(3-Trifluormethylbenzolsulfonyl)piperidin-2-carbonsäure[(S)-2-(3,4-dimethoxyphenyl)-1-(tetrahydropyran-4-ylcarbamoyl)ethyl]amid;
(S)-1-(3-Trifluormethylbenzolsulfonyl)piperidin-2-carbonsäure[(S)-2-(4-methoxyphenyl)-1-(4-morpholin-4-ylphenylcarbamoyl)ethyl]amid;
(S)-1-(3-Methoxybenzolsulfonyl)piperidin-2-carbonsäure((S)-1-carbamoyl-2-phenylethyl)amid;
(S)-1-(3-Hydroxybenzolsulfonyl)piperidin-2-carbonsäure((S)-1-carbamoyl-2-phenylethyl)amid;
(S)-1-(3-Propoxybenzolsulfonyl)piperidin-2-carbonsäure((S)-1-carbamoyl-2-phenylethyl)amid;
(S)-1-(3-Trifluormethylbenzolsulfonyl)piperidin-2-carbonsäure[(S)-2-[4-(1-hydroxyethyl)phenyl]-1-(4-morpholin-4-ylphenylcarbamoyl)ethyl]amid;
(S)-1-(3-Trifluormethylbenzolsulfonyl)piperidin-2-carbonsäure{(S)-1-(4-ethoxyphenylcarbamoyl)-2-[4-(1-hydroxyethyl)phenyl]ethyl}amid;
(S)-1-(3-Methoxybenzolsulfonyl)piperidin-2-carbonsäure[(S)-2-(indol-3-yl)-1-(naphthalin-2-ylcarbamoyl)ethyl)amid;
(S)-1-(3-Trifluormethylbenzolsulfonyl)piperidin-2-carbonsäure{(S)-1-(4-dimethylaminophenylcarbamoyl)-2-[4-(1-hydroxyethyl)phenyl]ethyl}amid;
(S)-1-(3,5-Dichlorbenzolsulfonyl)piperidin-2-carbonsäure[(S)-2-(1H-indol-3-yl)-1-(methylcarbamoylethyl]amid;
(S)-1-(3-Trifluormethylbenzolsulfonyl)piperidin-2-carbonsäure[(S)-2-(4-acetylphenyl)-1-(4-morpholin-4-ylphenylcarbamoyl)ethyl]amid;
(S)-1-(3-Fluorbenzolsulfonyl)piperidin-2-carbonsäure[(S)-2-(4-cyclopentyloxyphenyl-1-methyl-1-(tetrahydropyran-4-ylcarbamoyl)ethyl]amid;
(S)-1-(3-Fluorbenzolsulfonyl)piperidin-2-carbonsäure[(S)-2-(4-isopropoxyphenyl)-1-(2,2,6,6-tetramethylpiperidin-4-ylcarbamoyl)ethyl]amid;
(S)-1-(3-Fluorbenzolsulfonyl)-(2S)piperidin-2-carbonsäure[(S)-2-[4-(3-fluorpropoxy)phenyl]-1-(2,2,6,6-tetramethylpiperidin-4-ylcarbamoyl)ethyl]amid;
(S)-1-(3-Fluorbenzolsulfonyl)piperidin-2-carbonsäure[(S)-2-[4-(3-fluorpropoxyphenyl]-1-((S)-2-methoxy-1-methylethylcarbamoyl)ethyl]amid;
(S)-1-(3-Trifluormethylbenzolsulfonyl)piperidin-2-carbonsäure[(S)-2-(4-isopropoxyphenyl)-1-methylcarbamoylethyl]amid;
(S)-1-(3-Trifluormethylbenzolsulfonyl)piperidin-2-carbonsäure[(S)-2-(4-isopropoxyphenyl)-1-(tetrahydropyran-4-ylcarbamoyl)ethyl]amid;
(S)-1-(3-Trifluoromethylbenzolsulfonyl)piperidin-2-carbonsäure[(S)-2-(phenyl)-1-(benzimidazol-2-yl)ethyl]amid;
(S)-1-(3-Trifluormethylbenzolsulfonyl)piperidin-2-carbonsäure[(S)-2-(4-(benzyloxy)phenyl)-1-(benzimidazol-2-yl)ethyl]amid;
(S)-1-(3-Fluorbenzolsulfonyl)piperidin-2-carbonsäure[(S)-2-(4-methoxyphenyl)-1-(tetrahydropyran-4-ylcarbamoyl)ethyl]amid;
(S)-1-Benzolsulfonylpiperidin-2-carbonsäure((S)-1-amido-2-(2-methylphenyl)ethyl)amid;
(S)-1-Benzolsulfonylpiperidin-2-carbonsäure((S)-1-cyclohexylamido-2-(4-methylphenyl)ethyl)amid;
(S)-1-Benzolsulfonylpiperidin-2-carbonsäure[(S)-2-(4-allyloxyphenyl)-1-cyclohexylamidoethyl]amid;
(S)-1-(3-Trifluormethylbenzolsulfonyl)piperidin-2-carbonsäure[(S)-2-(4-allyloxyphenyl)-1-cyclohexylcarbamoylethyl]amid;
(S)-1-(3-Trifluormethylbenzolsulfonyl)piperidin-2-carbonsäure((S)-1-cyclohexylamido-2-(4-methylphenyl)ethyl)amid;
(S)-1-((E)-2-Phenylethensulfonyl)piperidin-2-carbonsäure[(S)-1-amido-2-(1H-indol-3-yl)ethyl]amid;
(S)-1-(4,5-Dibromthiophen-2-sulfonyl)piperidin-2-carbonsäure[(S)-1-amido-2-(1H-indol-3-yl)ethyl]amid;
(S)-1-Benzolsulfonylpiperidin-2-carbonsäure[(S)-1-tert.-butylamido-2-(1H-indol-3-yl)ethyl]amid;
(S)-1-Benzolsulfonylpiperidin-2-carbonsäure[(S)-1-(3-hydroxy-2,2-dimethylpropylamido)-2-(4-isopropylphenyl)ethyl]amid;
(S)-1-Benzolsulfonylpiperidin-2-carbonsäure((S)-1-methylamido-2- phenyl)ethyl)amid;
(S)-1-Benzolsulfonylpiperidin-2-carbonsäure[(S)-1-(3,3-dimethylamido)-2-(4-methoxyphenyl)ethyl]amid;
(S)-1-Benzolsulfonylpiperidin-2-carbonsäure[(S)-1-(2,2-dimethylpropylamido)-2-(phenyl)ethyl]amid;
(4-{(S)-2-[((S)-1-Benzolsulfonylpiperidin-2-carbonyl)amino]-2-amidoethyl}benzyl)phosphonsäure-diethylester;
(S)-1-Benzolsulfonylpiperidin-2-carbonsäure[(S)-2-(4(acetylamino)phenyl)-1-amidoethyl]amid;
(S)-1-Benzolsulfonylpiperidin-2-carbonsäure[(S)-1-amido-2-(4-(hydroxymethyl)phenyl)ethyl]amid;
(S)-1-(Naphthalin-2-sulfonyl)piperidin-2-carbonsäure[(S)-1-amido-2-(1H-indol-3-yl)ethyl]amid;
(S)-1-(3-Trifluormethylbenzolsulfonyl)piperidin-2-carbonsäure[(S)-1-(cyclohexylmethylamido)-2-(4-methylphenyl)ethyl]amid;
(S)-1-(3-Trifluormethylbenzolsulfonyl)piperidin-2-carbonsäure[(S)-1-(4-aminobutylamido)-2-(4-methylphenyl)ethyl]amid;
(S)-1-Benzolsulfonylpiperidin-2-carbonsäure[(S)-1-amido-2-(1H-indol-3-yl)ethyl]amid;
(S)-1-Benzolsulfonylpiperidin-2-carbonsäure((S)-1-amido-2-(pyridin-4-yl)ethyl)amid;
(S)-1-Benzolsulfonylpiperidin-2-carbonsäure((S)-1-amido-2-cyclohexylethyl)amid;
(S)-1-Benzolsulfonylpiperidin-2-carbonsäure{(S)-2-phenyl-1-[(thiophen-2-ylmethyl)amido]ethyl)amid;
(S)-1-(3-Fluorbenzolsulfonyl)piperidin-2-carbonsäure[(S)-1-(4-dimethylaminobutylcarbamoyl)-2-(4-fluorphenyl)ethyl]amid;
(S)-1-(3-Fluorbenzolsulfonyl)piperidin-2-carbonsäure[(S)-2-[4-(3-methoxypropoxy)phenyl]-1-(tetrahydropyran-4-ylcarbamoyl)ethyl]amid;
(S)-1-(3-Trifluormethylbenzolsulfonyl)piperidin-2-carbonsäure{(S)-1-cyclohexylcarbamoyl)-2-[4-(2-dimethylaminoethoxy)phenyl]ethyl}amid;
(S)-1-(3-Trifluormethylbenzolsulfonyl)piperidin-2-carbonsäure[(S)-2-(4-methoxyphenyl)-1-(2-piperazin-1-ylethylcarbamoyl)ethyl]amid;
(S)-1-(3-Trifluormethylbenzolsulfonyl)piperidin-2-carbonsäure[(S)-1-(2-hydroxy-1,1-dimethylethylcarbamoyl)-2-(4-methoxyphenyl)ethyl]amid;
(S)-1-Benzolsulfonylpiperidin-2-carbonsäure((S)-1-carbamoyl-2-phenylethyl)amid;
(S)-1-(3-Fluorbenzolsulfonyl)piperidin-2-carbonsäure{(S)-2-[4-(3-methoxypropoxy)phenyl]-1-methylcarbamoylethyl}amid;
(S)-1-(3-Fluorbenzolsulfonyl)piperidin-2-carbonsäure{(S)-1-((S)-2-methoxy-1-methylethyl-carbamoyl)-2-[4-(3-methoxypropoxy)phenyl]ethyl}amid; und
(S)-1-(3-Hydroxybenzolsulfonyl)piperidin-2-carbonsäure[(S)-2-(1H-indol-3-yl)-1-(naphthalin-2-ylcarbamoyl)ethyl]amid.

## Revendications

1. composé, comprenant des énantiomères, des stéréo-isomères, des rotamères et des tautomères desdits composés et des sels ou des solvates pharmaceutiquement acceptables de ceux-ci, lesdits composés présentant la structure générale indiquée dans la formule I : 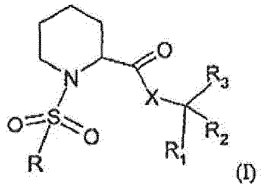 où :
R représente fluoroalkyl de formule CₙHₓF_{y}, où n représente un entier de 1 à 6, x représente un entier de 0 à 12, y représente un entier de 1 à 13 et la somme de x et y vaut 2n+1 ; aryle, aryl-C₁₋₆-alkyle, aryl-C₂₋₄-alcényle, aryle étant choisi parmi phényle, biphényle et naphtyle ; hétéroaryle ou hétéroaryl-C₁₋₄-alkyle, hétéroaryle étant choisi parmi pyridyle, thiophényle et benzothiophényle ; et hétérocyclyle choisi parmi pipéridinyle et pipérazinyle,
R₁ représente hydrogène ou C₁₋₆-alkyle
R₂ représente CONHR₄, benzimidazol-2-yle, CR₅R₆OH ou CR₇R₈NHR₉ ;
R₃ représente aryle, aryl-C₁₋₄-alkyle ou aryl-C₂₋₄-alcényle, aryle étant choisi parmi phényle,
X biphényle, naphtyle, bisarylméthyle, hétéroaryle et hétéroaryl-C₁₋₄-alkyle ; hétéroaryle étant choisi parmi indolyle, thiophényle, benzothiophényle, furannyle, benzofurannyle, 1,3-benzodioxolyle, 1,4-benzodioxanyle ; et C₅₋₇-cycloalkyl-C₁-alkyle ; représente NR₁₀ ou CH₂ ;
où :
R₄ est choisi dans le groupe constitué par hydrogène, C₁₋₁₀-alkyle, C₂₋₁₀-alcényle, C₃₋₈-cycloalkyle, C₈₋₁₂-bicycloalkyl, C₁₀₋₁₄-tricycloalkyle, C₃₋₈-cycloalkyl-C₁₋₆-alkyle, C₈₋₁₂-bicycloalkyl-C₁₋₆-alkyle, C₈₋₁₂-bicycloalkylamino-C₁₋₆-alkyle, C₈₋₁₂-tricycloalkylamino-C₁₋₆-alkyle, C₃₋₈-cycloalcényle, C₃₋₈-cycloalcényl-C₁₋₆-alkyle, fluoroalkyl de formule CₙHₓF_{y}, où n représente un entier de 1 à 20, x représente un entier de 0 à 40, y représente un entier de 1 à 41 et la somme de x et y vaut 2n+1, amino-C₂₋₂₀-alkyle, monoalkylamino-C₂₋₂₀-alkyle ou dialkylamino-C₂₋₂₀-alkyle, monohydroxy-C₂₋₂₀-alkyle, dihydroxy-C₂₋₂₀-alkyle ou trihydroxy-C₂₋₂₀-alkyle, C₁₋₆-alcoxy-C₂₋₂₀-alkyle, C₁₋₂₀-thioalkyle, C₁₋₆-alkylthio-C₂₋₂₀-alkyle, hydroxy-C₂₋₆-alcoxy-C₂₋₆-alkyle, C₁₋₆-alkylbis-(hydroxy-C₁₋₆-alkyle), aryle, aryl-C₁₋₁₀-alkyle, aryl-C₃₋₄-alcényle, aryle étant choisi parmi phényle, biphényle, indanyle et naphtyle ; hétéroaryle ou hétéroaryl-C₁₋₁₀-alkyle, hétéroaryle étant choisi parmi pyridyle, thiophényle, furannyle, benzofurannyle, benzothiophényle, pyrrolidinyle et indolyle ; hétérocycloalkyle ou hétérocycloalkyl-C₁₋₁₀-alkyle, hétérocycloalkyle étant choisi parmi tétrahydrofurannyle, tétrahydropyrannyle, morpholinyle, pipéridinyle, pyrrolidinyle, tétrahydrothiophényle, pipérazinyle, dioxolanyle, sulfolanyle et 1,1-dioxotétrahydrothiopyrannyle ; hétérobicyclyle de formule : 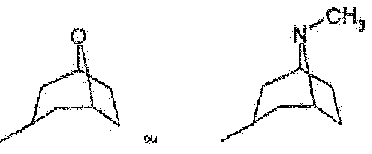 comprenant C₅₋₈-cycloalkyle, C₈₋₁₂-bicycloalkyle, et/ou hétérocyclyle, éventuellement substitué par un ou plusieurs substituents choisis dans le groupe constitué par C₁₋₁₀-alkyle, C₁₋₁₀-alcoxy, hydroxy, hydroxy-C₁₋₁₀-alcoxy, hydroxy-C₁₋₁₀-alkyle, C₁₋₁₀-acyloxy, amino, C₁₋₁₀-alkylcarbonylamino, hydroxy-C₁₋₂₀-alkylcarbonylamino, amino-C₂₋₁₀-alcoxy, mono-C₁₋₆-alkylamino-C₂₋₁₀-alcoxy, di-C₁₋₆-alkylamino-C₂₋₁₀-alcoxy, mono-(C₁₋₁₀-alkyl)amino, di-(C₁₋₁₀-alkyl)amino, amino-C₁₋₁₀-alkylcarbonylamino, C₁₋₁₀-alkylaminocarbonyle, hydroxy-C₁₋₁₀-alkylaminocarbonyle, amino-C₂₋₁₀-alkylaminocarbonyle, monoalkylamino-C₁₋₁₀-alkylaminocarbonyle ou dialkylamino-C₁₋₁₀-alkylaminocarbonyle, oxo, aryle éventuellement substitué et hétéroaryle éventuellement substitué, aryle étant choisi parmi phényle, naphtyle et biphényle et hétéroaryle étant choisi parmi pyridyle et thiophényle ; et
où : les groupes aryle ou hétéroaryle de R sont éventuellement substitués par un ou plusieurs substituents choisis dans le groupe constitué par fluor, chlore, brome, C₁₋₂₀-alkyle, fluoroalkyle ou fluoroalcoxy de formule CₙHₓF_{y} ou OCₙHₓF_{y}, où n représente un entier de 1 à 4, x représente un entier de 0 à 8, y représente un entier de 1 à 9 et la somme de x et y vaut 2n+1, C₁₋₂₀-alcoxy, C₁₋₂₀-alcényloxy, C₁₋₁₀-alcoxy-C₁₋₁₀-alcoxy, hydroxy, hydroxy-C₁₋₂₀-alcoxy, hydroxy-C₁₋₂₀-alkyle, C₁₋₂₀-acyloxy, hydroxy-C₁₋₂₀-acyle, nitro, amino, aminosulfonyle, C₁₋₂₀-alkylcarbonylamino, hydroxy-C₁₋₂₀-alkylcarbonylamino, amino-C₂₋₂₀-alcoxy, amino-C₁₋₂₀-alkylcarbonylamino, C₁₋₂₀-alkylaminocarbonyle, hydroxy-C₁₋₂₀-alkylaminocarbonyle, amino-C₁₋₂₀-alkylaminocarbonyle, monoalkylamino-C₁₋₂₀-alkylaminocarbonyle ou dialkylamino-C₁₋₂₀-alkylaminocarbonyle, aryle ou hétéroarylsulfonyle éventuellement substitué, aminosulfonyle, mono(C₁₋₆)alkylaminosulfonyle ou di(C₁₋₆)alkylaminosulfonyle, phényle ou thiophényle éventuellement substitué, phényl-C₁₋₁₀-alkyloxy ou thiophényl-C₁₋₁₀-alkyloxy éventuellement substitué et cyano ;
où : le substituant phényle ou thiophényle sur le groupe aryle ou hétéroaryle de R est éventuellement substitué par un ou plusieurs substituents choisis dans le groupe constitué par fluor, chlore, brome, C₁₋₅-alkyle, fluoroalkyl ou fluoroalkoxy de formule CₙHₓF_{y} ou OCₙHₓF_{y}, où n représente un entier de 1 à 4, x représente un entier de 0 à 8, y représente un entier de 1 à 9 et la somme de x et y vaut 2n+1, C₁₋₁₀-alcoxy, C₁₋₁₀-alcényloxy, C₁₋₅-alcoxy-C₁₋₅-alcoxy, hydroxy, hydroxy-C₁₋₁₀-alcoxy, hydroxy-C₁₋₁₀-alkyle, hydroxy-C₁₋₂₀-alkyloxo, nitro, amino, aminosulfonyle, C₁₋₁₀-alkylcarbonylamino, hydroxy-C₁₋₁₀-alkylcarbonylamino, amino-C₂₋₁₀-alcoxy, amino-C₁₋₁₀-alkylcarbonylamino, C₁₋₁₀-alkylaminocarbonyle, hydroxy-C₁₋₁₀-alkylaminocarbonyle, amino-C₁₋₁₀-alkylaminocarbonyle, monoalkylamino-C₁₋₁₀-alkylaminocarbonyle ou dialkylamino-C₁₋₁₀-alkylaminocarbonyle, aryl-C₁₋₁₀-alkyloxy éventuellement substitué et cyano ;
où : le radical aryle ou hétéroaryle de R₃ est éventuellement substitué par un ou plusieurs substituents choisis parmi les suivants : C₃₋₈-cycloalkyloxy, C₁₋₁₀-alcoxy, C₁₋₁₀-alkyle, C₂₋₁₀-alcényle, C₃₋₁₀-alcényloxy, mono-(C₁₋₆-alkyl)amino-C₂₋₆-alkyloxy, di-(C₁₋₆-alkyl)amino-C₂₋₆-alkyloxy, amino-C₂₋₆-alkyloxy, hétérocyclyl-C₂₋₆-alkyloxy, aryl-C₂₋₆-alkyloxy, hétéroaryl-C₂₋₆-alkyloxy, fluoroalkyl ou fluoroalkoxy de formule CₙHₓF_{y} ou OCₙHₓF_{y}, où n représente un entier de 1 à 4, x représente un entier de 0 à 8, y représente un entier de 1 à 9 et la somme de x et y vaut 2n+1, C₁₋₆-alcoxy-C₂₋₆-alcoxy, aryl-C₁₋₆-alkyloxy, hydroxy, chloro, fluoro, bromo, hydroxy-C₁₋₆-alcoxy, hydroxy-C₁₋₁₀-alkyle, C₁₋₆-alkyloxo, cyano, amino, 4-aryloxo, di-C₁₋₅-alkyphosphityl-C₁₋₅-alkyle, C₁₋₆-alkyloxoamino, C₁₋₆-alkyloxo(C₁₋₆-alkyl)amino, C₁₋₁₀-alkylsulfonyle, arylsulfonyle, carboxy, C₁₋₂₀-carboalcoxy, dialkylamino-C₁₋₁₀-alkyle, monoalkylamino-C₁₋₁₀-alkyle et amino-C₁₋₁₀-alkyle ;
où : le groupe substituant hétérocycloalkyle attaché au groupe alkyloxy, de R₃ est choisi parmi les suivants : pyrrolidinyle, morpholinyle, pipéridinyle et pipérazinyle ;
où : le groupe substituant hétéroaryle attaché au groupe alkyloxy, de R₃ est choisi parmi pyridinyle et pyrrolidinyle ;
où : le radical aryle du substituant arylsulfonyle sur le radical aryle de R₃ est choisi parmi phényle, indolyle, thiophényle et furannyle ;
où : les groupes aryle ou hétéroaryle de R₄ sont éventuellement substitués par un ou plusieurs substituants choisis parmi les suivants : mono-(C₁₋₆-alkyl) amino ou di-(C₁₋₆-alkyl) amino, mono-(C₁₋₆)alkylamino-C₂₋₆-alkyle ou di-(C₁₋₆)alkylamino-C₂₋₆-alkyle, amino-C₁₋₆-alkyle, hétérocycloalkyle, amino, C₁₋₂₀-alcoxy, chloro, fluoro, bromo, C₁₋₁₀-alkyl, aryle et hétéroaryle ;
où : le groupe hétérocycloalkyle attaché au groupe aryle de R₄ est choisi parmi les suivants : morpholinyle, pyrrolidinyle, pipérazinyle et pipéridinyle ;
où : le groupe hétéroaryle attaché au radical aryle de R₄ est choisi parmi pyridyle, pyrrolyle et furannyle ;
R₅, R₆ = indépendamment, H ou C₁₋₆-alkyle :
R₇, R₈ = indépendamment, H ou C₁₋₆-alkyle :
R₉ = di-(C₁₋₅-alkyl)amino-C₂₋₄-alkyle ; mono(C₁₋₅-alkyl)amino-C₂₋₄-alkyle ; amino-C₂-₄-alkyle ou hétérocycloalkyl-méthyle, où hétérocycloalkyle est choisi dans le groupe constitué par thiophényle, furannyle, pyrrolyl, et pyridinyle ; et
R₁₀ = H ou C₁₋₆-alkyle, à l'exception du composé dans lequel R représente un groupe phényle substitué en position para par un groupe méthyle, R₁ représente hydrogène, R₂ représente CR₅R₆OH, R₃ représente phénylméthyle, X représente NR₁₀, R₅, R₆ et Rio représentent hydrogène.

2. Composé selon la revendication 1, où
R est choisi dans le groupe constitué par aryle, aryl-C₁₋₆-alkyle, aryl-C₂₋₄-alcényle, où aryle est choisi parmi phényle, biphényle ou naphtyle ; hétéroaryle ou hétéroaryl-C₁₋₄-alkyle, où hétéroaryle est choisi parmi pyridyle, thiophényle ou benzothiophényle ; et hétérocyclyle choisi parmi pipéridinyle ou pipérazinyle
R₁ représente hydrogène ou méthyle ;
R₂ représente CONHR₄, benzimidazol-2-yle, CR₅R₆OH ou CR₇R₈NHR₉ ;
R₃ est choisi dans le groupe constitué par phényle et aryl-C₁₋₄-alkyle ou aryl-C₂₋₄-alcényle, où aryle est choisi parmi phényle, biphényle, naphtyle ou bisarylméthyle ; et hétéroaryle ou hétéroaryl-C₁₋₄-alkyle, ou hétéroaryle est choisi parmi indolyle et thiophényle.
X représente NR₁₀ ou CH₂ ; où R₄ est choisi dans le groupe constitué par hydrogène, C₁₋₁₀-alkyle, C₂₋₁₀-alcényle, C₃₋₈-cycloalkyle, C₈₋₁₂-bicycloalkyle, C₁₀₋₁₄-tricycloalkyle, C₃₋₈-cycloalkyl-C₁₋₆-alkyle, C₈₋₁₂-bicycloalkyl-C₁₋₆-alkyle, C₈₋₁₂-bicycloalkylamino-C₁₋₆-alkyle, C₈₋₁₂-tricycloalkylamino-C₁₋₆-alkyle, C₃₋₈-cycloalcényle, C₃₋₈-cycloalcényl-C₁₋₆-alkyle, fluoroalkyl de formule CₙHₓF_{y}, où n représente un entier de 1 à 10, x représente un entier de 0 à 20, y représente un entier de 1 à 21 et la somme de x et y vaut 2n+1, amino-C₂₋₁₀-alkyle, monoalkylamino-C₂₋₁₀-alkyle ou dialkylamino-C₂₋₁₀-alkyl, monohydroxy-C₂₋₁₀-alkyle, dihydroxy-C₂₋₁₀-alkyl ou trihydroxy-C₂₋₁₀-alkyle, C₁₋₆-alcoxy-C₂₋₁₀-alkyle, C₁₋₁₀-thioalkyle, C₁₋₆-alkylthio-C₂₋₁₀-alkyle, hydroxy-C₂₋₆-alcoxy-C₂₋₆-alkyle, C₁₋₆-alkylbis-(hydroxy-C₁₋₆-alkyle), aryle, aryl-C₁₋₆-alkyle et aryl-C₃₋₄-alcényle, aryle étant choisi parmi phényle, biphényle, indanyle et naphtyle ; hétéroaryle ou hétéroaryl-C₁₋₁₀-alkyle, hétéroaryle étant choisi parmi pyridyle, thiophényle, furannyle, benzofurannyle, benzothiophényle, pyrrolidinyl, et indolyle ; hétérocycloalkyle ou hétérocycloalkyl-C₁₋₁₀-alkyle, hétérocycloalkyle étant choisi parmi tétrahydrofurannyle, tétrahydropyrannyle, morpholinyl, pipéridinyle, pyrrolidinyle, pipérazinyle, dioxolanyle, sulfolanyle et 1,1-dioxotétrahydrothiopyrannyle ; où C₅₋₈-cycloalkyle, C₈₋₁₂-bicycloalkyle ou hétérocyclyle est éventuellement substitué par un ou plusieurs substituents choisis dans lé groupe constitué par C₁₋₆-alkyle, C₁₋₆-alcoxy, hydroxy, hydroxy-C₁₋₆-alcoxy, hydroxy-C₁₋₆-alkyle, amino, C₁₋₆-alkylcarbonylamino, hydroxy-C₁₋₆-alkylcarbonylamino, amino-C₂₋₆-alcoxy, mono-C₁₋₆-alkylamino-C₂₋₆-alcoxy, di-C₁₋₆-alkylamino-C₂₋₆-alkoxy, mono-(C₁₋₆-alkyl) amino di-(C₁₋₆-alkyl) amino, amino-C₁₋₆-alkylcarbonylamino, C₁₋₆-alkylaminocarbonyle, hydroxy-C₁₋₆- alkylaminocarbonyle, amino-C₂-₆-alkylaminocarbonyle, monoalkylamino-C₁₋₆-alkylaminocarbonyle ou dialkylamino-C₁₋₆-alkylaminocarbonyle, oxo, aryle éventuellement substitué et hétéroaryle éventuellement substitué, aryle étant choisi parmi phényle, naphtyle et diphényle et hétéroaryle étant choisi parmi pyridyl et thiophényle ; où : les groupes aryle ou hétéroaryle de R sont éventuellement substitués par un ou plusieurs substituants choisis dans le groupe constitué par fluor, chlore, brome, C₁₋₁₀-alkyle, fluoroalkyl ou fluoroalkoxy de formule CₙHₓF_{y} ou OCₙHₓF_{y}, où n représente un entier de 1 à 4, x représente un entier de 0 à 8, y représente un entier de 1 à 9 et la somme de x et y vaut 2n+1, C₁₋₁₀-alcoxy, C₁₋₁₀-alcényloxy, C₁₋₁₀-alcoxy-C₁₋₁₀-alcoxy, hydroxy, hydroxy-C₁₋₁₀-alcoxy, hydroxy-C₁₋₁₀-alkyle, hydroxy-C₁₋₁₀-acyle, nitro, amino, aminosulfonyle, C₁₋₁₀-alkylcarbonylamino, hydroxy-C₁₋₁₀-alkylcarbonylamino, amino-C₂₋₁₀-alcoxy, amino-C₁₋₁₀-alkylcarbonylamino, C₁₋₁₀-alkylaminocarbonyle, hydroxy-C₁₋₁₀-alkylaminocarbonyle, amino-C₁₋₁₀-alkylaminocarbonyle, monoalkylamino-C₁₋₁₀-alkylaminocarbonyle ou dialkylamino-C₁₋₁₀-alkylaminocarbonyle, aryle ou hétéroarylsulfonyle éventuellement substitué, aminosulfonyl, mono(C₁₋₆)alkylaminosulfonyle ou di(C₁₋₆)alkylaminosulfonyle, phényle ou thiophényle éventuellement substitué, phényl-C₁₋₁₀-alkyloxy ou thiophényl-C₁₋₁₀-alkyloxy éventuellement substitué et cyano ; où : le substituant phényle ou thiophényle sur le groupe aryle ou hétéroaryle de R est éventuellement substitué par un ou plusieurs substituants choisis dans le groupe constitué par fluor, chlore, brome, C₁₋₅-alkyle, CF₃, OCF₃, C₁₋₆-alkoxy, C₁₋₆-alcényloxy, C₁₋₅-alcoxy-C₁₋₅-alcoxy, hydroxy, hydroxy-C₁₋₁₀-alcoxy, hydroxy-C₁₋₁₀-alkyle, nitro, amino, aminosulfonyl, C₁₋₁₀-alkylcarbonylamino, hydroxy-C₁₋₆-alkylcarbonylamino, amino-C₂₋₆-alcoxy, amino-C₁₋₆-alkylcarbonylamino, C₁₋₆-alkylaminocarbonyle, hydroxy-C₁₋₆-alkylaminocarbonyle, aminc-C₁₋₆-alkylaminocarbonyle, monoalkylamino-C₁₋₆-alkylaminocarbonyle ou dîalkylamino-C₁₋₆-alkylaminocarbonyle, aryl-C₁₋₆-alkyloxy éventuellement substitué et cyano ; où : le radical aryle ou hétéroaryle de R₃ est éventuellement substitué par un ou plusieurs substituants choisis parmi les suivants : C₃₋₆-cycloalkyloxy, C₁₋₆-alcoxy, C₁₋₆-alkyle, C₂₋₆-alcényle, C₃₋₆-alcényloxy, mono-(C₁₋₆-alkyl)amino-C₂₋₆-alkyloxy, di-(C₁₋₆-alkyl)amino-C₂₋₆-alkyloxy, amino-C₂₋₆-alkyloxy, hétérocyclyl-C₂₋₆-alkyloxy, aryl-C₂₋₆-alkyloxy, hétéroaryl-C₂₋₆-alkyloxy, fluoroalkyle ou fluoroalcoxy de formule CₙHₓF_{y} ou OCₙHₓF_{y}, où n représente un entier de 1 à 4, x représente un entier de 0 à 8, y représente un entier de 1 à 9 et la somme de x et y vaut 2n+1, C₁₋₆-alcoxy-C₂₋₆-alkyloxy, aryl-C₁₋₆-alcoxy, hydroxy, chlore, fluoro, bromo, hydroxy-C₁₋₆-alcoxy, hydroxy-C₁₋₁₀-alkyle, C₁₋₆-alkyloxo, cyano, amino, 4-aryloxo, C₁₋₆-alkyloxoamino, C₁₋₆-alkyloxo(C₁₋₆-alkyl)amino, carboxy, dialkylamino-C₁₋₆-alkyle, monoalkylamino-C₁₋₆-alkyle et amino-C₁₋₆-alkyle ; où : le groupe substituant hétérocyclyle attaché au groupe alkyloxy de R₃ est choisi parmi les suivants : pyrrolidinyle, morpholinyle, pipéridinyle et pipérazinyle ; où : le groupe substituant hétéroaryle attaché au groupe alkyloxy de R₃ est choisi parmi pyridinyle et pyrrolidinyle ; où : les groupes aryle ou hétéroaryle de R₄ sont éventuellement substitués par un ou plusieurs substituants choisis parmi les suivants : mono-(C₁₋₆-alkyl)amino ou di-(C₁₋₆-alkyl)amino, mono- (C₁₋₆)alkylamino-C₂₋₆-alkyle ou di-(C₁₋₆)alkylamino-C₂₋₆-alkyle, amino-C₁₋₆-alkyle, hétérocyclyle, amino, C₁₋₁₀-alcoxy, chlore, fluor, bromo, C₁₋₁₀-alkyle, aryle et hétéroaryle ; où : le groupe hétérocyclyle attaché au groupe aryle de R₄ est choisi parmi les suivants : morpholinyle, pyrrolidinyl, pipérazinyle et pipéridinyle ; où : le groupe hétéroaryle attaché au radical aryle de R₄ est choisi parmi pyridyle, pyrrolyle, et furannyle ;
R₅, R₆ = H ;
R₇, R₈ = H ;
R₉ = di-(C₁₋₅-alkyl)amino-C₂₋₄-alkyle ; mono(C₁₋₅-alkyl)amino-C₂₋₄-alkyle ; amino-C₂₋₄-alkyle, hétérocycloalkyl-méthyle, où hétérocycloalkyle est choisi dans le groupe constitué par thiophényle, furannyle, pyrrolyl, et pyridinyl ; et
R₁₀ = H, CH₃, à l'exception du composé dans lequel R représente un groupe phényle substitué en position para par un groupe méthyle, R₁ représente hydrogène, R₂ représente CR₅R₆OH, R₃ représente phénylméthyle, X représente NR₁₀, R₅, R₆ et R₁₀ représentent hydrogène.

3. Composé selon la revendication 2, où
R représente aryle choisi parmi phényle, diphényle et naphtyle, ou hétéroaryle choisi parmi pyridyle et thiophényle,
R₁ représente hydrogène ou méthyle,
R₂ représente CONHR₄,
R₃ représente aryl-C₁-alkyle, où aryle est choisi parmi phényle, biphényle et naphtyle, ou hétéroaryl-C₁₋alkyle, où hétéroaryle est choisi parmi indolyle et thiophényle.
X représente NR₁₀, où R₄ est choisi dans le groupe constitué par hydrogène, C₁₋₁₀-alkyle, C₂₋₁₀-alcényle, C₃₋₈- cycloalkyl, C₈₋₁₂-bicycloalkyle, C₁₀₋₁₄-tricycloalkyle, C₃-₈-cycloalkyl-C₁₋₆-alkyle, C₈₋₁₂-bicycloalkyl-C₁₋₆-alkyle, C₈₋₁₂-bicycloalkylamino -C₁₋₆-alkyle, C₈-₁₂-tricycloalkylamino-C₁₋₆-alkyle, C₃₋₈-cycloalcényle, C₁₋₈-cycloalcényl-C₁₋₆-alkyle, fluoroalkyle de formule CₙHₓF_{y}, où n représente un entier de 1 à 6, x représente un entier de 0 à 12, y représente un entier de 1 à 13 et la somme de x et y vaut 2n+1, amino-C₂₋₈-alkyle, monoalkylamino-C₂₋₈-alkyle ou dialkylamino-C₂₋₈-alkyle, monohydroxy-C₂₋₁₀-alkyle, dihydroxy-C₂₋₁₀-alkyle ou trihydroxy-C₂₋₁₀-alkyle, C₁₋₆-alcoxy-C₂₋₁₀-alkyle, C₁₋₆-thioalkyle, C₁₋₆-alkylthio-C₂₋₆-alkyle, hydroxy-C₂₋₆-alcoxy-C₂₋₆-alkyle, C₁₋₆-alkylbis-(hydroxy-C₁₋₆-alkyle), aryle, aryl-C₁₋₆-alkyle, aryle étant choisi parmi phényle, diphényle et naphtyle ; hétéroaryle ou hétéroaryl-C₁₋₆-alkyle, hétéroaryle étant choisi parmi pyridyle, thiophényle ; hétérocycloalkyle ou hétérocycloalkyl-C₁₋₆-alkyle, hétérocycloalkyle étant choisi parmi tétrahydrofurannyle, tétrahydropyrannyle, morpholinyl, pipéridinyle, pyrrolidinyle et pipérazinyle ; où : les groupes aryle ou hétéroaryle de R sont éventuellement substitués par un ou plusieurs substituants choisis dans le groupe constitué par fluor, chlore, brome, C₁₋₁₀-alkyle, CF₃, OCF₃, C₁₋₁₀-alkoxy, C₁₋₁₀-alcényloxy, C₁₋₅-alcoxy-C₁₋₁₀-alcoxy, hydroxy, hydroxy-C₁₋₁₀-alcoxy, hydroxy-C₁₋₁₀-alkyle, nitro, amino, C₁₋₁₀-alkylcarbonylamino, hydroxy-C₁₋₁₀-alkylcarbonylamino, amino-C₂₋₁₀-alcoxy, amino-C₁₋₁₀-alkylcarbonylamino, phényle ou thiophényle éventuellement substitué, phényl-C₁₋₁₀-alkyloxy ou thiophényl-C₁₋₁₀-alkyloxy éventuellement substitué et cyano ; où : le substituant phényle ou thiophényle sur le groupe aryle ou hétéroaryle de R est éventuellement substitué par un ou plusieurs substituants choisis dans le groupe constitué par fluor, chlore, brome, C₁₋₅-alkyle, CF₃, OCF₃, C₁₋₆-alcoxy, C₁₋₆-alcényloxy, C₁₋₅-alcoxy-C₁₋₅-alcoxy, hydroxy, hydroxy-C₁₋₁₀-alcoxy, hydroxy-C₁₋₁₀-alkyle, nitro, aryl-C₁₋₆-alkyloxy éventuellement substitué et cyano ; où : le radical aryle ou hétéroaryle de R₃ est éventuellement substitué par un ou plusieurs substituants choisis parmi : C₃₋₆-cycloalkyloxy, C₁₋₆-alcoxy, C₁₋₆-alkyle, C₂₋₆-alcényle, C₃₋₆-alcényloxy, mono-(C₁₋₆-alkyl)amino-C₂₋₆-alkyloxy, di-(C₁₋₆-alkyl)amino-C₂₋₆-alkyloxy, amino-C₂₋₆-alkyloxy, hétérocyclyl-C₂₋₆-alkyloxy, aryl-C₂₋₆-alkyloxy, hétéroaryl-C₂₋₆-alkyloxy, fluoroalkyle ou fluoroalcoxy de formule CₙHₓF_{y} ou OCₙHₓF_{y}, où n représente un entier de 1 à 4, x représente un entier de 0 à 8, y représente un entier de 1 à 9 et la somme de x et y vaut 2n+1, C₁₋₆-alcoxy-C₂₋₆-alcoxy, aryl-C₁₋₆-alkyloxy, hydroxy, chloro, fluor, brome, nitro, hydroxy-C₁₋₆-alcoxy, hydroxy,-C₁₋₆-alkyle, C₁₋₆-alkyloxo, cyano, amino, 4-aryloxo, C₁₋₆-alkyloxoamino, carboxy, dialkylamino-C₁₋₆-alkyle, monoalkylamino-C₁₋₆-alkyle et amino-C₁₋₆-alkyle ; où : le groupe substituant hétérocycloalkyle attaché au groupe alkyloxy de R₃ est choisi parmi : pyrrolidinyle, morpholinyle et pipéridinyle ; où : le groupe substituant hétéroaryle attaché au groupe alkyloxy de R₃ est choisi parmi pyridinyle et pyrrolidinyle ; où : les groupes aryle ou hétéroaryle de R₄ sont éventuellement substitués par un ou plusieurs substituants choisis parmi les suivants : mono-(C₁₋₆-alkyl)amino ou di-(C₁₋₆-alkyl)amino, mono-(C₁₋₆)alkylamino-C₂₋₆-alkyle ou di-(C₁₋₆)alkylamino-C₁₋₆-alkyle, amino-C₁₋₆-alkyle, hétérocycloalkyle, amino, C₁₋₆-alcoxy, chloro, fluoro, bromo, C₁₋₆-alkyle, aryle et hétéroaryle ; où : le groupe hétérocycloalkyle attaché au groupe aryle de R₄ est choisi parmi les suivants : morpholinyle, pyrrolidinyle, pipérazinyle et pipéridinyle ; et où : le groupe hétéroaryle attaché au radical aryle de R₄ est choisi parmi pyridyle et pyrrolyle.

4. Composé selon la revendication 3, où
R représente aryle, aryle étant choisi parmi phényle et naphtyle,
R₁ représente hydrogène ou méthyle,
R₂ représente CONHR₄,
R₃ représente aryl-C₁-alkyle, aryle étant choisi parmi phényle, indolyle et thiophényle ;
x représente NH où R₄ est choisi dans le groupe constitué par hydrogène, C₁₋₁₀-alkyle, C₃₋₆-cycloalkyle, adamantyl, adamantyl-C₁-alkyle, 2-adamantylaminoéthyle, C₃₋₆-cycloalkyl-C₁-alkyle, 3-propényle, trifluorométhyl-C₁-alkyle, 4-amino-n-butyl, 4-(méthylamino)-n-butyle, 4-(diméthylamino)-n-butyle, 4-diéthylaminobutyle, 2-hydroxyéthyle, 2-(2-hydroxyéthoxy)éthyle, 3-hydroxypropyle, 2-méthoxyéthylaryle, aryle étant choisi parmi phényle et naphtyle ; aryl-C₁-alkyle, aryle étant choisi parmi phényle et diphényle ; hétéroaryl-C₁-alkyle, hétéroaryle étant choisi parmi 2-thiophényle et 2-pyridinyl ; hétérocycloalkyle, hétérocycloalkyle étant choisi parmi 4-tétrahydropyrannyle, 4-pipéridinyle, 2,2,6,6-tétraméthylpipéridinyle et 1-éthyl-4-pipéridinyle ; hétérocycloalkyl-C₁-alkyle, hétérocycloalkyle étant choisi parmi 4-tétrahydropyrannyle et 2-tétrahydrofurannyle ; 2-hétérocycloalkyl-C₂-alkyle, hétérocycloalkyle étant choisi parmi 4-tétrahydropyrannyle et 1-pipérazinyle ; où : le radical phényle de R est éventuellement substitué par un ou deux substituants dans les positions 3, 4 ou 5, choisis dans le groupe constitué par fluor, chlore, brome, trifluorométhyle, C₁₋₇-alcoxy, C₁₋₇-alcényloxy, C₁₋₈-alkyl et C₂₋₃-hydroxypropyle ; le radical naphtyle de R est éventuellement substitué par un des substituants choisis parmi chlore, fluor et diméthylamino ; le substituant phényle de R₃ est éventuellement substitué par un ou deux substituants choisis dans le groupe constitué par C₁₋₅-alkyle, C₁₋₅-alkyloxy, 2-diméthylaminoéthoxy, 2-pyrrolidinoéthoxy, 3-fluoropropyloxy, 3-méthoxypropyloxy, cyclopentoxy, benzyloxy, hydroxy, chloro, fluor, bromo, trifluorométhyle, 1-hydroxyéthyle, acétyle, alkyloxy, cyano, amino, 4-benzoyle et acétoxy ; le radical phényle de R₄ est éventuellement substitué par un ou deux substituants choisis dans le groupe constitué par 4-diméthylamino, 4-méthylamino, 4-amino-, méthoxy, éthoxy, chloro, 4-morpholino, fluoro et méthyle.

5. Composé selon la revendication 1, choisi parmi :
le [(S)-1-(4-diméthylaminophénylcarbamoyl)-2-(4-méthoxyphényl)-éthyl]-amide de l'acide (S)-1-(3-trifluorométhylbenzènesulfonyl)-pipéridine-2-carboxylique ;
le [(S)-2-4-acétylphényl)-1-(4-éthoxyphénylcarbamoyl)-éthyl]-amide de l'acide (S)-1-(3-trifluorométhylbenzènesulfonyl)-pipéridine-2-carboxylique ;
le [(S)-1-(4-morpholin-4-yl-phénylcarbamoyl)-2-(4-trifluorométhylphényl)-éthyl]-amide de l'acide (S)-1-(3-trifluorométhylbenzènesulfonyl)-pipéridine-2-carboxylique ;
le [(S)-2-(4-méthoxyphényl)-1-(tétrahydropyrann-4-ylcarbamoyl)-éthyl]-amide de l'acide (S)-1-(3-trifluorométhylbenzènesulfonyl)-pipéridine-2-carboxylique ;
le [(S)-2-(2-méthyl-4-méthoxyphényl)-1-(tétrahydropyrann-4.ylcarbamoyl)-éthyl]-amide de l'acide (S)-1-(3-trifluorométhylbenzènesulfonyl)-pipéridine-2-carboxylique ;
le [(S)-2-(3,4-diméthoxyphényl)-1-(tétrahydropyrann-4-ylcarbamoyl)-éthyl]-amide de l'acide (S)-1-(3-trifluorométhylbenzènesulfonyl)-pipéridine-2-carboxylique ;
le [(S)-2-(4-méthoxyphényl)-1-(4-morpholin-4-ylphénylcarbamoyl)-éthyl]-amide de l'acide (S)-1-(3-trifluorométhylbenzènesulfonyl)-pipéridine-2-carboxylique ;
le ((S)-1-carbamoyl-2-phényléthyl)-amide de l'acide (S)-1-(3-méthoxybenzènesulfonyl)-pipéridine-2-carboxylique ;
le ((S)-1-carbamoyl-2-phényléthyl)-amide de l'acide (S)-1-(3-hydroxybenzènesulfonyl)-pipéridine-2-carboxylique ;
le ((S)-1-carbamoyl-2-phényléthyl)-amide de l'acide (S)-1-(3-propoxybenzènesulfonyl)-pipéridine-2-carboxylique ;
le [(S)-2-[4-(-hydroxyéthyl)-phényl]-1-(4-morpholin-4-ylphénylcarbamoyl)-éthyl]-amide de l'acide (S)-1-(3-trifluorométhylbenzénesulfonyl)-pipéridine-2-carboxylique ;
le {(S)-1-(4-éthoxyphénylcarbamoyl)-2-[4-(1-hydroxyéthyl)-phényl]-éthyl}-amide de l'acide (S)-1-(3-trifluorométhylbenzènesulfonyl)-pipéridine-2. carboxylique ;
le [(S)-2-(indol-3-yl)-1-(naphtalén-2-ylcarbamoyl)-éthyl]-amide de l'acide (S)-1-(3-méthoxybenzènesulfonyl)-pipéridine-2-carboxylique ;
le {(S)-1-(4-diméthylaminophénylcarbamoyl)-2-[4-(1-hydroxyéthyl)-phényl]-éthyl}-amide de l'acide (S)-1-(3-trifluorométhylbenzènesulfonyl)-pipéridine-2-carboxylique ;
le [(S)-2-(1H-indol-3-yl)-1-méthylcarbamoyléthyl]-amide de l'acide (S)-1-(3,5-dichlorobenzènesulfonyl)-pipéridine-2-carboxylique ;
le [(S)-2-(4-acétylphényl)-1-(4-morpholin-4-ylphénylcarbamoyl)-éthyl]-amide de l'acide (S)-1-(3-trifluorométhylbenzènesulfonyl)-pipéridine-2-carboxylique ;
le [(S)-2- (4-cyclopentyloxyphényl)-1-méthyl-1-(tétrahydropyrann-4-ylcarbamoyl)-éthyl]-amide de l'acide (S)-1-(3-fluorobenzénesulfonyl)-pipéridine-2-carboxylique ;
le [(S)-2-(4-isopropoxyphényl)-1-(2,2,6,6-tétraméthylpipéridin-4-ylcarbamoyl)-éthyl]-amide de l'acide (S)-(-(3-fluorobenzènesulfonyl)-pipéridine-2-carboxylique ;
le [(S)-2-[4-(3-fluoropropoxy)-phényl]-1-(2,2,6,6-tétraméthylpipéridin-4-ylcarbamoyl)-éthyl]-amide de l'acide (S)-1-(3-fluorobenzènesulfonyl)-(2S)-pipéridine-2-carboxylique ;
le [(S)-2-[4-3-fluoropropoxy-phényl]-1-(S)-2-méthoxy-1-méthyléthylcarbamoyl)-éthyl]-amide de l'acide (S)-1-(3-fluorobenzènesulfonyl)-pipéridine-2-carboxylique ;
le [(S)-2-{4-isopropoxyphényl)-1-méthylcarbamoyléthyl]-amide de l'acide (S)-1-(3-trifluorométhylbenzènesulfonyl)pipéridine-2-carboxylique ;
le [(S)-2-4-isopropoxyphényl)-1-(tétrahydropyrann-4-ylcarbamoyl)-éthyl]-amide de l'acide (S)-1-(3-trifluorométhylbenzènesulfonyl)-pipéridine-2-carboxylique ;
le [(S)-2- phényl)-1-(benzimidazol-2-yl)-éthyl]-amide de l'acide (S)-1-(3-trifluorométhylbenzènesulfonyl)-pipéridine-2-carboxylique ;
le [(S)-2-4-(benzyloxy)phényl)-1-benzimidazol-2-yl)-éthyl]-amide de l'acide (S)-1-(3-trifluorométhylbenzènesulfonyl)-pipéridine-2-carboxylique ;
le [(S)-2-(4-méthoxyphényl)-1-(tétrahydropyrann-4-ylcarbamoyl)-éthyl]-amide de l'acide (S)-1-(3-fluorobenzènesulfonyl)-pipéridine-2-carboxylique ;
le ((S)-1-amido-2-(2-méthylphényl)-éthyl)-amide de l'acide (S)-1-benzènesulfonylpipéridine-2-carboxylique ;
le ((S)-1-cyclohexylamido-2-(4-méthylphényl)-éthyl)-amide de l'acide (S)-1-benzénesulfonylpipéridine-2-carboxylique ;
le [(S)-2-(4-allyloxyphényl)-1-cyclohexylamido-éthyl]-amide de l'acide (S)-1-benzénesulfonylpipéridine-2-carboxylique ;
le [(S)-2-(4-allyloxyphényl)-1-cyclohexylcarbamoyléthyl]-amide de l'acide (S)-1-(3-trifluorométhylbenzènesulfonyl)-pipéridine-2-carboxylique ;
le ((S)-1-cyclohexylamido-2-(4-méthylphényl)-éthyl)-amide de l'acide (S)-1-(3-trifluorométhylbenzènesulfonyl)-pipéridine-2-carboxylique ;
le [(S)-1-amido-2-(1H-indol-3-yl)-éthyl]-amide de l'acide (S)-1-((E)-2-phényléthènesulfonyl)-pipéridine-2-carboxylique ;
le [(S)-1-amido-2-(1H-indol-3-yl)-éthyl]-amide de l'acide (S)-1-(4,5-dibromothiophène-2-sulfonyl)-pipéridine-2-carboxylîque ;
le [(S)-1-tertbutylamido-2-(1H-indol-3-yl)-éthyl]-amide de l'acide (S)-1-benzènesulfonylpipéridine-2-carboxylique ;
le [(S)-1-(3-hydroxy-2,2-diméthylpropylamido)-2-(4-isopropylphényl)-éthyl]-amide de l'acide (S)-1-benzènesulfonylpipéridine-2-carboxylique ;
le méthyl-((S)-1-méthylamido-2-(phényl)-éthyl)-amide de l'acide (S)-1-benzènesulfonylpipéridine-2-carboxylique ;
le [(S)-1-(3,3-diméthylbutylamido)-2-(4-méthoxyphényl)-éthyl]-amide de l'acide (S)-1-benzènesulfonylpipéridine-2-carboxylique ;
le [(S)-1-(2,2-diméthylpropylamido)-2-(phényl)-éthyl]-amide de l'acide (S)-1-benzènesulfonylpipéridine-2-carboxylique ;
l'ester diéthylique de l'acide (4-{(S)-2-[((S)-1-benzènesulfonylpipéridine-2-carbonyl)-amino]-2-amido-éthyl}-benzyl)-phosphonique ;
le [(S)-2-(4-(acétylamino)-phenyl)-1-amido-éthyl]-amide de l'acide (S)-1-benzènesulfonylpipéridine-2-carboxylique ;
le [(S)-1-amido-2-(4-(hydroxyméthyl)-phényl)-éthyl]-amide de l'acide (S)-1-benzènesulfonylpipéridine-2-carboxylique ;
le [(S)-1-amido-2-(1H-indol-3-yl)-éthyl]-amide de l'acide (S-1-naphtalène-2-sulfonyl)-pipéridine-2-carboxylique ;
le [(S)-1-(cyclohexylméthylamido)-2-(4-méthylphényl)éthyl]-amide de l'acide (S)-1-(3-trifluorométhylbenzènesulfonyl)-piperidine-2-carboxylique ;
le [(S)-1-(4-aminobutylamido)-2-(4-méthylphényl)-éthyl]-amide de l'acide (S)-1-(-3-trifluorométhylbenzènesulfonyl)-pipéridine-2-carboxylique ;
le [(S)-1-amido-2-(1H-indol-3-yl)-éthyl]-amide de l'acide (S)-1-benzènesulfonylpipéridine-2-carboxylique ;
le ((S)-1-amido-2-(pyridin-4-yl)-éthyl)-amide de l'acide (S)-1-benzènesulfonylpipéridine-2-carboxylique ;
le ((S)-1-amido-2-cyclohexyléthyl)-amide de l'acide (S)-1-benzènesulfonylpipéridine-2-carboxylique ;
le ((S)-2-phényl-1-[(thiophén-2-ylméthyl)-amido]-éthyl}-amide de l'acide (S)-1-benzènesulfonylpipéridine-2-carboxylique ;
le [(S)-1-(4-diméthylaminobutylcarbamoyl)-2-(4-fluorophényl)-éthyl]-amide de l'acide (S)-1-(3-fluorobenzènesulfonyl)-pipéridine-2-carboxylique ;
le [(S)-2-[4-(3-méthoxypropoxy)-phényl]-1-(tétrahydropyrann-4-ylcarbamoyl)-éthyl]-amide de l'acide (S)-1-(3-fluorobenzènesulfonyl)-pipéridine-2-carboxylique ;
le {(S)-1-cyclohexylcarbamoyl-2-[4-(2-diméthylaminoéthoxy)-phényl]-éthyl}-amide de l'acide (S)-1-(3-trifluorométhylbenzènesulfonyl)-pipéridine-2-carboxylique ;
le [(S)-2-(4-méthoxyphényl)-1-(2-pipérazin-1-yléthylcarbamoyl)-éthyl]-amide de l'acide (S)-1-(3-trifluorométhylbenzènesulfonyl)-pipéridine-2-carboxylique ;
le [(S-1-(2-hydroxy-1,1-diméthyléthylcarbamoyl)-2-(4-méthoxyphényl)-éthyl]-amide de l'acide (S)-1-(3-trifluorométhylbenzènesulfonyl)-pipéridine-2-carboxylique ;
le ((S)-1-carbamoyl-2-phényléthyl)-amide de l'acide (S)-1-benzénesulfonylpipéridine-2-carboxylique ;
le ({S)-2-[4-(3-méthoxypropoxy)-phényl]-1-méthylcarbamoyléthyl}-amide de l'acide (S)-1-(3-fluorobenzènesulfonyl)-pipéridine-2-carboxylique ;
le {(S)-1-((S)-2-méthoxy-1-méthyléthylcarbamoyl)-2-[4-(3-méthoxypropoxy)-phényl]-éthyl}-amide de l'acide (S)-1-(3-fluorobenzénesulfonyl)-pipéridine-2-carboxylique ; et
le [(S)-2-1H-indol-3-yl)-(naphtalén-2-ylcarbamoyl)-éthyl]-amide de l'acide (S)-1-(3-hydroxybenzènesulfonyl)-pipéridine-2-carboxylique.

6. Composition pharmaceutique, comprenant un composé, y compris les énantiomères, les stéréo-isomères, les rotamères et les tautomères dudit composé et les sels ou les solvates pharmaceutiquement acceptables de ceux-ci, présentant la structure générale indiquée dans la formule I, telle que définie dans la revendication 1.

7. Composition pharmaceutique selon la revendication 6, où ledit composé de formule I est tel que défini dans la revendication 2.

8. Composition pharmaceutique selon la revendication 7, où ledit composé de formule I est tel que défini dans la revendication 3.

9. Composition pharmaceutique selon la revendication 8, ou ledit composé de formule 1 est tel que défini dans la revendication 4.

10. Composition pharmaceutique selon la revendication 7, dans laquelle le composé est choisi dans le groupe formé par :
le [(S)-1-(4-diméthylaminophénylcarbamoyl)-2-(4-méthoxyphényl)-éthyl]-amide de l'acide (S)-1-(3-trifluorométhylbenzènesulfonyl)-pipéridine-2-carboxylique ;
le [(S)-2-(4-acétylphényl)-1-(4-éthoxyphénylcarbamoyl - éthyl]-amide de l'acide (S)-1-(3-trifluorométhylbenzènesulfonyl)-pipéridine-2-carboxylique ;
le [(S)-1-4-morpholin-4-ylphenylcarbamoyl-2-4-trifluorométhylphényl)-éthyl]-amide de l'acide (S)-1-(3-trifluorométhylbenzènesulfonyl)-pipéridine-2-carboxylique ;
le [(S)-2-(4-méthoxyphényl)-1-(tétrahydropyrann-4-ylcarbamoyl)-éthyl]-amide de l'acide (S)-1-(3-trifluorométhylbenzènesulfonyl)-pipéridine-2-carboxylique ;
le [(S)-2-(2-méthyl-4-méthoxyphényl)-1-(tétrahydropyrann-4-ylcarbamoyl)-éthyl]-amide de l'acide (S)-1-(3-trifluorométhylbenzènesulfonyl)-pipéridine-2-carboxylique ;
le [(S)-2-(3,4-diméthoxyphényl)-1-(tétrahydropyrann-4-ylcarbamoyl)-éthyl]-amide de l'acide (S)-1-(3-trifluorométhylbenzènesulfonyl-pipéridine-2-carboxylique ;
le [(S)-2-(4-méthoxyphényl)-1-(4-morpholin-4-ylphénylcarbamoyl)-éthyl]-amide de l'acide (S)-1-(3-trifluorométhylbenzènesulfonyl)-pipéridine-2-carboxylique ;
le ((S)-1-carbamoyl-2-phényléthyl)-amide de l'acide (S)-1-(3-méthoxybenzènesulfonyl)-pipéridine-2-carboxylique ;
le ((S)-1-carbamoyl-2-phényléthyl)-amide de l'acide (S)-1-(3-hydroxybenzènesulfonyl)-pipéridine-2-carboxylique ;
le ((S)-1-carbamoyl-2-phényléthyl)-amide de l'acide (S)-1-(3-propoxybenzènesulfonyl)-pipéridine-2-carboxylique ;
le [(S)-2-[4-(1-hydroxyéthyl)-phenyl]-1-(4-morpholin-4-ylphénylcarbamoyl)-éthyl]-amide de l'acide (S)-1-(3-trifluorométhylbenzènesulfonyl)-pipéridine-2-carboxylique ;
le ((S)-1-(4-éthoxyphénylcarbamoyl)-2-[4-(1-hydroxyéthyl)-phényl]-éthyl}-amide de l'acide (S)-1-(3-trifluorométhylbenzènesulfonyl)-pipéridine-2-carboxylique ;
le [(S)-2-(indol-3-yl)-1-(naphtalén-2-ylcarbamoyl)-éthyl]-amide de l'acide (S)-1-(3-méthoxybenzènesulfonyl)-pipéridine-2-carboxylique ;
le ((S)-1-(4-diméthylaminophénylcarbamoyl)-2-[4-(1-hydroxyéthyl)-phényl]-éthyl}-amide de l'acide (S)-1-(3-trifluorométhylbenzènesulfonyl)-pipéridine-2-carboxylique ;
le [(S)-2-(1H-indol-3-yl)-1-méthylcarbamoyléthyl]-amide de l'acide (S)-1-(3,5-dichlorobenzènesulfonyl)-pipéridine-2-carboxylique ;
le [(S)-2-(4-acétylphényl)-1-(4-morpholin-4-ylphénylcarbamoyl)-éthyl]-amide de l'acide (S)-1-(3-trifluorométhylbenzènesulfonyl)-pipéridine-2-carboxylique ;
le [(S)-2-(4-cyclopentyloxyphényl)-1-méthyl-1-(tétrahydropyrann-4-ylcarbamoyl)-éthyl]-amide de l'acide (S)-1-(3-fluorobenzènesulfonyl)-pipéridine-2-carboxylique ;
le [(S)-2-(4-isopropoxyphênyl)-1-(2,2,6,6-tétraméthylpipéridin-4-ylcarbamoyl)-éthyl]-amide de l'acide (S)-1-(3-fluorobenzènesulfonyl)-pipéridine-2-carboxylique ;
le [(S)-2-[4-(3-fluoropropoxy)-phényl]-1-(2,2,6,6-tétraméthylpipéridin-4-ylcarbamoyl)-éthyl]-amide de l'acide (S)-1-(3-fluorobenzènesulfonyl)-(2S)-pipéridine-2-carboxylique ;
le [(S)-2-[4-(3-fluoropropoxy)-phényl]-1-((S)-2-méthoxy-1-méthyléthylcarbamoyl)-éthyl]-amide de l'acide (S)-1-(3-fluorobenzènesulfonyl)-pipéridine-2-carboxylique ;
le [(S)-2-(4-isopropoxyphényl)-1-méthylcarbamoyléthyl]-amide de l'acide (S)-1-(3-trifluorométhylbenzènesulfonyl)-pipéridine-2-carboxylique ;
le [(S)-2-4-isopropoxyphényl)-1-(tétrahydropyrann-4-ylcarbamoyl)-éthyl]-amide de l'acide (S)-1-(3-trifluorométhylbenzènesulfonyl)-pipéridine-2-carboxylique ;
le [(S)-2-(phényl)-1-(benzimidazol-2-yl)-éthyl]-amide de l'acide (S)-1-(3-trifluorométhylbenzènesulfonyl)-pipéridine-2-carboxylique ;
le [(S)-2-(4-(benzyloxy)phényl)-1-(benzimidazol-2-yl)-éthyl]-amide de l'acide (S)-1-(3-trifluorométhylbenzénesulfonyl)-pipéridine-2-carboxylique ;
le [(S)-2-(4-méthoxyphényl)-1-(tétrahydropyrann-4-ylcarbamoyl)-éthyl]-amide de l'acide (S)-1-(3-fluorobenzènesulfonyl)-pipéridine-2-carboxylique ;
le ((S)-1-amido-2-(2-méthylphényl)-éthyl)-amide de l'acide (S)-1-benzènesulfonylpipéridine-2-carboxylique ;
le ((S)-1-cyclohexylamido-2-(4-méthylphényl)-éthyl)-amide de l'acide (S)-1-benzènesulfonylpipéridine-2-carboxylique ;
amide [(S)-2-(4-allyloxyphényl)-1-cyclohexylamido-éthyl]-amide de l'acide (S)-1-benzènesulfonylpipéridine-2-carboxylique ;
le [(S)-2-(4-allyloxyphényl)-1-cyclohexylcarbamoyléthyl]-amide de l'acide (S)-1-(3-trifluorométhylbenzénesulfonyl)-pipéridine-2-carboxylique ;
le ((S)-1-cyclohexylamido-2-(4-méthylphényl)-éthyl)-amide de l'acide (S)-1-(3-trifluorométhylbenzénesulfonyl)-pipéridine-2-carboxylique ;
le [(S)-1-amido-2-(1H-indol-3-yl)-éthyl]-amide de l'acide (S)-1-((E)-2-phényléthènesulfonyl)-pipéridine-2-carboxylique ;
le [(S)-1-amido-2-(1H-indol-3-yl)-éthyl]-amide de l'acide (S)-1-(4,5-dibromothiophène-2-sulfonyl)-pipéridine-2-carboxylique ;
le [(S)-1-tertbutylamido-2-(1H-indol-3-yl)-éthyl]-amide de l'acide (S)-1-benzènesulfonylpipéridine-2-carboxylique ;
le [(S)-1-(3-hydroxy-2,2-diméthylpropylamido)-2-(4-isopropylphényl)-éthyl]-amide de l'acide (S)-1-benzènesulfonylpipéridine-2-carboxylique ;
le méthyl-((S)-1-méthylamido-2-(phényl)-éthyl)-amide de l'acide (S)-1-benzènesulfonylpipéridine-2-carboxylique ;
le [(S)-1-3,3-diméthylbutylamido-2-(4-méthoxyphényl)-éthyl]-amide de l'acide (S)-1-benzènesulfonylpipéridine-2-carboxylique ;
le f(S)-1-(2,2-diméthylpropylamido)-2-(phényl)-éthyl]-amide de l'acide (S)-1-benzénesulfonylpipéridine-2-carboxylique ;
l'ester diéthylique de l'acide (4-{(S)-2-[((S)-1-benzènesulfonylpipéridine-2-carbonyl)-amino]-2-amido-éthyl}-benzyl)-phosphonique ;
le [(S)-2-(4-(acétylamino)-phényl)-1-amido-éthyl]-amide de l'acide (S)-1-benzènesulfonylpipéridine-2-carboxylique ;
le [(S)-1-amido-2-(4-(hydroxyméthyl)-phényl)-éthyl]-amide de l'acide (S)-1-benzènesulfonylpipéridine-2-carboxylique ;
le [(S)-1-amido-2-(1H-indol-3-yl)-éthyl]-amide de l'acide (S)-1-(naphtalène-2-sulfonyl)-pipéridine-2-carboxylique ;
le [(S)-1-(cyclohexylméthylamido)-2-(4-méthylphényl)éthyl]-amide de l'acide (S)-1-(3-trifluorométhylbenzènesulfonyl)-pipéridine-2-carboxylique ;
le [(S)-1-(4-aminobutylamido)-2-(4-méthylphényl)-éthyl]-amide de l'acide (S)-1-(3-trifluorométhylbenzènesulfonyl)-pipéridine-2-carboxylique ;
le [(S)-1-amido-2-(1H-indol-3-yl)-éthyl]-amide de l'acide (S)-1-benzènesulfonylpipéridine-2-carboxylique ;
le ((S)-1-amido-2-(pyridin-4-yl)-éthyl)-amide de l'acide (S)-1-benzènesulfonylpipéridine-2-carboxylique ;
le ((S-1-amido-2-cyclohexyléthyl)-amide de l'amide (S)-1-benzènesulfonylpipéridine-2-carboxylique ;
le {(S)-2-phényl-1-[(thiophén-2-ylméthyl)-amido]-éthyl}-amide de l'acide (S)-1-benzènesulfonylpipéridine-2-carboxylique ;
le [(S)-1-(4-diméthylaminobutylcarbamoyl)-2-(4-fluorophényl)-éthyl]-amide de l'acide (S)-1-(3-fluorobenzènesulfonyl)-pipéridine-2-carboxylique ;
le [(S)-2-[4-(3-méthoxypropoxy)-phényl]-1-(tétrahydropyrann-4-ylcarbamoyl)-éthyl]-amide de l'acide (S)-1-(3-fluorobenzènesulfonyl)-pipéridine-2-carboxylique ;
le {(S)-1-cyclohexylcarbamoyl-2-[4-(2-diméthylaminoéthoxy)₋phényl]-éthyl}-amide de l'acide (S)-1-(3-trifluorométhylbenzènesulfonyl)-pipéridine-2-carboxylique ;
le [(S)-2-(4-méthoxyphényl)-1-(2-pipérazin-1-yléthylcarbamoyl)-éthyl]-amide de l'acide (S)-1-(3-trifluorométhylbenzènesulfonyl)-pipéridine-2-carboxylique ;
le [(S)-1-(2-hydroxy-1,1-diméthyléthylcarbamoyl)-2-(4-méthoxyphényl)-éthyl]-amide de l'acide (S)-1-(3-trifluorométhylbenzènesulfonyl)-pipéridine-2-carboxylique ;
le ((S)-1-carbamoyl-2-phényléthyl)-amide de l'acide (S)-1-benzènesulfonylpipéridine-2-carboxylique ;
le {(S)-2-[4-(3-méthoxypropoxy)-phényl]-1-méthylcarbamoyléthyl}-amide de l'acide (S)-1-(3-fluorobenzènesulfonyl)-pipéridine-2-carboxylique ;
le {(S)-1-((S)-2-méthoxy-1-méthyléthylcarbamoyl)-2-[4-(3-méthoxypropoxy)-phényl]-éthyl}-amide de l'acide (S)-1-(3-fluorobenzènesulfonyl-pipéridine-2-carboxylique ;
et
le [(S)-2-(1H-indol-3-yl)-1-(naphtalén-2-ylcarbamoyl)-éthyl]-amide de l'acide (S)-1-(3-hydroxybenzènesulfonyl)-pipéridine-2-carboxylique .

11. Composé présentant la structure générale représentée dans la formule I, telle que définie dans la revendication 1, y compris les énantiomères, les stéréo-isomères, les rotamères et les tautomères dudit composé et les sels ou les solvates pharmaceutiquement acceptables de ceux-ci, pour la préparation d'un médicament destiné au traitement d'un trouble impliquant une lésion neurologique.

12. Composé selon la revendication 11, où le trouble est la maladie de Parkinson.

13. Composé selon la revendication 11, où le trouble est la sclérose en plaques.

14. Composé selon la revendication 11, où le trouble est la maladie d'Alzheimer.

15. Composé selon la revendication 11, où le trouble est un AVC.

16. Composé selon la revendication 11, où le trouble est une lésion de la moelle épinière.

17. Composé selon la revendication 11, où ledit composé est tel que défini dans la revendication 2.

18. Composé selon la revendication 11, où ledit composé est tel que défini dans la revendication 3.

19. Composé selon la revendication 11, où ledit composé est tel que défini dans la revendication 4.

20. Composé selon la revendication 11, le composé étant choisi parmi
le [(S)-1-(4-diméthylaminophénylcarbamoyl)-2-(4-méthoxyphényl)-éthyl]-amide de l'acide (S-1-3-trifluorométhylbenzènesulfonyl)-pipéridine-2-carboxylique ;
le [(S)-2-(4-acétylphényl)-1-(4-éthoxyphénylcarbamoyl)-éthyl]-amide de l'acide (S)-1-(3-trifluorométhylbenzènesulfonyl)-pipéridine-2-carboxylique ;
le [(S)-1-(4-morpholin-4-ylphénylcarbamoyl)-2-(4-trifluorométhylphényl)-éthyl]-amide de l'acide (S)-1-(3-trifluorométhylbenzènesulfonyl)-pipéridine-2-carboxylique ;
le [(S)-2-(4-méthoxyphényl)-1-(tétrahydropyrann-4-ylcarbamoyl)-éthyl]-amide de l'acide (S)-1-(3-trifluorométhylbenzènesulfonyl)-pipéridine-2-carboxylique ;
le [(S)-2-(2-méthyl-4-méthoxyphényl)-1-(tétrahydropyrann-4-ylcarbamoyl)-éthyl]-amide de l'acide (S)-1-(3-trifluorométhylbenzènesulfonyl)-pipéridine-2-carboxylique ;
le [(S)-2-(3,4-diméthoxyphényl)-1-(tétrahydropyrann-4-ylcarbamoyl)-éthyl]-amide de l'acide (S)-1-(3-trifluorométhylbenzènesulfonyl)-pipéridine-2-carboxylique ;
le [(S)-2-(4-méthoxyphényl)-1-(4-morpholin-4-ylphénylcarbamoyl)-éthyl]-amide de l'acide (S)-1-(3-trifluorométhylbenzènesulfonyl)-pipéridine-2-carboxylique ;
le ((S)1-carbamoyl-2-phényléthyl)-amide de l'acide (S)-1-(3-méthoxybenzènesulfonyl)-pipéridine-2-carboxylique ;
le ((S)-1-carbamoyl-2-phényléthyl)-amide de l'amide (S)-1-(3-hydroxybenzènesulfonyl)-pipéridine-2-carboxylique ;
le ((S)-1-carbamoyl-2-phényléthyl)-amide de l'acide (S)-1-(3-propoxybenzènesulfonyl)-pipéridine-2-carboxylique ;
le [(S)-2-[4-(1-hydroxyéthyl)-phényl]-1-(4-morpholin-4-ylphénylcarbamoyl)-éthyl]-amide de l'acide (S)-1-(3-trifluorométhylbenzènesulfonyl)-pipéridine-2-carboxylique ;
le {(S)-1-(4-éthoxyphénylcarbamoyl)-2-[4-(1-hydroxyéthyl)-phényl]-éthyl}-amide de l'acide (S)-1-(3-trifluorométhylbenzènesulfonyl)-pipéridine-2-carboxylique ;
le [(S)-2-(indol-3-yl)-1-(naphtalén-2-ylcarbamoyl)-éthyl]-amide de l'acide (S)-1-(3-méthoxybenzènesulfonyl-pipéridine-2-carboxylique ;
le ((S)-1-(4-diméthylaminophénylcarbamoyl)-2-[4-(1-hydroxyéthyl)-phényl]-éthyl}-amide de l'acide (S)-1-(3-trifluorométhylbenzènesulfonyl)-pipéridine-2-carboxylique ;
le [(S)-2-(1H-indol-3-yl)-1-méthylcarbamoyléthyl]-amide de l'acide (S)-1-(3,5-dichlorobenzènesulfonyl)-pipéridine-2-carboxylique ;
le [(S)-2-(4-acétylphényl)-1-(4-morpholin-4-ylphénylcarbamoyl)-éthyl]-amide de l'acide (S)-1-(3-trifluorométhylbenzènesulfonyl)-pipéridine-2-carboxylique ;
le [(S)-2-(4-cyclopentyloxyphényl)-1-méthyl-1-(tétrahydropyrann-4-ylcarbamoyl)-éthyl]-amide de l'acide (S)-1-(3-fluorobenzènesulfonyl)-pipéridine-2-carboxylique ;
le [(S)-2-(4-isopropoxyphényl)-1-(2,2,6,6-tétraméthylpipéridin-4-ylcarbamoyl)-éthyl]-amide de l'acide (S)-1-(3-fluorobenzènesulfonyl)-pipéridine-2-carboxylique ;
le [(S)-2-[4-(3-fluoropropoxy)-phényl]-1-(2,2,6,6-tétraméthylpipéridin-4-ylcarbamoyl)-éthyl]-amide de l'acide (S)-1-(3-fluorobenzènesulfonyl)-(2S)-pipéridine-2-carboxylique ;
le [(S)-2-[4-(3-fluoropropoxy)-phényl]-1-((S)-2-méthoxy-1-méthyléthylcarbamoyl)-éthyl]-amide de l'acide (S)-1-(3)-fluorobenzènesulfonyl)-pipéridine-2-carboxylique ;
le [(S)-2-(4-isopropoxyphényl)-1-méthylcarbamoyléthyl]-amide de l'acide (S)-1-(3-trifluorométhylbenzènesulfonyl)-pipéridine-2-carboxylique ;
le [(S)-2-(4-isopropoxyphényl)-1-(tétrahydropyrann-4-ylcarbamoyl)-éthyl]-amide de l'acide (S)-1-(3-trifluorométhylbenzènesulfonyl)-pipéridine-2-carboxylique ;
le [(S)-2-(phényl)-1-(benzimidazol-2-yl)-éthyl]-amide de l'acide (S)-1-(3-trifluorométhylbenzènesulfonyl)-pipéridine-2-carboxylique ;
le [(S)-2-(4-(benzyloxy)phényl)-1-(benzimidazol-2-yl)-éthyl]-amide de l'acide (S)-1-(3-trifluorométhylbenzènesulfonyl)-pipéridine-2-carboxylique ;
le [(S)-2-(4-méthoxyphényl)-1-(tétrahydropyrann-4-ylcarbamoyl)-éthyl]-amide de l'acide (S)-1-(3-fluorobenzènesulfonyl-pipéridine-2-carboxylique ;
le ((S)-1-amido-2-(2-méthylphényl)-éthyl)-amide de l'acide (S)-1-benzènesulfonylpipéridine-2-carboxylique ;
le ((S)-1-cyclohexylamido-2-(4-méthylphényl)-éthyl)-amide de l'acide (S)-1-benzènesulfonylpipéridine-2-carboxylique ;
le [(S)-2-(4-allyloxyphényl)-1-cyclohexylamido-éthyl]-amide de l'acide (S)-1-benzènesulfonylpipéridine-2-carboxylique ;
le [(S)-2-(4-allyloxyphényl)-1-cyclohexylcarbamoyléthyl]-amide de l'acide (S)-1-3-trifluorométhylbenzènesulfonyl)-pipéridine-2-carboxylique ;
le ((S)-1-cyclohexylamido-2-(4-méthylphényl)-éthyl)-amide de l'acide (S)-1-(3-trifluorométhylbenzènesulfonyl)-pipéridine-2-carboxylique ;
le [(S)-1-amido-2-(1H-indol-3-yl)-éthyl]-amide de l'acide (S)-1-((E)-2-phényléthènesulfonyl)-pipéridine-2-carboxylique ;
le [(S)-1-amido-2-(1H-indol-3-yl)-éthyl]-amide de l'acide (S)-1-(4,5-dibromothiophène-2-sulfonyl)-pipéridine-2-carboxylique ;
le [(S)-1-tertbutylamido-2-(1H-indol-3-yl)-éthyl]-amide de l'acide (S)-1-benzènesulfonylpipéridine-2-carboxylique ;
le [(S)-1-(3-hydroxy-2,2-diméthylpropylamido-2-(4-isopropylphényl)-éthyl]-amide de l'acide (S)-1-benzènesulfonylpipéridine-2-carboxylique ;
le méthyl-((S)-1-méthylamido-2-(phényl)-éthyl)-amide de l'acide (S)-1-benzénesulfonylpipéridine-2-carboxylique ;
le [(S)-1-(3,3-diméthylbutylamido)-2-(4-méthoxyphényl)-éthyl]-amide de l'acide (S)-1-benzènesulfonylpipéridine-2-carboxylique ;
le [(S)-1-(2,2-diméthylpropylamido)-2-(phényl)-éthyl]-amide de l'acide (S)-1-benzénesulfonylpipéridine-2-carboxylique ;
l'ester diéthylique de l'acide (4-{(S)-2-[((S)-1-benzènesulfonylpipéridine-2-carbonyl)-amino]-2-amido-éthyl}-benzyl)-phosphonique ;
le [(S)-2-(4-(acétylamino)-phényl)-1-amido-éthyl]-amide de l'acide (S)-1-benzènesulfonylpipéridine-2-carboxylique ;
le [(S)-1-amido-2-(4-(hydroxyméthyl)-phényl)-éthyl]-amide de l'acide (S)-1-benzénesulfonylpipéridine-2-carboxylique ;
le [(S)-1-amido-2-(1H-indol-3-yl)-éthyl]-amide de l'acide (S)-1-(naphtalène-2-sulfonyl)-pipéridine-2-carboxylique ;
le [(S)-1-(cyclohexylméthylamido)-2-(4-méthylphényl)éthyl]-amide de l'acide (S)-1-(3-trifluorométhylbenzènesulfonyl)-pipéridine-2-carboxylique ;
le [(S)-1-(4-aminobutylamido)-2-(4-méthylphényl)-éthyl]-amide de l'acide (S)-1-(3-trifluorométhylbenzènesulfonyl)-pipéridine-2-carboxylique ;
le [(S)-1-amido-2-(1H-indol-3-yl)-éthyl]-amide de l'acide (S)-1-benzènesulfonylpipéridine-2-carboxylique ;
le ((S)-1-amido-2-(pyridin-4-yl)-éthyl)-amide de l'acide (S)-1-benzènesulfonylpipéridine-2-carboxylique ;
le ((S)-1-amido-2-cyclohexyléthyl)-amide de l'amide (S)-1-benzènesulfonylpipéridine-2-carboxylique ;
le ((S)-2-phényl-1-[(thiophén-2-ylméthyl)-amido] éthyl)-amide de l'acide (S)-1-benzènesulfonylpipéridine-2-carboxylique ;
le [(S)-1-(4-diméthylaminobutylcarbamoyl)-2-(4-fluorophényl)-éthyl]-amide de l'acide (S)-1-(3-fluorobenzènesulfonyl)-pipéridine-2-carboxylique ;
le [(S)-2-[4-(3-méthoxypropoxy)-phényl]-1-(tétrahydropyrann-4-ylcarbamoyl)-éthyl]-amide de l'acide (S)-1-(3-fluorobenzènesulfonyl)-pipéridine-2-carboxylique ;
le ((S)-1-cyclohexylcarbamoyl-2-[4-(2-diméthylaminoéthoxy)-phényl]-éthyl}-amide de l'acide (S)-1-3-trifluorométhylbenzènesulfonyl)-pipéridine-2-carboxylique ;
le [(S)-2-(4-méthoxyphényl)-1-(2-pipérazin-1-yléthylcarbamoyl)-éthyl]-amide de l'acide (S)-1-(3-trifluorométhylbenzènesulfonyl)-pipéridine-2-carboxylique ;
le [(S)-1-(2-hydroxy-1,1-diméthyléthylcarbamoyl)-2-(4-méthoxyphényl)-éthyl]-amide de l'acide (S)-1-(3-trifluorométhylbenzènesulfonyl-pipéridine-2-carboxylique ;
le ((S)-1-carbamoyl-2-phényléthyl)-amide de l'acide (S)-1-benzènesulfonylpipéridine-2-carboxylique ;
le ((S)-2-[4-(3-méthoxypropoxy)-phényl]-1-méthylcarbamoyléthyl)-amide de l'acide (S)-1-(3-fluorobenzènesulfonyl)-pipéridine-2-carboxylique ;
le {(S)-1-({S)-2-méthoxy-1-méthyléthylcarbamoyl)-2-[4-(3-méthoxypropoxy)-phényl]-éthyl)-amide de l'acide (S)-1-(3-fluorobenzénesulfonyl-pipéridine-2-carboxylique ; et
le [(S)-2-(1H-indol-3-yl)-1-(naphtalén-2-ylcarbamoyl)-éthyl]-amide de l'acide (S)-1-(3-hydroxybenzènesulfonyl-pipéridine-2-carboxylique.
